(19) Europäisches Patentamt
European Patent Office
Office européen des brevets

(11) EP 4 112 727 A2

(12) # EUROPÄISCHE PATENTANMELDUNG

(43) Veröffentlichungstag:
**04.01.2023 Patentblatt 2023/01**

(21) Anmeldenummer: **22168282.6**

(22) Anmeldetag: **17.11.2011**

(51) Internationale Patentklassifikation (IPC):
***C12N 9/90*** *(2006.01)* ***C12N 15/52*** *(2006.01)*
***C07C 29/56*** *(2006.01)*

(52) Gemeinsame Patentklassifikation (CPC):
(C-Sets verfügbar)
**C12N 9/90; C07C 29/17; C07C 29/56; C12P 7/02;**
C07C 2601/14 (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.11.2010 EP 10191454**
**21.06.2011 EP 11170812**
**28.09.2011 EP 11183176**

(62) Dokumentnummer(n) der früheren Anmeldung(en) nach Art. 76 EPÜ:
**18203725.9 / 3 470 515**
**11785004.0 / 2 640 835**

(71) Anmelder: **BASF SE**
**67056 Ludwigshafen am Rhein (DE)**

(72) Erfinder:
- **BREUER, Michael**
  **64285 Darmstadt (DE)**
- **HAUER, Bernhard**
  **67136 Fußgönheim (DE)**
- **JENDROSSEK, Dieter**
  **72070 Tübingen (DE)**
- **SIEDENBURG, Gabriele**
  **70569 Stuttgart (DE)**
- **PLEISS, Jürgen**
  **71679 Asperg (DE)**
- **SIRIM, Demet**
  **70180 Stuttgart (DE)**
- **RACOLTA, Silvia**
  **70569 Stuttgart (DE)**

(74) Vertreter: **Reitstötter Kinzebach**
**Patentanwälte**
**Sternwartstrasse 4**
**81679 München (DE)**

Bemerkungen:
•Das gesamte Dokument mit Referenztabelle(n) und Sequenzliste(n) kann von der Webseite des EPA heruntergeladen werden
•Diese Anmeldung ist am 14.04.2022 als Teilanmeldung zu der unter INID-Code 62 erwähnten Anmeldung eingereicht worden.

(54) **VERFAHREN ZUR BIOKATALYTISCHEN CYCLISIERUNG VON TERPENEN UND DARIN EINSETZBARE CYCLASE-MUTANTEN**

(57) Die vorliegende Erfindung betrifft neuartige Mutanten mit Cyclase-Aktivität und deren Anwendung in einem Verfahren zur biokatalytischen Cyclisierung von Terpenen, wie insbesondere zur Herstellung von Isopulegol durch Cyclisierung von Citronellal; ein Verfahren zur Herstellung von Menthol sowie Verfahren zur biokatalytischen Umsetzung weiterer Verbindungen mit Terpenähnlichen Strukturmotiven.

EP 4 112 727 A2

(52) Gemeinsame Patentklassifikation (CPC): (Forts.)

C-Sets
**C07C 29/17, C07C 35/12;**
**C07C 29/56, C07C 35/17**

**Beschreibung**

Die vorliegende Erfindung betrifft neuartige Verfahren zur Cyclisierung von Terpenen unter Verwendung von Cyclasen sowie neuartige Mutanten mit Cyclase-Aktivität und deren Anwendung in einem Verfahren zur biokatalytischen Cyclisierung von Terpenen, wie insbesondere zur Herstellung von Isopulegol durch Cyclisierung von Citronellal; ein Verfahren zur Herstellung von Menthol sowie Verfahren zur biokatalytischen Umsetzung weiterer Verbindungen mit Terpen-ähnlichen Strukturmotiven.

## Hintergrund der Erfindung

Isopulegol der Formel (II) (2-Isopropenyl-5-methyl-cyclohexanol), ist ein Terpen, das als Aromachemikalie eingesetzt wird, um "Blütennoten" zu generieren. Außerdem ist es ein Zwischenprodukt in der Synthese von Menthol aus Citral.

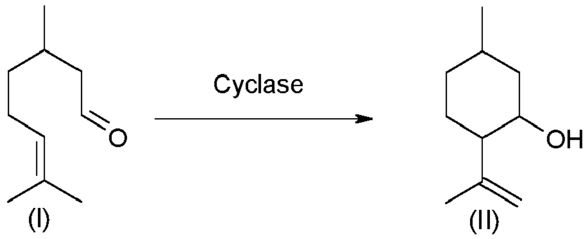


(I) Citronellal

(II) Isopulegol

Isopulegol-Isomere findet man in der Natur in einer großen Anzahl von etherischen Ölen. Da Isopulegol relativ leicht aus Citronellal, der Verbindung der Formel (I) (3,7-Dimethyloct-6-en-1-al), entsteht, kommt es häufig in Begleitung von Citronellal vor bzw. wird bei Gewinnung des etherischen Öls gebildet. Isopulegol, das technisch aus (+)-Citronellal hergestellt wird, ist in der Regel eine Mischung verschiedener Isomere mit einem großen Anteil an (-)-Isopulegol.

Die industrielle Herstellung von Isopulegol wird überwiegend durch die chemische Zyklisierung von (+)-Citronellal durchgeführt. Ursprünglich wurde 80-85% reines, aus Citronellöl gewonnenes Ausgangsmaterial verwendet. Seit den neunziger Jahren wird dieses zunehmend durch das optisch reinere (+)-Citronellal (97,5%) aus dem sog. Takasago-Verfahren ersetzt. Hier wird Geranyldiethyldiamin asymmetrisch unter Verwendung eines Rh-BINAP-Komplexkatalysators (Rh-Komplex mit 2,2'-Bis(diphenylphosphino)-1,1'-binaphtyl) zu (+)-Citronellal isomerisiert.

Ausgehend vom Citronellal wurde die chemische Synthese von Isopulegol mehrfach beschrieben. (+)-Citronellal kann unter Verwendung von einem Kupfer-Chrom-Katalysator, Zinkbromid, Alkylaluminiumchlorid, einem Rhodiumkomplex, einem festen Säure-Base-Katalysator, Zeolith oder Silicagel zyklisiert werden. Dabei wird das Silicagelverfahren in der letzten Zeit immer mehr vom Verfahren mit Zinkbromid abgelöst, da dieses eine höhere Selektivität aufweist.

Allgemein ist die Cyclisierung von Terpenen mit Hilfe von speziellen Cyclasen bekannt. So wird beispielsweise in der Natur Squalen mit Hilfe einer Squalen-Hopen-Cyclase (SHC) zu dem pentacyclischen Hopen cyclisiert.

Die Gen- und Proteinsequenzen der aus dem Bakterium Zymomonas mobilis stammenden Squalen-Hopen-Cyclase (Zm-SHC) sind bekannt (Genpept Accession No AAV90172 2004 und Nat Biotechnol 2005, 23:63-68, vgl. SEQ ID NO:1 und 2).

In der internationalen Anmeldung PCT/EP2010/057696 (WO2010139719 A2), auf deren vollständige Offenbarung hierin ausdrücklich Bezug genommen wird, werden Polypeptide als Biokatalysatoren für die Cyclisierung von Homofarnesol zu Ambroxan vorgeschlagen.

Die Biosynthese von zahlreichen Monoterpenen in den entsprechenden Produktionsorganismen wurde bereits aufgeklärt. Häufig werden dabei lineare Vorläufermoleküle durch hochspezifische Biokatalysatoren zyklisiert. Bei den Vorläufern handelt es sich in der Regel um Ester linearer Terpenalkohole und Diphosphorsäure. Ein typisches Beispiel für eine solche Vorstufe ist Geranylpyrophosphat. Die Pyrophosphatgruppe wird enzymatisch aus dem Molekül eliminiert und anschließend in zwei Phosphationen hydrolysiert. Auf der anderen Seite entsteht dabei eine Carbokation, das nun intramolekular weiterreagieren kann und, z.B. unter Abspaltung eines Protons, zu einem zyklischen Monoterpen rekombiniert (Curr. Opin. Chem. Biol. 2009, 13:180-188).

Es war weiterhin Aufgabe der vorliegenden Erfindung, eine Alternative zu den bekannten chemischen Zyklisierungsverfahren für Terpene zu finden, mit der es möglich ist, Terpenverbindungen mittels enzymatischer Katalyse, wie z.B. das lineare Citronellal zu Isopulegol, zu cyclisieren.

Es war weiterhin Aufgabe der vorliegenden Erfindung, neuartige Biokatalysatoren bereitzustellen, die zur Cyclisierung von Terpenen, wie z.B. von Citronellal unter Bildung von Isopulegol einsetzbar sind.

## Kurzfassung der Erfindung

Obige erste Aufgabe wird durch ein Verfahren zur Herstellung von Isopulegol der allgemeinen Formel (I)

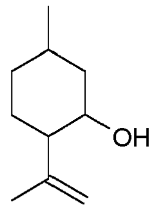

(I)

umfassend einen Reaktionsschritt,
wobei man Citronellal der allgemeinen Formel (II)

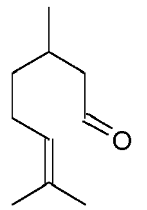

(II)

biokatalytisch zu dem entsprechenden Isopulegol der Formel (I) mittels eines Enzyms mit der Aktivität einer Citronellal-Isopulegol-Cyclase cyclisiert,
gelöst.

Obige zweite Aufgabe konnte überraschenderweise durch Bereitstellung von Mutanten von Wildtypenzymen, wie der Zm-SHC-1 (SEQ ID NO:2), gelöst werden. Insbesondere wurde nämlich festgestellt, dass durch gezielte Einführung von Mutationen in wenigstens einer bei solchen Cyclasen, insbesondere Squalen-Hopen Cyclasen, hochkonservierten Sequenzposition (vgl Alignment der SEQ ID NOs. 2 bis 326, unten) die enzymatische Aktivität in der gewünschten Weise beeinflussbar ist.

## Figurenbeschreibung

Figur 1a zeigt die Wildtyp-Aminosäuresequenz (SEQ ID NO:2) der Squalen-Hopen-Cyclase 1 aus *Zymomonas mobilis* (Zm-SHC-1) Die Position 486 der Sättigungsmutagenese ist markiert.
Figur 1b zeigt die Wildtyp-Nukleinsäuresequenz (SEQ ID NO:1) der Zm-SHC-1. Die Positionen 1456-1458 der Sättigungsmutagenese sind markiert.
Figur 2 zeigt den Umsatz des SHC_1 WT Proteins im Vergleich zur F486A Mutante im zeitlichen Verlauf mit 10mM R(+)- und S(-)-Citronellal als Substrat. Dargestellt ist jeweils die prozentuale Verteilung von Substrat und Isopulegol-Produkt-Isomeren nach Inkubation für verschiedene Zeiten bei 30°C. Citronellal (Rauten), Isopulegol I (Quadrate), Isopulegol II (Dreiecke) und Isopulegol III (Kreuze).
Figur 3 zeigt den Umsatz der verschiedenen Mutanten der Zm-SHC-1 im Vergleich zum Wildtyp (wt) und der Kontrolle ohne Enzym (K) mit 10mM Citronellal-Racemat als Substrat. Dargestellt ist jeweils die prozentuale Verteilung von Substrat und Isopulegol-Produkt-Isomeren nach Inkubation über Nacht bei 30°C.
Figur 4 zeigt den Umsatz der verschiedenen Zm-SHC-1 Mutanten im Vergleich zum Wildtyp (wt) und der Kontrolle ohne Enzym (K) mit 25mM Squalen als Substrat in Gegenwart von 1% Triton. Dargestellt ist jeweils die prozentuale Verteilung von Squalen und Hopen nach Inkubation für 70h bei 30°C.

Die Figuren 5 bis 7 zeigen die Umsetzung von jeweils 20mM Substrat nach über Nacht Inkubation mit den Mutanten Ap-SHC: F481C, Bj-SHC: F447C, Sc-SHC: F449C, Zm SHC-2: F438C und Zm SHC-1 im Vergleich zur Kontrolle; Substrate waren in Figur 5 Citronellal-Racemat, in Figur 6 R(+)-Citronellal und in Figur 7 S(-)-Citronellal.

## Detaillierte Beschreibung der Erfindung

### A. Allgemeine Definitionen

"Cyclasen" im Sinne der vorliegenden Erfindung sind allgemein Enzyme bzw. Enzymmutanten, welche insbesondere die Aktivität einer Citronellal-Isopulegol-Cyclase zeigen. Als Enzyme mit der Aktivität einer Citronellal-Isopulegol-Cyclase sind intramolekulare Transferasen aus der Subklasse der Isomerasen; also Proteine mit der EC-Nummer EC 5.4 geeignet. (Enzymcode gemäß Eur. J. Biochem. 1999, 264, 610-650) Insbesondere handelt es sich um Vertreter von EC 5.4.99.17. Als Enzyme mit der Aktivität einer Citronellal-Isopulegol-Cyclase sind insbesondere solche Cyclasen geeignet, die auch die Cyclisierung von Homofarnesol zu Ambroxan oder von Squalen zu Hopen (daher auch zuweilen die Bezeichnung "SHC" Squalen Hopen Cyclase) bewirken und die ausführlich in der internationalen Anmeldung PCT/EP2010/057696 beschrieben werden, worauf hier ausdrücklich Bezug genommen wird. Insbesondere sind erfindungsgemäße Cyclasen solche, die durch Mutation von SHCs abgeleitet sind.

Aufgrund der Reversibilität enzymatischer Reaktionen betrifft die vorliegende Erfindung die hierin beschriebenen enzymatischen Umsetzungen in beiden Umsetzungsrichtungen.

"Funktionale Mutanten" einer "Cyclase" umfassen die unten definierten "funktionalen Äquivalente" solcher Enzyme.

Der Begriff "biokatalytisches Verfahren" betrifft jegliches in Gegenwart von katalytischer Aktivität einer erfindungsgemäßen "Cyclase" bzw. eines Enzyms mit "Cyclase Aktivität" durchgeführtes Verfahren, d.h. Verfahren in Gegenwart von rohem, oder gereinigtem, gelöstem, dispergiertem oder immobilisiertem Enzym, oder in Gegenwart ganzer mikrobieller Zellen, welche derartige Enzymaktivität aufweisen oder exprimieren. Biokatalytische Verfahren umfassen somit enzymatische als auch mikrobielle Verfahren.

Der Begriff "stereospezifisch" bedeutet, dass eines von mehreren möglichen Stereoisomeren einer erfindungsgemäß hergestellten Verbindung mit wenigstens eine Asymmetriezentrum durch die Wirkung eines erfindungsgemäßen Enzyms in hohem "Enatiomerenüberschuß" oder hoher "Enantiomerenreinheit", wie z.B. wenigstens 90%ee, insbesondere wenigstens 95 %ee, oder wenigstens 98 %ee, oder wenigstens 99 %ee produziert wird. Der ee% Wert wird nach folgender Formel berechnet:

$$ee\% = [X_A\text{-}X_B]/[\, X_A+X_B]*100,$$

worin $X_A$ und $X_B$ für den Molenbruch der Enantiomere A bzw B stehen.

"Erste-Sphäre-Reste" und "Zweite-Sphäre-Reste" sind Aminosäurereste, denen, basierend auf Strukturanalysen des Proteins, eine besondere Nähe zum reaktiven Zentrum der Cyclase zugeordnet werden. Das Kriterium für die erste Sphäre ist die Distanz zum Liganden 2-Azasqualen, der in einer publizierten Röntgenstruktur angegeben ist (pdb: 1ump). Diese Reste wurden automatisch mit einem Computerprogramm ermittelt (http://ligin.weizmann.ac.il/cgibin/lpccsu/LpcCsu.cgi ; Sobolev V, Sorokine A, Prilusky J, Abola EE, Edelman M. Automated analysis of interatomic contacts in proteins. Bioinformatics 1999;15(4):327-332.). Dieses Programm geht davon aus, dass zwei Moleküle in Kontakt miteinander stehen wenn die Entfernung ihrer Atome der Summe ihrer van der Waals Radien ± 1 Ä entspricht. Zur zweiten Sphäre werden alle Aminosäuren gezählt, die in einem Radius von 5 Ä zu jedem Rest der ersten Sphäre liegen. Derartige Reste sind daher für die Vornahme von gezielten Mutation in besonders geeignet erscheinen, um die Enzymaktivität weiter gezielt zu modifizieren.

Unter einer "Cyclase-Aktivität", die mit einem "Referenzsubstrat unter Standardbedingungen" bestimmt wurde, ist z.B. eine Enzymaktivität, welche die Bildung eines cyclischen Produkts aus einem nicht-cyclischen Substrat beschreibt. Standardbedingungen sind z.B. Substratkonzentrationen von 10 mM bis 0,2 M, insbesondere 15 bis 100mM, wie z.B. etwa 20 bis 25 mM; bei pH 4 bis 8, und bei Temperaturen von z.B. 15 bis 30 oder 20 bis 25 °C. Die Bestimmung kann dabei mit rekombinanten Cyclase-exprimierenden Zellen, aufgeschlossenen Cyclase-exprimierenden Zellen, Fraktionen davon oder angereichertem oder gereinigtem Cyclase-Enzym erfolgen. Insbesondere ist Referenzsubstrat ein Citronellal der Formel (II); insbesondere R(+)-Citronellal, oder ein Citronellal-Racemat, in einer Konzentration von 15 bis 100mM oder etwa 20 bis 25 mM, bei 20 bis 25 °C und pH 4 - 6, wie etwa 4,5; wie auch in den Bespielen näher beschrieben.

Eine "F486-analoge" Position entspricht die Position F486 gemäß SEQ ID NO:2 in funktioneller Sicht und ist durch Sequenzalignment von SHCs aus anderen Organismen als Zymomonas mobilis wie hierin erläutert ermittelbar. Beispielsweise ist die F486-analoge Position von SEQ ID NO:3 die Position F449 und von SEQ ID NO:4 die Position F481 und von SEQ ID NO:5 die Position F447 und von SEQ ID NO:6 die Position F438. Entsprechende Analogien gelten für die anderen hierin für SEQ ID NO: 2 konkret beschriebenen Sequenzpositionen, wie die sogenannten "Erste-" und "Zweite-Sphäre-Reste" oder des DXDD-Motivs und deren analogen Positionen in SEQ ID NO:3 bis 326).

"Terpene" sind Kohlenwasserstoffe die aus Isopreneinheiten (C5-Einheiten) aufgebaut sind, insbesondere nicht-cyclische Terpene, wie z.B. Squalen, der Kohlenstoffzahl durch 5 teilbar ist.

"Terpenoide" sind Substanzen, die von Terpenen, insbesondere nichtcyclischenTerpenen, abgeleitet sind, z.B. durch zusätzliche Einfügung von C-Atomen und/ oder Heteroatomen, wie z.B. Citonellal.

"Terpen-ähnliche" Verbindungen im Sinne der vorliegenden Erfindung umfassen insbesondere solche Verbindungen, welche unter die allgemeine Strukturformel (IV), wie im folgenden definiert, fallen.

Generell mit umfasst sind erfindungsgemäß alle isomeren Formen der hierin beschriebenen Verbindungen, wie Konstitutionsisomere und insbesondere Stereoisomere und Gemische davon, wie z.B. optische Isomere oder geometrische Isomere, wie E- und Z- Isomere., sowie Kombinationen davon. Sind mehrere Asymmetriezentren in einem Molekül vorhanden, so umfasst die Erfindung sämtliche Kombinationen von unterschiedlichen Konformationen dieser Asymmetriezentren, wie z.B. Enantiomerenpaare.

"Menthol" umfasst alle stereoisomeren Formen wie (+)-Menthol, (+)-Isomenthol, (+)-Neomenthol, (+)- Neoisomentol, (-)-Menthol, (-)-Isomenthol, (-)-Neomenthol, (-)- Neoisomentol und beliebige Gemische davon.

Citronellal der Formel (II) ist kommerziell sowohl als R(+)-Citronellal der Formel (R-II) als auch als S(-)-Citronellal der Formel (S-II) als auch als Racemat der Formel (II) erhältlich.

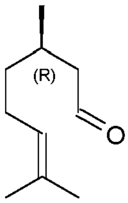
(R-II)

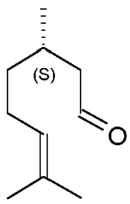
(S-II)

Isopulegol der Formel (I)

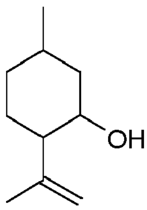
(I)

hat in den Positionen 1, 3 und 6 jeweils ein optisch aktives Zentrum, sodass prinzipiell 4 verschiedene Diastereomere mit jeweils 2 Enantiomeren, also insgesamt 8 Stereoisomere, denkbar sind, wenn von dem Racemat des Citronellals der Formel (I) ausgegangen wird.

Isopulegol

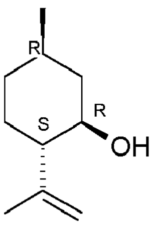
*1R,3R,6S*

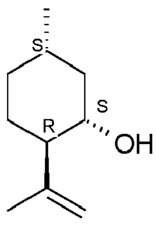
*1S,3S,6R*

Neo-Isopulegol

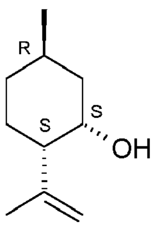
*1S,3R,6S*

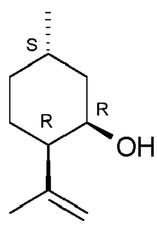
*1R,3S,6R*

Iso-Isopulegol

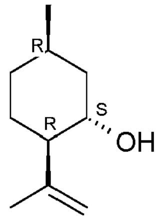

*1S,3R,6R*

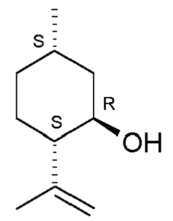

*1R,3S,6S*

Epi-Isopulegol

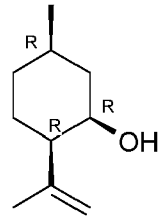

1R,3R,6R

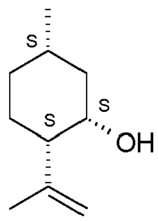

*1S,3S,6R*

Isopulegol wird auch als Isopulegol I bezeichnet, Neo-Isopulegol wird auch als Isopulegol II bezeichnet; Iso-Isopulegol wird auch als Isopulegol III bezeichnet; Epi-Isopulegol oder Neoiso-Isopulegol wird auch als Isopulegol IV bezeichnet;

Sofern keine abweichenden Angaben gemacht werden gelten hierin folgende allgemeine chemische Definitionen:

**Alkyl sowie alle Alkylteile in davon abgeleiteten Resten, wie z.B. Hydroxyalkyl:** gesättigte, geradkettige oder verzweigte Kohlenwasserstoffreste mit 1 bis 4, 1 bis 6, 1 bis 8 oder 1 bis 10 Kohlenstoffatomen, z. B.

- **$C_1$-$C_6$-Alkyl:** wie Methyl, Ethyl, Propyl, 1-Methylethyl, Butyl, 1-Methyl-propyl, 2-Methylpropyl, und 1,1-Dimethylethyl als beispielhafte Vertreter für $C_1$-$C_4$-Alkyl; sowie Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.
- **Hydroxy-$C_1$-$C_6$-alkyl,** umfassend **Hydroxy-$C_1$-$C_4$-Alkyl,** wie z.B. Hydroxymethyl, 1-oder 2-Hydroxyethyl, 1-, 2- oder 3-Hydroxypropyl, 1-Hydroxymethylethyl, 1-, 2-, 3- oder 4-Hydroxybutyl, 1-Hydroxymethylpropyl und 2-Hydroxymethylpropyl.

**Alkenyl** steht für ein- oder mehrfach, insbesondere einfach ungesättigte, geradkettige oder verzweigte Kohlenwasserstoffreste mit 2 bis 4, 2 bis 6, 2 bis 8, 2 bis 10 oder 2 bis 20 Kohlenstoffatomen und einer Doppelbindung in einer beliebigen Position, z. B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

"Oxo" steht z.B. für einen Rest, der zusammen mit dem C-Atom an das er gebunden ist eine Ketogruppe (C=O) bildet.

"Methylen" ($=CH_2$) steht z.B. für einen Rest, der zusammen mit dem C-Atom an das er gebunden ist einen Vinylrest ($-CH=CH_2$) bildet.

## B. Spezielle Ausgestaltungen der Erfindung

Die vorliegende Erfindung betrifft insbesondere folgende spezielle Ausführungsformen:

1. Enzymmutante mit Cyclase-Aktivität, ausgewählt unter Mutanten eines Wildtyp-Enzyms, das eine Aminosäuresequenz, ausgewählt unter SEQ ID NO: 2 bis 326 oder eine Teilsequenz davon umfasst; wobei die Mutante wenigstens die Cyclisierung wenigstens eines Citronellal-Isomers (oder eines Isomerengemischs, wie z.B. Racemats) gemäß obiger Definition zu wenigstens einem Isopulegol-Isomer (oder zu eine Diastereomerenpaar I bis IV, wie z.B. I und /oder II) gemäß obiger Definition katalysiert, wobei die Teilsequenz oder Kurzform der Cyclase z.B.

mindestens 50, 100, 150, 200, 250, 300, 350, 400, 450, 500, 550, 600, 650 oder 700 zusammenhängenden Aminosäurereste einer dieser Sequenzen umfasst, und z.B. durch N- und/oder C-terminale Verkürzung der konkreten Sequenzen zugänglich ist.

2. Enzymmutante nach Ausführungsform 1, umfassend

a) eine Mutation in Position F486 von SEQ ID NO: 2 oder
b) eine Mutation in einer unter SEQ ID NO: 3 bis 326 ausgewählten Sequenzen, wobei die mutierte Position der Position F486 von SEQ ID NO: 2 entspricht (d.h. eine "F486-analoge" Position ist);

wobei durch die Mutation wenigstens die Cyclisierung wenigstens eines Citronellal-Isomers zu wenigstens einem Isopulegol-Isomer ermöglicht wird (d.h. dass das entsprechende Ausgangs- oder Wildtypprotein diese Umsetzung nicht katalysierte) oder modifiziert wird, (d.h. dass das entsprechende Ausgangs- oder Wildtypprotein diese Umsetzung katalysierte, jedoch z.B. mit geringerer Produktausbeute, Umsatzrate und/ oder Stereospezifität). Auch die Teilsequenz oder Kurzform der Cyclase weist dabei diese Cyclase-typische Mutation in einer F486 aus SEQ ID NO: 2 entsprechenden Position auf. Beispielsweise stellt eine N-terminal verkürzte Version der Cyclase gemäß SEQ ID NO: 2 ein Beispiel einer derartigen Kurzversion dar. Diese ist durch folgenden N-Terminus gekennzeichnet: (M)**K**IFGAEKTSYKPASDTIIGTDTLKRPN......wobei das N-terminale **K** der Position 16 von SEQ ID NO:2 entspricht.

3. Enzymmutante nach einer der vorhergehenden Ausführungsformen bei der bis zu 25% oder bis zu 20, 15, 10, 9, 8, 7, 6, 5 4, 3, 2 oder 1% der Aminosäurereste, wie z.B. 1 bis 30, 2 bis 25, 3 bis 20 oder 4 bis 15 oder 5 bis 10 der Aminosäurereste, jeweils gegenüber der nichtmutierten Wildtypsequenz gemäß SEQ ID NO: 2 bis 326, durch Deletion, Insertion, Substitution, Addition, Inversion oder einer Kombination davon verändert sind.

4. Enzymmutante nach einer der vorhergehenden Ausführungsformen, bei der die Mutation in der Position F486 von SEQ ID NO:2 oder in einer dieser Positionen entsprechenden Position in einer der Sequenzen gemäß SEQ ID NO: 3 bis 326, eine Substitution ausgewählt unter F486N, F486Q, F486L, F486M, F486E, F486G, F486S, F486V, F486T, F486C, F486I und F486A oder ggf. ausgewählt unter F486H, F486Y, F486W und F486D ist.

5. Enzymmutante nach einer der vorhergehenden Ausführungsformen, bei der zusätzlich (oder alternativ, insbesondere aber zusätzlich) wenigstens eine, wie z.B. 1, 2, 3, 4, 5 ,6, 7 oder 8, Mutationen in einer der Positionen W374, D437, D440, F428, W555, Y561, Y702, Y705 (die sogenannten "Erste-Sphäre-Reste") der SEQ ID NO: 2 oder in wenigstens einer aus diesen Positionen ausgewählten, entsprechenden Position in einer der Sequenzen gemäß SEQ ID NO: 3 bis 326, vorliegt.

6. Enzymmutante nach einer der vorhergehenden Ausführungsformen, bei der keine Mutation in der Position D437 und/oder D439 und/oder D440 von SEQ ID NO: 2 (DXDD-Motiv) oder der jeweils entsprechenden Position in einer der Sequenzen gemäß SEQ ID NO: 3 bis 326, vorliegt.

7. Enzymmutante nach einer der vorhergehenden Ausführungsformen, bei der keine Mutation in der Position Y702 von SEQ ID NO: 2 oder in der entsprechenden Position in einer der Sequenzen gemäß SEQ ID NO: 3 bis 326, vorliegt oder falls eine Mutation vorliegt, diese eine Substitution Y702F oder ggf. Y702E oder Y702D oder entsprechende Substitution ist.

8. Enzymmutante nach einer der vorhergehenden Ausführungsformen, welche gegebenenfalls weiterhin mutiert ist in wenigstens einer, wie z.B. 1 bis 15, 1bis 10 oder 1 bis 5, wie 1, 2, 3 oder 4 , der Positionen P229, D439, D508, E601, G553, G556, N432, P436, P499, R224, S371, T376, T563, W414 oder W624 (die sogenannten "Zweite Sphäre Reste") von SEQ ID NO: 2 oder in wenigstens einer aus diesen Positionen ausgewählten, entsprechenden Position in einer der Sequenzen gemäß SEQ ID NO: 3 bis 326; sowie ggf. eine weitere Mutation in Position E429, L700 und R554 von SEQ ID NO: 2 oder den analogen Positionen von SEQ ID NO: 3 bis 326.

9. Enzymmutante nach einer der vorhergehenden Ausführungsformen, ausgewählt unter

a) den Einfachmutanten

F486X mit X = N, Q, L, M, E, G, S, V, T, C, I oder A gemäß SEQ ID NO: 2 oder einer Kurzversion davon;
Y702X mit X = F, A, C oder S gemäß SEQ ID NO: 2 oder einer Kurzversion davon;
Y561X mit X= A oder S gemäß SEQ ID NO: 2 oder einer Kurzversion davon;

wobei die Kurzversion z.B. die folgende N-terminale Sequenz umfasst: (M)KIFGAEKTSYKPASDTIIGTDTLKRPN......

b) den Mehrfachmutanten F486A / Y702A, F486A / Y561A oder F486A / Y705A gemäß SEQ ID NO: 2
c) den zu a) oder b) korrespondierenden, von einer der SEQ ID NO: 3 bis 325 abgeleiteten Mutanten.

10. Enzymmutante nach einer der vorhergehenden Ausführungsformen, die mindestens 50%, wie z. B. 50 bis 100% oder mehr als 100%, wie z.B. >100 bis 1000%, jeweils bestimmt unter Standardbedingungen unter Verwendung eines Referenzsubstrates, der Citronellal-Isopulegol-Cyclase Aktivität eines Enzyms zeigt, das eine Aminosäuresequenz gemäß SEQ ID NO: 2 von Position 1 bis 725, 2 bis 725 oder 16 bis 725, ggf. N-terminal verlängert durch einen Methioninrest, umfasst.

11. Enzymmutante nach Ausführungsform 10, wobei die Citronellal-Isopulegol-Cyclase Aktivität unter Verwendung eines Citronellals, wie z.B. des Racemats oder der R(+)-Form, als Referenzsubstrat unter Standardbedingungen bestimmt wird.

12. Enzymmutante nach einer der vorhergehenden Ausführungsformen, wobei die Mutation in einem Enzym erfolgt, die eine Aminosäuresequenz gemäß SEQ ID NO: 2 von Position 1 bis 725, 2 bis 725 oder 16 bis 725, ggf. N-terminal verlängert durch einen Methioninrest, umfasst.

13. Nukleinsäuresequenz, kodierend für eine Mutante nach einer der vorhergehenden Ausführungsformen.

14. Expressionskassette, umfassend eine Nukleinsäuresequenz nach Ausführungsform 13.

15. Rekombinanter Vektor, umfassend unter der Kontrolle wenigstens eines regulativen Elements wenigstens eine Nukleinsäuresequenz nach Ausführungsform 13 oder wenigstens eine Expressionskassette nach Ausführungsform 14.

16. Rekombinanter Mikroorganismus, enthaltend wenigstens eine Nukleinsäuresequenz nach Ausführungsform 13 oder wenigstens eine Expressionskassette nach Ausführungsform 14 oder wenigstens einen Vektor nach Ausführungsform 15.

17. Biokatalytisches Verfahren zur Herstellung von Isopulegol der allgemeinen Formel (I)

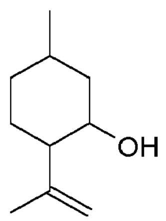

(I)

wobei man Citronellal der allgemeinen Formel (II)

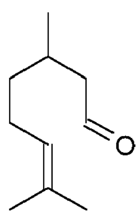

(II)

zu Isopulegol der Formel (I) mittels eines Enzyms der EC Klasse EC 5.4.99, insbesondere der EC Klasse EC 5.4.99.17, oder in Gegenwart eines dieses Enzym exprimieren Miroorganismus cyclisiert.

18. Biokatalytisches Verfahren zur Herstellung von Isopulegol der allgemeinen Formel (I)

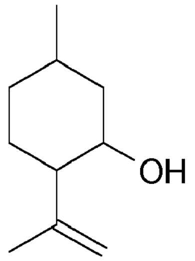

(I)

wobei man Citronellal der allgemeinen Formel (II)

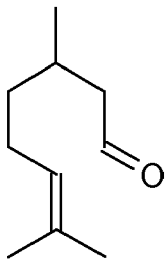

(II)

zu Isopulegol der Formel (I) mittels einer Enzymmutante nach einer der Ausführungsformen 1 bis 12, oder in Gegenwart eines diese Enzymmutante exprimieren Miroorganismus nach Ausführungsform 16 cyclisiert.

19. Verfahren zur Herstellung von Menthol, der Formel III

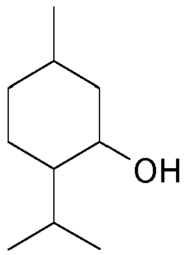

(III)

wobei man

a) Citronellal zu Isopulegol nach einem Verfahren gemäß Anspruch 17 oder 18 cyclisiert und
b) Isopulegol katalytisch zu Menthol hydriert.

20. Verfahren nach Anspruch 19, wobei die Hydrierung in Gegenwart von Wasserstoff und einem Katalysator erfolgt, der

- 30 bis 70 Gew.-% sauerstoffhaltige Verbindungen des Nickels, berechnet als NiO,
- 15 bis 45 Gew.-% sauerstoffhaltige Verbindungen des Zirkons, berechnet als $ZrO_2$,
- 5 bis 30 Gew.-% sauerstoffhaltige Verbindungen des Kupfers berechnet als CuO und
- 0,1 bis 10 Gew.-% sauerstoffhaltige Verbindungen des Molybdäns, berechnet als $MoO_3$,

umfasst, wobei sich die Gew.-%-Angaben auf den trockenen, nicht reduzierten Katalysator beziehen.

21. Verfahren zu enzymatischen oder biokatalytischen Umsetzungen von Verbindungen der allgemeinen Formel IV

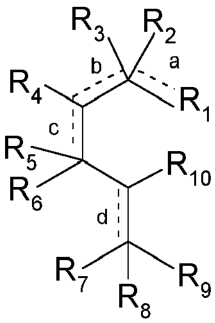

(IV)

worin

"a", "b", "c" und "d", jeweils unabhängig voneinander, eine C-C-Einfach- oder Doppelbindung darstellen, mit der Maßgabe, dass kumulierte Doppelbindungen ausgeschlossen sind; und den folgenden Maßgaben:

$R_1$ folgende Bedeutungen besitzt:

(1) wenn "a" eine Doppelbindung ist:
$R_1$ ausgewählt ist unter

Oxo (=O), oder
$CH-(CH_2)_n-Z$,

worin n für 0, 1 oder 2 steht und
Z für OH, CHO, C(O) Alkyl, wie $C(O)C_1-C_4$-Alkyl, insbesondere $C(O)-CH_3$ oder $C(O)-CH_2CH_3$; COOH, $C(CH_2)-CH=CH_2$;
$C(OH)(CH_3)-CH=CH_2$; $C(CH_3)=CH-CH=CH_2$; oder einen Rest der Formel $C(CH_3)=CH-CH_2Y$ steht
worin
Y für OH, $CH_2OH$, COOH, oder $CH_2C(O)CH_3$ steht; oder

(2) wenn "a" eine Einfachbindung ist:
$R_1$ ausgewählt ist unter
$CH_3$; CHO; $CH_2CH_2OH$; $CH=CH_2$; $CH_2C(O)OH$; $CH_2CHO$ oder $C_3H_6CH(CH_3)CHO$ steht; wobei, wenn "a" eine Doppelbindung ist, diese E- oder Z- Konfiguration aufweist;

$R_2$ und $R_3$ folgende Bedeutungen besitzen:

(1) wenn "a" und "b"jeweils eine Einfachbindung sind:
$R_2$ und $R_3$ unabhängig voneinander für H, Alkyl, wie $C_1-C_4$-Alkyl, oder OH stehen oder $R_2$ und $R_3$ zusammen für eine Methylen (=$CH_2$)- oder Oxo (=O)-Gruppe stehen; oder
(2) wenn "a" oder "b" eine Doppelbindung ist, einer der Reste $R_2$ und $R_3$ fehlt und der andere der beiden Reste für H, $C_1-C_4$-Alkyl, insbesondere Methyl, oder OH steht;

$R_4$ für H oder Hydroxy-$C_1-C_4$-alkyl, insbesondere Hydroxymethyl, steht;

$R_5$ und $R_6$ folgende Bedeutungen besitzen:

(1) wenn "c" eine Einfachbindung ist:
$R_5$ und $R_6$ jeweils für H stehen oder $R_5$ und $R_6$ zusammen für eine Oxo (=O)-Gruppe stehen; oder
(2) wenn "c" eine Doppelbindung ist, einer der Reste $R_5$ und $R_6$ fehlt und der andere der beiden Reste für H steht;

$R_7$, $R_8$ und $R_9$ folgende Bedeutungen besitzen:

(1) wenn "d" eine Einfachbindung ist:
zwei der Reste $R_7$, $R_8$ und $R_9$ jeweils unabhängig voneinander für H oder Alkyl, wie $C_1-C_4$-Alkyl, insbeson-

dere Methyl oder Ethyl, stehen und der andere der Reste für OH steht; oder
(2) wenn "d" eine Doppelbindung ist, einer der Reste $R_7$, $R_8$ und $R_9$ fehlt und die andere der beiden Reste jeweils unabhängig voneinander für H oder Alkyl, wie $C_1$-$C_4$-Alkyl, insbesondere Methyl oder Ethyl, stehen, und insbesondere zwei Reste für Methyl stehen;

$R_{10}$ für H oder Hydroxy-$C_1$-$C_6$-Alkyl, wie Hydroxy-$C_1$-$C_4$-Alky, oder ein oder mehrfach ungesättigtes $C_2$-$C_6$-Alkenyl, wie insbesondere H oder $CH=CH-C(CH_3)=CH_2$ steht;

wobei man eine Verbindung der Formel IV in stereoisomerenreiner Form oder ein Stereoisomerengemisch davon unter Verwendung eines Enzyms der Klasse EC 5.4.99, insbesondere der Klasse EC 5.4.99.17, oder eine Enzymmutante nach einer der Ausführungsformen 1 bis 12 oder in Gegenwart einen diese Enzyme oder Enzymmutanten exprimierenden Mikroorganismus nach Ausführungsform 16 umsetzt.

22. Verfahren nach Ausführungsform 21, worin eine Verbindung umgesetzt wird, die ausgewählt ist unter Verbindungen der Formel IVa

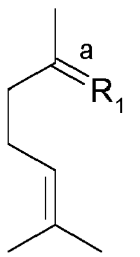

(IVa)

worin $R_1$ die oben angegebenen Bedeutungen besitzt und insbesondere für den Rest $CH-(CH_2)_n-Z$ steht
worin

n = 0 und Z = CHO, oder COOH ist; oder
n = 1 und Z = OH ist; oder
n = 2 und Z = $C(O)CH_3$; COOH, $C(CH_2)-CH=CH_2$; $C(CH_3)=CH-CH=CH_2$; oder ein Rest der Formel $C(CH_3)=CH-CH_2Y$ ist
worin Y für OH, $CH_2OH$, COOH, oder $CH_2C(O)CH_3$ steht;

und "a" ggf. E- oder Z-Konfiguration aufweist;
oder der Formel IVb

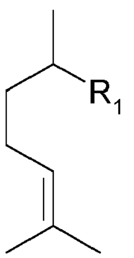

(IVb)

worin $R_1$ die oben angegebenen Bedeutungen besitzt; und insbesondere für $CH_2CHO$ steht;
oder der Formel IVc

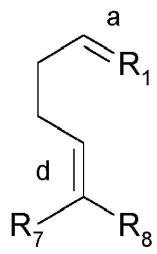

(IVc)

worin
$R_1$ die oben angegebenen Bedeutungen besitzt, und insbesondere für CH-CHO steht; und einer der Reste $R_7$ und $R_8$ für H und der andere für $C_1$-$C_4$-Alkyl, wobei insbesondere $R_7$ für Ethyl steht und die Doppelbindung "a" und "d" Z-Konfiguration aufweisen.

23. Verfahren nach einer der Ausführungsformen 20 bis 22, worin die Verbindung der Formel IV ausgewählt ist unter Citronellal; Citral; Farnesol; Homofarnesol; Homofarnesolderivaten, wie Homofarnesylsäure; Geranylaceton, Melonal; Nonadienal; und Trimethyldecatetraen.

24. Verwendung eines Enzyms aus der EC Klasse EC 5.4.99, insbesondere der EC Klasse EC 5.4.99.17 zur Cyclisierung von Terpenen und/oder Terpenoiden, insbesondere zur Umsetzung von Citronellal zu Isopulegol.

25. Verwendung einer Enzymmutante nach einer der Ausführungsformen 1 bis 12, einer Nukleinsäure nach Ausführungsform 13, eines Expressionskonstruktes nach Ausführungsform 14, eines rekombinanten Vektors nach Ausführungsform 15 oder eines rekombinanten Mikroorganismus nach Ausführungsform 1 zur Cyclisierung von Terpenen und/oder Terpenoiden, sowie zur Umsetzung von Verbindungen der allgemeinen Formel IV gemäß der Definition in einer der Ausführungsformen 20 bis 23.

25. Verwendung nach Ausführungsform 25 zur Umsetzung von Citronellal zu Isopulegol; oder zur Umsetzung von Squalen zu Hopen.

26. Verfahren zur Herstellung von Isopulegol der allgemeinen Formel (I)

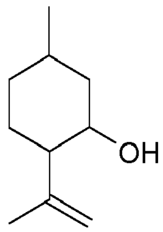

(I)

umfassend einen Reaktionsschritt,
wobei man Citronellal der allgemeinen Formel (II)

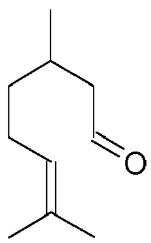

(II)

biokatalytisch zu dem entsprechenden Isopulegol der Formel (I) mittels eines Enzyms mit der Aktivität einer Citronellal-Isopulegol-Cyclase cyclisiert.

27. Verfahren nach Ausführungsform 26, dadurch gekennzeichnet, dass das Enzym eine Polypeptidsequenz besitzt, die entweder

a) SEQ ID NO:2 ist, oder
b) bei der bis zu 25%, wie z.B. bis zu 20, 15, 10, 9, 8, 7, 6, 5, 4, 3, 2 oder 1% der Aminosäurereste gegenüber SEQ ID NO:2 durch Deletion, Insertion, Substitution oder einer Kombination davon verändert sind, und die noch mindestens 50%, wie z.B. mindestens 60, 65, 70, 75, 80, 85, 90 oder 95% der enzymatischen Aktivität von SEQ ID NO:2 aufweist.

28. Verfahren nach Ausführungsform 26 oder 27, dadurch gekennzeichnet, dass das Enzym von einer Nukleinsäuresequenz gemäß SEQ ID NO:1 oder einem funktionalen Äquivalent davon kodiert wird.

29. Verfahren nach einer der Ausführungsformen 26 bis 28, dadurch gekennzeichnet, dass das Enzym von einer Nukleinsäuresequenz gemäß SEQ ID NO:1 oder einem funktionalen Äquivalent davon kodiert wird, wobei die Nukleinsäuresequenz Bestandteil eines Genkonstrukts oder Vektors ist.

30. Verfahren nach einer der Ausführungsformen 26 bis 29, dadurch gekennzeichnet, dass das Enzym von einer Nukleinsäuresequenz gemäß SEQ ID NO:1 oder einem funktionalen Äquivalent davon kodiert wird, wobei die Nukleinsäuresequenz Bestandteil eines Genkonstrukts oder Vektors ist, welche in einer Wirtszelle enthalten sind.

31. Verfahren nach einer der Ausführungsformen 26 bis 30, dadurch gekennzeichnet, dass das Enzym in einer Form vorliegt, ausgewählt aus der Gruppe bestehend aus:

a) freies, gegebenenfalls gereinigtes oder teilweise gereinigtes Polypeptid mit der Aktivität einer Citronellal-Isopulegol-Cyclase;
b) immobilisiertes Polypeptid mit der Aktivität einer Citronellal-Isopulegol-Cyclase;
c) aus Zellen isoliertes Polypeptid gemäß a) oder b);
d) ganze Zelle, gegebenenfalls ruhende oder aufgeschlossene Zellen, enthaltend mindestens ein Polypeptid mit der Aktivität einer Citronellal-Isopulegol-Cyclase;
e) Zelllysat oder Zellhomogenisat der Zellen beschrieben unter d).

32. Verfahren nach Ausführungsform 31, dadurch gekennzeichnet, dass die Zellen Mikroorganismen sind, bevorzugt transgene Mikroorganismen exprimierend mindestens ein heterologes Nukleinsäuremolekül kodierend für ein Polypeptid mit der Aktivität einer Citronellal-Isopulegol-Cyclase.

33. Verfahren nach einer der Ausführungsformen 26 bis 32, dadurch gekennzeichnet, dass die Herstellung von Isopulegol in einphasigen wässrigen Systemen oder in zweiphasigen Systemen erfolgt.

34. Verfahren nach einer der Ausführungsformen 26 bis 33, wobei die Reaktion von Citronellal zu Isopulegol bei einer Temperatur im Bereich von 20 bis 40 °C und/oder einem pH-Wert im Bereich von 4 bis 8 erfolgt.

35. Verfahren nach einer der Ausführungsformen 26 bis 34, dadurch gekennzeichnet, dass das Enzym mit der Aktivität einer Citronellal-Isopulegol-Cyclase von einem Gen kodiert wird, das aus einem Mikroorganismus isoliert wurde, ausgewählt aus der Gruppe der Mikroorganismen bestehend aus Zymomonas mobilis, Methylococcus capsulatus, Rhodopseudomonas palustris, Bradyrhizobium japonicum, Frankia spec. und Streptomyces coelicolor insbesondere Zymomonas mobilis.

36. Verfahren nach einer der Ausführungsformen 26 bis 35, dadurch gekennzeichnet, dass das Enzym mit der Aktivität einer Citronellal-Isopulegol-Cyclase von einem Mikroorganismus erzeugt wurde, der das Enzym mit der Aktivität einer Citronellal-Isopulegol-Cyclase überproduziert und der ausgewählt wurde aus der Gruppe der Mikroorganismen bestehend aus den Gattungen Escherichia, Corynebacterium, Ralstonia, Clostridium, Pseudomonas, Bacillus, Zymomonas, Rhodobacter, Streptomyces, Burkholderia, Lactobacillus und Lactococcus.

37. Verfahren nach einer der Ausführungsformen 26 bis 36, dadurch gekennzeichnet dass das Enzym mit der Aktivität einer Citronellal-Isopulegol-Cyclase von transgenen Mikroorganismen der Spezies Escherichia coli, Pseudomonas putida, Burkholderia glumae, Corynebacterium glutamicum, Saccharomyces cerevisiae, Pichia pastoris, Streptomyces lividans, Streptomyces coelicolor, Bacillus subtilis oder Zymomonas mobilis erzeugt wurde, welche das Enzym mit der Aktivität einer Citronellal-Isopulegol-Cyclase überproduzieren.

38. Verwendung eines Enzyms mit der Aktivität einer Citronellal-Isopulegol-Cyclase zur biokatalytischen Umsetzung von Citronellal zu Isopulegol.

39. Verwendung nach Ausführungsform 38, dadurch gekennzeichnet, dass das Enzym eine Polypeptidsequenz besitzt, die entweder

a) SEQ ID NO:2 ist, oder
b) bei der bis zu 25%, wie z.B. bis zu 20, 15, 10, 9, 8, 7, 6, 5, 4, 3, 2 oder 1% der Aminosäurereste gegenüber SEQ ID NO:2 durch Deletion, Insertion, Substitution oder einer Kombination davon verändert sind, und die noch mindestens 50%, wie z.B. mindestens 60, 65, 70, 75, 80, 85, 90 oder 95% der enzymatischen Aktivität von SEQ ID NO:2 aufweist.

40. Verwendung nach Ausführungsform 38 oder 39, dadurch gekennzeichnet, dass das Enzym von einer Nukleinsäuresequenz gemäß SEQ ID NO:1 oder einem funktionalen Äquivalent davon kodiert wird.

41. Verwendung eines Genkonstrukts oder Vektors umfassend eine Nukleinsäuresequenz gemäß SEQ ID NO:1 oder ein funktionales Äquivalent davon, die ein Polypeptid mit der Aktivität einer Citronellal-Isopulegol-Cyclase kodieren, das zur biokatalytischen Umsetzung von Citronellal zu Isopulegol dient, in einem Verfahren zur Herstellung von Isopulegol durch Cyclisierung von Citronellal.

42. Verwendung einer Wirtszelle, die ein Genkonstrukt oder einen Vektor enthält, welche eine Nukleinsäuresequenz gemäß SEQ ID NO:1 oder ein funktionales Äquivalent davon umfassen, für die Herstellung eines Enzyms mit der Aktivität einer Citronellal-Isopulegol-Cyclase für die biokatalytische Umsetzung von Citronellal zu Isopulegol.

**C. Weitere Ausgestaltungen der Erfindung**

**1. Besonders geeignete Wildtyp-Sequenzen**

Erfindungsgemäß brauchbare SHC Wildtypsequenzen, deren SEQ ID NO, Quellorganismus, Genbank-Referenznummer, der der Position F486 von SEQ ID NO:2 "entsprechende", d.h. F486-analoge, Aminosäurerest ("Aa") und dessen Sequenzposition sind in folgender Tabelle zusammengefasst. Die Angaben beruhen dabei auf einem Sequenzalignment, das folgendermaßen erstellt wurde:

| | |
|---|---|
| Programm: CLUSTALW, | |
| Default-Parameter: | |
| Protein Gap Open Penalty | 10.0 |
| Protein Gap Extension Penalty | 0.2 |
| Protein weight matrix: | Gonnet series |

| S_ID DB | SEQ ID NO | Organism | GI-Nr. der Referenz Sequenzen | Aa | Position |
|---|---|---|---|---|---|
| s1 | seq_ID 2 | ***Zymomonas mobilis*** | AAV90172.1 | F | 486 |
| s20 | seq_ID 3 | ***Streptomyces coelicolor*** | CAB39697.1 | F | 449 |
| s911 | seq_ID 4 | ***Acetobacter pasteurianus*** | BAH99456.1 | F | 481 |
| s2 | seq_ID 5 | ***Bradyrhizobium sp.*** | ABQ33590.1 | F | 447 |
| s940 | seq_ID 6 | ***Zymomonas mobilis*** | EER62728.1 | F | 438 |
| s949 | seq_ID 7 | ***Acidithiobacillus caldus*** | EET25937.1 | Y | 432 |
| s167 | seq_ID 8 | ***Acidithiobacillus ferrooxidans*** | ACH84004.1 | Y | 429 |
| s41 | seq_ID 9 | ***Acidobacterium capsulatum*** | AC034244.1 | F | 458 |
| s36 | seq_ID 10 | ***Acidothermus cellulolyticus*** | ABK53469.1 | F | 426 |
| s83 | seq_ID 11 | ***Adiantum capillus-veneris*** | BAF93209.1 | Y | 436 |
| s143 | seq_ID 12 | ***Ajellomyces capsulatus*** | EDN09769.1 | F | 496 |
| s995 | seq_ID 13 | ***Ajellomyces capsulatus*** | EER40510.1 | - | 432 |

(fortgesetzt)

| S_ID DB | SEQ ID NO | Organism | GI-Nr. der Referenz Sequenzen | Aa | Position |
|---|---|---|---|---|---|
| s163 | seq_ID 14 | ***Ajellomyces capsulatus*** | EEH02950.1 | F | 429 |
| s13 | seq_ID 15 | ***Alicyclobacillus acidocaldarius*** | EED08231.1 | Y | 420 |
| s14 | seq_ID 16 | ***Alicyclobacillus acidocaldarius*** | P33247.4 | Y | 420 |
| s1193 | seq_ID 17 | ***Alicyclobacillus acidocaldarius*** | AAT70690.1 | Y | 116 |
| s21 | seq_ID 18 | ***Alicyclobacillus acidoterrestris*** | CAA61950.1 | Y | 420 |
| s1189 | seq_ID 19 | ***Alicyclobacillus acidoterrestris*** | AAT70691.1 | Y | 121 |
| s51 | seq_ID 20 | ***Anabaena variabilis*** | ABA24268.1 | F | 423 |
| s76 | seq_ID 21 | ***Anaeromyxobacter sp.*** | ABS28257.1 | F | 440 |
| s159 | seq_ID 22 | ***Aspergillus clavatus*** | EAW07713.1 | F | 446 |
| s131 | seq_ID 23 | ***Aspergillus flavus*** | EED48353.1 | F | 444 |
| s176 | seq_ID 24 | ***Aspergillus fumigatus*** | EDP50814.1 | F | 502 |
| s126 | seq_ID 25 | ***Aspergillus fumigatus*** | EAL84865.1 | F | 449 |
| s178 | seq_ID 26 | ***Aspergillus fumigatus*** | EAL86291.2 | F | 406 |
| s121 | seq_ID 27 | ***Aspergillus niger*** | CAK43501.1 | F | 441 |
| s115 | seq_ID 28 | ***Aspergillus niger*** | CAK45506.1 | F | 440 |
| s124 | seq_ID 29 | ***Aspergillus oryzae*** | BAE63941.1 | F | 444 |
| s119 | seq_ID 30 | ***Azotobacter vinelandii*** | EAM07611.1 | F | 442 |
| s223 | seq_ID 31 | ***Bacillus amyloliquefaciens*** | ABS74269.1 | F | 413 |
| s221 | seq_ID 32 | ***Bacillus anthracis*** | AAP27368.1 | F | 409 |
| s976 | seq_ID 33 | ***Bacillus cereus*** | EEK66523.1 | F | 423 |
| s225 | seq_ID 34 | ***Bacillus cereus*** | EAL12758.1 | F | 423 |
| s972 | seq_ID 35 | ***Bacillus cereus*** | EEL44583.1 | F | 412 |
| s977 | seq_ID 36 | ***Bacillus cereus*** | EEK43841.1 | F | 412 |
| s985 | seq_ID 37 | ***Bacillus cereus*** | EEK82938.1 | F | 412 |
| s988 | seq_ID 38 | ***Bacillus cereus*** | EEK99528.1 | F | 412 |

(fortgesetzt)

| S_ID DB | SEQ ID NO | Organism | GI-Nr. der Referenz Sequenzen | Aa | Position |
|---|---|---|---|---|---|
| s981 | seq_ID 39 | ***Bacillus cereus*** | EEK77935.1 | F | 412 |
| s987 | seq_ID 40 | ***Bacillus cereus*** | EEL81079.1 | F | 412 |
| s960 | seq_ID 41 | ***Bacillus cereus*** | EEK88307.1 | F | 412 |
| s979 | seq_ID 42 | ***Bacillus cereus*** | EEL63943.1 | F | 412 |
| s974 | seq_ID 43 | ***Bacillus cereus*** | EEL59884.1 | F | 412 |
| s956 | seq_ID 44 | ***Bacillus cereus*** | EEL69857.1 | F | 412 |
| s951 | seq_ID 45 | ***Bacillus cereus*** | EEL92663.1 | F | 412 |
| s986 | seq_ID 46 | ***Bacillus cereus*** | EEL49968.1 | F | 411 |
| s227 | seq_ID 47 | ***Bacillus cereus*** | AAU16998.1 | F | 409 |
| s224 | seq_ID 48 | ***Bacillus cereus*** | AAS42477.1 | F | 409 |
| s212 | seq_ID 49 | ***Bacillus cereus*** | ACK95843.1 | F | 409 |
| s289 | seq_ID 50 | ***Bacillus coahuilensis*** | 205373680 | F | 276 |
| s219 | seq_ID 51 | ***Bacillus cytotoxicus*** | ABS22481.1 | F | 411 |
| s230 | seq_ID 52 | ***Bacillus licheniformis*** | AAU23777.1 | F | 414 |
| s955 | seq_ID 53 | ***Bacillus mycoides*** | EEL98438.1 | F | 412 |
| s990 | seq_ID 54 | ***Bacillus mycoides*** | EEM04821.1 | F | 411 |
| s989 | seq_ID 55 | ***Bacillus pseudomycoides*** | EEM16144.1 | F | 411 |
| s247 | seq_ID 56 | ***Bacillus pumilus*** | ABV62529.1 | F | 409 |
| s250 | seq_ID 57 | ***Bacillus pumilus*** | EDW21137.1 | F | 409 |
| s249 | seq_ID 58 | ***Bacillus sp.*** | EAR64404.1 | F | 425 |
| s218 | seq_ID 59 | ***Bacillus sp.*** | EDL66148.1 | F | 412 |
| s241 | seq_ID 60 | ***Bacillus subtilis*** | Q796C3.1 | F | 415 |
| s284 | seq_ID 61 | ***Bacillus subtilis*** | AAB84441.1 | F | 415 |
| s215 | seq_ID 62 | ***Bacillus thuringiensis*** | ABK86448.1 | F | 423 |
| s984 | seq_ID 63 | ***Bacillus thuringiensis*** | EEM21409.1 | F | 412 |

(fortgesetzt)

| S_ID DB | SEQ ID NO | Organism | GI-Nr. der Referenz Sequenzen | Aa | Position |
|---|---|---|---|---|---|
| s957 | seq_ID 64 | ***Bacillus thuringiensis*** | EEM82653.1 | F | 412 |
| s980 | seq_ID 65 | ***Bacillus thuringiensis*** | EEM52372.1 | F | 412 |
| s961 | seq_ID 66 | ***Bacillus thuringiensis*** | EEM27851.1 | F | 412 |
| s969 | seq_ID 67 | ***Bacillus thuringiensis*** | EEM40716.1 | F | 412 |
| s959 | seq_ID 68 | ***Bacillus thuringiensis*** | EEM46814.1 | F | 409 |
| s965 | seq_ID 69 | ***Bacillus thuringiensis*** | EEM94969.1 | F | 409 |
| s202 | seq_ID 70 | ***Bacillus weihenstephanensis*** | ABY44436.1 | F | 409 |
| s63 | seq_ID 71 | ***Bacterium Ellin514*** | EEF57225.1 | F | 461 |
| s72 | seq_ID 72 | ***Bacterium Ellin514*** | EEF59508.1 | Y | 435 |
| s87 | seq_ID 73 | ***Beijerinckia indica*** | ACB96717.1 | F | 441 |
| s69 | seq_ID 74 | ***Blastopirellula marina*** | EAQ81955.1 | F | 475 |
| s543 | seq_ID 75 | ***Blastopirellula marina*** | EAQ78122.1 | F | 389 |
| s156 | seq_ID 76 | ***Bradyrhizobium japonicum*** | CAA60250.1 | F | 439 |
| s938 | seq_ID 77 | ***Acetobacter pasteurianus*** | BAH98349.1 | F | 437 |
| s3 | seq_ID 78 | ***Bradyrhizobium sp.*** | CAL79893.1 | F | 447 |
| s201 | seq_ID 79 | ***Brevibacillus brevis*** | BAH44778.1 | F | 448 |
| s148 | seq_ID 80 | ***Burkholderia ambifaria*** | EDT05097.1 | F | 450 |
| s158 | seq_ID 81 | ***Burkholderia ambifaria*** | EDT37649.1 | F | 450 |
| s149 | seq_ID 82 | ***Burkholderia ambifaria*** | ACB68303.1 | F | 446 |
| s100 | seq_ID 83 | ***Burkholderia ambifaria*** | EDT42454.1 | F | 436 |
| s146 | seq_ID 84 | ***Burkholderia cenocepacia*** | EAY66961.1 | F | 451 |
| s139 | seq_ID 85 | ***Burkholderia cenocepacia*** | ACA95661.1 | F | 451 |
| s147 | seq_ID 86 | ***Burkholderia cenocepacia*** | CAR57099.1 | F | 451 |
| s95 | seq_ID 87 | ***Burkholderia cenocepacia*** | CAR56694.1 | F | 436 |
| s102 | seq_ID 88 | ***Burkholderia dolosa*** | EAY71311.1 | F | 437 |

(fortgesetzt)

| S_ID DB | SEQ ID NO | Organism | GI-Nr. der Referenz Sequenzen | Aa | Position |
|---|---|---|---|---|---|
| s941 | seq_ID 89 | ***Burkholderia glumae*** | ACR32572.1 | F | 555 |
| s945 | seq_ID 90 | ***Burkholderia glumae*** | ACR30752.1 | F | 449 |
| s132 | seq_ID 91 | ***Burkholderia graminis*** | EDT12320.1 | F | 462 |
| s104 | seq_ID 92 | ***Burkholderia mallei*** | ABM48844.1 | F | 436 |
| s140 | seq_ID 93 | ***Burkholderia multivorans*** | ABX19650.1 | F | 450 |
| s116 | seq_ID 94 | ***Burkholderia multivorans*** | ABX16859.1 | F | 436 |
| s91 | seq_ID 95 | ***Burkholderia oklahomensis*** | 167567074 | F | 447 |
| s111 | seq_ID 96 | ***Burkholderia phymatum*** | ACC73258.1 | F | 456 |
| s127 | seq_ID 97 | ***Burkholderia phytofirmans*** | ACD21317.1 | F | 455 |
| s120 | seq_ID 98 | ***Burkholderia pseudomallei*** | EEC32728.1 | F | 436 |
| s137 | seq_ID 99 | ***Burkholderia sp.*** | EEA03553.1 | F | 460 |
| s144 | seq_ID 100 | ***Burkholderia sp.*** | ABB06563.1 | F | 450 |
| s98 | seq_ID 101 | ***Burkholderia sp.*** | ABB10136.1 | F | 436 |
| s944 | seq_ID 102 | ***Burkholderia sp. CCGE1002*** | EFA54357.1 | F | 473 |
| s89 | seq_ID 103 | ***Burkholderia thailandensis*** | 167840988 | F | 451 |
| s113 | seq_ID 104 | ***Burkholderia thailandensis*** | 167617352 | F | 442 |
| s154 | seq_ID 105 | ***Burkholderia ubonensis*** | 167589807 | F | 445 |
| s93 | seq_ID 106 | ***Burkholderia ubonensis*** | 167584986 | F | 436 |
| s96 | seq_ID 107 | ***Burkholderia vietnamiensis*** | AB056791.1 | F | 436 |
| s150 | seq_ID 108 | ***Burkholderia xenovorans*** | ABE35912.1 | F | 457 |
| s54 | seq_ID 109 | ***Candidatus Koribacter*** | ABF40741.1 | F | 435 |
| s171 | seq_ID 110 | ***Candidatus Kuenenia*** | CAJ71215.1 | F | 273 |
| s79 | seq_ID 111 | ***Candidatus Solibacter*** | ABJ82180.1 | F | 439 |
| s99 | seq_ID 112 | ***Candidatus Solibacter*** | ABJ82254.1 | F | 429 |
| s917 | seq_ID 113 | ***Catenulispora acidiphila*** | ACU75510.1 | F | 418 |

(fortgesetzt)

| S_ID DB | SEQ ID NO | Organism | GI-Nr. der Referenz Sequenzen | Aa | Position |
|---|---|---|---|---|---|
| s65 | seq_ID 114 | ***Chthoniobacter flavus*** | EDY15838.1 | F | 433 |
| s637 | seq_ID 115 | ***Chthoniobacter flavus*** | EDY22035.1 | F | 384 |
| s38 | seq_ID 116 | ***Crocosphaera watsonii*** | EAM53094.1 | F | 426 |
| s186 | seq_ID 117 | ***Cupriavidus taiwanensis*** | CAQ72562.1 | F | 454 |
| s32 | seq_ID 118 | ***Cyanothece sp.*** | ACB53858.1 | F | 441 |
| s40 | seq_ID 119 | ***Cyanothece sp.*** | ACK71719.1 | F | 430 |
| s30 | seq_ID 120 | ***Cyanothece sp.*** | EDY02410.1 | F | 429 |
| s29 | seq_ID 121 | ***Cyanothece sp.*** | ACK66841.1 | F | 429 |
| s47 | seq_ID 122 | ***Cyanothece sp.*** | EDX97382.1 | F | 428 |
| s35 | seq_ID 123 | ***Cyanothece sp.*** | EAZ91809.1 | F | 426 |
| s39 | seq_ID 124 | ***Cyanothece sp.*** | ACL45896.1 | F | 423 |
| s925 | seq_ID 125 | ***Cyanothece sp. PCC 8802*** | ACV02092.1 | F | 429 |
| s64 | seq_ID 126 | ***Desulfovibrio salexigens*** | EEC62384.1 | F | 475 |
| s74 | seq_ID 127 | ***Dryopteris crassirhizoma*** | BAG68223.1 | F | 444 |
| s59 | seq_ID 128 | ***Frankia alni*** | CAJ61140.1 | Y | 533 |
| s48 | seq_ID 129 | ***Frankia alni*** | CAJ60090.1 | F | 493 |
| s56 | seq_ID 130 | ***Frankia sp.*** | ABD10207.1 | F | 530 |
| s60 | seq_ID 131 | ***Frankia sp.*** | ABW15063.1 | F | 512 |
| s31 | seq_ID 132 | ***Frankia sp.*** | ABW14125.1 | Y | 481 |
| s948 | seq_ID 133 | ***Frankia sp. Eul1c*** | EFA59873.1 | F | 557 |
| s919 | seq_ID 134 | ***Frankia sp. Eul1c*** | EFA59089.1 | F | 553 |
| s628 | seq_ID 135 | ***Gemmata obscuriglobus*** | 168700710 | F | 387 |
| s209 | seq_ID 136 | ***Geobacillus sp.*** | EED61885.1 | F | 404 |
| s206 | seq_ID 137 | ***Geobacillus sp.*** | EDY05760.1 | F | 403 |
| s964 | seq_ID 138 | ***Geobacillus sp. Y412MC52*** | EEN95021.1 | F | 404 |

(fortgesetzt)

| S_ID DB | SEQ ID NO | Organism | GI-Nr. der Referenz Sequenzen | Aa | Position |
|---|---|---|---|---|---|
| s993 | seq_ID 139 | ***Geobacillus sp. Y412MC61*** | ACX79399.1 | F | 404 |
| s205 | seq_ID 140 | ***Geobacillus thermodenitrificans*** | AB067242.1 | F | 403 |
| s15 | seq_ID 141 | ***Geobacter bemidjiensis*** | ACH40355.1 | F | 468 |
| s8 | seq_ID 142 | ***Geobacter lovleyi*** | ACD95949.1 | F | 470 |
| s62 | seq_ID 143 | ***Geobacter metallireducens*** | ABB30662.1 | F | 493 |
| s12 | seq_ID 144 | ***Geobacter metallireducens*** | ABB33038.1 | F | 467 |
| s73 | seq_ID 145 | ***Geobacter sp.*** | ACM21577.1 | F | 487 |
| s10 | seq_ID 146 | ***Geobacter sp.*** | EDV72707.1 | F | 468 |
| s11 | seq_ID 147 | ***Geobacter sp.*** | ACM22003.1 | F | 467 |
| s913 | seq_ID 148 | ***Geobacter sp. M18*** | EET34621.1 | F | 468 |
| s914 | seq_ID 149 | ***Geobacter sp. M21*** | ACT16952.1 | F | 468 |
| s58 | seq_ID 150 | ***Geobacter sulfurreducens*** | AAR36453.1 | F | 493 |
| s7 | seq_ID 151 | ***Geobacter sulfurreducens*** | AAR34018.1 | F | 467 |
| s9 | seq_ID 152 | ***Geobacter uraniireducens*** | ABQ25226.1 | F | 467 |
| s46 | seq_ID 153 | ***Gloeobacter violaceus*** | BAC91998.1 | F | 425 |
| s67 | seq_ID 154 | ***Gluconacetobacter diazotrophicus*** | ACI51585.1 | F | 444 |
| s165 | seq_ID 155 | ***Gluconacetobacter diazotrophicus*** | CAP55563.1 | F | 444 |
| s68 | seq_ID 156 | ***Gluconobacter oxydans*** | AAW61994.1 | F | 445 |
| s80 | seq_ID 157 | ***Granulibacter bethesdensis*** | ABI63005.1 | F | 429 |
| s937 | seq_ID 158 | ***Hyphomicrobium denitrificans*** | EET65847.1 | F | 444 |
| s932 | seq_ID 159 | ***Leptospirillum ferrodiazotrophum*** | EES53667.1 | F | 460 |
| s24 | seq_ID 160 | ***Leptospirillum rubarum*** | EAY57382.1 | F | 448 |
| s25 | seq_ID 161 | ***Leptospirillum sp.*** | EDZ38599.1 | F | 448 |
| s174 | seq_ID 162 | ***Magnaporthe grisea*** | EDK02551.1 | F | 445 |
| s153 | seq_ID 163 | ***Magnetospirillum magnetotacticum*** | 46203107 | F | 447 |

(fortgesetzt)

| S_ID DB | SEQ ID NO | Organism | GI-Nr. der Referenz Sequenzen | Aa | Position |
|---|---|---|---|---|---|
| s49 | seq_ID 164 | ***Methylacidiphilum infernorum*** | ACD82457.1 | F | 456 |
| s169 | seq_ID 165 | ***Methylobacterium chloromethanicum*** | ACK83067.1 | F | 447 |
| s75 | seq_ID 166 | ***Methylobacterium chloromethanicum*** | ACK86232.1 | F | 426 |
| s946 | seq_ID 167 | ***Methylobacterium extorquens*** | CAX24364.1 | F | 447 |
| s141 | seq_ID 168 | ***Methylobacterium nodulans*** | ACL61886.1 | F | 442 |
| s152 | seq_ID 169 | ***Methylobacterium populi*** | ACB79998.1 | F | 447 |
| s162 | seq_ID 170 | ***Methylobacterium radiotolerans*** | ACB27373.1 | F | 445 |
| s180 | seq_ID 171 | ***Methylobacterium sp.*** | ACA20611.1 | F | 442 |
| s175 | seq_ID 172 | ***Methylocella silvestris*** | ACK52150.1 | F | 451 |
| s181 | seq_ID 173 | ***Methylococcus capsulatus*** | CAA71098.1 | F | 439 |
| s55 | seq_ID 174 | ***Microcystis aeruginosa*** | CA086472.1 | F | 423 |
| s101 | seq_ID 175 | ***Neosartorya fischeri*** | EAW20752.1 | F | 448 |
| s129 | seq_ID 176 | ***Nitrobacter hamburgensis*** | ABE63461.1 | F | 433 |
| s161 | seq_ID 177 | ***Nitrobacter sp.*** | EAQ34404.1 | F | 430 |
| s160 | seq_ID 178 | ***Nitrobacter winogradskyi*** | ABA05523.1 | F | 433 |
| s157 | seq_ID 179 | ***Nitrococcus mobilis*** | EAR22397.1 | F | 436 |
| s164 | seq_ID 180 | ***Nitrosococcus oceani*** | ABA57818.1 | F | 446 |
| s170 | seq_ID 181 | ***Nitrosomonas europaea*** | CAD85079.1 | F | 452 |
| s173 | seq_ID 182 | ***Nitrosomonas eutropha*** | ABI59752.1 | F | 456 |
| s943 | seq_ID 183 | ***Nitrosomonas sp. AL212*** | EET32702.1 | F | 452 |
| s142 | seq_ID 184 | ***Nitrosospira multiformis*** | ABB75845.1 | F | 439 |
| s52 | seq_ID 185 | ***Nostoc punctiforme*** | ACC84529.1 | F | 423 |
| s45 | seq_ID 186 | ***Nostoc sp.*** | BAB72732.1 | F | 423 |
| s122 | seq_ID 187 | ***Oligotropha carboxidovorans*** | ACI93782.1 | F | 433 |
| s233 | seq_ID 188 | ***Paenibacillus sp.*** | EDS49994.1 | F | 399 |

(fortgesetzt)

| S_ID DB | SEQ ID NO | Organism | GI-Nr. der Referenz Sequenzen | Aa | Position |
|---|---|---|---|---|---|
| s991 | seq_ID 189 | ***Paenibacillus sp. JDR-2*** | ACS99948.1 | F | 399 |
| s950 | seq_ID 190 | ***Paenibacillus sp. oral taxon 786*** | EES74793.1 | F | 428 |
| s1280 | seq_ID 191 | ***Paramecium tetraurelia*** | 145542269 | F | 400 |
| s71 | seq_ID 192 | ***Pelobacter carbinolicus*** | ABA87701.1 | F | 494 |
| s5 | seq_ID 193 | ***Pelobacter carbinolicus*** | ABA87615.1 | F | 435 |
| s66 | seq_ID 194 | ***Pelobacter propionicus*** | ABK98395.1 | F | 486 |
| s16 | seq_ID 195 | ***Pelobacter propionicus*** | ABK98811.1 | F | 467 |
| s136 | seq_ID 196 | ***Penicillium chrysogenum*** | CAP99707.1 | F | 440 |
| s936 | seq_ID 197 | ***Planctomyces limnophilus*** | EE067214.1 | F | 490 |
| s1158 | seq_ID 198 | ***Planctomyces limnophilus*** | EE068341.1 | F | 412 |
| s526 | seq_ID 199 | ***Planctomyces maris*** | EDL58855.1 | F | 392 |
| s992 | seq_ID 200 | ***Polypodiodes niponica*** | BAI48071.1 | Y | 521 |
| s942 | seq_ID 201 | ***Polypodiodes niponica*** | BAI48070.1 | F | 443 |
| s1202 | seq_ID 202 | ***Populus trichocarpa*** | EEF12098.1 | F | 162 |
| s168 | seq_ID 203 | ***Ralstonia eutropha*** | AAZ64302.1 | F | 452 |
| s190 | seq_ID 204 | ***Ralstonia eutropha*** | CAJ96989.1 | F | 451 |
| s81 | seq_ID 205 | ***Ralstonia metallidurans*** | ABF11015.1 | F | 448 |
| s110 | seq_ID 206 | ***Ralstonia metallidurans*** | ABF11268.1 | F | 430 |
| s123 | seq_ID 207 | ***Rhizobium sp.*** | P55348.1 | F | 433 |
| s657 | seq_ID 208 | ***Rhodopirellula baltica*** | CAD74517.1 | F | 428 |
| s4 | seq_ID 209 | ***Rhodopseudomonas palustris*** | ABJ08391.1 | F | 445 |
| s130 | seq_ID 210 | ***Rhodopseudomonas palustris*** | CAA71101.1 | F | 433 |
| s155 | seq_ID 211 | ***Rhodopseudomonas palustris*** | ABD06434.1 | F | 433 |
| s97 | seq_ID 212 | ***Rhodopseudomonas palustris*** | ABD87279.1 | F | 433 |
| s135 | seq_ID 213 | ***Rhodopseudomonas palustris*** | ACF02757.1 | F | 432 |

(fortgesetzt)

| S_ID DB | SEQ ID NO | Organism | GI-Nr. der Referenz Sequenzen | Aa | Position |
|---|---|---|---|---|---|
| s84 | seq_ID 214 | ***Rhodospirillum rubrum*** | ABC20867.1 | F | 437 |
| s1279 | seq_ID 215 | ***Rubrobacter xylanophilus*** | ABG05671.1 | F | 372 |
| s915 | seq_ID 216 | ***Saccharomonospora viridis*** | ACU97316.1 | F | 428 |
| s42 | seq_ID 217 | ***Saccharopolyspora erythraea*** | CAM03596.1 | F | 421 |
| s82 | seq_ID 218 | ***Schizosaccharomyces japonicus*** | EEB08219.1 | F | 437 |
| s923 | seq_ID 219 | ***Sphaerobacter thermophilus*** | ACZ39437.1 | F | 404 |
| s924 | seq_ID 220 | ***Streptomyces albus*** | 239983547 | F | 371 |
| s23 | seq_ID 221 | ***Streptomyces avermitilis*** | BAC69361.1 | F | 450 |
| s44 | seq_ID 222 | ***Acaryochloris marina*** | ABW29816.1 | F | 423 |
| s921 | seq_ID 223 | ***Streptomyces filamentosus*** | 239945642 | F | 447 |
| s934 | seq_ID 224 | ***Streptomyces flavogriseus*** | EEW70811.1 | F | 447 |
| s920 | seq_ID 225 | ***Streptomyces ghanaensis*** | 239927462 | F | 448 |
| s922 | seq_ID 226 | ***Streptomyces griseoflavus*** | 256812310 | F | 448 |
| s28 | seq_ID 227 | ***Streptomyces griseus*** | BAG17791.1 | F | 447 |
| s926 | seq_ID 228 | ***Streptomyces hygroscopicus*** | 256775136 | F | 414 |
| s916 | seq_ID 229 | ***Streptomyces lividans*** | 256783789 | F | 449 |
| s33 | seq_ID 230 | ***Streptomyces peucetius*** | ACA52082.1 | F | 455 |
| s27 | seq_ID 231 | ***Streptomyces pristinaespiralis*** | EDY61772.1 | F | 455 |
| s933 | seq_ID 232 | ***Streptomyces scabiei*** | CBG68454.1 | F | 447 |
| s37 | seq_ID 233 | ***Streptomyces sp.*** | EDX25760.1 | F | 453 |
| s34 | seq_ID 234 | ***Streptomyces sp.*** | EDY46371.1 | F | 453 |
| s931 | seq_ID 235 | ***Streptomyces sp. AA4*** | 256668250 | F | 428 |
| s918 | seq_ID 236 | ***Streptomyces sp. C*** | 256770952 | F | 454 |
| s929 | seq_ID 237 | ***Streptomyces sp. Mg1*** | 254385931 | F | 453 |
| s928 | seq_ID 238 | ***Streptomyces sp. SPB74*** | 254379682 | F | 453 |

(fortgesetzt)

| S_ID DB | SEQ ID NO | Organism | GI-Nr. der Referenz Sequenzen | Aa | Position |
|---|---|---|---|---|---|
| s930 | seq_ID 239 | ***Streptomyces sp. SPB78*** | 256680470 | F | 404 |
| s26 | seq_ID 240 | ***Streptomyces sviceus*** | EDY55942.1 | F | 453 |
| s927 | seq_ID 241 | ***Streptomyces viridochromogenes*** | 256805984 | F | 447 |
| s61 | seq_ID 242 | ***Synechococcus sp.*** | EDX84551.1 | F | 426 |
| s935 | seq_ID 243 | ***Synechococcus sp. PCC 7335*** | 254422098 | F | 426 |
| s53 | seq_ID 244 | ***Synechocystis sp.*** | BAA17978.1 | F | 428 |
| s22 | seq_ID 245 | ***Syntrophobacter fumaroxidans*** | ABK18414.1 | F | 478 |
| s6 | seq_ID 246 | ***Syntrophobacter fumaroxidans*** | ABK17672.1 | F | 457 |
| s912 | seq_ID 247 | ***Teredinibacter turnerae*** | ACR13362.1 | F | 438 |
| s57 | seq_ID 248 | ***Thermosynechococcus elongatus*** | BAC09861.1 | F | 425 |
| s43 | seq_ID 249 | ***Trichodesmium erythraeum*** | ABG50159.1 | F | 418 |
| s1178 | seq_ID 250 | ***Uncultured organism*** | ACA58560.1 | F | 118 |
| s1176 | seq_ID 251 | ***Uncultured organism*** | ABL07557.1 | F | 118 |
| s1165 | seq_ID 252 | ***Uncultured organism*** | ACA58559.1 | F | 116 |
| s1166 | seq_ID 253 | ***Uncultured organism*** | ACA58558.1 | F | 116 |
| s1168 | seq_ID 254 | ***Uncultured organism*** | ABL07560.1 | F | 116 |
| s1169 | seq_ID 255 | ***Uncultured organism*** | ABL07565.1 | F | 116 |
| s1170 | seq_ID 256 | ***Uncultured organism*** | ABL07566.1 | F | 116 |
| s1167 | seq_ID 257 | ***Uncultured organism*** | ACA58545.1 | F | 116 |
| s1171 | seq_ID 258 | ***Uncultured organism*** | ACA58535.1 | F | 116 |
| s1180 | seq_ID 259 | ***Uncultured organism*** | ACA58549.1 | F | 116 |
| s1179 | seq_ID 260 | ***Uncultured organism*** | ACA58554.1 | F | 116 |
| s1181 | seq_ID 261 | ***Uncultured organism*** | ACA58555.1 | F | 116 |
| s1182 | seq_ID 262 | ***Uncultured organism*** | ACA58556.1 | F | 116 |
| s1235 | seq_ID 263 | ***Uncultured organism*** | ACA58530.1 | F | 116 |

(fortgesetzt)

| S_ID DB | SEQ ID NO | Organism | GI-Nr. der Referenz Sequenzen | Aa | Position |
|---|---|---|---|---|---|
| s1188 | seq_ID 264 | ***Uncultured organism*** | ACA58534.1 | F | 115 |
| s1237 | seq_ID 265 | ***Uncultured organism*** | ACA58552.1 | F | 115 |
| s1223 | seq_ID 266 | ***Uncultured organism*** | ABL07558.1 | F | 115 |
| s1200 | seq_ID 267 | ***Uncultured organism*** | ABL07542.1 | F | 115 |
| s1236 | seq_ID 268 | ***Uncultured organism*** | ACA58539.1 | F | 114 |
| s1238 | seq_ID 269 | ***Uncultured organism*** | ACA58537.1 | F | 114 |
| s1233 | seq_ID 270 | ***Uncultured organism*** | ACA58543.1 | F | 114 |
| s1173 | seq_ID 271 | ***Uncultured organism*** | ABL07553.1 | F | 114 |
| s1241 | seq_ID 272 | ***Uncultured organism*** | ABL07540.1 | F | 114 |
| s1242 | seq_ID 273 | ***Uncultured organism*** | ABL07544.1 | F | 114 |
| s1225 | seq_ID 274 | ***Uncultured organism*** | ACA58557.1 | F | 114 |
| s1183 | seq_ID 275 | ***Uncultured organism*** | ACA58520.1 | F | 113 |
| s1197 | seq_ID 276 | ***Uncultured organism*** | ACA58524.1 | F | 113 |
| s1185 | seq_ID 277 | ***Uncultured organism*** | ACA58522.1 | F | 113 |
| s1190 | seq_ID 278 | ***Uncultured organism*** | ACA58525.1 | F | 113 |
| s1187 | seq_ID 279 | ***Uncultured organism*** | ACA58523.1 | F | 113 |
| s1184 | seq_ID 280 | ***Uncultured organism*** | ACA58521.1 | F | 113 |
| s1204 | seq_ID 281 | ***Uncultured organism*** | ACA58547.1 | F | 113 |
| s1221 | seq_ID 282 | ***Uncultured organism*** | ACA58544.1 | F | 113 |
| s1198 | seq_ID 283 | ***Uncultured organism*** | ACA58546.1 | F | 112 |
| s1226 | seq_ID 284 | ***Uncultured organism*** | ACA58527.1 | F | 112 |
| s1227 | seq_ID 285 | ***Uncultured organism*** | ABL07537.1 | F | 112 |
| s1232 | seq_ID 286 | ***Uncultured organism*** | ACA58510.1 | F | 112 |
| s1230 | seq_ID 287 | ***Uncultured organism*** | ACA58538.1 | F | 112 |
| s1229 | seq_ID 288 | ***Uncultured organism*** | ACA58542.1 | F | 112 |

(fortgesetzt)

| S_ID DB | SEQ ID NO | Organism | GI-Nr. der Referenz Sequenzen | Aa | Position |
|---|---|---|---|---|---|
| s1231 | seq_ID 289 | ***Uncultured organism*** | ACA58540.1 | F | 112 |
| s1207 | seq_ID 290 | ***Uncultured organism*** | ABL07564.1 | F | 112 |
| s1212 | seq_ID 291 | ***Uncultured organism*** | ABL07563.1 | F | 112 |
| s1208 | seq_ID 292 | ***Uncultured organism*** | ABL07562.1 | F | 112 |
| s1209 | seq_ID 293 | ***Uncultured organism*** | ABL07559.1 | F | 112 |
| s1214 | seq_ID 294 | ***Uncultured organism*** | ABL07556.1 | F | 112 |
| s1216 | seq_ID 295 | ***Uncultured organism*** | ACA58528.1 | F | 112 |
| s1219 | seq_ID 296 | ***Uncultured organism*** | ACA58536.1 | F | 112 |
| s1192 | seq_ID 297 | ***Uncultured organism*** | ABL07533.1 | F | 112 |
| s1195 | seq_ID 298 | ***Uncultured organism*** | ABL07536.1 | F | 112 |
| s1174 | seq_ID 299 | ***Uncultured organism*** | ABL07545.1 | F | 112 |
| s1186 | seq_ID 300 | ***Uncultured organism*** | ABL07548.1 | F | 112 |
| s1196 | seq_ID 301 | ***Uncultured organism*** | ACA58561.1 | F | 112 |
| s1172 | seq_ID 302 | ***Uncultured organism*** | ABL07555.1 | F | 112 |
| s1194 | seq_ID 303 | ***Uncultured organism*** | ABL07541.1 | F | 112 |
| s1211 | seq_ID 304 | ***Uncultured organism*** | ABL07554.1 | F | 112 |
| s1220 | seq_ID 305 | ***Uncultured organism*** | ABL07547.1 | F | 112 |
| s1203 | seq_ID 306 | ***Uncultured organism*** | ABL07550.1 | F | 112 |
| s1199 | seq_ID 307 | ***Uncultured organism*** | ABL07551.1 | F | 112 |
| s1228 | seq_ID 308 | ***Uncultured organism*** | ACA58509.1 | F | 111 |
| s1201 | seq_ID 309 | ***Uncultured organism*** | ACA58514.1 | F | 111 |
| s1205 | seq_ID 310 | ***Uncultured organism*** | ABL07543.1 | F | 111 |
| s1206 | seq_ID 311 | ***Uncultured organism*** | ABL07534.1 | F | 111 |
| s1177 | seq_ID 312 | ***Uncultured organism*** | ABL07546.1 | F | 111 |
| s1210 | seq_ID 313 | ***Uncultured organism*** | ABL07535.1 | F | 111 |

(fortgesetzt)

| S_ID DB | SEQ ID NO | Organism | GI-Nr. der Referenz Sequenzen | Aa | Position |
|---|---|---|---|---|---|
| s1175 | seq_ID 314 | ***Uncultured organism*** | ABL07552.1 | F | 111 |
| s1191 | seq_ID 315 | ***Uncultured organism*** | ABL07549.1 | F | 111 |
| s1222 | seq_ID 316 | ***Uncultured organism*** | ACA58553.1 | F | 111 |
| s1244 | seq_ID 317 | ***Uncultured organism*** | ABL07539.1 | F | 111 |
| s1213 | seq_ID 318 | ***Uncultured organism*** | ACA58532.1 | F | 110 |
| s1239 | seq_ID 319 | ***Uncultured organism*** | ACA58548.1 | F | 110 |
| s1215 | seq_ID 320 | ***Uncultured organism*** | ABL07561.1 | F | 110 |
| s1240 | seq_ID 321 | ***Uncultured organism*** | ACA58533.1 | F | 110 |
| s1234 | seq_ID 322 | ***Uncultured organism*** | ABL07538.1 | F | 109 |
| s1224 | seq_ID 323 | ***Uncultured organism*** | ACA58541.1 | F | 109 |
| s1217 | seq_ID 324 | ***Uncultured organism*** | ACA58529.1 | F | 109 |
| s596 | seq_ID 325 | ***Verrucomicrobium spinosum*** | 171910093 | F | 395 |
| s70 | seq_ID 326 | ***Acidiphilium cryptum*** | ABQ30890.1 | F | 430 |

Ausgehend davon sind anhand der Befunde für Mutanten der Zm-SHC-1 weitere potentielle Cyclase-Mutanten mit den gewünschten Substrat-Eigenschaften herstellbar.

**2. Weitere erfindungsgemäße Proteine/Enzymmutanten**

Die vorliegende Erfindung ist nicht auf die hierin konkret offenbarten Mutanten mit Cyclase-Aktivität beschränkt, sondern erstreckt sich vielmehr auch auf funktionale Äquivalente davon.

"Funktionale Äquivalente" oder Analoga der konkret offenbarten Enzyme und Enzymmutanten (F486- und "F486-analoge" Mutanten, abgeleitet von SEQ ID NO:2 bis 326, insbesondere SEQ ID NO: 2 bis 6) sind im Rahmen der vorliegenden Erfindung davon verschiedene Polypeptide, welche weiterhin die gewünschte biologische Aktivität, wie z.B. Cyclase-Aktivität, besitzen.

So versteht man beispielsweise unter "funktionalen Äquivalenten" Enzyme und Mutanten, die in einem verwendeten Test auf "Cyclase-Aktivität" im Sinne der Erfindung (d.h. mit einem Referenzsubstrat unter Standardbedingungen) eine um mindestens 1%, insbesondere um mindestens etwa 5 bis 10 % wie z.B. mindestens 10% oder mindestens 20 %, wie z.B. mindestens 50 % oder 75% oder 90 % höhere oder niedrigere Aktivität eines Enzyms, umfassend eine hierin konkret definierte Aminosäuresequenz (z.B. einer F486- und "F486-analogen" Mutante, abgeleitet von SEQ ID NO:2 bis 326; insbesondere SEQ ID NO: 2 bis 6) aufweisen.

Die Aktivitätsangaben für funktionale Äquivalente beziehen sich hierin, wenn nichts anderes angegeben ist) auf Aktivitätsbestimmungen, durchgeführt mittels eines Referenzsubstrates unter Standardbedingungen, wie hierin definiert.

Die "Cyclase-Aktivität" im Sinne der Erfindung kann mit Hilfe verschiedener bekannter Tests nachgewiesen werden. Ohne darauf beschränkt zu sein, sei ein Test unter Verwendung eines Referenzsubstrates, wie z. B. Citronellal-Racemat oder R(+)-Form, unter Standardbedingungen, wie oben beschrieben und im experimentellen Teil erläutert, zu nennen.

Funktionale Äquivalente sind außerdem z.B. zwischen pH 4 bis 11 stabil und besitzen vorteilhaft ein pH-Optimum in einem Bereich von pH 5 bis 10, wie insbesondere 6,5 bis 9,5 oder 7 bis 8 oder etwa bei 7,5, sowie ein Temperaturoptimum im Bereich von 15°C bis 80°C oder 20°C bis 70°C, wie z.B. etwa 30 bis 60°C oder etwa 35 bis 45°C, wie etwa bei 40°C.

Unter "funktionalen Äquivalenten" versteht man erfindungsgemäß insbesondere auch "Mutanten", welche, neben der (den) konkret genannten Mutation(en) (z.B. einer F486- und "F486-analoge" Mutante, abgeleitet von SEQ ID NO:2 bis 326, insbesondere SEQ ID NO: 2 bis 6), in wenigstens einer Sequenzposition der oben genannten Aminosäuresequenzen eine andere als die konkret genannte Aminosäure aufweisen aber trotzdem eine der oben genannten biologischen Aktivitäten besitzen.

"Funktionale Äquivalente" umfassen die durch eine oder mehrere, wie z.B. 1 bis 50, 2 bis 30, 2 bis 15, 4 bis 12 oder 5 bis 10 "zusätzliche Mutationen", wie Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Derartige "zusätzliche Mutationen" erfolgen dabei an einer von Position F486 gemäß SEQ ID NO:2 oder von der F486-analogen Position gemäß einer der SEQ ID NOs: 3 bis 326 insbesondere SEQ ID NO: 3 bis 6,verschiedenen Position der jeweiligen Aminosäuresequenz.

Nichtlimitierende Beispiele für geeignete Aminosäuresubstitutionen sind in folgender Tabelle zusammengefasst:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

Erfindungsgemäß mit umfasste "funktionale Äquivalente" sind Homologe zu den konkret offenbarten Proteinen. Diese besitzen wenigstens 60 %, vorzugsweise wenigstens 75% ins besondere wenigsten 85 %, wie z.B. 90, 91, 92, 93, 94, 95, 96, 97,98 oder 99%, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäßen homologen Polypeptids bedeutet insbesondere prozentuale Identität der Aminosäurereste bezogen auf die Gesamtlänge einer der hierin konkret beschriebenen Aminosäuresequenzen. Insbesondere weisen diese Homologen aber weiterhin die F486- oder "F486-analoge" Mutation, abgeleitet von SEQ ID NO:2 bis 326, insbesondere SEQ ID NO: 2 bis 6, auf.

Die prozentualen Identitätswerte können auch anhand von BLAST Alignments, Algorithmus blastp (protein-protein BLAST), oder durch Anwendung der unten angegebenen Clustal Einstellungen ermittelt werden.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

### 3. Nukleinsäuren und Konstrukte

### 3.1 Nukleinsäuren

Gegenstand der Erfindung sind auch Nukleinsäuresequenzen, die für ein wie oben beschriebenes Enzym bzw. eine oben beschriebenen Mutante davon mit Cyclase-Aktivität kodieren.

Die vorliegende Erfindung betrifft auch Nukleinsäuren mit einem bestimmten Identitätsgrad zu den hierin beschriebenen konkreten Sequenzen.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

Multiple alignment parameters:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighting | 0 |

Pairwise alignment parameter:

| | |
|---|---|
| FAST algorithm | on |
| K-tuple size | 1 |
| Gap penalty | 3 |
| Window size | 5 |
| Number of best diagonals | 5 |

Alternativ dazu kann die Identität auch nach Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Internetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalente, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder

5'-Ende des kodierenden Genbereichs enthalten.

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinander folgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Erfindungsgemäße Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den erfindungsgemäßen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology,

John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 x SSC (750 mM NaCl, 75 mM Tri-Natrium-citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Gegenstand der Erfindung sind auch Derivate der konkret offenbarten oder ableitbaren Nukleinsäuresequenzen.

So können weitere erfindungsgemäße, für Cyclase-Mutanten kodierende Nukleinsäuresequenzen z.B. von SEQ ID NO:1 oder von den kodierenden Sequenzen zu SEQ ID NO: 2 bis 326, insbesondere SEQ ID NO: 2 bis 6, durch eine F486 oder F486-analoge Mutation abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der erfindungsgemäßen, für Cyclase-Mutanten kodierende Nukleinsäuresequenzen abgeleitet von Sequenz SEQ ID NO: 1 oder von einer der kodierenden Sequenzen zu SEQ ID NO: 2 bis 326, insbesondere SEQ ID NO: 2 bis 6, sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der erfindungsgemäßen Nukleinsäuresequenzen, beispielsweise pilzliche oder bakterielle Homologe, verkürzte Sequenzen, Einzelstrang-DNA oder RNA der kodierenden und nichtkodierenden DNA-Sequenz, zu verstehen.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertion, Inversion und/oder Deletion verändert sein, ohne dass aber die Funktionalität bzw. Wirksamkeit der Promotoren beeinträchtigt sind. Des Weiteren können die Promotoren durch Veränderung ihrer Sequenz in ihrer Wirksamkeit erhöht oder komplett durch wirksamere Promotoren auch artfremder Organismen ausgetauscht werden.

### 3.2 Generierung funktionaler Mutanten

Dem Fachmann sind darüber hinaus Verfahren zur Erzeugung funktionaler Mutanten erfindungsgemäßer Enzyme bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der von diesen codierten Protein sind dem Fachmann seit langem geläufig, wie beispielsweise

- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcärel R, Stunnenberg HG (1994) Nucleic Acids Res 22:541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739);
- die SeSaM-Methode (Sequence Saturation Method), bei der bevorzugte Austausche durch die Polymerase verhindert werden. Schenk et al., Biospektrum, Vol. 3, 2006, 277-279

- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique using an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747).

Unter Anwendung der so genannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise können stufenweise eine begrenzte Anzahl von Mutationen, wie z.B. 1, 2, 3, 4 oder 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die erfindungsgemäßen Ergebnisse liefern auch wichtige Informationen in Bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

Ebenfalls sind Informationen ableitbar bezüglich Aminosäure-Sequenzpositionen, in deren Bereich Mutationen durchgeführt werden können, die voraussichtlich wenig Einfuß auf die Enzymaktivität haben sollten, und als potentielle "silent mutations bezeichnet werden können.

### 3.3 Konstrukte

Gegenstand der Erfindung sind außerdem, insbesondere rekombinante, Expressionskonstrukte, enthaltend unter der genetischen Kontrolle regulativer Nukleinsäuresequenzen eine für ein erfindungsgemäßes Polypeptid kodierende Nukleinsäuresequenz; sowie, insbesondere rekombinante, Vektoren, umfassend wenigstens eines dieser Expressionskonstrukte.

Unter einer "Expressionseinheit" wird erfindungsgemäß eine Nukleinsäure mit Expressionsaktivität verstanden, die einen Promotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird erfindungsgemäß eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird erfindungsgemäß eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäße Nukleinsäurekonstrukte umfassen insbesondere eine für eine Cyclase-Mutante kodierende Sequenz, z.B. abgeleitet von SEQ ID NO: 1 oder kodierend für eine Mutante der SEQ ID NO: 2 bis 326 oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, lpp-, lac-, lpp-lac-, $lacI^{q-}$, T7-, T5-, T3-, gal-, trc-, ara-, rhaP ($rhaP_{BAD}$)SP6-, lambda-$P_R$- oder im lambda-$P_L$-Promotor enthalten, die vorteilhafterweise in gram-negativen Bakterien Anwendung finden. Weitere vorteilhafte Regulationssequenzen sind beispielsweise in den grampositiven Promotoren amy und SPO2, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-$III^{113}$-B1, $\lambda$gt11 oder pBdCI, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, $pGHlac^+$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### 4. Mikroorganismen

Je nach Zusammenhang kann unter dem Begriff "Mikroorganismus" der Wildtyp-Mikroorganismus oder ein genetisch veränderter, rekombinanter Mikroorganismus oder beides verstanden werden.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit Phenylethanol Dehydrogenase-Aktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

### 5. Rekombinante Herstellung von erfindungsgemäßen Enzymen

Gegenstand der Erfindung sind weiterhin Verfahren zur rekombinanten Herstellung erfindungsgemäße Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die erfindungsgemäß hergestellten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz

oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen werden. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, T. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

Für die Expression erfindungsgemäßer Mutanten kann auf die Beschreibung der Expression des Wildtypenzyms EbN1 und der dafür brauchbaren Expressionssysteme in der WO2005/108590 und der WO2006/094945, zurückgegriffen werden, worauf hiermit ausdrücklich Bezug genommen wird.

## 6. Enzymimmobilisierung

Die erfindungsgemäßen Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes"" in K. Drauz und H. Waldmann, Enzyme Catalysis in Organic Synthesis 2002, Vol.III, 991-1032, Wiley-VCH, Weinheim beschrieben. Weitere Informationen zu Biotransformationen und Bioreaktoren zur Durchführung erfindungsgemäßer Verfahren findet man z.B. auch in Rehm et al (Ed) Biotechology, 2nd Edn, Vol 3, Chapter 17, VCH, Weinheim.

## 7. Enzymatische Cyclisierung von Terpenen

### 7.1 Allgemeine Beschreibung

Insbesondere wird das erfindungsgemäße Cyclisierungsverfahren in Gegenwart eines Enzyms durchgeführt, wobei das Enzym von einer Nukleinsäuresequenz gemäß SEQ ID NO:1 oder einem funktionalen Äquivalent davon kodiert wird, wobei die Nukleinsäuresequenz Bestandteil eines Genkonstrukts oder Vektors ist. Solche Genkonstrukte oder Vektoren werden ausführlich in der internationalen Anmeldung PCT/EP2010/057696 auf den Seiten 16 bis 20 beschrieben, worauf hier ausdrücklich Bezug genommen wird. Derartige funktionalen Äquivalente, insbesondere solche

mit Citronellal-Isopulegol-Cyclase-Aktivität, umfassen insbesondere eine F486- oder F486-analoge Mutation, wie hierin definiert.

Die Wirtszelle, die ein Genkonstrukt oder einen Vektor enthält, worin die Nukleinsäuresequenz enthalten ist, die das Enzym mit der gewünschten Aktivität kodiert, bezeichnet man auch als transgenen Organismus. Die Herstellung solche transgenen Organismen ist prinzipiell bekannt und wird beispielsweise in internationalen Anmeldung PCT/EP2010/057696auf Seite 20 diskutiert, worauf hier ausdrücklich Bezug genommen wird.

Als transgene Organismen werden bevorzugt Zellen aus der Gruppe bestehend aus Bakterien, Cyanobakterien, Pilzen und Hefen ausgewählt. Bevorzugt ist die Zelle ausgewählt aus Pilzen der Gattung Pichia oder Bakterien der Gattungen Escherichia, Corynebacterium, Ralstonia, Clostridium, Pseudomonas, Bacillus, Zymomonas, Rhodobacter, Streptomyces, Burkholderia, Lactobacillus oder Lactococcus. Besonders bevorzugt ist die Zelle ausgewählt aus Bakterien der Spezies Escherichia coli, Pseudomonas putida, Burkholderia glumae, Streptomyces lividans, Streptomyces coelicolor oder Zymomonas mobilis.

Bevorzugt ist ein erfindungsgemäßes Verfahren, dadurch gekennzeichnet, dass das Enzym mit der Aktivität einer Citronellal-Isopulegol-Cyclase von einem Gen kodiert wird, das aus einem Mikroorganismus isoliert wurde, ausgewählt unter Zymomonas mobilis, Methylococcus capsulatus, Rhodopseudomonas palustris, Bradyrhizobium japonicum, Frankia spec, Streptomyces coelicolor sowie Acetobacter pasteurianus. Besonders zu nennen sind die betreffenden Gene aus Zymomonas mobilis, Streptomyces coelicolor, Bradyrhizobium japonicum und Acetobacter pasteurianus isoliert.

Weiterhin bevorzugt ist ein erfindungsgemäßes Verfahren, dadurch gekennzeichnet, dass das Enzym mit der Cyclase-Aktivität von einem Mikroorganismus generiert wurde, der das Enzym überproduziert und der ausgewählt wurde aus der Gruppe der Mikroorganismen bestehend aus den Gattungen Escherichia, Corynebacterium, Ralstonia, Clostridium, Pseudomonas, Bacillus, Zymomonas, Rhodobacter, Streptomyces, Burkholderia, Lactobacillus und Lactococcus.

Besonders zu erwähnen ist ein erfindungsgemäßes Verfahren, dadurch gekennzeichnet dass das Enzym mit der Cyclase-Aktivität von transgenen Mikroorganismen der Spezies Escherichia coli, Pseudomonas putida, Burkholderia glumae, Corynebacterium glutamicum, Saccharomyces cerevisiae, Pichia pastoris, Streptomyces lividans, Streptomyces coelicolor, Bacillus subtilis oder Zymomonas mobilis erzeugt wurde, welche das Enzym mit der Cyclase-Aktivität überproduzieren.

Weitere Ausgestaltungen zur Durchführung des erfindungsgemäßen biokatalytischen Cyclisierungsverfahrens, wie z.B. des Verfahrens zur Herstellung von Isopulegol:
Das erfindungsgemäße Verfahren ist dadurch gekennzeichnet, dass das Enzym in wenigstens einer der folgenden Formen vorliegt:

a) freies, gegebenenfalls gereinigtes oder teilweise gereinigtes Polypeptid;
b) immobilisiertes Polypeptid;
c) aus Zellen isoliertes Polypeptid gemäß a) oder b);
d) ganze Zelle, gegebenenfalls ruhende oder wachsende Zellen, enthaltend mindestens ein solches Polypeptid;
e) Lysat oder Homogenisat der Zellen gemäß d).

Eine weitere Ausführungsform des erfindungsgemäßen Verfahrens ist dadurch gekennzeichnet, dass die Zellen Mikroorganismen sind, bevorzugt transgene Mikroorganismen exprimierend mindestens ein heterologes Nukleinsäuremolekül kodierend für ein Polypeptid mit der Cyclase-Aktivität.

Eine bevorzugte Ausführungsform des erfindungsgemäßen Verfahrens umfasst wenigsten die folgenden Schritte a), b) und d):

a) einen ein Enzym mit Cyclase-Aktivität produzierenden Mikroorganismus aus einer natürlichen Quelle zu isolieren oder rekombinant herzustellen,
b) diesen Mikroorganismus zu vermehren,
c) aus dem Mikroorganismus das Enzym mit Cyclase-Aktivität gegebenenfalls zu isolieren oder eine dieses Enzym enthaltende Proteinfraktion herzustellen, und
d) den Mikroorganismus gemäß Stufe b) oder das Enzym gemäß Stufe c) in ein Medium zu überführen, das Substrat, z.B. Citronellal der allgemeinen Formel (I), enthält.

In dem erfindungsgemäßen Verfahren wird Substrat, wie z.B. Citronellal, mit dem Enzym, das die Aktivität einer Citronellal-Isopulegol-Cyclase besitzt, in einem Medium in Kontakt gebracht und/oder so inkubiert, dass eine Umsetzung des Substrats, wie z.B. von Citronellal, zu Isopulegol, in Gegenwart des Enzyms erfolgt. Bevorzugt handelt es sich bei dem Medium um ein wässriges Reaktionsmedium.

Der pH-Wert des wässrigen Reaktionsmediums, in dem das erfindungsgemäße Verfahren bevorzugt durchgeführt wird, wird dabei vorteilhaft zwischen pH 4 und 12, bevorzugt zwischen pH 4,5 und 9, besonders bevorzugt

zwischen pH 5 und 8 gehalten.

Bei den wässrigen Reaktionsmedien handelt es sich vorzugsweise um gepufferte Lösungen, die in der Regel einen pH-Wert von vorzugsweise von 5 bis 8, aufweisen. Als Puffer kann ein Citrat-, Phosphat-, TRIS- (Tris(hydroxymethyl)-aminomethan) oder MES-Puffer (2-(N-Morpholino)ethansulfonsäure) verwendet werden. Ferner kann das Reaktionsmedium noch weitere Zusätze enthalten, wie z.B. Detergentien (beispielsweise Taurodeoxycholat).

Das Substrat, wie z.B. Citronellal, wird vorzugsweise in einer Konzentration von 2 - 200mM, besonders bevorzugt von 5 - 25mM in die enzymatische Umsetzung eingesetzt und kann kontinuierlich oder diskontinuierlich nachgeführt werden.

Die enzymatische Cyclisierung erfolgt in der Regel bei einer Reaktionstemperatur unterhalb der Desaktivierungstemperatur des eingesetzten Enzyms und oberhalb von -10°C. Bevorzugt wird das erfindungsgemäße Verfahren bei einer Temperatur zwischen 0°C und 95°C, besonders bevorzugt bei einer Temperatur zwischen 15°C und 60°C, insbesondere zwischen 20 und 40°C, z.B. bei etwa 25 bis 30°C durchgeführt.

Besonders bevorzugt ist ein erfindungsgemäßes Verfahren, wobei die Reaktion von Citronellal zu Isopulegol bei einer Temperatur im Bereich von 20 bis 40 °C und/oder einem pH-Wert im Bereich von 4 bis 8 erfolgt.

Neben diesen einphasigen wässrigen Systemen werden in einer weiteren Variante der Erfindung auch zweiphasige Systeme eingesetzt. Dabei werden neben einer wässrigen Phase als zweite Phase, organische, nicht-wassermischbare Reaktionsmedien verwendet. Dadurch akkumulieren die Reaktionsprodukte in der organischen Phase. Nach der Reaktion ist das Produkt, wie zB. Isopulegol, in der organische Phase leicht von der wässrigen Phase, die den Biokatalysator enthält, abtrennbar.

Bevorzug ist ein erfindungsgemäßes Verfahren, dadurch gekennzeichnet, dass die Herstellung von Isopulegol in einphasigen wässrigen Systemen oder in zweiphasigen Systemen erfolgt.

Das Reaktionsprodukt, wie z.B. Isopulegol, kann mit organischen Lösungsmitteln extrahiert werden und gegebenenfalls zur Aufreinigung destilliert werden.

Geeignete organische Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Ethanol, Isopropanol, Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Heptan, Methyltertbutylether, Diisopropylether, Tetrahydrofuran, Ethylacetat verwendet.

Die erfindungsgemäß eingesetzten Cyclasen können im erfindungsgemäßen Verfahren als freies oder immobilisiertes Enzym, wie oben bereits beschrieben, verwendet werden.

Für das erfindungsgemäße Verfahren können ruhende oder wachsende, freie oder immobilisierte Zellen verwendet werden, die für die Cyclase kodierenden Nukleinsäuren, Nukleinsäurekonstrukte oder Vektoren enthalten. Auch aufgeschlossene Zellen, wie Zelllysate oder Zellhomogenate, können verwendet werden. Unter aufgeschlossenen Zellen sind beispielsweise Zellen zu verstehen, die über eine Behandlung beispielsweise mit Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder teilweise gereinigte Enzyme können für das Verfahren verwendet werden.

Werden für das erfindungsgemäße Verfahren freie Organismen oder Enzyme verwendet, so werden diese vor der Extraktion zweckmäßigerweise abgetrennt, beispielsweise über eine Filtration oder Zentrifugation.

Das erfindungsgemäße Verfahren kann batchweise, semi-batchweise oder kontinuierlich betrieben werden.

**7.2. Enzymatische Cyclisierung von Citronellal**

Das erfindungsgemäß eingesetzte Citronellal der Formel (II), das mittels eines Enzyms mit Citronellal-Isopulegol-Cyclose-Aktivität umgesetzt wird, ist kommerziell sowohl als (+)-R-Citronellal der Formel (R-II) als auch als (-)-S-Citronellal der Formel (S-II) als auch als Racemat der Formel (II) erhältlich.

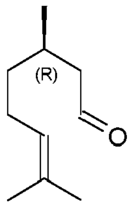

(R-II)

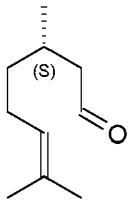

(S-II)

Das erfindungsgemäß gebildete Isopulegol der Formel (I)

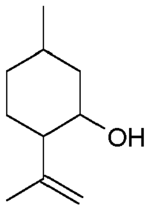

(I)

hat in den Positionen 1, 3 und 6 jeweils ein Stereozentrum, sodass prinzipiell 4 verschiedene Diastereomere mit jeweils 2 Enantiomeren, also insgesamt 8 Stereomere, denkbar sind, wenn von dem Racemat des Citronellals der Formel (I) ausgegangen wird.

Isopulegol

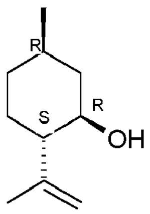

*1R,3R,6S*

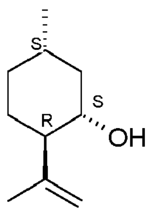

*1S,3S,6R*

Neo-Isopulegol

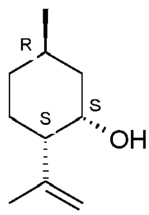

*1S,3R,6S*

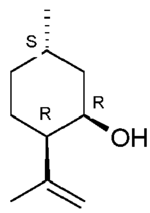

*1R,3S,6R*

Iso-Isopulegol

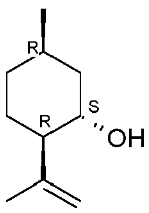

*1S,3R,6R*

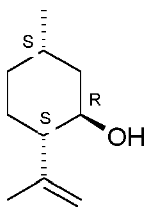

*1R,3S,6S*

Epi-Isopulegol

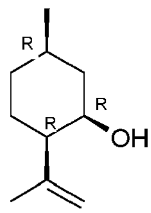

1R,3R,6R

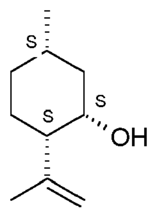

*1S,3S,6R*

Als Enzyme mit der Aktivität einer Citronellal-Isopulegol-Cyclase sind intramolekulare Transferasen aus der Subklasse der Isomerasen; also Proteine mit dem Enzymcode EC 5.4 geeignet. (Enzymcode gemäß Eur. J. Biochem. 1999, 264, 610-650) Bevorzugt handelt es sich um Vertreter mit dem Enzymcode 5.4.99.17. Als Enzyme mit der Aktivität einer Citronellal-Isopulegol-Cyclase sind insbesondere auch solche Cyclasen geeignet, die auch die Cyclisierung von Homofarnesol zu Ambroxan oder von Squalen zu Hopen bewirken und die ausführlich in der internationalen Anmeldung PCT/EP2010/057696 beschrieben werden, worauf hier ausdrücklich Bezug genommen wird; weiterhin sind geeignet die hier beschriebenen Enzyme und Mutanten.

Eine besonders geeignete Ausführungsform des erfindungsgemäßen Verfahren besteht darin, dass das in dem erfindungsgemäßen Verfahren eingesetzte Enzym mit der Aktivität einer Citronellal-Isopulegol-Cyclase eine Polypeptidsequenz besitzt, die entweder

a) SEQ ID NO:2 ist, oder

b) bei der bis zu 25% der Aminosäurereste gegenüber SEQ ID NO:2 durch Deletion, Insertion, Substitution oder einer Kombination davon verändert sind, und die noch mindestens 50% der enzymatischen Aktivität von SEQ ID NO:2 aufweist.

Geeignete Enzyme mit Citronellal-Isopulegol-Cyclase-Aktivität, die eine Aminosequenz gemäß SEQ ID NO: 2 umfassen, sowie "funktionale Äquivalente" oder Analoga der konkret offenbarten Enzyme (E) mit Citronellal-Isopulegol-Cyclase-Aktivität, werden wie oben bereits angesprochen ausführlich in der internationalen Anmeldung PCT/EP2010/057696 beschrieben, worauf hier ausdrücklich Bezug genommen wird.

In einer besonders bevorzugten Ausführungsform des Verfahrens ist das Enzym mit Citronellal-Isopulegol-Cyclase-Aktivität ausgewählt unter Enzymen, welche eine Aminosäuresequenz gemäß SEQ ID NO: 2 umfassen oder eine davon abgeleitete Sequenz, bei der bis zu 25%, bevorzugt bis zu 20%, besonders bevorzugt bis zu 15% insbesondere bis zu 10, 9, 8, 7, 6, 5, 4, 3, 2, 1 % der Aminosäurereste durch eine Deletion, eine Substitution eine Insertion oder eine Kombination aus Deletion, Substitution und Insertion verändert worden sind, wobei die gegenüber SEQ ID NO: 2 veränderten Polypeptidsequenzen noch mindestens 50%, bevorzugt 65%, besonders bevorzugt 80% , insbesondere mehr als 90 % der enzymatischen Aktivität von SEQ ID NO:2 besitzten. In diesem Zusammenhang soll unter enzymatischer Aktivität von SEQ ID NO:2 die Fähigkeit verstanden werden, Citronellal der allgemeinen Formel (II) biokatalytisch zu dem entsprechenden Isopulegol der Formel (I) zu cyclisieren.

Das erfindungsgemäße Verfahren wird bevorzugt in Gegenwart eines Enzyms durchgeführt, wobei das Enzym von einer Nukleinsäuresequenz gemäß SEQ ID NO:1 oder einem funktionalen Äquivalent davon kodiert wird.

Funktionelle Äquivalente beschreiben prinzipiell hier Nukleinsäuresequenzen, die unter Standardbedingungen mit einer Nukleinsäuresequenz oder Teilen einer Nukleinsäuresequenz hybridisieren und befähigt sind, die Expression eines Proteins mit den gleichen Eigenschaften wie die des Enzyms mit Citronellal-Isopulegol-Cyclase-Aktivität in einer Zelle oder einem Organismus zu bewirken.

Unter einem funktionellen Äquivalent versteht man weiterhin auch Nukleinsäuresequenzen, die mit einer bestimmten Nukleinsäuresequenz ("ursprüngliche Nukleinsäuresequenz) bis zu einem definierten Prozentsatz homolog bzw. identisch sind und die gleiche Aktivität wie die ursprünglichen Nukleinsäuresequenzen aufweisen, ferner insbesondere auch natürliche oder künstliche Mutationen dieser Nukleinsäuresequenzen.

Die Nukleinsäuresequenzen, die für die Kodierung der in dem erfindungsgemäßen Verfahren verwendbaren Enzyme mit Citronellal-Isopulegol-Cyclase-Aktivität eingesetzt werden können, werden ebenfalls ausführlich in der internationalen Anmeldung PCT/EP2010/057696 beschrieben, worauf hier ausdrücklich Bezug genommen wird.

Besonders bevorzugt wird das erfindungsgemäße Verfahren in Gegenwart eines Enzyms durchgeführt, wobei das Enzym von einer Nukleinsäuresequenz gemäß SEQ ID NO:1 oder einem funktionalen Äquivalent davon kodiert wird, wobei die Nukleinsäuresequenz Bestandteil eines Genkonstrukts oder Vektors ist. Solche Genkonstrukte oder Vektoren werden ausführlich in der internationalen Anmeldung PCT/EP2010/057696 auf den Seiten 16 bis 20 beschrieben, worauf hier ausdrücklich Bezug genommen wird.

Ganz besonders bevorzugt wird das erfindungsgemäße Verfahren in Gegenwart eines Enzyms durchgeführt, wobei das Enzym von einer Nukleinsäuresequenz gemäß SEQ ID NO:1 oder einem funktionalen Äquivalent davon kodiert wird, wobei die Nukleinsäuresequenz Bestandteil eines Genkonstrukts oder Vektors ist, welche in einer Wirtszelle enthalten sind.

Die Wirtszelle, die ein Genkonstrukt oder einen Vektor enthält, worin die Nukleinsäuresequenz enthalten ist, die das Enzym mit der Citronellal-Isopulegol-Cyclase-Aktivität kodiert, bezeichnet man auch als transgenen Organismus. Die Herstellung solche transgenen Organismen ist prinzipiell bekannt und wird beispielsweise in internationalen Anmeldung PCT/EP2010/057696 auf Seite 20 diskutiert, worauf hier ausdrücklich Bezug genommen wird.

Als transgene Organismen werden bevorzugt Zellen aus der Gruppe bestehend aus Bakterien, Cyanobakterien, Pilzen und Hefen ausgewählt. Bevorzugt ist die Zelle ausgewählt aus Pilzen der Gattung Pichia oder Bakterien der Gattungen Escherichia, Corynebacterium, Ralstonia, Clostridium, Pseudomonas, Bacillus, Zymomonas, Rhodobacter, Streptomyces, Burkholderia, Lactobacillus oder Lactococcus. Besonders bevorzugt ist die Zelle ausgewählt aus Bakterien der Spezies Escherichia coli, Pseudomonas putida, Burkholderia glumae, Streptomyces lividans, Streptomyces coelicolor oder Zymomonas mobilis.

Bevorzugt ist ein erfindungsgemäßes Verfahren, dadurch gekennzeichnet, dass das Enzym mit der Aktivität einer Citronellal-Isopulegol-Cyclase von einem Gen kodiert wird, das aus einem Mikroorganismus isoliert wurde, ausgewählt aus der Gruppe der Mikroorganismen bestehend aus Zymomonas mobilis, Methylococcus capsulatus, Rhodopseudomonas palustris, Bradyrhizobium japonicum, Frankia spec. und Streptomyces coelicolor. Besonders bevorzugt wurde das betreffende Gen aus Zymomonas mobilis isoliert.

Weiterhin bevorzugt ist ein erfindungsgemäßes Verfahren, dadurch gekennzeichnet, dass das Enzym mit der Aktivität einer Citronellal-Isopulegol-Cyclase von einem Mikroorganismus erzeugt wurde, der das Enzym mit der Aktivität einer Citronellal-Isopulegol-Cyclase überproduziert und der ausgewählt wurde aus der Gruppe der Mikroorganismen bestehend aus den Gattungen Escherichia, Corynebacterium, Ralstonia, Clostridium, Pseudomonas, Bacillus, Zymomonas, Rhodobacter, Streptomyces, Burkholderia, Lactobacillus und Lactococcus.

Besonders bevorzugt ist ein erfindungsgemäßes Verfahren, dadurch gekennzeichnet dass das Enzym mit der Aktivität einer Citronellal-Isopulegol-Cyclase von transgenen Mikroorganismen der Spezies Escherichia coli, Pseudomonas putida, Burkholderia glumae, Corynebacterium glutamicum, Saccharomyces cerevisiae, Pichia pastoris, Streptomyces lividans, Streptomyces coelicolor, Bacillus subtilis oder Zymomonas mobilis erzeugt wurde, welche das Enzym mit der Aktivität einer Citronellal-Isopulegol-Cyclase überproduzieren.

Die oben beschriebenen weiteren Ausgestaltungen zur Durchführung des erfindungsgemäßen biokatalytischen Verfahrens zur Cyclisierung von Terpenen gelten entsprechend für die Herstellung von Isopulegol.

Ein weiterer Gegenstand der vorliegenden Erfindung ist auch die Verwendung eines Enzyms mit der Aktivität einer Citronellal-Isopulegol-Cyclase zur biokatalytischen Umsetzung von Citronellal zu Isopulegol.

Bevorzugt ist die Verwendung eines Enzyms mit der Aktivität einer Citronellal-Isopulegol-Cyclase zur biokatalytischen Umsetzung von Citronellal zu Isopulegol, dadurch gekennzeichnet, dass das Enzym eine Polypeptidsequenz besitzt, die entweder

a) SEQ ID NO:2 ist, oder

b) bei der bis zu 25% der Aminosäurereste gegenüber SEQ ID NO:2 durch Deletion, Insertion, Substitution oder einer Kombination davon verändert sind, und die noch mindestens 50% der enzymatischen Aktivität von SEQ ID NO:2 aufweist.

Bevorzugt ist auch die Verwendung eines Enzyms mit der Aktivität einer Citronellal-Isopulegol-Cyclase zur biokatalytischen Umsetzung von Citronellal zu Isopulegol, dadurch gekennzeichnet, dass das Enzym von einer Nukleinsäuresequenz gemäß SEQ ID NO:1 oder einem funktionalen Äquivalent davon kodiert wird.

Ein weiterer Gegenstand vorliegenden Erfindung ist auch die Verwendung eines Genkonstrukts oder Vektors umfassend eine Nukleinsäuresequenz gemäß SEQ ID NO:1 oder ein funktionales Äquivalent davon, die ein Polypeptid mit der Aktivität einer Citronellal-Isopulegol-Cyclase kodieren, das zur biokatalytischen Umsetzung von Citronellal zu Isopulegol dient, in einem Verfahren zur Herstellung von Isopulegol durch Cyclisierung von Citronellal.

Ebenfalls ein weiterer Gegenstand vorliegenden Erfindung ist auch die Verwendung einer Wirtszelle, die ein Genkonstrukt oder einen Vektor enthält, welche eine Nukleinsäuresequenz gemäß SEQ ID NO:1 oder ein funktionales Äquivalent davon umfassen, für die Herstellung eines Enzyms mit der Aktivität einer Citronellal-Isopulegol-Cyclase für die biokatalytische Umsetzung von Citronellal zu Isopulegol.

Das vorangehend beschriebene Verfahren eröffnet erstmal die Möglichkeit, Citronellal mit Hilfe eines Enzyms zu Isopulegol zu cyclisieren.

**8. Verfahren zur Herstellung von Menthol**

Das in erfindungsgemäßer Weise hergestellte Isopulegol kann in an sich bekannte Weise durch katalytische Hydrierung in Menthol überführt werden. Dazu eignet sich neben herkömmlichen Hydrierungsverfahren, insbesondere ein katalytisches Verfahren, wie in der WO 2009/013192 beschrieben.

Insbesondere setzt man zur Durchführung des erfindungsgemäßen Verfahrens solche Katalysatoren ein, umfassend

- 45 bis 55 Gew.-% sauerstoffhaltige Verbindungen des Nickels, berechnet als NiO,
- 25 bis 35 Gew.-% sauerstoffhaltige Verbindungen des Zirkons, berechnet als $ZrO_2$,
- 5 bis 20 Gew.-% sauerstoffhaltige Verbindungen des Kupfers, berechnet als CuO, -
- 1 bis 3 Gew.-% sauerstoffhaltige Verbindungen des Molybdäns, berechnet als $MoO_3$ und
- 0 bis 5 Gew.-% weiterer Komponenten,

wobei sich die Angaben in Gew.-% zu 100 Gew.-% ergänzen und sich auf den trockenen, nicht reduzierten Katalysator beziehen.

Ein besonders bevorzugter Katalysator besteht zu 49 bis 53 Gew.-% aus NiO, zu 15 bis 19 Gew.-% aus CuO, zu 28 bis 32 Gew.-% aus $ZrO_2$ und zu 1 bis 2 Gew.-% aus $MoO_3$ sowie gegebenenfalls zu 0 bis 3 Gew.-% aus weiteren Komponenten wie beispielsweise Graphit, wobei sich die jeweils gewählten Gewichtsanteile der einzelnen Komponenten auf den trockenen, nicht reduzierten Katalysator beziehen und zu 100 Gew.-% ergänzen. Derartige Katalysatoren sind bekannt und können beispielsweise wie in der EP 0 696 572 oder in der WO 2009/013192 beschrieben, hergestellt werden.

Im allgemeinen werden die Katalysatoren bevorzugt in Form von Vollkatalysatoren eingesetzt. Mit dem Begriff "Vollkatalysator" wird ein Katalysator bezeichnet, der im Gegensatz zu einem Trägerkatalysator nur aus katalytisch aktiver Masse besteht. Vollkatalysatoren können dergestalt eingesetzt werden, dass man die katalytisch aktive, zu Pulver

vermahlene Masse in das Reaktionsgefäß einbringt oder, dass man die katalytisch aktive Masse nach Mahlung, Vermischung mit Formhilfsmitteln, Formung und Temperung als Katalysatorformkörper - beispielsweise als Kugeln, Zylinder, Tabletten, Ringe, Spiralen, Stränge und dergleichen mehr - im Reaktor anordnet.

Im Rahmen einer bevorzugten Ausführungsform des erfindungsgemäßen Hydrierverfahrens setzt man den gewählten heterogenen Katalysator in Form eines Festbettkatalysators ein.

Zur Durchführung des erfindungsgemäßen Verfahrens bringt man den wie vorstehend beschriebenen Ausgangsstoff Isopulegol mit Wasserstoff und dem gewählten Katalysator in Kontakt. Der Wasserstoff kann dabei in unverdünnter Form, üblicherweise in einer Reinheit von etwa 99,9 Vol.-% oder in verdünnter Form, d.h. in Form von Gemischen mit inerten Gasen wie beispielsweise Stickstoff oder Argon, eingesetzt werden. Bevorzugt setzt man Wasserstoff in unverdünnter Form ein. Die Umsetzung kann mit gutem Erfolg ohne Zusatz von Lösungsmittel oder in Gegenwart von unter den Reaktionsbedingungen inerten organischen Lösungsmitteln, wie beispielsweise Methanol, Ethanol, Isopropanol, Hexan, Heptan, Cyclohexan und dergleichen mehr durchgeführt werden. Bevorzugt führt man die Reaktion ohne Zusatz von Lösungsmittel durch.

Die erfindungsgemäße Hydrierung von Isopulegol kann bei einem Wasserstoffdruck (absolut) im Bereich von 1 bis 200 bar, wie von 2 oder 3 bis 200 bar, vor allem von 4 oder 5 bis 150 bar, wie von 5 bis 100 bar oder im Bereich von 5 bis 50 bar durchgeführt werden. Als Reaktionstemperatur zur Durchführung der erfindungsgemäßen Hydrierung wählt man vorteilhaft eine Temperatur im Bereich von 20 bis 150°C, wie von 40 bis 130°C, oder von 60 bis 110°C und insbesondere von 70 bis 100°C.

Praktisch geht man bei der Durchführung im allgemeinen so vor, dass man dem Katalysator, der sich üblicherweise in einem insbesondere von außen beheizten Festbettreaktor wie beispielsweise einem Rohrreaktor, Autoklaven oder Rohrbündelreaktor befindet, bei der gewünschten Reaktionstemperatur und dem gewünschten Druck das umzusetzende Isopulegol zuführt. Dabei belastet man den Katalysator im Allgemeinen mit 0,1 bis 1 ,0, wie mit 0,1 bis 0,6 oder mit 0,2 bis 0,4 kg Isopulegol pro kg Katalysator und pro Stunde. Hierbei kann es zweckmäßig sein, das einzusetzende Isopulegol bereits vor der Zuführung in das Reaktionsgefäß bzw. den Reaktor zu erwärmen und zwar bevorzugt auf die Reaktionstemperatur.

Der Reaktor kann sowohl in der Sumpf- als auch in der Rieselfahrweise betrieben werden, d.h. man kann die Ausgangsstoffe sowohl von unten nach oben als auch von oben nach unten durch den Reaktor leiten. Das erfindungsgemäße Hydrierverfahren kann sowohl diskontinuierlich als auch kontinuierlich durchgeführt werden. In beiden Fällen kann nicht umgesetztes Edukt zusammen mit dem Wasserstoff im Kreis geführt werden.

Die erfindungsgemäße Hydrierung kann auch stufenweise in einer Kaskade von mehreren, d.h. 2 bis in der Regel 4, wie z.B. 2 oder 3 hintereinander geschalteten Reaktoren, bevorzugt Festbettreaktoren durchgeführt werden. Dabei wird in dem ersten, üblicherweise als Hauptreaktor bezeichneten Reaktor unter den vorstehend beschriebenen Reaktionsbedingungen der Hauptumsatz der Reaktion erzielt und das erhaltene Rohprodukt einem zweiten, üblicherweise als Nachreaktor bezeichneten Reaktor zugeführt, in dem das noch nicht umgesetzte Ausgangsmaterial in erfindungsgemäßer Weise zumindest weitgehend in L-Menthol überführt wird. Dabei können die Reaktionsbedingungen unabhängig voneinander vorzugsweise in den vorstehend genannten Bereichen gewählt werden.

Das erfindungsgemäße Verfahren kann diskontinuierlich, halb- oder vollkontinuierlich durchgeführt werden. Bevorzugt führt man das Verfahren kontinuierlich, insbesondere vollkontinuierlich durch, wobei die Ausgangsstoffe kontinuierlich in den Reaktor eingetragen und das erhaltenen Reaktionsgemisch bzw. Reaktionsprodukt kontinuierlich aus dem Reaktor ausgetragen werden. Es hat sich weiterhin als vorteilhaft erwiesen, aufgrund der Lage des Schmelzpunktes des erfindungsgemäßen Reaktionsproduktes Menthol, speziell L-Menthol für eine Beheizung der eingesetzten Transportleitungen zu sorgen.

Das erfindungsgemäße Verfahren erlaubt die Herstellung von Menthol durch katalytische Hydrierung von Isopulegol, wobei es üblicherweise nur in geringem Ausmaß zur Bildung von unerwünschten Diastereomeren des Menthols kommt. Das erfindungsgemäße Verfahren liefert dementsprechend, bei Einsatz von Isopulegol mit einer entsprechenden Reinheit, Menthol der Formel (III) in einer chemischen Reinheit von 97 Gew.-% oder darüber, bevorzugt von 98 bis 100 Gew.-%, besonders bevorzugt von 98,5 bis 99,9 Gew.-%, ganz besonders bevorzugt von mindestens 99 bis 99,9 Gew.-%. Dabei umfasst der Begriff chemische Reinheit auch die Diastereomerenreinheit des erhaltenen Menthols bezüglich der Diastereomere Neoiso-Menthol der Formel (IIIa), NeoMenthol der Formel (IIIb) und Iso-Menthol der Formel (IIIc). Dementsprechend liefert das erfindungsgemäße Verfahren im Rahmen bevorzugt Menthol mit einer Diastereomerenreinheit von 97 Gew.-% oder darüber, bevorzugt von 98 bis 100 Gew.-%, besonders bevorzugt von 98,5 bis 99,9 Gew.-% und ganz besonders bevorzugt von mindestens 99 bis 99,9 Gew.-%.

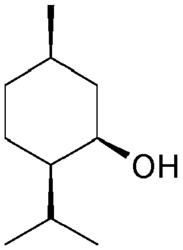

(IIIa)

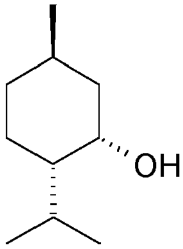

(IIIb)

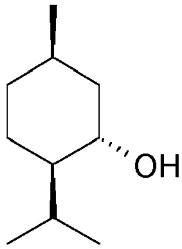

(IIIc)

Setzt man Isopulegol in optisch aktiver Form, erfindungsgemäß bevorzugt solche Gemische, die zum überwiegenden Teil das Enantiomer L-Isopulegol enthalten ein, erhält man als erfindungsgemäßes Verfahrensprodukt in der Regel Menthol in optisch aktiver Form, bevorzugt in Form des (-)- bzw-L-Menthols. Die erfindungsgemäße Hydrierung verläuft im Regelfall weitgehend ohne nennenswerte Racemisierung des eingesetzten Materials. Demnach erhält man, in Abhängigkeit vom Enantiomerenüberschuss des eingesetzten optisch aktiven Isopulegols optisch aktives, bevorzugt bei Einsatz von L-Ispulegol als Produkt, L-Menthol mit einem Enatiomerenüberschuss (ee) von 80% ee oder dar-über, bevorzugt von 85 oder 90% ee oder darüber, besonders bevorzugt von 95 bis 100% ee, besonders bevorzugt von 96 bis 99,9% ee, ganz besonders bevorzugt von 97 bis 99,8% ee, noch mehr bevorzugt von 98 bis 99,7% ee und insbesondere bevorzugt von 98,5 bis 99,6% ee ein.

Das erfindungsgemäß erhaltene Menthol zeichnet sich darüber hinaus durch einen besonders geringen Gehalt der unerwünschten Nebenprodukte Menthon der Formel (IIId) und Isomenthon der Formel (IIIe) und Neoiso-Menthol der Formel (IIIa) aus.

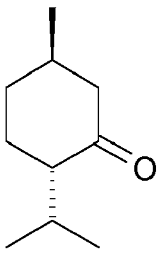

(IIId)

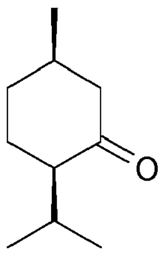

(IIIe)

Diese Nebenprodukte werden im Regelfall im Rahmen des erfindungsgemäßen Verfahrens nur in einem auf die Menge an erhaltenem Menthol bezogenen Anteil von bis zu 0,5 Gew.-%, bevorzugt 0,4 Gew.-%, besonders bevorzugt 0,3 Gew.-%, insbesondere 0,2 Gew.-% und ganz besonders bevorzugt 0,1 bis 0 Gew.-% erhalten.

**9. Beispiele braucharer Substrate für erfindungsgemäße enzymatische oder biokatalytische Umsetzungen:**

Dier hierin beschriebenen Enzyme und Mikroorganismen eigne sich insbesondere Zur umsetzung von Verbindungen der obigen allgemeinen Formel IV. Nichtlimitierende Beispiele davon sind in folgender Tabelle A unter Angabe der Strukturformel und des chemischen Namens zusammengefasst.

Tabelle A: Weitere Substrate

| Formel | Name |
|---|---|
| 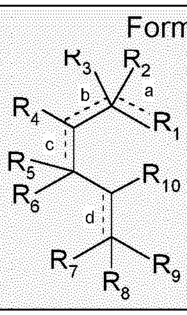 (IV) | |
| 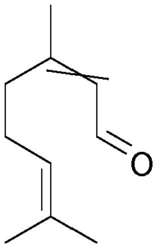 | Citral |
| 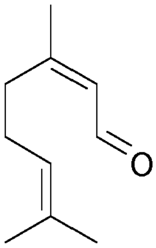 | Neral |
| 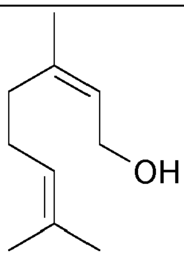 | Nerol |
| 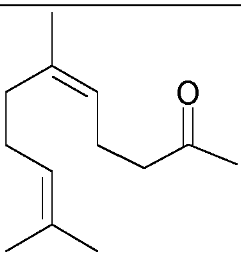 | Nerylaceton |
| 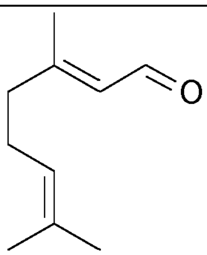 | Geranial |

| Formel | Name |
|---|---|
| 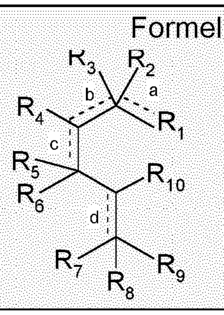 (IV) | |
| 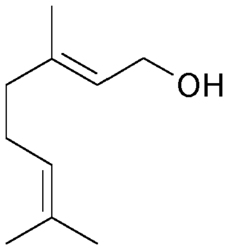 | Geraniol |
| 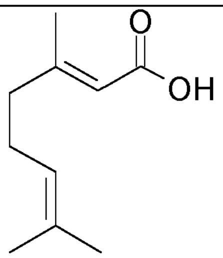 | Geranylsäure |
| 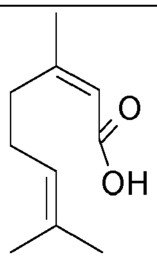 | cis-Geranylsäure |
| 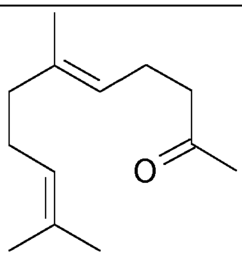 | Geranylaceton |
| 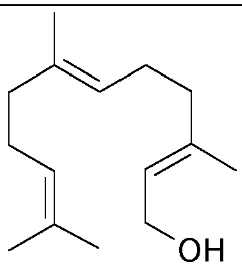 | Farnesol |

| Formel | Name |
| --- | --- |
| 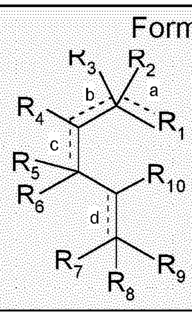 (IV) | |
| 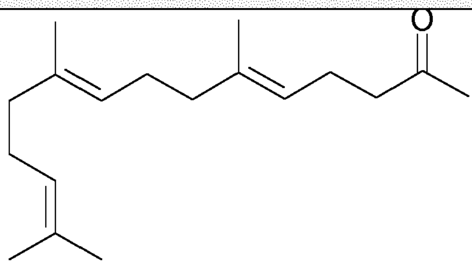 | Farnesylaceton |
| 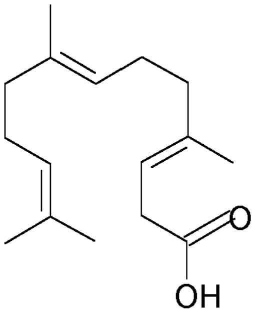 | Homofarnesylsäure |
| 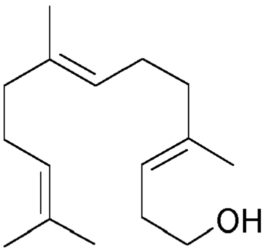 | Homofarnesol |
| 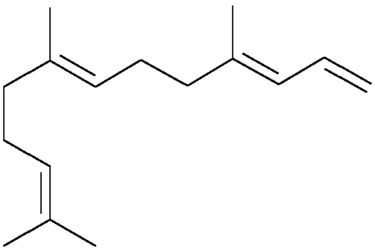 | Trimethyltridecatetraen |
| 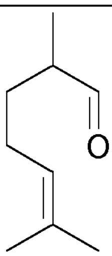 | Melonal |

| Formel | Name |
|---|---|
| 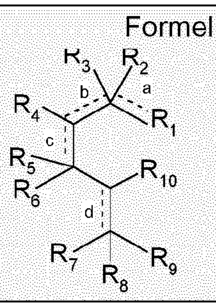 (IV) | |
| 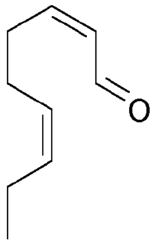 | Nonadienal |
| 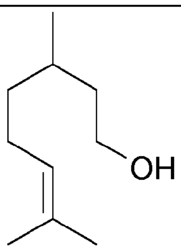 | Citronellol |
| 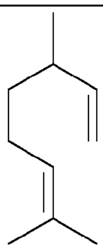 | β-Citronellen |
| 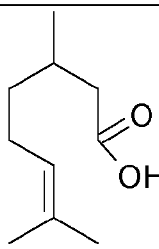 | Citronell~~ol~~säure |
| 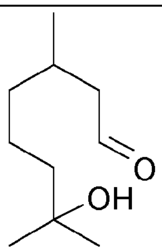 | Hydroxycitronellal |
| 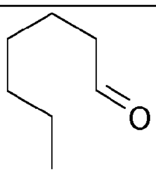 | Heptanal |

| Formel | Name |
|---|---|
| 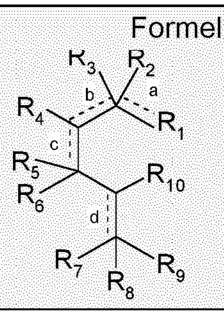 (IV) | |
| 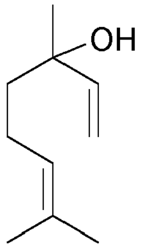 | Linalool |
| 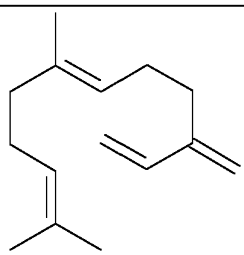 | Farnesen (β) |
| 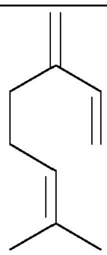 | Myrcen |
| 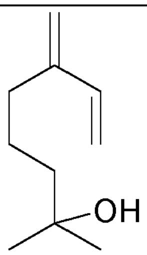 | Myrcenol |
| 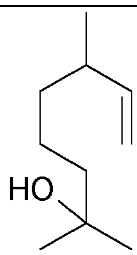 | Dihydromyrcenol |

| Formel | Name |
|---|---|
| 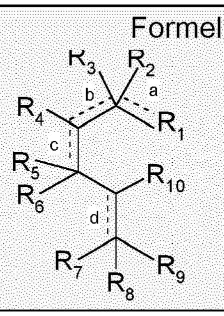 (IV) | |
| 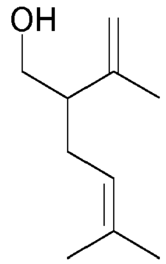 | Lavandulol |
| 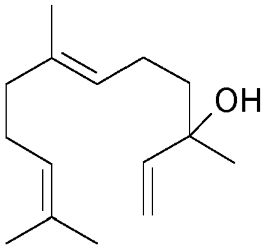 | Nerolidol |
| 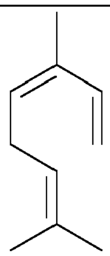 | (E)-β-Ocimen (4 Isomere vorhanden) |
| 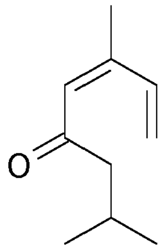 | Tagetone |
| 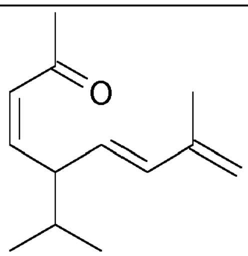 | Solanone |

| Formel | Name |
|---|---|
| 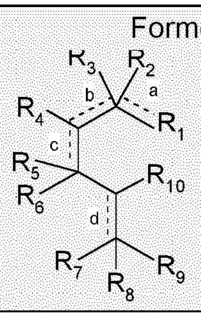 (IV) | |
| 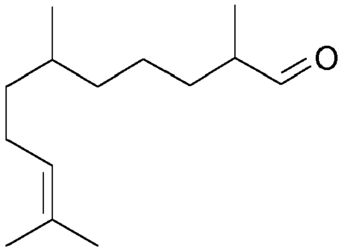 | 2,6,10-Trimethyl-9-undecanal |

Die bei deren Umsetzung anfallenden Reaktionsprodukte können in an sich bekannter Weise unter Anwendung analytischer Standardmethoden, wie Chromatographie, HPLC, Gaschromatographie, Massenspekrometrie, GC/MS oder MALDI-TOF und Kombinationen davon, nachgewiesen und quantifiziert werden.

Werden für das erfindungsgemäße Verfahren nicht-immobilisierte Organismen oder Enzyme verwendet, so werden diese vor der Extraktion zweckmäßigerweise abgetrennt, beispielsweise über eine Filtration oder Zentrifugation.

Das erfindungsgemäße Verfahren kann batchweise, semi-batchweise oder kontinuierlich betrieben werden.

## Experimenteller Teil

Sofern keine speziellen Angaben in den folgenden Beispielen gemacht werden, gelten die folgenden allgemeinen Angaben.

### A. Allgemeine Angaben

Sämtliche eingesetzte Materialien und Mikroorganismen sind im Handel erhältliche Produkte.

Soweit nicht anderes angegeben wird, erfolgt die Klonierung und Expression von rekombinanten Proteinen nach Standardmethoden, wie z.B. in Sambrook, J., Fritsch, E.F. und Maniatis, T., Molecular cloning: A Laboratory Manual, 2. Auflage, Cold Spring Harbor Laboratory, Cold Spring Harbor Laboratory Press, Cold Spring Harbor, NY, 1989 beschrieben.

### a) Bakterienstämme, Plasmide und Wachstumsbedingungen

Sämtliche Experimente wurden mit *E. coli* durchgeführt. Die SHC-Proteine wurden in *E. coli* BL21 (DE3) pLysS oder *E. coli* Rosetta pLysRAR62, enthaltend pET16b-Konstrukte mit dem jeweiligen *shc*-Gen, durch Wachstum in Luria-Bertani Medium, ergänzt mit Ampicillin (100 $\mu$g/ml), Chloramphenicol (34 $\mu$g/ml), und 0,5 mM Isopropylthio-$\beta$-D-galactosid bei einer $OD_{600}$ von 0,4 und zusätzlichem Wachstum über 4 Stunden bei 30°C exprimiert.

### b) Vektorkonstrukte

Das jeweilige Squalen-Hopen Cyclasegen (z.B. *Zymomonas mobilis* ZMO1548 [NC_006526.2, Region: 1578816..1580993]) wurde aus chromosomaler DNA PCR-amplifiziert, wobei entsprechende Primerpaare verwendet wurden (z.B. ZMO1548-fwd (5'-gcgctgtttcatatgggtattgaca-3') (SEQ ID NO: 327) und ZMO1548-rev (5'-gcgcttaccctggatcctcgaaaat-3') (SEQ ID NO: 328)). Das Restriktionsenzym verdaute (z.B. mit NdeI/BamHI) PCR Produkt wurde in pET16b einkloniert, (wobei man z.B.) pET1584 erhält. Die Konstrukte wurden durch DNA Sequenzierung überprüft und in *E. coli* XL1-blue transformiert

Das *shc-Gen* anderer Mikroorganismen (z.B. von *A. acidocaldarius)* wurde in analoger Weise kloniert.

Sämtliche Plasmide wurden einzeln in *E. coli* BL21 (DE3) pLysS oder *E. coli* Rosetta pLysRAR62 transformiert.

### c) Cyclisierungs-Assay mit verschiedenen Substraten (Standardbedingungen)

Rekombinante *E. coli* Zellen wurden in 20 mM Tris-HCl pH 8.0 (3 ml pro g feuchter Zellen) suspendiert. Das Zyklisierungsgemisch enthielt 250 µl Zellsuspension, 50 µl 1 M Citratpuffer (pH 4.5), 20 mM (Endkonzentration) Substrat und Wasser ad 500 µl. Bei Zyklisierung von Squalen wurde 1% (v/v) Triton-X100 zugesetzt. Für die Homofarnesol Zyklisierung wurden *E. coli* Zellen (6 g feuchte Zellen) in Solubilisierungspuffer (50 mM Phosphat, 10 mM $MgCl_2$ (pH 6,5; Gesamtvolumen: 25 ml) suspendiert. Die Zellen wurden bei 1500 bar unter Verwendung eines Manton-Gaulin Homogenisators aufgeschlossen. Unlösliche Zelldebris wurde abzentrifugiert (15 min bei 4°C and 7150*g). Das Zyklisierungsgemisch enthielt 1 ml rohen Zellextrakt und 20 mM Homofarnesol in 1,25 ml Puffer (50 mM Kaliumphosphat, 45 mM $MgCl_2$ (pH 6,5). Das Reaktionsgemisch wurde bei 30°C mittels eines Magnetrührers gerührt. Die Reaktion wurde durch Extraktion mit Heptan beendet. Die organische Phase wurde mittels Gaschromatographie analysiert. Kontrollen wurden mit *E. coli* Zellen durchgeführt, die einen leeren Vektor trugen sowie mit Hitze-inaktivierten SHC-exprimierenden Zellen. Die Bildung von Zyklisierungsprodukten wurde bei den Kontrollen niemals beobachtet (Daten nicht gezeigt).

### d) Gaschromatographie

Terpenoide wurden qualitativ und quantitativ durch Gaschromatographie unter Verwendung einer Agilent 7890A GC-Apparatur, ausgestattet mit einer DB-5 Säule (20 m × 0.1 mm × 0.1 µm) und einem Ionisationsdetektor analysiert. 3 µl des Lösungsmittelextraktes wurden auf die Säule aufgetragen (Splitverhältnis 1:5, Heliumflussrate 0,25 oder 0,5 ml/min, Injektortemperatur 250°C).

Zur Trennung von linearen und zyklischen Monoterpenoiden wurde die anfängliche Ofentemperatur (60°C) auf 130°C mit 40°C/min, mit 2°C/min auf 150°C und dann mit 40°C/min auf 200°C erhöht. Die Retentionszeiten der Terpenoide waren wie folgt: (*R*, *S*)-Citronellal (7,55 min), Isopulegol (7,70 min), Neo-Isopulegol (7,90 min), *Iso*-Isopulegol (8,10 min), *Neoiso*-Isopulegol (8,25 min), 1-Decanol (9,91 min).

Für die Detektion von Triterpenen wurde die Injektortemperatur auf 300°C eingestellt. Die Ofentemperatur betrug ursprünglich 60°C, wurde mit 40°C/min auf 220°C erhöht und anschließend mit 6°C/min auf 310°C und dort 10 min konstant gehalten. Squalen und Hopen eluierten nach 19,2 min bzw. 26,9 min.

Homofarnesol und Ambroxan wurden auf einer 10 m Optima 1 Säule (Macherey&Nagel, Düren, Deutschland) analysiert. Die anfängliche Ofentemperatur (100°C) wurde mit 5°C/min auf 200°C erhöht und bei dieser Temperatur 5 min gehalten. Anschließend wurde sie mit 30°C/min auf 320°C erhöht. Eine Analyse dauerte 40 min. Die Retentionszeiten waren wie folgt: Homofarnesol (10,8 min), Ambroxan (9,9 min).

Alternativ dazu wurde ein Shimadzu GC-MS QP 2010-System mit einer FS Supreme 5 Säule (30 m × 0.25 mm × 0.25 µm) für eine gekoppelte GC/MS-Analyse (Splitverhältnis 1:20; 3 min 120°C, Erhöhung auf 135°C mit 2°C/min und weitere Erhöhung auf 365°C mit 10°C/min, gefolgt von einer Abkühlung auf 300°C mit 70°C/min) verwendet. Die GC-MS-Daten wurden unter Verwendung einer LabSolutions GCsolutions Postrun Software analysiert. Es gilt zu beachten, dass die Substrate Citronellal-Racemat, (*R*)-Citronellal und (*S*)-Citronellal, immer geringe Mengen an Isopulegol und Neo-Isopulegol als Verunreinigungen enthalten. Die GC-Flächenwerte für diese linearen Terpenoide wurden als 100% festgesetzt. Die Flächenwerte für die Isopulegol-Isomere in dem Produkt wurden durch die Menge an Isopulegol-Isomer, die bereits in dem Substrat vorlag, korrigiert. Die Standardabweichung wurde anhand von 24 einzelnen Tests unter Verwendung von zwei separat gezüchteten *E. coli* Kulturen berechnet.

## B. Beispiele

### Beispiel 1: Herstellung von Mutanten des Typs F486X der Squalen-Hopen-Cyclasen durch rationales Proteindesign mittels Quick Change Mutagenese

Die Mutanten verschiedener Squalen-Hopen-Cyclasen wurden mittels "Quick Change" Mutagenese in das entsprechende Gen eingebaut. Dabei wurde weitgehend nach den Angaben des Herstellers (Fa. Agilent Technologies, Waldbronn) vorgegangen. Zunächst wurde eine PCR durchgeführt:

| | | |
|---|---|---|
| PCR-Ansatz: | 1,8µl | DMSO |
| | 2µl | dNTP's (je 2,5mM) |
| | 1,5µl | Primer forward (10pmol/µl) |
| | 1,5µl | Primer reverse (10pmol/µl) |
| | 1µl | Template (1 µg/µL; rekombinantes Plasmid, das SHC-Gen trägt, wie z.B. pETZmSHC_1) |
| | 0,2µl | Prime-Star Polymerase (Takara, 2,5Units/µl) |

(fortgesetzt)

| | |
|---|---|
| 6µl | 5x Puffer |
| 16µl | $H_2O$ |

PCR-Programm:

| | |
|---|---|
| (1) 95°C | 3 Minuten |
| (2) 95°C | 45 Sekunden |
| (3) 53°C | 1 Minute |
| (4) 68°C | 17 Minuten |
| 5x | Wiederholung der Schritte (2), (3) und (4) |

Im Anschluss an die PCR wurden 10µl des Ansatzes mit dem Restriktionsenzym DpnI für mindestens 1 Stunde bei 37°C verdaut. Danach erfolgte die Transformation in *E. coli* XL1-blue-Zellen. Nach DNA-Sequenzierung fand die Transformation in den Expressionsstamm z. B. *E. coli* Rosetta pLysRAR62 statt. Analog kann das Gen auch in anderen Expressionsplasmiden modifiziert werden.

Folgende Primer wurden für die Quick-Change-PCR verwendet. Der jeweilige Austausch ist anhand des Fettdrucks in den Primernamen ersichtlich. Die Gene, die durch die jeweiligen Primer modifiziert werden, sind durch Kursivdruck im Primernamen markiert; dabei entspricht

| | |
|---|---|
| *ZmSHC_1* | SEQ ID NO: 2; |
| *ZmSHC_2* | SEQ ID NO: 6; |
| *Ap* | SEQ ID NO: 4; |
| *Bj* | SEQ ID NO: 5 und |
| *Sc* | SEQ ID NO: 3. |

| **Primemame** | **Sequenz** | **SEQ ID NO** |
|---|---|---|
| *ZmSHC_***1F486Ile**for | GTTATTATCCTTATCGATGGCTCCCCAACCG | 329 |
| *ZmSHC_1***F486Ile**rev | GGTTGGGGAGCCATCGATAAGGATAATAACAG | 330 |
| *ZmSHC_1***F486Met**for | GTTATTATCCTTATCCATGGCTCCCCAACCG | 331 |
| *ZmSHC_1***F486Met**rev | GGTTGGGGAGCCATGGATAAGGATAATAACAG | 332 |
| *ZmSHC_1***F486Thr**for | GTTATTATCCTTATCGGTGGCTCCCCAACCG | 333 |
| *ZmSHC_1***F486Thr**rev | GGTTGGGGAGCCACCGATAAGGATAATAACAG | 334 |
| *ZmSHC_1***F486Gln**for | GTTATTATCCTTATCCTGGGCTCCCCAACCG | 335 |
| *ZmSHC_1***F486Gln**rev | GGTTGGGGAGCCCAGGATAAGGATAATAACAG | 336 |
| *ZmSHC_1F***486Asn**for | GTTATTATCCTTATCGTTGGCTCCCCAACCG | 337 |
| *ZmSHC_1***F486Asn**rev | GGTTGGGGAGCCAACGATAAGGATAATAACAG | 338 |
| *ZmSHC_1***F486Lys**for | GTTATTATCCTTATCTTTGGCTCCCCAACCG | 339 |
| *ZmSHC_1***F486Lys**rev | GGTTGGGGAGCCAAAGATAAGGATAATAACAG | 340 |
| *ZmSHC_1***F486Asp**for | GTTATTATCCTTATCATCGGCTCCCCAACCG | 341 |
| *ZmSHC_1***F486Asp**rev | GGTTGGGGAGCCGATGATAAGGATAATAACAG | 342 |
| *ZmSHC_1***F486Glu**for | GTTATTATCCTTATCTTCGGCTCCCCAACCG | 343 |
| *ZmSHC_1***F486Glu**rev | GGTTGGGGAGCCGAAGATAAGGATAATAACAG | 344 |
| *ZmSHC_1***F486Trp**for | GTTATTATCCTTATCCCAGGCTCCCCAACCG | 345 |
| *ZmSHC_1***F486Trp**rev | GGTTGGGGAGCCTGGGATAAGGATAATAACAG | 346 |
| *ZmSHC_1***F486Arg**for | GTTATTATCCTTATCACGGGCTCCCCAACCG | 347 |
| *ZmSHC_1***F486Arg**rev | GGTTGGGGAGCCCGTGATAAGGATAATAACAG | 348 |
| *ZmSHC_1***F486Cys**for | GTTATTATCCTTATCGCAGGCTCCCCAACCG | 349 |
| *ZmSHC_1***F486Cys**rev | GGTTGGGGAGCCTGCGATAAGGATAATAACAG | 350 |
| *ZmSHC_1***F486G**for | GTTATTATCCTTATCACCGGCTCCCCAACCG | 351 |
| *ZmSHC_1***F486G**rev | GGTTGGGGAGCCGGTGATAAGGATAATAACAG | 352 |
| *ZmSHC_1***F486S**for | GTTATTATCCTTATCGCTGGCTCCCCAACCG | 353 |
| *ZmSHC_1***F486S**rev | GGTTGGGGAGCCAGCGATAAGGATAATAACAG | 354 |

(fortgesetzt)

| Primername | Sequenz | SEQ ID NO |
|---|---|---|
| *ZmSHC_1*F486Pfor | GTTATTATCCTTATCCGGGGCTCCCCAACCG | 355 |
| *ZmSHC_1*F486Prev | GGTTGGGGAGCCCCGGATAAGGATAATAACAG | 356 |
| *ZmSHC_1*F486Hfor | GTTATTATCCTTATCATGGGCTCCCCAACCG | 357 |
| *ZmSHC_7*F486Hrev | GGTTGGGGAGCCCATGATAAGGATAATAACAG | 358 |
| *ZmSHC_1*F486Lfor | GTTATTATCCTTATCCAGGGCTCCCCAACCG | 359 |
| *ZmSHC_1*F486Lrev | GGTTGGGGAGCCCTGGATAAGGATAATAACAG | 360 |
| *ZmSHC_*1F486Vfor | GTTATTATCCTTATCAACGGCTCCCCAACCG | 361 |
| *ZmSHC_1*F486Vrev | GGTTGGGGAGCCGTTGATAAGGATAATAACAG | 362 |
| *ZmSHC_1*F486Afor | GTTATTATCCTTATCCGCGGCTCCCCAACCG | 363 |
| *ZmSHC_1*F486Arev | GGTTGGGGAGCCGCGGATAAGGATAATAACAG | 364 |
| *ZmSHC_1*F486Yfor | GTTATTATCCTTATCATAGGCTCCCCAACCG | 365 |
| *ZmSHC_1*F486Yrev | GGTTGGGGAGCCTATGATAAGGATAATAACAG | 366 |
| *ZmSHC_1*Y702Cfor | GCCGATAAAAATCGCAACGCAGCATAAACG | 367 |
| *ZmSHC_1*Y702Crev | CGTTTATGCTGCGTTGCGATTTTTATCGGC | 368 |
| *ZmSHC_1*Y702Ffor | GCCGATAAAAATCTTTACGCAGCATAAACG | 369 |
| *ZmSHC_1*Y702Frev | CGTTTATGCTGCGTAAAGATTTTTATCGGC | 370 |
| *ZmSHC_1*Y702Afor | GCCGATAAAAATCCGCACGCAGCATAAACG | 371 |
| *ZmSHC_1*Y702Arev | CGTTTATGCTGCGTGCGGATTTTTATCGGC | 372 |
| *ZmSHC_1*Y702Sfor | GCCGATAAAAATCGCTACGCAGCATAAACG | 373 |
| *ZmSHC_1*Y702Srev | CGTTTATGCTGCGTAGCGATTTTTATCGGC | 374 |
| *ZmSHC_1*Y561Afor | GAACCGCACCGGTGCCATAGATCGCATTAACG | 375 |
| *ZmSHC_1*Y561Arev | GGTTTGGTCGTTGGGGCGTTAATGCGATCTATGG | 376 |
| *ZmSHC_1*Y705Afor | CCATAATCGGGAAGAATTGCCGCGCAAAATC | 377 |
| *ZmSHC_1*Y705Arev | CTGCGTTATGATTTTGCGCGGCAATTCTTC | 378 |
| *ZmSHC_2*F486Cfor | GGCGGTTGGGGCGCTTGCGATGCCAATAACAG | 379 |
| *ZmSHC_2*F486*Crev* | CTGTTATTGGCATCGCAAGCGCCCCAACCGCC | 380 |
| *Ap*F486Crev | CATTATCTTTATCGCATGCACCCCAACCACC | 381 |
| *ApF*486Cfor | GGTGGTTGGGGTGCATGCGATAAAGATAATG | 382 |
| *Bj*F486Cfor | CGGCTGGGGCGCGTGCGATAAAGATAAC | 383 |
| *Bj*F486Crev | GTTATCTTTATCGCACGCGCCCCAGCCG | 384 |
| *Sc*F486Cfor | CGGCGCCTGGGGCGCCTGCGACGTCGACAAC | 385 |
| *Sc*F486Crev | GTTGTCGACGTCGCAGGCGCCCCAGGCGCCG | 386 |

**Beispiel 2: Aktivitätstests mit Mutanten der Squalen-Hopen-Cyclase-1 (SHC-1) aus *Zymomonas mobilis***

Der Einfluss von verschiedenen Einfachmutationen, hergestellt gemäß Beispiel 1, in der Sequenz-Position entsprechend F486 auf die Cyclase-Aktivität wurde für verschiedene Substrate bestimmt.

a) Citronellal

Nach dem generellen Nachweis einer geringen Zyklisierungsaktivität der Squalen-Hopen-Cyclase-1 aus *Zymomonas mobilis* (SEQ ID NO:2) gegenüber Citronellal, wurde die Umsatzrate durch rationales Proteindesign deutlich verbessert. Der Austausch des Phenylalaninrestes F486 gegen Alanin, führte in ersten Versuchen (vgl. **Figur 2)** zu einer deutlich gesteigerten Produktion von Isopulegol **(2)** ausgehend vom Citronellal **(1).**

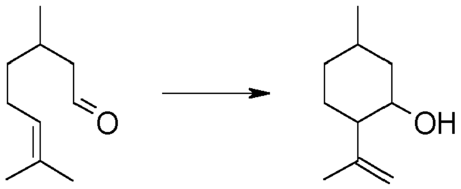

(**1**) (**2**)

Die gesteigerte Aktivität der SHC_1-F486A Mutante wurde im Weiteren näher untersucht. Dabei konnte neben einer wesentlich besseren Umsetzung des Citronellal-Substrates auch festgestellt werden, dass diese das R(+)- Isomer als Substrat bevorzugt und im Vergleich zum WT dieses auch in wesentlich kürzerer Zeit umsetzt (vgl. **Figur 2).** Während beim WT Enzym die Reaktion mit R(+)-Citronellal erst nach längerer Inkubation messbar ist, zeigt die F486A Mutante insbesondere zu Beginn der Reaktion hohe Umsätze. Dieser Effekt ist bei S(-)-Citronellal als Substrat nicht zu beobachten. Auffällig ist, dass die F486A Mutante nur Isopulegol I und II bildet, unabhängig von der Stereokonfiguration des Substrates. Der WT hingegen ist abhängig von der Stereokonfiguration des Substrates und bildet neben Isopulegol I überwiegend Isopulegol II aus R(+)-Citronellal und fast ausschließlich Isopulegol III aus S(-)-Citronellal.

Basierend auf diesen Ergebnissen wurde in weiterführenden Experimenten die Bedeutung des Aminosäurerestes an der Position 486 genauer untersucht. Dazu wurde mittels Mutagenese der Phenylalaninrest gegen jede weitere Aminosäure ausgetauscht und die Aktivität der verschiedenen Muteine mit Citronellal als Substrat getestet (Sequenzen siehe **Figur 1a** und **b).** Es wurde festgestellt, dass einige Aminosäuren an dieser Position nicht nur den Umsatz von Citronellal durch das Enzym verbessern, sondern zusätzlich zu einer höheren Produktspezifität in der Umsetzung führen, dergestalt, dass weniger Isomere des Isopulegol produziert werden (siehe **Figur 3).**

Der Austausch gegen Arginin, Prolin und Lysin führt zu einem Verlust der Aktivität gegenüber Citronellal. Die ermittelten Produktmengen sind in gleicher Verteilung auch als Verunreinigung in der Negativkontrolle zu finden **(‚K'** siehe **Figur 3).** Die höchste Aktivität wurde nach Austausch gegen Valin, Threonin, Cystein, Isoleucin und Alanin beobachtet. Insgesamt auffällig ist das veränderte Produktspektrum einiger Muteine. Nicht alle zeigen die Bildung von drei Isopulegol Peaks wie der Wildtyp und auch die Mengenverteilung unterscheidet sich.

Es existieren insgesamt $2^3$ Isopulegol-Isomere:

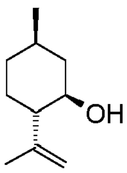 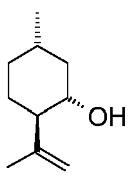 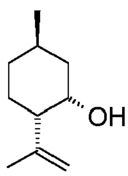 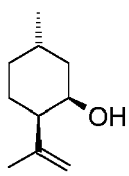

(*1R*, *3R*, *6S*) (*1S*, *3S*, *6R*) (*1S*, *3R*, *6S*) (*1R*, *3S*, *6R*)

Isopulegol
(Isopulegol I)

*neo*-Isopulegol
(Isopulegol II)

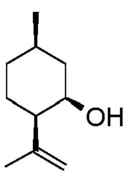 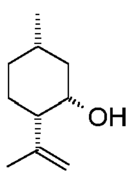 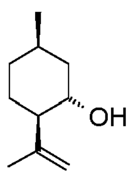 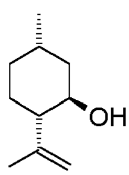

(*1R*, *3R*, *6R*) (*1S*, *3S*, *6S*) (*1S*, *3R*, *6R*) (*1R*, *3S*, *6S*)

*iso*-Isopulegol
(*epi*-Isopulegol)
(Isopulegol III)

*neo-iso*-Isopulegol
(Isopulegol IV)

Bisher konnte das Hauptprodukt (Isopulegol I) dem Enantiomerenpaar (*1R,3R,6S*)-Isopulegol bzw. (1*S*,3*S*,6*R*)-Isopulegol zugeordnet werden.

Die höchste Ausbeute an Isopulegol mit den wenigsten Nebenprodukten (bestehend aus weiteren Isomeren) begleitet von hoher Enzymaktivität zeigt die Zm-SHC-1 F486C Mutante.

b) Squalen

Die deutlichen Veränderungen in der Aktivität nach Mutation an Position F486 zeigen sich auch mit Squalen als Substrat. Interessanterweise sorgt in diesem Fall der Austausch des Phenylalanins gegen Tyrosin fast zu einer Verdopplung des Umsatzes (siehe **Figur 4).**

**Beispiel 3: Aktivitätstests mit Mutanten weiterer Squalen-Hopen-Cyclasen**

Der Einfluss von verschiedenen Einfachmutationen, hergestellt gemäß Beispiel 1, in der Sequenz-Position entsprechend F486 auf die Cyclase-Aktivität verschiedener weiterer SHCs wurde für verschiedene Citronellal-Substrate (jeweils 20mM über Nacht Inkubation) bestimmt:
Es handelt sich dabei um folgende Mutanten:

Ap-SHC: F481C,
Bj-SHC: F447C,
Sc-SHC: F449C,
Zm SHC-2: F438C

Die Phenylalaninreste sitzen dabei an Positionen, die dem F486 der Zm-SHC-1 (SEQ ID NO:2) analog sind.

Die Ergebnisse sind der **Figur 5** (Citronellal-Racemat als Substrat), der **Figur 6** (R(+)-Citronellal als Substrat), und der **Figur 7** (S(-)-Citronellal als Substrat) zu entnehmen. Als Kontrolle diente ein Ansatz ohne aktiven Biokatalysator.

Man beobachtet, dass die Wildtyp-Enzyme durch Mutation an der angegebenen F486 (von Zm SHC-1) entsprechenden Position nunmehr Citronellal zu Isopulegol zyklisieren können und zudem die R(+)-Form mit erhöhter Selektivität im Vergleich zur S(-)-Form umsetzten.

**Beispiel 4: Umsetzung von Verbindungen der Formel IV**

Diese Substanzen wurden unter Bedingungen umgesetzt, entsprechend denjenigen, die für die Umsetzung von Citronellal, wie oben beschriebenen, angewandt wurden.

**Beispiel 5: Isolierung und Charakterisierung der Squalen-Hopen-Cyclase aus *Zymomonas mobilis* (Zm-SHC)**

In der internationalen Anmeldung PCT/EP2010/057696, worauf hier ausdrücklich Bezug genommen wird, wird beschrieben, wie mit Hilfe spezifischer Oligonukleotide das Gen der Zm-SHC aus der genomischen DNA von Zymomonas mobilis amplifiziert werden und in Escherichia coli exprimiert werden konnte.

**a) Material und Methoden:**

Im Folgenden wird nur auf Material und Methoden eingegangen, die in der internationalen Anmeldung PCT/EP2010/057696 nicht in dieser Form erwähnt wurden.

**b) Stämme, Plasmide und Kulturbedingungen:**

Es wurde der E. coli Stamm DH5α, der E. coli Stamm BL21 (DE3)pLysS (Novagen) und der Stamm E. coli Rosetta verwendet. Das Plasmid pET16b (Novagen) wurde für die Klonierung verwendet. Für die Überexpression der SHC wurde des Weiteren das Plasmid pLysRAR62 zusätzlich zur Anpassung des Codon Usage an E. coli transformiert. Des Weiteren wurde das Plasmid pDHE+ZmSHC-1 aus E. coli Lu15568 ver-wendet (internationalen Anmeldung PCT/EP2010/057696). Die Anzucht der Stämme fand mit LB-Medium bei 30°C statt.

**c) Chemikalien:**

Squalen, (+/-)-Citronellal, (+)-R-Citronellal und (-)-S-Citronellal wurden von Sigma (Sigma-Aldrich Chemie GmbH, München) bezogen. Restriktionsenzyme, T4-Ligase, DNA-Polymerase kamen von New England Biolabs (New England Biolabs GmbH, Frankfurt).

**d) Isolierung der DNA und Transformation:**

Plasmide wurden aus E. coli mittels des Qiaprep Spin Miniprep Kits von Qiagen (Qia-gen, GmbH, Hilden) isoliert. Für Gel Extraktionen oder PCR-Aufreingungen wurden das Qiaquick Gel Extraction Kit von Qiagen genutzt. Alle verwendeten E. coli Stämme wurden mittels der $CaCl_2$-Methode transformiert.

e) **PCR und Sequenzierung:**

Die DNA von Zymomonas mobilis subspec. mobilis CP4 wurde von Herrn Prof. Dr. Sprenger (Institut für Mikrobiologie, Universität Stuttgart) gestellt. Die PCR wurde mit der Prime Star Polymerase durchgeführt. Folgende Primer wurden zur Synthese des Squalen-Hopen-Cyclasen-Gens aus Zymomonas mobilis verwendet:

| | | |
|---|---|---|
| SHC_1: | SHC-for | TATGCATATGGGTATTGACAGAAT (SEQ ID NO: 387) |
| | SHC-rev | CCGGATCCTCAATTATTCAAATCAATC (SEQ ID NO: 388) |

Die Korrektheit der klonierten Gene wurde mittels Sequenzierung durch die Firma GATC Biotech überprüft. Sequenzanalysen wurden mittels des Programms Clone Ma-nager7.0 durchgeführt. Nach Restriktion der entsprechenden Amplifikate wurden diese in frame in den pET16b-Vektor MIT N-terminal kodiertem His-tag kloniert. Die Plasmide wurden im Anschluss zunächst in E. coli DH5α und im Anschluss in E. coli BL21 (DE3)pLysS und E. coli Rosetta transformiert. Zur besseren Expression wurde in die E. coli Rosetta Stämme zusätzlich zu den pET16b-Konstrukten das Plasmid pLysRAR62 transformiert. Parallel wurden entsprechende Klonierungen mit Leervektoren durchge-führt. Außerdem wurde das Plasmid pDHE+ZmSHC_1 (entspricht SHC_1 mit an E. coli angepasstem Codon-Usage) in E. coli BL21 (DE3)pLysS transformiert.

**f) Expression und Zellaufschluss:**

Die entsprechenden E. coli Bl21 (DE3) pLysS bzw. E. coli Rosetta Transformanten wurden in LB-Medium mit Ampicillin und Chloramphenicol (100µg/ml bzw. 32µg/ml) bei 30°C kultiviert. Die Synthese der Squalen-Hopen-Cyclasen wurde durch Zugabe von 0,5-1mM IPTG bzw. 0,1% Rhamnose (bei Verwendung der pDHE-Derivate) bei einer $OD_{600}$ von 0,4-0,6 induziert. Die Zellen wurden für 4-6h weiter wachsen gelassen und im Anschluss geerntet. Dazu wurden die Zellen abzentrifugiert und in 5ml/g Nassge-wicht 25mM Tris/HCl mit 40% Glycerin aufgenommen. Wurden die Zellen nicht sofort weiter verwendet, fand eine Lagerung bei -20°C statt. Zum Aufschluss der Zellen wur-den diese jeweils 2x mit durch die French Press gegeben und entweder direkt oder nach Abtrennen der Zelltrümmer durch Zentrifugation für die Aktivitätsbestimmungen eingesetzt. Alternativ fand der Zellaufschluss mit Ultraschall statt. Nach Zentrifugation wurden die SHC-Proteine im Anschluss mit Solubilisationspuffer (50mM Tris/HCl pH 8, 10mM $MgCl_2$, 1% Triton X-100) aus den Zelltrümmern gelöst und somit partiell ange-reichert.

**g) Aktivitätsbestimmungen:**

Jeder Ansatz zur Ermittlung der Aktivität der Squalen-Hopen-Cyclasen hatte ein Endvolumen von 1ml. Dieses setzte sich aus 600µl mittels French Press aufgeschlossenen Zellen (alternativ 800µl nach Solubilisation aus der Zellmembran), 100mM Na-Citrat-Puffer mit verschiedenen pH-Werten (getestet wurden pH 4.0 bis pH 8.0) und 10mM Substratlösung [(+/- )Citronellal, (+)-R-Citronellal und (-)-S-Citronellal] zusammen. Ne-ben dem Substrat und $H_2O$ enthielt die Substratlösung noch Triton X-100, welches im Aktivitätsansatz jeweils in einer Konzentration von 0,2% vorlag.

Die Ansätze wurden für 6h bis 24h bei Temperaturen von 22°C, 30°C und 37°C unter Schütteln inkubiert. Die Extraktion des Substrates und möglicher Produkte erfolgte mit einem Volumen Chloroform oder Hexan/Prpopanol im Verhältnis 2:3. Der Extrakt wurde direkt für die gaschromatographische Analyse eingesetzt.

**h) GC-Messungen:**

Die Gaschromatographischen Messungen fanden auf einem Agilent 7890A Gaschromatographen mit Flammenionisationsdetektor statt. Als Säule wurde eine DB-5 (Agilent Technologies) mit einer Länge von 20m, einem Durchmesser von 0,1mm und 0,25µM Beschichtung verwendet. Eine Identifizierung erfolgte über den Vergleich der Retentionszeiten mit vorhandenen Standard-Lösungen.

Zur Absicherung wurden die Proben auf einem Shimadzu Gaschromatographen mit Massenspektrometer parallel untersucht. Unter Verwendung der Säule FS Supreme Colum mit 30m Länge, Innendurchmesser von 0,25mm und 0,25µm Beschichtung konnten die Retentionszeiten wiederum mit Standard-Lösungen abgeglichen werden und

die jeweiligen Massenspektren der Inhaltsstoffe analysiert werden.

Das im folgenden mit Isopulegol I bezeichnete Diastereomer konnte mit Hilfe eines Standards dem *(1R,3R,6S)* bzw. (*1S,3S,6R*) Isopulegol zugeordnet werden, während für die als Isopulegol II und Isopulegol III bezeichneten Isomere noch keine Zuordnung getroffen werden konnte.

**i) Ergebnisse der Aktivitätsbestimmungen:**

1. Test 1a: (Vergleich) (Kontrollen *i.e.* Ergebnisse mit abgekochtem Protein, mit Leervektor und ohne Protein)

| | pH 4,0 | pH 4,5 | pH 5,0 | pH 5,5 | pH 6,0 | pH 6,5 | pH 7,0 |
|---|---|---|---|---|---|---|---|
| Citronellal | 85,4 | 85,4 | 86,0 | 85,6 | 84,4 | 84,7 | 85,1 |
| Isopulegol I | 10,8 | 10,8 | 10,4 | 10,8 | 11,7 | 11,5 | 11,2 |
| Isopulegol II | 3,8 | 3,8 | 3,6 | 3,6 | 3,9 | 3,8 | 3,7 |
| Isopulegol III | 0 | 0 | 0 | 0 | 0 | 0 | 0 |

In den folgenden Angaben für das Substrat *rac*-Citronellal erfolgen bereits abzüglich der in den Kontrollen nachgewiesenen Mengen Isopulegol.

2. Test 1b: Vergleich der beiden überexprimierten SHC_1-Proteine (aus pDHE und pET16b-Vektor und Einfluss des His-tags auf Aktivität bei pH 4,5)

| | pDHE | pET16b |
|---|---|---|
| Citronellal | 95,2 | 95,2 |
| Isopulegol I | 0,7 | 0,8 |
| Isopulegol II | 1,7 | 1,6 |
| Isopulegol III | 2,4 | 2,4 |

3. Test 1c: pH-Abhängigkeit

| | pH 4,0 | pH 4,5 | pH 5,0 | pH 5,5 | pH 6,0 | pH 6,5 | pH 7,0 |
|---|---|---|---|---|---|---|---|
| Citronellal | 95,9 | 94,9 | 94,7 | 94,4 | 95,1 | 98,7 | 98,8 |
| Isopulegol I | 0,4 | 0,8 | 0,8 | 1,0 | 1,1 | 0,8 | 0,5 |
| Isopulegol II | 1,1 | 2,4 | 2,1 | 2,1 | 1,6 | 0,5 | 0,7 |
| Isopulegol III | 2,6 | 1,9 | 2,4 | 2,5 | 2,2 | 0 | 0 |

4. Test 1d: Einfluss von Salzen bei pH 4,5

| | ohne | $BaCl_2$ | $CaCl_2$ | $MgCl_2$ |
|---|---|---|---|---|
| Citronellal | 94,9 | 95,2 | 94,9 | 95,0 |
| Isopulegol I | 0,7 | 0,8 | 1,0 | 0,9 |
| Isopulegol II | 2,5 | 2,4 | 2,4 | 2,5 |
| Isopulegol III | 1,9 | 1,6 | 1,7 | 1,6 |

5. Test 1e: Einfluss der Temperatur bei pH 4,5

| | 22°C | 30°C | 37°C |
|---|---|---|---|
| Citronellal | 95,3 | 94,9 | 95,4 |

(fortgesetzt)

| | 22°C | 30°C | 37°C |
|---|---|---|---|
| Isopulegol I | 0,8 | 1,0 | 0,8 |
| Isopulegol II | 1,8 | 2,2 | 1,6 |
| Isopulegol III | 2,1 | 1,9 | 2,2 |

6. Test 2: S(-)-Citronellal als Substrat

| | pH 4,0 | CTRL | pH 4,5 | CTRL | pH 5,0 | CTRL | pH 5,5 | CTRL |
|---|---|---|---|---|---|---|---|---|
| Citronellal | 90,8 | 95,5 | 90,8 | 95,7 | 91,7 | 96,2 | 92,4 | 96,2 |
| Isopulegol I | 4,9 | 4,5 | 4,7 | 4,3 | 4,4 | 3,8 | 4,1 | 3,8 |
| Isopulegol II | 0 | 0 | 0 | 0 | 0 | 0 | 0 | 0 |
| Isopulegol III | 4,3 | 0 | 4,5 | 0 | 3,9 | 0 | 3,5 | 0 |

| | pH 6,0 | CTRL | pH 6,5 | CTRL | pH 7,0 | CTRL |
|---|---|---|---|---|---|---|
| Citronellal | 94,1 | 96,6 | 96,4 | 96,5 | 96,5 | 96,4 |
| Isopulegol I | 3,8 | 3,4 | 3,6 | 3,5 | 3,5 | 3,6 |
| Isopulegol II | 0 | 0 | 0 | 0 | 0 | 0 |
| Isopulegol III | 2,1 | 0 | 0 | 0 | 0 | 0 |

7. Test 3: R-(+)-Citronellal als Substrat

| | pH 4,0 | CTRL | pH 4,5 | CTRL | pH 5,0 | CTRL | pH 5,5 | CTRL |
|---|---|---|---|---|---|---|---|---|
| Citronellal | 80,0 | 84,2 | 78,4 | 83,8 | 81,1 | 85,6 | 81,7 | 86,8 |
| Isopulegol I | 15,9 | 15,8 | 16,0 | 16,2 | 14,1 | 14,4 | 13,5 | 13,2 |
| Isopulegol II | 4,1 | 0 | 5,6 | 0 | 4,8 | 0 | 4,8 | 0 |
| Isopulegol III | 4,3 | 0 | 4,5 | 0 | 3,9 | 0 | 3,5 | 0 |

| | pH 6,0 | CTRL | pH 6,5 | CTRL | pH 7,0 | CTRL |
|---|---|---|---|---|---|---|
| Citronellal | 81 | 85,5 | 80,8 | 85,8 | 81,4 | 86,2 |
| Isopulegol I | 14,3 | 14,5 | 14,5 | 14,2 | 14,0 | 13,8 |
| Isopulegol II | 4,7 | 0 | 4,7 | 0 | 4,6 | 0 |
| Isopulegol III | 2,1 | 0 | 0 | 0 | 0 | 0 |

j) **Zusammenfassung der Ergebnisse:**

Die Squalen-Hopen-Cyclase aus Zymomonas mobilis konnte in E. coli rekombinant hergestellt werden. Das Enzym ist in der Lage, Citronellal zu Isopulegol umzusetzen.

Dabei zeigten die beiden überproduzierten Zm-SHC-1 Proteine, einmal ohne und einmal mit N-terminal angehängtem His-tag keine Unterschiede in ihrer Aktivität unter den getesteten Bedingungen (vgl. Test 1b).

Diese Reaktion wurde nach 12h mit den beschriebenen Methoden nachgewiesen. Dabei hatte die Reaktion eine geringe Abhängigkeit vom pH-Wert. In einem pH-Bereich von pH 4 bis pH 6 konnten nach 20-stündiger Inkubation Umsatzraten von insgesamt etwa 5% zu verschiedenen Isopulegol-Isomeren gemessen werden.

Dabei war es nicht entscheidend, ob die Ansätze bei RT, 30°C oder 37°C inkubiert wurden. Auch die Zugabe zweiwertiger Ionen, wie z.B. $MgCl_2$, erhöhte nicht den Umsatz (vgl. Test 1d) . Entscheidend war jedoch, dass die Zellextrakte bei Messungen über einem pH-Wert von pH 5 entweder vor Zugabe des Substrates dialysiert wurden oder EDTA zu den Ansätzen gegeben wurde, um eine Reduktion des Substrates Citronellal zu Citronellol durch Enzyme des Wirtes zu unterdrücken. Ein Einfluss auf die Aktivität der Zm-SHC-1 durch diese Behandlung konnte nicht gezeigt werden. Blieb diese Behandlung aus, so wurde das Substrat innerhalb von 20h fast komplett zu Citronellol reduziert und es fand keine messbare Cyclisierung zu Isopulegol mehr statt. Die Zm-SHC-1 kann somit kein Citronellol, jedoch Citronellal zu Isopulegol zyklisieren. Sehr wahrscheinlich sind unspezifische Dehydrogenasen für die Reduktionsreaktion verantwortlich.

Um auszuschließen, dass eine chemische Reaktion für die Zyklisierung verantwortlich ist, wurden abgekochte Zellextrakte eingesetzt. In diesen Kontrollen und Kontrollen mit Zellextrakten aus Anzuchten mit Leervektoren konnte jedoch kein entsprechender Umsatz festgestellt werden (vgl. Test 1a).

Mit (+/-)-Citronellal als Substrat konnten im Anschluss an die Reaktion verschiedene, noch nicht genau identifizierte, Isomere von Isopulegol nachgewiesen werden (vgl. Test 2 und 3). Zur Kontrolle, ob diese Isomere aus den verschiedenen Isomeren des Ausgangssubtrates entstanden oder nur ein Isomer als Substrat akzeptiert und unterschiedlich umgesetzt wurde, wurden dieselben Studien mit (+)-R- Citronellal und (-)-S- Citronellal durchgeführt. Dabei zeigte sich, dass abhängig vom Substrat unterschiedliche Isopulegolisomere gebildet werden. Die Umsetzung von (+)-R-Citronellal fand dabei interessanterweise von pH 4 bis pH 7 ohne deutliche Unterschiede mit einer Rate von etwa 5% statt. Das Enantiomer wurde hingegen nur bis zu einem pH-Wert von pH 6 mit Umsatzraten von ca. 4,5% umgesetzt. Auch hier schwankte die Umsatzrate bei den einzelnen pH-Werten zwischen pH 4 und pH 6 kaum.

**Sequenzen:**

SEQ ID NO: 1- 326 Nukleinsäure/Aminosäuresequenzen verschiedner SHC Gene
SEQ ID NO: 327-388 PCR Primer

Auf die Offenbarung der hierin zitierten Druckschriften wird ausdrücklich Bezug genommen.

Es folgt eine Auflistung erfindungsgemäß brauchbarer SHC-Enzymsequenzen:

Enzymsequenzen

>seq ID 4

```
MNMASRFSLKKILRSGSDTQGTNVNTLIQSGTSDIVRQKPAPQEPADLSALKAMGNSLTHTLS-
SACEWLMKQQKPDGHWVGSVGSNASMEAEWCLALWFLGLEDHPLRPRLGKALLEMQRPDGS-
WGTYYGAGSGDINATVESYAALRSLGYAEDDPAVSKAAAWIISKGGLKNVRVFTRYWLALI-
GEWPWEKTPNLPPEIIWFPDNFVFSIYNFAQWARATMM-
PLAILSARRPSRPLRPQDRLDALFPGGRANFDYELPTKEGRDVIADFFRLADKGLHWLQSS-
FLKRAPSREAAIKYVLEWIIWHQDADGGWGGIQPPWVYGLMALHGEGYQFHHPVMAKAL-
DALNDPGWRHDKGDASWIQATNSPVWDTMLSLMALHDANAEERFTPEMDKALD-
WLLSRQVRVKGDWSVKLPNTEPGGWAFEYANDRYPDTDDTAVALIAIASCRNRPEWQAKGVEE-
AIGRGVRWLVAMQSSCGGWGAFDKDNNKSILAKIPFCDFGEALDPPSVDVTAHVLEAFGLL-
GLPRDLPCIQRGLAYIRKEQDPTGPWFGRWGVNYLYGTGAVLPALAALGEDMTQPYIS-
KACDWLINCQQENGGWGESCASYMEVSSIGHGATTPSQTAWALMGLIAANRPQDYEAIAKGC-
RYLIDLQEEDGSWNEEEFTGTGFPGYGVGQTIKLDDPAISKRLMQGAELSRAF-
MLRYDLYRQLFPIIALSRASRLIKLGN
```

>seq_ID 2

MGIDRMNSLSRLLMKKIFGAEKTSYKPASDTIIGTDTLKRPNRRPEPTAKVDKTI-
FKTMGNSLNNTLVSACDWLIGQQKPDGHWVGAVESNASMEAEWCLALWFLGLEDH-
PLRPRLGNALLEMQREDGSWGVYFGAGNGDINATVEAYAALRSLGYSADNPVLKKAAA-
WIAEKGGLKNIRVFTRYWLALIGEWPWEKTPNLPPEIIWFPDNFVFSIYNFAQWA-
RATMVPIAILSARRPSRPLRPQDRLDELFPEGRARFDYELPKKEGIDLWSQFFRTT-
DRGLHWVQSNLLKRNSLREAAIRHVLEWIIRHQDADGGWGGIQPPWVYGLMALH-
GEGYQLYHPVMAKALSALDDPGWRHDRGESSWIQATNSPVWDTMLALMALKDAKAEDRFT-
PEMDKAADWLLARQVKVKGDWSIKLPDVEPGGWAFEYANDRYPDTDDTAVALIALSSYRD-
KEEWQKKGVEDAITRGVNWLIAMQSECGGWGAFDKDNNRSILSKIPFCDFGESI-
DPPSVDVTAHVLEAFGTLGLSRDMPVIQKAIDYVRSEQEAEGAWFGRWGVNYIYGTGAVLPA-
LAAIGEDMTQPYITKACDWLVAHQQEDGGWGESCSSYMEIDSIGKGPTTPSQTAWALM-
GLIAANRPEDYEAIAKGCHYLIDRQEQDGSWKEEEFTGTGFPGYGVGQTIKLD-
DPALSKRLLQGAELSRAFMLRYDFYRQFFPIMALSRAERLIDLNN

>seq ID 5

MTVTSSASARATRDPGNYQTALQSTVRAAADWLI-
ANQKPDGHWVGRAESNACMEAQWCLALWFMGLEDHPLRKRLGQSLLDSQRPDGAWQVYF-
GAPNGDINATVEAYAALRSLGFRDDEPAVRRAREWIEAKGGLRNIRVFTRYWLALIGE-
WPWEKTPNIPPEVIWFPLWFPFSIYNFAQWARATLMPIAVLSARRPSRPLPPENRLDALFPH-
GRKAFDYELPVKAGAGGWDRFFRGADKVLHKLQNLGNRLNLGLFRPAATSRVLEWMIRHQDFD-
GAWGGIQPPWIYGLMALYAEGYPLNHPVLAKGLDALNDPGWRVDVGDATYIQATNSPVWD-
TILTLLAFDDAGVLGDYPEAVDKAVDWVLQRQVRVPGDWSMKLPHVKPGGWAFEYANNY-
YPDTDDTAVALIALAPLRHDPKWKAKGIDEAIQLGVDWLIGMQSQGGGWGAFDKDNNQKILT-
KIPFCDYGEALDPPSVDVTAHIIEAFGKLGISRNHPSMVQALDYIRREQEPSGPWF-
GRWGVNYVYGTGAVLPALAAIGEDMTQPYIGRACDWLVAHQQADGGWGESCASYMDV-
SAVGRGTTTASQTAWALMALLAANRPQDKDAIERGCMWLVERQSAGTWDEPEFT-
GTGFPGYGVGQTIKLNDPALSQRLMQGPELSRAFMLRYGMYRHYFPLMALGRALRPQSHS

>seq_ID 78

MTLTSSASARAPRDPGNYQTALQSTVRAAADWLI-
ANQKPDGHWVGRAESNACMEAQWCLALWFMGLEDHPLRKRLGQSLLDTQRPDGAWQVYF-
NAPNGDINATVEAYAALRSLGYPDSEPAVRRAREWIEAKGGLRNIRVFTRYWLALIGE-
WPWEKTPNIPPEVIWFPLWFPFSIYNFAQWARATLMPIALLSARRPSRPLPPENRLDTLF-
PRGRDAFDYELPVKANAGGWDKFFRGADKVLHALQNFGNRLNLGLFRPAATSRV-
LEWMIRHQDFDGAWGGIQPPWIYGLMALYAEGYPLNHPVLAKGLDALND-
PGWRVDVGEATYIQATNSPVWDTILTLLAFDDAGVLGDYPDAVD-
KAVNWVLARQVRVPGDWSMKLPHVKPGGWAFEYANNHYPDTDDTAVALIAL-
APLRHDPKWKAKGIDEAIQLGVDWLIGMQSQGGGWGAFDKDNNQQILTKIPFCDYGEALD-
PPSVDVTAHIVEAFGKLGISRNHPSMVQALDYIRKEQEPSGPWFGRWGVNYVYGTGAVLPA-
LAAIGEDMTQPYIGRACDWLVAHQQPDGGWGESCASYMDISAVGRGTTTASQTAWAL-
MALLAANRPQDKDAIERGCMWLVERQSAGTWDEPEFTGTGFPGYGVGQTIKLTDPS-
LQERLMQGPELSRAFMLRYGMYRHYFPLMALGRALRPQGHG

>seq ID 209

MDSILAPRADAPRNIDGALRESVQQAADWLVANQKPDGHWVGRAETNAT-
MEAQWCLALWFLGLEDHPLRVRLGRALLDTQRPDGAWHVFYGAPNGDINATVEAYAALRSLG-

HRDDEEPLRKARDWILSKGGLANIRVFTRYWLALIGEWPWEKTPNILPEVIWLPTWFPFSIYN-
FAQWARATLMPIAVLSAHRPSRPLAPQDRLDALFPQGRDSFNYDLPARLGAGVWDVIFRKID-
TILHRLQDWGARRGPHGIMRRGAIDHVLQWIIRHQDYDGSWGGIQPPWIYGLMALHTEGYA-
MTHPVMAKALDALNEPGWRIDIGDATFIQATNSPVWDTMLSLLAFDDAGL-
GERYPEQVERAVRWVLKRQVLVPGDWSVKLPDVKPGGWAFEYANNFYPDTDDTSVALMAL-
APFRHDPKWQAEGIEDAIQRGIDWLVAMQCKEGGWGAFDKDNDKKILAKIPFCDFGEALD-
PPSADVTAHIIEAFAKVGLDRNHPSIVRALDYLKREQEPEGPWFGRWGVNYVYGTGAVLPA-
LAAIGEDMRQPYIARACDWLIARQQANGGWGESCVSYMDAKQAGEGTATASQTAWALMA-
LIAADRPQDRDAIERGCLYLTETQRDGTWQEVHYTGTGFPGYGVGQTIKLND-
PLLSKRLMQGPELSRSFMLRYDLYRHYFPMMAIGRVLRQRGDRSGH

>seq ID 193

MNVIRQLNSGVNAAKSLDDGIESAIEWLAENQDKEGFWVGMLESNSCIEAEWILAMHLL-
GVKDDPKYDKVVQAILNEQREDGSWAVYYDAPAGDINATVEAYAALRTAGFGAGDERLIKARN-
WIFSHGGLKNVRVFTRYWLALIGEWPWDETPALAPEIIYLPAWCPLNIYDFACWARAT-
LVPLSVLSVRRPVKPLPAESRLDELFPEGRENADYSLPESEK-
GLAERFFLVVDWFLKKYNRLPMQFGREKAIRLCLEWIVRHQDYDGGWGGIQPPLIYSLIALN-
TEGYGINHPVISKGLDAFNPPWAYEKNGGVYLQCSESPVWDTLFTMLALFESGCSFDDTPMMR-
PALDWILSKQITSWGDWQVKVRGVRPGGWAFERANTAYPDVDDTALAL-
VVLAEARRHVKDSAAVDAALERAEEWILGLQCRNGGWAAFDRDNNSAIVTKIPFCDFGEVLD-
PPSVDVTAHVVEALAALGRDRHDPVVARALKYIRSEQEPGGSWFGRWGVNHIYGTCAVLPA-
LAAIGEDMRAPYVLRAADWLVRHQNDDGGWGESCASYMDDSQCGQGSSTASQTGWALMAL-
VAMSSHDYDEAIRRGLDYLLSHQKSGTWDEPQYTGTGFPGYGVGERTNLKEAGATLDQGCE-
LARGFMINYNMYRHYFPLIAMARARRHLGLAANPRHQDSRSSVEVAPEALRGRACG

>seq ID 246

MRRLDTFPPEIPTGSRDKPPSGEEHSCSTPAEPLRSRLDEGILRAVDWLVCDQHPDGFWAG-
MLQSNSCMEAEWVLAMHFLGIDDDPKYDGVIRAILGEQRADGSWGVFHKAPNGDINTTVE-
CYAALRASGLAPESAPLSSAREWILAGGGLANIRNFTKYWLALIGEWPWEGTPTIP-
PELIFFPPRMPLNIYHFASWARSTIVPLSILSARRPVRPLPEDRRLDELFPQGRSAFD-
FRLPRKDGWLSWEGFFHVCDRILRLYARTRRAPFRETAIRVCLEWIIRRQETDGAWSGIQPP-
WIYALLALHAEGYGLDHPILRAGLRAFDSHWSYERDGGIYLQASESPVWDTVLSLRALADCGE-
ERKASVSIASALEWLLNRQISVPGDWAVRVPSVPCGGWAFQRANSFYPDVDDTAVAIEV-
LARLRPFTANQSAVDRAIRSARDWVLAMQCSNGGWAAFDRDNDFKLVTKIPFCDFGELLD-
PPSVDVTAHVIEALAALGWDMTSREIEAAVSFIRREQEAEGSWFGRWGVNHIYGTATVLPAL-
RAIGEDMSSAYVLRAADWLASRQNADGGWGETPASYMDDSLRGVGESTASQTAWAIM-
GLVAVGSGAHDDTVRRGIDFLLFAQHGGTWEEPQYTGTGFPGYSVGERIRLRD-
MGASLKQGTELQRAFMINYNLYRHYFPLMALGRARYHLQLRRSAREGGNGETTPNGSAL

>seq ID 151

MKISKNPISHALTSFNDAARETADNSAARKSGKIHHLPATIWKKKESTVSSPLDIAI-
ERTQEFFFREQLPAGYWWAELESNATITAEYIMLFHFMGLVNREKERKMANYLLRQQTTE-
GYWTIWHGGPGDLSTTIEAYFALKLAGYPADHPSMSKARAFILEHGGILKARVFT-
KIFLALFGEFSWLGVPSMPIEMMLLPAGFTFNMYEFSSWSRATIIPLSIVMAERPVRKLPP-
WARVQELYVRPPRPTDYTFTKEDGILTWKNIFIGIDHVLKVYEASPIRPGRKKA-
MAIAEKWVLEHQEPTGDWGGIQPAMLNSVLALHVLGYANDHPAVAKGLQALANFCIEGEDEL-
VLQSCVSPVWDTALGLMAMVDSGVPTDHPSLSKAAQWLLDREVRRPGDWKIKCPDLEPGG-
WAFEFMNDWYPDVDDSGIVMMAIKNVKVKDQRAKEDTITRGIAWCLGMQSKNG-
GWGAFDKDNTKHILNKIPFADLEALIDPPTADLTGRMLELMGTYGYPKDHPAAVRAL-
KFIRETQEPDGPWWGRWGVNYIYGTWSVMSGLAAFGEDMSQPWIRKAVDWLVEHQNEDGGW-
GECCESYADPRLAGVGPSTASQTGWALLTLLAAGEVASSSVVRGVQYLLDTQKPDGTWDE-
DAFTGTGFPKFFMIKYHIYRNCFPLMALGRYRTLAGKGL

>seq_ID 142

MKSRKYPISHALTSFNHTTVAPVEAPAPISVKSPAKVHRLPSSIWKKMEGSAGNPLDKAVEL-
TRDFFFREQLPDGYWWAELESNVTITAEYIMLFHFLGMVDKDKERKMANYLLRQQTEE-
GYWTVWHNGPGDLSTTIEAYFALKLAGYHADHIALRKARDFILANGGILKSRVFTKT-
FLAMFGEFSWLGVPSMPIELMLLPDWAYLNVYEFSSWARATIIPMSVLMAN-
RPVYKLPPHARVQELYVRPPRPTDYTFTKEDGIFSLKNFFIGVDHLLKIYESSPIRPFK-
KRATEKVEQWILEHQEKTGDWGGIQPAMLNAILALHCLGYANDHPAVAKGLEALANF-
TIEDSDSLVLQSCISPVWDTALVLQAMQEASVPLDHPSLIKASQWLLDREVRIKGDWKIKSPD-
LEPGGWAFEFQNDWYPDVDDSTAVMIAIKDIKVKNTKARQDAIRRGIDWCLGMQSENGG-
WAAFDKDNTKHMLNKIPFADLEALIDPPTADLTGRMLELMGNFGYTKDHPQAVSALEFLKNE-
QEPEGPWFGRWGVNYIYGTWYVLIGLEAIGEDMNSPYIKKSVNWIKSRQNLDGGWGEVCD-

SYWDRTLMGCGPSTASQTSWALMALMAAGEVGCQAVERGIQYLLATQNSDGTWDEEAFT-
GTGFPKYFMIKYHIYRNCFPLTALGRYRRLTAGTHAQ

>seq_ID 152

MNSCKHPISHALTSFNGETADAAKKQPVKPGAKIHHLPASIWKKKEGESKSPLDIAIENSRD-
FFFREQLPDGYWWAELESNCTITAEYLMLYHFMGIVDQERERKMATYLLSKQTAEG-
FWTIYFGGPGDLSTTVEAYFALKLAGYPADHPAMAKARAFILDNGGIIKCRVFTKIFLALFGE-
FAWFGVPSMPIELILLPNWAYFNMYELSSWSRATIIPLSIVMTER-
PVRKLPPSSRVQELYVRPPRPIDYTFSKEDGIITWKNFFIGVDHILKVYESNPIRPFKKRALA-
TAENWVLDHQESTGDWGGIQPAMLNSVLALHCLGYANDHPAVAKGLEALANFCIETEDS-
LVLQSCISPIWDTALALKALVDSDVPTDHPALVKAAQWLLDKEVRKPGDWKIKCPELESGGWA-
FEFLNDWYPDVDDSGFVMMALKDVAVKDRKSMDGAIKRGINWCLGMQSKNG-
GWGAFDKDNTKYLLNKIPFADLEALIDPPTADLTGRM-
LELMGTFGYSKDYPAAVRALEFIKKNQEPEGSWWGRWGVNYIYGTWSVLGGLAAIGED-
LNQPYIRKAVNWLKSRQNMDGGWGETCESYHDTSLAGIGESTPSQTGWALLSLMSAGEANS-
STVARGIQYLIANQKSDGTWDEEQYTGTGFPKFFMIKYHIYRNCFPLTALGTYRKLTGGMA

>seq ID 146

MTSPFKHPISNALTSFNGNFAEPEQCVEQQTGAKVHHLPASIWKRKMGKAKSPLDVAIEGS-
RDFFFQEQLPKGYWWAELESNVTITAEYIMLFHFLGLVDRERQRKMSNYLLSKQTEEG-
FWPIYYGGPGDLSTTIEAYFALKLSGYPADHPALAKARAFILEQGGVVKSRVFTKIFLALFGE-
FEWQGVPSMPVELNLLPDWAYINIYEFSSWARATIVPLS-
VVMHSRPVRRVPPSARVQELFVRQPTAADYSFAKNDGIFTWENFFLGLDRV-
LKVYEKSPLRPFKNMALAKAEEWVLEHQEPTGDWGGIQPAMLNAVLALNVLGYQNDHPA-
VEQGLRALANFCIETEDQLVLQSCVSPVWDTALALKALLDAGVPPDHPSLVKGAQWLLD-
KEVTRPGDWRVKSPALEPGGWAFEFLNDWYPDVDDSGFVMIALKGIQVKDRKS-
MDAAIKRGINWCLGMQSKNGGWGAFDKDNTRHVLNKIPFADLEALIDPPTADLTGRM-
LELMGTFNYPITLPAAQRAIEFLKKNQEPEGPWWGRWGVNYLYGTWSVLCGLAAIGE-
DMDQPYIRKAVNWIKSRQNIDGGWGETCQSYHDRTLAGVGESTPSQTGWALLGLLAAGEMH-
SATVVRGVQYLISTQNSDGTWDEQQYTGTGFPKYFMIKYHIYRNCFPLMALGTYRTLTRTQP

>seq_ID 147

MSPCKHPISHALTSFNGETADSVPVQTPKTGAKIHHLPPSIWKKKEGELKSPLDIAIENSRD-
FFFREQLPDGYWWAELESNCTITAEYVMLYHFMDLVDRERERKMANYLLSKQTEEG-
FWTIYYGGPGDLSTTVEAYFALKLAGYPADHPAMVKARAFILDNGGIIKTRVFTKIFLALFGE-
FAWFGVPSMPIELILLPNWAYFNMYELSSWSRATIIPLSIVMTQRPVRKLPPAS-
RVQELYVRPPSPIDYTFTKEDGIFTWKNFFIGVDHILKVYESNPIRPFKKKAMLAAEN-
WVLEHQEATGDWGGIQPAMLNSVLALHCLGYANNHPAVAKGLEALENFCIESEDS-
LVLQSCISPVWDTALALKALVDSDVPNDHPALVKAAQWLLDKEIRKAGDWKVKSPELEPGGWA-
FEFLNDWYPDVDDSGFVMMALKDVAVKDRKSMDTAIKRGISWCLGMQSKNG-
GWGAFDKDNTKYLLNKIPFADLEALIDPPTVDLTGRMMELMGTFGYAKDYPPAVRALD-
FIKRNQEPDGSWWGRWGVNYIYGTWSVLCGLSAMGEDLNQPYIRKAINWLKSRQNIDGGWGE-
TCESYHDSSLAGIGASTASQTGWALLALMAVGEENASAVARGVQYLLATQKSDGTWDED-
LYTGTGFPKFFMIKYHIYRNCFPLTALGTYRRKTGGRAEMQVSEHNK

>seq ID 144

MKISKHPISHALTSFNETAKETKEEPQKKRGGKVHHLPASIWKKRDVET-
TSPLDQAIKRSQEFFLREQLPAGYWWAELESNVTITAEYVILFHFMGLVNRDKDRK-
MATYLLSKQTEEGCWCIWHGGPGDLSTTIEAYFALKLAGYPADHPAMQKARTFILGKGGIL-
KARVFTKIFLALFGEFSWLGVPSMPIEMMLLPNGFTFNLYEFSSWSRATIIPLSIVMA-
ERPVRKLPPWARVQELYVRPPRPMDYTFTKEDGILTWKNIFIGIDHILKVYEASPIRPGMKKA-
MAIAEQWVLDHQEPTGDWGGIQPAMLNSVLALHCLGYANDHPAVAKGLQALANFCIESDDEI-
VLQSCISPVWDTALALMAMVDSEVPTDHPALVKAAQWLLDREVRKVGDWKIKAPNLEPGG-
WAFEFQNDWYPDVDDSGIVMMAIKDVKVKDSKAKAEAIQRGIAWCIGMQSKNG-
GWGAFDKDNTKHILNKIPFADLEALIDPPTADLTGRMLELMGTFGYPKDHPAAVRALQFVKEN-
QEPDGPWWGRWGVNYIYGTWSVLCGLKAYGEDMGQPYVRKAVEWLAAHQNPDGGWGECCE-
SYCDQKLAGTGPSTASQTGWALLSMLAAGDVDHPAVARGIRYLIETQQPDGTWDEDQFT-
GTGFPKYFMIKYHIYRNCFPLMAMGRYRALKGHKG

>seq_ID 15

MAEQLVEAPAYARTLDRAVEYLLSCQKDEGYWWGPLLSNVTMEAEYVLLCHILDRVDRDRME-
KIRRYLLHEQREDGTWALYPGGPPDLDTTIEAYVALKYIGMSRDEEPMQKALRFIQSQG-
GIESSRVFTRMWLALVGEYPWEKVPMVPPEIMFLGKRMPLNIYEFGSWARATVVAISIV-
MSRQPVFPLPERARVPELYDTDVPPRRRGAKGGGGRIFDALDRALHGYQKLSVHPFRRAAEI-
RALDWLLERQAGDGSWGGIQPPWFYTLIALKILDMTQHPAFIKGWEGLELYGVDLDY-

GGWMFQASISPVWDTGLAVLALRAAGLPADHDRLVKAGEWLLDRQITVPGDWAVKRPNLKPGG-
FAFQFDNVYYPDVDDTAVVVWALNSLRLPDERRRRDVMTKGFRWIVGMQSSNG-
GWGAYDVDNTSDLPNHIPFCDFGEVTDPPSEDVTAHVLECFGSFGYDDAWKVIRRAVEY-
LKREQRPDGSWFGRWGVNYLYGTGAVVPALKAVGIDVREPFIQKALDWVEQHQNPDGGWGE-
DCRSYEDPAYAGKGASTPSQTAWALMALIAGGRAESDSVRRGVQYL-
VETQRPDGGWDEPYYTGTGFPGDFYLGYTMYRHVFPTLALGRYKQAIERR

>seq ID 16

MAEQLVEAPAYARTLDRAVEYLLSCQKDEGYWWGPLLSNVTMEAEYVLLCHILDRVDRDRME-
KIRRYLLHEQREDGTWALYPGGPPDLDTTIEAYVALKYIGMSRDEEPMQKALRFIQSQG-
GIESSRVFTRMWLALVGEYPWEKVPMVPPEIMFLGKRMPLNIYEFGSWARATVVALSIV-
MSRQPVFPLPERARVPELYETDVPPRRRGAKGGGGWIFDALDRALHGYQKLSVHPFRRAAEI-
RALDWLLERQAGDGSWGGIQPPWFYALIALKILDMTQHPAFIKGWEGLELYGVELDY-
GGWMFQASISPVWDTGLAVLALRAAGLPADHDRLVKAGEWLLDRQITVPGDWAVKRPNLKPGG-
FAFQFDNVYYPDVDDTAVVVWALNTLRLPDERRRRDAMTKGFRWIVGMQSSNG-
GWGAYDVDNTSDLPNHIPFCDFGEVTDPPSEDVTAHVLECFGSFGYDDAWKVIRRAVEY-
LKREQKPDGSWFGRWGVNYLYGTGAVVSALKAVGIDTREPYIQKALDWVEQHQNPDGGWGE-
DCRSYEDPAYAGKGASTPSQTAWALMALIAGGRAESEAARRGVQYL-
VETQRPDGGWDEPYYTGTGFPGDFYLGYTMYRHVFPTLALGRYKQAIERR

>seq ID 141

MTSPFKHPISNALTSFNGNVAEPEQSVEQQSGAKVHHLPASIWKRKMGRAKSPLDVAIEGS-
RDFFFQEQLPKGYWWAELESNVTITAEYIMLFHFLGLVDPERQRKMSTYLLSKQTEEG-
FWTIYYGGPGDLSTTIEAYFALKLSGYPEDHPALAKARAFILEQGGVVKSRVFT-
KIFLALFGEFDWQGIPSMPVELNLLPDWAYINIYEFSSWARATIVPLS-
VVMHSRPVRRVPPSARVQELFVRQPTAADYSFAKNDGLFTWEKFFLGLDRV-
LKVYEKSPLRPFKKTALAKAEEWVLEHQEPTGDWGGIQPAMLNAILALNVLGYRNDHPA-
VEQGLRALANFCIETEDQLVLQSCVSPVWDTALALKALLDAGVPPDHPSLVKGAQWLLD-
KEVTRAGDWRVKSPNLEAGGWAFEFLNDWYPDVDDSGFVMIALKGIQVKDH-
KAMDAAIKRGINWCLGMQSKNGGWGAFDKDNTKHVLNKIPFADLEALIDPPTADLTGRM-
LELMGTFDYPVTFPAAQRAIEFLKKNQEPEGPWWGRWGVNYLYGTWSVLCGLAAIGE-
DMDQPYIRKAVNWIKSRQNIDGGWGETCQSYHDRTLAGVGESTPSQTGWALLSLLAAGEMH-
SATVVRGVQYLISTQNSDGTWDEQQYTGTGFPKYFMIKYHIYRNCFPLMALGTYRTLTRTQP

>seq_ID 195

MNPAKYKISSSLTSLNAEPVEQAPLPAKRTGSKVHRLPPSIWKKMVAEAKSPLDKGIERTRD-
FFLREQLPDGYWWAELESNVTISAEYVMLFHFLGMVDRERERKLANYILAKQTSEG-
FWSLWHNGPGDLSTTIEAYFALKLAGYSADHPAMAKARAFVLANGGIIKARVFTKIFLALFGE-
FAWFGVPSMPIELMLLPDWAYFNMYEFSSWSRATIIPLSVVMSERPVRKLPPRA-
QVQELFVRPPRPTDYTITREDGLFTWKNFFIGADHLIKVYESSPIRPFKKRAVALAEN-
WILEHQEQSGDWGGIQPAMLNSILALHCLGYANDHPAVAKGLDALANFCIEDDDCIVLQSCV-
SPVWDTALALVALQEADVPADHPALVKAAQWLLNLEVRRKGDWQVKCPELEPGGWAFEFLND-
WYPDVDDSGFVMLSIKNIKVRDRKHREEAIKRGIAWCLGMQSENGGWGAF-
DRNNTKYLLNKIPFADLEALIDPPTADLTGRMLELMGNFDYPKSHPAAERALAFLK-
KEQESEGPWWGRWGVNYLYGTWSVLCGLEAIGEDMNQPYIRKAVNWIKSRQNNDGGWGEVCE-
SYFDRSLMGSGPSTASQTGWALLALMAAGEANSRAAAQGVKYLLETQNEDGTWDEDAFT-
GTGFPKFFMIKYHIYRNCFPLTALGRYRRLTAAKG

>seq ID 3

MTATTDGSTGASLRPLAASASDTDITIPAAAAGVPEAAARATRRATDFLLAKQDAEGWWKGD-
LETNVTMDAEDLLLRQFLGIQDEETTRAAALFIRGEQREDGTWATFYGGPGELSTTIE-
AYVALRLAGDSPEAPHMARAAEWIRSRGGIASARVFTRIWLALFGWWKWDDLPELP-
PELIYFPTWVPLNIYDFGCWARQTIVPLTIVSAKRPVRPAPFPLDELHTDPARPNPPR-
PLAPVASWDGAFQRIDKALHAYRKVAPRRLRRAAMNSAARWIIERQENDGCWGGIQPPA-
VYSVIALYLLGYDLEHPVMRAGLESLDRFAVWREDGARMIEACQSPVWDTCLATIAL-
ADAGVPEDHPQLVKASDWMLGEQIVRPGDWSVKRPGLPPGGWAFEFHND-
NYPDIDDTAEVVLALRRVRHHDPERVEKAIGRGVRWNLGMQSKNGAWGAFDVDNTSAFPNRL-
PFCDFGEVIDPPSADVTAHVVEMLAVEGLAHDPRTRRGIQWLLDAQETDGSWF-
GRWGVNYVYGTGSVIPALTAAGLPTSHPAIRRAVRWLESVQNEDGGWGEDLRSYRYV-
REWSGRGASTASQTGWALMALLAAGERDSKAVERGVAWLAATQREDGSWDEPYFT-
GTGFPWDFSINYNLYRQVFPLTALGRYVHGEPFAKKPRAADAPAEAAPAEVKGS

>seq ID 18

MTKQLLDTPMVQATLEAGVAHLLRRQAPDGYWWAPLLSNVCMEAEYVLLCHCLGKKN-
PEREAQIRKYIISQRREDGTWSIYPGGPSDLNATVEAYVALKYLGEPASDPQMVQAKE-

FIQNEGGIESTRVFTRLWLAMVGQYPWDKLPVIPPEIMHLPKSVPLNIYDFASWARATIVTL-
SYRHESPTCDATSGLCKGSGIVRGEGPPKRRSAKGGDSGFFVALDKFLKAYNKWPIQPGRKS-
GEQKALEWILAHQEADGCWGGIQPPWFYALLALKCLNMTDHPAFVKGFEGLEAYGVHTS-
DGGWMFQASISPIWDTGLTVLALRSAGLPPDHPALIKAGEWLVSKQILKDGDWKVRRR-
KAKPGGWAFEFHCENYPDVDDTAMVVLALNGIQLPDEGKRRDALTRGFRWLREMQSSNG-
GWGAYDVDNTRQLTKSDSIFATSGEVIDPPSEDVTAHVLECFGSFGYDEAWKVIRKAVEY-
LKAQQRPDGSWFGRWGVNYVYGIGAVVPGLKAVGVDMREPWVQKSLDWLVEHQNEDGGWGE-
DCRSYDDPRLAGQGVSTPSQTAWALMALIAGGRVESDAVLRGVTYLHDTQRADGGWDEE-
VYTGTGFPGDFYLAYTMYRDILPVWALGRYQEAMQRIRG

>seq ID 245

MNPIRGKRGSAADFLEEEYQWENLADHGESGRTPGGGHPAALKEYEAGSATEHTGHHCVHHL-
GVRNSWLRKIEKAIDNACGQLFKTQYEDGYWWSELESNVTITSEYIMLLYLLEVSRPEQQKS-
MVKYLLNQQRPDGSWGLYYGDGGNLSTTIEAYFALKLAGEHCESEPMRRAREFILSKGGIE-
SARVFTKIWLALFSQYDWDKVPSMPVELVLLPSSLYFNIYEFSSWARGTVVPLSIV-
MSIRPRCPLPAKCSIKELYVPGSKHKNFASCTHKLFFLFDRIAKAFERRPVPSLRN-
KAVQAAETWVLDHQEDSGDWGGIQPPMVYSVLALYYLGYPLDHEVIVKGIKALDAF-
CMEDEEGTRMQSCVSPVWDTALTVLSMLDAGVAAEHPGLEKAGRWLLENQVLT-
GGDWQIKNDSLPGGWAFEFYNTRYPDVDDSAVVLSTLNRFNAERVEGLEFAKCRGMEWCLS-
MQSSNGGWAAFDKDNTLEILNRIPFADQEAMVDYPTADVTGRVLEAMGYLGYDGSH-
PRARKAIQFLKKRQERDGCWWGRWGVNYIYGTWSVLKGLISIGED-
PRAAYIRAAVRWVKDHQNSDGGWGETCESYENPELRGQGPSTPSQTAWALMSLIACGEM-
KSQEASRGIQYLLRTQKRDGTWEELHFTGTGFPKHFYIRYHNYRNCFPLMALGQYLRALER

>seq ID 221

MTATTDGSTGALPPRAASASEPHDTIPQAAGSVGIQDAAARATQRATDFLLSRQDAEGWWKGD-
LETNVTMDAEDLLLRQFLGIQDEKTTRAAGLFIRGEQRADGTWATFYGGPGDLSATIE-
AYVALRLAGDGPDEPHMAKASAWIRERGGIASARVFTRIWLALFGWWKWDDLPELP-
PELIYFPKWMPLNIYDFGCWARQTIVPLTVVSAKRPVRPAPFPLDELHADANDPNPAK-
PLAPMVSWDGLFQRLDVALHTYRKVAPRRLRKAAMNTAARWIIERQENDGCWGGIQPPA-
VYSVIALYLLGYDLEHPVMREGLASLDRFAVWRDDGARMIEACQSPVWDTCLATIAL-
ADAGVPADHPQLVRAADWMLGEEIVRPGDWAVKRPQLPPGGWAFEFHND-
NYPDIDDTAEVVLALRRVKHHDPERLDNAIRRGVRWNLGMQSKDGGWGAFDVDNTSPFPNRL-
PFCDFGEVIDPPSADVTAHVVEMLAFEGLSHDPRTRRGIQWLLSAQEANGSWF-
GRWGVNYVYGTGSVVPALVAAGLPASHPAIRRAVTWLETVQNDDGGWGEDLR-
SYPEAAEWSGKGASTASQTGWALLALLAAGERESKAVERGIEWLAQTQRPDGSWDEPYFT-
GTGFPWDFSINYHLYRQVFPLTALGRYVNGEPLVEVKGG

>seq ID 160

MKGKEPTREELLSFSSGIQMDSSAENTTPVSTEELQEKVRLAAESLISRQVE-
EGYWVEPLEADVTITSEYILLQYLLGRERDEFFRRAAPFILESQGEDGGWPLYHGGPAEI-
SATVKAYLALKLLGYDADHPAMQRARALVLERGGAINVNVFTRITLALFGQYDWKGVPALP-
PEMILLPRWFPLSIYTVSYWSRTVIVPLLFIYHYKPLLELPPEKGVQELFITPMSEVRVHYA-
WDKHWVSWKNLFFVLDRILQAWNRHPPSFLRRKALKKAMEWMIPRLKGEGGLGAIYPAMANS-
VLALRLEGYAMDHPLVRRAIQSIDDLVFDLGEQQSVQPCHSPIWDTALALGALYEAGLDEGS-
PFVSRALDWFCRKEVRTVGDWSVRVPGVEAGGWAFQFENDYYPDIDDTSVVLMDFAKWVPEM-
GAYRDVFRRAIEWTLSMQGTDGGWGAFDKDNDFLFLNNIPFADHGALLDPSTSDVTGRVTELL-
GILGYDARTPVVRRALRFLRKEQEENGSWYGRWGVNYIYGTWSVVSALKAVGEDMSAPY-
VQKAMQFLFSRQNPDGGWGESCYSYFRKDTAGEGVSTSSQTAWALIALIHGGHVRHPA-
VSKGIDFLLSRQQADGKWLEQEYTGTGFPKVFYLRYNMYRDYFSLWALSLYRNVLLDGQSR-
VERLARRWKGNPYPVRSRFLA

>seq ID 161

MEGKDPTREELLSFTSGIQMDSRVGNTNPVSTEELQEKVRLAAESLIS-
RQGEEGYWVEPLEADITITSEYVLLQYLLGRERDEFFRRAAPFILESQGEDGGWPLYNGG-
PAEISATVKAYLALKLLGYDADHPAMQRARALVLERGGAINVNVFTRIT-
LALFGQYDWKGVPALPPEMILLPRWFPLSIYTVSYWSRTVIVPLLFIYHYKPLLEL-
PPEKGVQELFITPMSEVRVHYAWDKHWVSWKNLFFVLDRILQAWNRHPPSFLRRKALKKA-
MEWMIPRLKGEGGLGAIYPAMANSVLALRLEGYEMDHPLVRRAIQSIDDLVFDLGEQQSVQP-
CHSPIWDTALALGALYEAGLDEGSPFVSRALDWFCRKEVRTVGDWSVRVPGVEAGGWAFQFEN-
DYYPDIDDTSVVLMDFAKWVPEMGAYRDVFRRAIEWTLSMQGTDGGWGAFDKDNDFLFLN-
NIPFADHGALLDPSTSDVTGRVTELLGILGYDARTPVVRRALRFLRKEQEENGSWYG-
RWGVNYIYGTWSVVSALKAVGEDMSAPYVQRAMQFLFSRQNPDGGWGESCYSYFRKDTAGE-
GVSTASQTAWALIALIHGGHVRHPAVSKGIDFLLSRQQADGKW-
LEQEYTGTGFPKVFYLRYNMYRDYFSLWALSLYRNVLLDGQSRVERLSRRWKGTPYPVRSRFLA

>seq ID 240

MHEGEAMTATTDGSTGALPPRAAAASETHLDTPVAAGIQEAAVRAVQRATEHLLARQDAEG-
WWKGDLETNVTMDAEDLLLRQFLGIRDESTTRAAAKFIRGEQREDGTWAGFYGGPGELSTT-
VEAYVALRLDGDAPDAPHMAKASAWIRAQGGIAAARVFTRIWLALFGWWKWEDLPELP-
PELIYFPKWAPLNIYDFGCWARQTIVPLTIVSAKRPVRPAPFPLDELHADPADPNPAK-
PLAPVASWDGAFQRLDKAMHQLRKVAPRRLRRAAMNSAARWIIERQENDGCWGGIQPPA-
VYSVIALHLLGYDLQHPVMRAGLESLDRFAIWREDGSRMIEACQSPVWDTCLATIAL-
VDAGVPADHPQLVKAADWMLGEEIVRPGDWSVKRPQLPPGGWAFEFHND-
NYPDIDDTAEVVLALRRVRHHDPDRVENAIGRGVRWNLGMQSKNGAWGAFDVDNTSPFPNRL-
PFCDFGEVIDPPSADVTAHVVEMLAVEGLSHDPRTRRGIEWLLAEQEPDGSWF-
GRWGVNYIYGTGSVVPALTAAGLPASHPAIRRAVAWLEKVQNDDGGWGEDLR-
SYKYVKEWSGRGASTASQTAWALMALLAAGERDSKAVERGVEWLASTQRADGSWDEPYFT-
GTGFPWDFSINYHLYRQVFPLTALGRYVHGEPFSRTEAL

>seq_ID 231

MTATTDGSSGPVRAGAATAGDTTTTTAARTTAPGTDVREAAGRAAERAVEHL-
LARQDAQGWWKGDLETNVTMDAEDLLLRQFLGIQDAATVEASARFIRGQQRDDGTWATFYGGP-
GELSTTIEAYVALRLAGDRPDDPHMQRAASWVRSRGGIAAARVFTRIWLALFGWWKWDDL-
PELPPELILLPKWVPLNIYDFGCWARQTIVPLTVVSAKRPVRPAPFALDELHT-
DPAMPNPQKRFAPAASWDGFFQRADKALHLYHKVAPRRLRRAAMNAAARWIIERQENDGCWG-
GIQPPAVYSVIALHLLGYDLEHPVMRAGLESLDRFAVHREEEGLPVRMIEACQSPVWDTCLA-
TIALADAGLPADHPALVKAADWMLSEQIVRPGDWAVRRPGLGPGGWAFEFHND-
NYPDIDDTAEVILALRRVKHPDPERVEAAVARGTRWNLGMQSLNGAWGAFDADNTSPFPNRL-
PFCDFGEVIDPPSADVTAHVVEMLAHEGMAEDPRTRRGVRWLLREQEANGAWF-
GRWGVNYVYGTGAVVPALIAAGLPASHPSVRRAVTWLESVQNEDGGWGEDLRSYREEQSIGR-
GASTASQTGWALLALLSAGERDGRAVERGVAWLARTQRPDGSWDEPYFTGTGFPWDFSI-
NYHLYRQVFPLTALGRFLHGEKPVGRAAAREGG

>seq ID 227

MTATTDGSTGAANPSEATAHDPTDTTTAADDLTVAARRAAERSVEHLLGRQDEQGWWKGD-
LATNVTMDAEDLLLRQFLSIQDPETTRAAALFIRGEQLGDGTWNTFYGGPGDLSATIE-
AYVALRLAGDRPDEPHMARAAGWIRDQGGIAAARVFTRIWLALFGWWKWDDLPELPPELM-
FFPKWVPLNIYDFGCWARQTIVPLTIVSAKRPVRPAPFALDELHTDPDHPNPPRKLAPPTS-
WDGLFQRLDKGLHLYHKVAPRPLRRVAMNLAARWIIERQENDGCWGGIQPPAVYSVIALHLL-
GYDLDHPVMKAGLASLDRFAVRREDGARMIEACQSPVWDTCLATIALADAGLRPDHPAL-
VKAADWMLAEEITRPGDWSVRKPELAPGGWAFEFHNDNYPDIDDTAEVVLAL-
RRVRHPDPARLQAAIDRGVRWNLGMQSRNGAWGAFDADNTSPFPNRLPFCDFGEVIDPPSAD-
VTGHVVEMLAVEGLASHPRTREGIEWLLAEQEACGAWFGRWGVNYVYGTGSVVPALITAGL-
PAGHPAIRRAVAWLESVQNDDGGWGEDLRSYQEEKWIGHGESTASQTA-
WALLALLAAGRRDTRPVARGVTWLTEAQQADGSWDEPYFTGTGFPWDFSINYHLYRQVF-
PLTALGRYVHGDPFADRAMAAEGA

>seq_ID 121

MQTQNRVTSTQKVELSNLTKAIIASQNYIMSRQYPEGYWWGELESNITLTAETILLH-KIWKTDKTRPFHKVETYLRRQQNEQGGWELFYGDGGELSTSVEAYMALRLL-GVTPEDPALIRAKDFILSQGGISKTRIFTKFHLALIGCYDWKGIPSIPPWIMLFPDNFPFTIY-EMSSWARESTVPLLIVFDKKPIFEIEPAFNLDELYAEGVENVKYALPRNHNWSDIFLGLD-KLFKWTEKNNLVPFHKKSLQAAERWMLNHQQESGDWGGIMPPMVNSLIA-FKVLNYDVADPSVQRGFEAIDRFSIEEEDTYRVQACVSPVWDTAWVIRALVDSGLKPDHPS-LVKAGEWLLDKQILEYGDWAIKNKQGKPGGWAFEFINRFYPDLD-DSAVVVMALNGIKLPDENCKKAAINRCLEWMATMQCKPGGWAAFDVDNDQAWINEIPYGDLKA-MIDPNTADVTARVLEMVGSCGLKMDENRVQKALFYLEKEQESDGSWFGRWGVNYIYGTSGVL-SALAVIAPNTHKPQMEKAVNWLISCQNEDGGWGETCWSYNDPSLKGTGVSTASQTAWALIGLL-DAGEALETLATDAIKRGINYLLDTQTPDGTWEEAEFTGTGFPCHFYIRYHLYRHYFPLIAL-GRYWKIGLKNLKG

>seq ID 120

MQTQNRVTSTQKVELSNLTQAIIASQNYILSRQYPEGYWWGELESNITLTAETVLLH-KIWKTDKTRPFHKVETYLRRQQNEQGGWELFYGDGGELSTSVEAYMALRLL-GVTPEDPALIRAKDFILSKGGISKTRIFTKFHLALIGCYDWKGIPSIPPWIMLFPDNFPFTIY-EMSSWARESTVPLLIVFDKKPIFEIEPAFNLDELYAEGVENVKYALPRNHNWSDIFLGLD-KLFKWTEKNNLVPFHKKSLQAAEKWMLNHQQESGDWGGIMPPMVNSLIA-FKVLNYDVADPSVQRGFEAIDRFSIEEEDTYRVQACVSPVWDTAWVIRALVDSGLKPDHPS-LVKAGEWLLDKQILEYGDWAIKNKQGKPGGWAFEFINRFYPDLDDSAVVVMALNGIKLPDEN-

RKKAAINRCLEWMATMQCKPGGWAAFDVDNDQAWINEIPYGDLKAMIDPNTADVTARV-LEMVGSCGLKMDENRVQKALFYLEKEQESDGSWFGRWGVNYIYGTSGVLSALA-VIAPNTHKPQMEKAVNWLISCQNEDGGWGETCWSYNDSSLKGTGISTASQTAWAIIGLLDA-GEALETLATDAIKRGIDYLLATQTPDGTWEEAEFTGTGFPCHFYIRYHLYRHYFPLIALGRY-WKIGLKTPSVIPLN

>seq_ID 132

MFQGSDRPPVTLVMNDMRGPDMNVSDTVSVTRESIPTQTSAGDATARDLTAAVGSELTRALR-LATDHLLALQDGTGWWKFDLETNTSMDAEDLLLREYLGIRTTEVTAASARFIRSRQSDDGS-WPQYFGGPGELSTTVESYIALRLAGDDASAPHMLSAATWVRD-HGGVPATRVFTRIWLALFGWWRWEDLPALPPEIMLLPRRAPLNIYSFGSWARQTLVSLTVV-SALRPVRPAPFDLDELYPDGPASAWSGAGPSNVLERISTRFTAKEIFLGIDRLLHVYHR-RPVRSMRNHALRAAERWIIARQEADGCFGGIQPPAVYSIIALRLLGYELDHPVLKAALRALD-DYSVTLPDGSRMVEASQSPVWDTALAVNALADAGATAAIAPDHPALVRAAGWLLGQEVRHRR-GDWAVNHPDVPASGWAFEFENDTYPDTDDTAEVLLALRRVRHPARDELDAAER-RAVAWLFGLQSSDGGWGAYDADNTSTIPYQIPFADFGALTDPPSADVTAHVVELLAEA-GLGGDDRTRRGVDWLLDHQEADGSWFGRWGVNYVYGTGSVMPALRAAGLEPSHPAMRAGAD-WLLTHQNADGGWGEDLRSYTDPEWSGRGESTASQTAWAMLALLTVGDQPEVSGALARGAR-WLADHQRPDGSWDEDQFTGTGFPGDFYINYHGYRLLWPIMALGRYLRG

>seq ID 118

MLTYKEYRRSVTEIAMQTRDRQTQKPALSLNDAITASQNYLLSLQYPQGYWWAELESNIT-
LTAETVLLHKIWGTDKTRPLHKVEAYLRQQREQGGWELFYGDGGEISTSVEAYMALRLL-
GVPQDDPALIRAKDFILSKGGISKTRIFTKFHLALIGCYSWKGIPSIPPWIMLFPNSFPFTIY-
EMASWARESTVPLIIVFNDKPVFAVDPIFNLDELYAEGIENVKYELPKNNNWGDIFLGLD-
KVFKFAEQVDLVPFRKKGLQAAERWMLNHQQETGDWGGIMPPMVNSLLAFRVLNYDVND-
PSVQRGFEAIDRFSIEENETYRVQACVSPVWDTAWCVRALTNSGLPKDHFSLVKAGKWL-
LEKQCLEYGDWAVKNKTGKPGGWAFEFTNRFYPDIDDSAVVVMALNGIKLPDEARKQAAIN-
RCVKWIETMQCKEGGWAAFDVDNDQAWLNEVPYGDLKAMIDPNTADVTARVVEMVGSCD-
LEISSKRLNKALNYLYKEQEKDGSWFGRWGVNYIYGTSGVLSALAVINPEKHQPQIEQGIN-
WLLSCQNKDGGWGETCWSYNDSNLKGKGISTASQTAWALIGLLDAGEALNHFETDSIQRGI-
SYLLNTQTEEGTWEESEFTGTGFPCHFYIRYHFYRHYFPLIALGRYQNLSSEFGIRNSEL

>seq ID 230

MTATTDGSSGPLRGGAATAGETTSTSAARTTEPGTDLREAAARAAERAVEHLLARQDAEG-
WWKGDLETNVTMDAEDLLLRQFLGIQDPATVGASARFIRGQQRDDGTWATFYGGPGELSTT-
VEAYVALRLAGDRPDDPHMQRAASWVRSRGGIAASRVFTRIWLALFGWWKWEDLPELP-
PELIFLPKWFPLNIYDFGCWARQTIVPLTVVSAKRPVRPAPFALDELHTDPALPNPGKRLA-
PAASWDGFFQRADKALHAYHKVAPRRLRRAAMNAAARWIIERQENDGCWGGIQPPAVYSVI-
ALHLLGYDLEHPVMRAGLESLDRFAVHHEEEGLPVRMIEACQSPVWDTCLATIALADAGL-
PADHPALVKAADWMLSEQIVRPGDWSVRRPGLPGGWAFEFHNDNYPDIDDTAEVVLAL-
RRVKHPDPERVDAAVARGTRWNLGMQSRDGAWGAFDADNTSPFPNRLPFCDFGEVIDPPSAD-
VTAHVVEILAHEGMAHDPRTRRGVRWLLAHQEANGAWFGRWGVNYVYGTGA-
VVPALTAAGLPGSHPAIRRAVAWLESVQNEDGGWGEDLRSYREEKSIGRGVSTASQTG-
WALLALLAAGERESKAVERGVAHLAQTQAPDGSWDEPYFTGTGFPWDFSINYHLYRQVF-
PLTALGRYVHGEKLPGRAGAREGR

>seq ID 234

MHEGEAMTATTDGSTGAATPPATTASAPLHLSPEARETHEATARATRRAVDFLLARQSDEG-
WWKGDLATNVTMDAEDLLLRQFLGIRDEATTRAAALFIRGEQQEDGTWNTFYGGPGDLSA-
TIEGYVALRLAGDSPEAPHMRKASAFVRAQGGVARARVFTRIWLALFGWWKWEDLPEMP-
PELMFFPKWAPLNIYDFGCWARQTIVPLTVVCAQRPVRPAPFALEELHTDPADPDPAQPAPP-
VVSWDNVFHKLDKLLHGYRRIAPRRVREAAMRAAATWIVERQENDGCWGGIQPPAVYSI-
MALNLLGYDLDHPVLRAGLASLDRFAVWREDGARMIEACQSPVWDTCLATVALADAGVPAD-
HPQMIKAADWMLAEQIVRPGDWVVRRPDLPPGGWAFEFHNDNYPDIDDTAEVVLALRR-
VAHPDATRVDKAVRRAVDWNVGMQSKNGAWGAFDADNTSPFPNRLPFSDFGEVIDPPSAD-
VTAHVVEMLAEEGLAHHPRTRRGIEWLLKNQEGNGSWFGRWGVNYVYGTGAVVPALVAAGL-
PASHPAIRRSVSWLGQVQNEDGGWGEDLRSYQDSAWHGRGHSTASQTAWALLALLAAGE-
RETEQVRRGIAYLVETQTEDGTWDEPWFTGTGFPWDFTINYHLYRQVFPVTALGRYLNGTGPGEN

>seq ID 123

MQTRDRQTHKPALSLNDAITASQNYLLSLQYPQGYWWAELESNITLTAETVLLH-
KIWGTDKTRPLHKVEAYLRQQQREHGGWELFYGDGGEISTSVEAYMALRLLGVPSND-
PALIRAKNFIISQGGISKTRIFTKFHLALIGCYSWKGIPSIPPWIMLFPNSFPFTIYEMASWA-
RESTVPLIIVFNDKPVFAIDPIFNLDELYAEGIENVKYELPKNNNWGDLFLGLDKVF-

KLAEQVDLVPFRKQGLQAAERWMLDHQQETGDWGGIMPPMVNSLLAFRVLNYDVADPSVQRG-
FEAIDRFSIEENDTYRVQACVSPVWDTAWCIRALTDSGLPKDHFSLVKAGKWL-
LEKQVLEYGDWAVKNKTGKPGGWAFEFTNRFYPDIDDSATVVMALNGIKLPDEALKQAAIN-
RCLKWIETMQCKAGGWAAFDVDNDQAWLNEIPYGDLKAMIDPNTAD-
VTARVVEMVGSCDLEMSSDRLNKALDYLYEEQEKDGSWFGRWGVNYIYGTSGVLSALAVIN-
PKQHKSQIEQGMNWLLSCQNEDGGWGETCWSYNDLSLKGKGVSTPSQTAWALIGLLDAGEV-
LNHFETDSIERGINYLLNTQTEEGTWEESEFTGTGFPCHFYIRYHFYRHYFPLIALGRYQQMLGS

>seq ID 10

MTQASVREDAKAALDRAVDYLLSLQDEKGFWKGELETNVTIEAEDLLLREFLGIRT-
PDITAETARWIRAKQRSDGTWATFYDGPPDLSTSVEAYVALKLAGDDPAAPHMEKAAAYIR-
GAGGVERTRVFTRLWLALFGLWPWDDLPTLPPEMIFLPSWFPLNIYDWGCWARQTVVPLTIV-
SALRPVRPIPLSIDEIRTGAPPPPRDPAWTIRGFFQRLDDLLRGYRRVADHGPARLFRRLAM-
RRAAEWIIARQEADGSWGGIQPPWVYSLIALHLLGYPLDHPVLRRGLDGLNGFTIREETAD-
GAVRRLEACQSPVWDTALAVTALRDAGLPADHPRVQAAARWLVGEEVR-
VAGDWAVRRPGLPPGGWAFEFANDNYPDTDDTAEVVLALRRVRLEDADQQALEAAVRRATT-
WVIGMQSTDGGWGAFDADNTRELVLRLPFCDFGAVIDPPSADVTAHIVEMLAALGMRDHPAT-
VAGVRWLLAHQEPDGSWFGRWGANHIYGTGAVVPALIAAGVSPDTPPIRRAIRW-
LEEHQNPDGGWGEDLRSYTDPALWVGRGVSTASQTAWALLALLAAGEEASPAVDRG-
VRWLVTTQQPDGGWDEPHYTGTGFPGDFYINYHLYRLVFPISALGRYVNR

>seq ID 233

MRRRRSPRGPGAGPEADYGPARASAPDRLRGDAARGDAARRVQDATARAIRNLLGRQDPAG-
WWKGDLETNVTMDAEDLLLRQFLGIRDEAVTQAAALFIRREQREDGTWATFHGGPPELSATIE-
AYVALRLAGDAPDAPHMATASAWIRAHGGLAAARVFTRIWLALFGWWDWENLPELPPELVLL-
PPWVPLNIYDFGCWARQTIVPLTVVSAMRPVRPAPFALDELHTDARVPVPPRRMAPPTT-
WNGAFQWMDRALHVYRRFAPRRLREAAMASAGRWIIERQENDGCWGGIQPPAVYSVIALHLL-
GYDLGHPVMRAGLESLDRFAVWREDGSRMIEACQSPVWDTCLAAIALADAGVRPDHPAL-
VKAADWMLGEEIVRTGDWAVRRPGLAPGGWAFEFHNDTYPDIDDTAEVVLALRRIRHPDPAR-
VEAAIARGVSWNLGMQSRGGAWGAFDADNTSPFPNRLPFCDFGEVIDPPSADVTAHVVEM-
LAAEGRAADPRTRRGIAWLLAEQEPEGPWFGRWGTNYVYGTGSVVPALTAAGLSPGH-
PAIRRAVLWLESVQNPDGGWGEDQRSYQDRAWAGKGESTPSQTAWALMALLSAGERDAKTVER-
GIAYLVETQLADGGWDEPHFTGTGFPWDFSINYHLYRHVFPLTALGRYLYGEPFGHDGRHI-
GAHLGDRTGVPAEGV

>seq_ID 116

MQTQDRLTQKQPLSLKDAITASQNYLLSLQYPQGYWWAELESNITLTAETVLLH-
KIWGTDKTRPLHKVEAYLRQQQREHGGWELFYGDGGEISTSVEAYMALRLL-
GVPQDDPALIRAKDFIISKGGISKTRIFTKFHLALIGCYDWKGIPSIPPWIMLFPDSFPFTIY-
EMASWARESTVPLIIVFNDKPVFSVDPVFNLDELYAEGVENVKYELPKNNNWGDIFL-
GIDQVFKFAEQVDLVPFRKEGLKAAEKWILNHQQETGDWGGIMPPMLNSLLAFRTLNYDVND-
PSVKLGFEAIDRFSIEEDDTYRLQACVSPIWDTAWCVRALTDSGLEKDHFSLVKAGKWLLD-
KQVMEYGDWAVKNKAGKPGGWAFEFTNRFYPDLDDSATVVMALNGIKLPDEARKQAAIN-
RCLQWIETMQCKEGGWAAFDLNNDQAWLNEVPYGDLKAMIDPNTADVTARVVEMLGSCDLEI-
ESDRLNKSLNYLYKEQEKDGSWFGRWGVNYIYGTSGVLSALAVINPEKHKTQMEQGINWLL-
SCQNKDGGWGETCRSYNDPSLKGKGVSTPSQTAWSLIGLLDAGEALNKFETDAIERGVNYLL-
DTQTEEGTWEESEFTGTGFPCHFYIRYHFYRHYFPLIALGRYQNLSSEFGVRS

>seq_ID 124

MQIRATVDTAKLEKAIAASQEHLLSTQYPEGYWWAELESNVTMTAEVVLLHKIWKTDGTRPMH-
KAEKYLRSEQREHGGWELFYGDGGDLSTSVETYTALRLLGVPASDPALLKAKDFILRRG-
GISKTRIFTKLHLALIGCYDWRGLPSLPPWVMLLPENFPFTIYELSSWARGSTVPLLIVM-
DRKPVFSVNPQINVDELYAEGRDRVKFELPRKGDWTDLFIELDGLFKFTEQNNLVPFREEGL-
RAAERWVLERQEATGDWGGIIPAMLNSLLALRALGYHPADPYVRRG-
MAAVDRFAIETADTYRVQPCVSPVWDTALVMRGLIDSGLPADHPAIVKAGEWL-
LEKQILAYGDWAVKNKTGQPGAWAFEFENRFYPDVDDSAVVVMALQAAQLPDEDLKQQAI-
ERCVKWIATMQCKPGGWAAFDVDNDQDWLNQIPYGDLKAMIDPNTADVTARVLEMIGRSGVTT-
GEASVERALAYLRREQEVEGCWFGRWGVNYIYGTSGVLAALALIAPKSDHAMIQRGADWLV-
RCQNADGGWGETCRSYNDPHLKGQGPSTASQTAWALIGLLAAGEATGEFAWGAIDRGI-
NYLLATQQQDGRWDEDWFTGTGFPGHFYLKYHLYQQHFPLTALGRYSSLTGLKQEL-
KIPLQLKSKPEVVMIEDSDLLSDEDAT

>seq_ID 119

MQIQDRNSSPQVTEVLNQVKDAIAASQDYLMSIQYPEGYWWAELESNVTITAEVVLLH-
KIWGTDKTRPLHKVETYLRRQQREHGGWELFYGDGGDLSTSVEAYMALRLLGVSIDDPALIRG-

REFILKRGGISKSRIFTKLHLALIGCYDWRGIPSIPPWIMLLPENFPFTIYEMSSWARS-
STVPLLIVFDKKPVYCCDPTINLDELYSEGIENVKYDLPKTGDWTDIFVWLDGVFK-
FAQDYNLVPLRQESLQAAERWVLERQEDSGDWGGIIPAMLNSLLALRALNYEAVDPIVHR-
GLQSVDNFAIETEDTYHVQPCISPVWDTAWAIRALVESGLKADDPRLVKGAQWLLDKQILDY-
GDWAVKNKQGTPGGWAFEFDNRWYPDLDDSAVVVMALDQVKMPNED-
LKNGAIRRCVRWMATMQCKDGGWGAFDLDNDQNWLNFLPYADLKAMIDPNTSDVTARV-
LEMLGTCGLIMDSNRVQKAIAYLEKEQEPDGSWFGRWGVNYIYGTSGVLSALA-
VIAPETHQKELKKGAAWLVGCQNADGGWGETCFSYNDSSLKGKGDSTASQTAWGLIGLLAA-
GEATGEFFKTAIERGVNYLLKTQREDGTWDENYFTGTGFPCHFYLKYHLYLQYFPLIALSRYQRLLT

>seq_ID 9

MSVSERAQPGGNPIPGSTSQSAVKFGRIDAALEDVKRAIAGAK-
DRVFAQQSKDGWWCGELEADSMLEADYIFAHTLLGTGDAGKMKRALTEMLRYQNEDGS-
WSIYPGGPGNISLTVKCYFSAKLMGMTADNPILVKAREWILAHGGVVECNTFT-
KIYLCFLGQYEYDAVPAIPPEIVLFPNWFYFNIYEISSWSRAILVPLSIAYAKKPFKKIP-
PEQGIDELFVGGREKANLHLRWDSKNLLSWRNFFLALDRVTHWFERVHIRPLRSIALKKA-
EKWMLARFEMSDGLGAIYPAMLNAIIALRCLGYSLDDPQVLRAMDEFEKLGIDEPEG-
TAEYAEPTFRMQPCVSPVWDTAQAVFALGEAGVPRNDPRMQKAAD-
WLLSKEVRHKGDWAMKVRNAQPGGWYFEFNNEFYPDVDDSAQVLLALNKVDN-
PRERYQYDVCQRAIDWIFAMQCRNGGWASFDKDNTKMIFQYVPFADHNAMLDPPTVDITGRI-
LEMLATYGYTRKDRRVEKAIKFIYDEQEPDGSWFGRWGVNYLYGTFLVLRGLEAIGVWN-
HEPQIQQAAEWIRSVQNADGGWGETCGSYDDPNTRGVGPSTPSQTAWAILGLLSAGDDRSDS-
VAKGIKWLLAHQKPDGGWDESTGSGSKHQALYTGTGFPRVFYLAYHQYRDYFPLLALTNYEKAMERGE

>seq ID 217

MTEEVLQRTAAPAEVLAAAREHLLSLQHERGWWKGELETNVTMDVEDLLLRRFLGILT-
TAETEQAARWIRSRQRADGTWAQFHGGPGDLSTTVEAYVGLKLAGDDVDSEHMAAARA-
WILERGGIEETRVFTRIWLALFGEWSWDDLPAMPPELVLLPPWVPLNLADWGCWARQTIV-
PLTVVCTLRPRRDLGVGLAELRSGRRRRKVPSPSWAGAFQVLDGALHGYQRHPLRGLREHAM-
RRAAEWIVARQEADGSWGGIQPPWVYSLLALHLLGYPLDHPVLRQGLAGLERFLIREET-
PEGTVRRLEACQSPVWDTVLSMQALRDAGLAADHPALRRAADFVLAEEIRVKGDWSVRRPD-
LAPGGWAFEFDNDGYPDIDDTAEVVLALNRVDHERPGAVNAAIDRGVRWMSGMQSADGGWGAF-
DADNTRELVNELPFCDFGAVIDPPSADVTAHVVEALCVLGRGDGEAVRRGVRWLLDHQELD-
GSWFGRWGANHVYGTGAAVPALVRAGLRRDHLALRRAVRWLEVHQNDDGGWGEDLR-
SYDDPVWVGRGRSTASQTAWALLALLAVDLHDTDAVRRGVGFLAETQRPDGTWDEPQFT-
GTGFPGDFYINYHLYRLVFPVTALGRYEQARREQSGGSG

>seq ID 249

MIEKNKVKQSILASQKHLLSLQETEGYWWGQLESNVTITAEIILLHKIWQTDKKIPLNKAKNY-
LISQQREHGGWELFYGDGGDLSTSIEAYMALRLLGVSRTDPIMIEAQNFIIKKG-
GISCSRIFTKLHLALIGCYSWQGIPSIPSSIMLLPEDFPFTIYEMSSWARSSTVPLLIV-
FDKKPIFSVNPTINLDELYAEGINNASFELPRKYDLTDLFLGLDKAFKFAENLNLM-
PLQQEGLKAAEKWILERQEVTGDWGGIIPAMLNSMLALKCLEYDVADPVVVRGLEAIDRFAIE-
NEDSYRVQACVSPVWDTAWVIRSLVDSGISPSHPAMVKAGQWLLQQQILDYGDWVFKNK-
FGKPGGWAFEFMNRFYPDIDDTAVVVMALDVVELPDEDLKGKAIARGMEWIASMQCEAGG-
WAAFDVDNNQDWLNATPYGDLKAMIDPNTADVTGRVLEMVGCCGLAMDSWRVKRGIDFLVRE-
QEEEGCWFGRWGVNYIYGTSGVILALAVMARESHRGYIERGASWLVGCQNSDGGWGE-
SCWSYNDPSLKGKGKSTASQTAWALIGLLAAGEGTGNFARDAIDGGVGFLVSTQNDDGSWLE-
DEFTGTGFPGHFYIKYHFYSQYFPLMALGRYESLLSG

>seq ID 222

MAVRDRVNPKTLEAAIAASQSYLLTQQDETGYWWAELESNVSITSEVVLLHKIWGTDRS-
RPLEKVETYLRSQQRDHGGWELYFDDGGEISVSVEAYMALKLLGVPMEDPAMVRARQFILEHG-
GISRTRVFTKLHLALIGCYEWRGIPSLPPWVMLLPEQFPFTIYEMSSWARGSTVPLLIVM-
DREPVYAVEAGFNLDELYVEGRHRAQFDLPLSNEWTDAFIYLDGLFKFAESTNLVPF-
REEGIRAAERWILERQEATGDWGGIIPAMLNSLLGLKALDYDVHDPIIERGMAALDAFALE-
TEDQYWIQPCISPVWDTALVVRGLAESGLAPDHPALVKAGEWLLNKQILDY-
GDWSVKNPGGLPGGWAFEFDNRFYPDVDDTAVVVMALNEVQLPDEQAKDAAIARAVN-
WIATMQCRPGGWAAFDINNDQDWLNALPYGDLKAMIDPNTADVTARVLEMIGRCHQTTGKNS-
VDRALRYLRTEQEPEGCWFGRWGVNYIYGTSGVLAALALIDPQGWQSQIQQAAAWLV-
SCQNTDGGWGETCASYDNPKLKGQGPSTASQTAWAIMGLLSAGEATSVYAEAAIERGVNYLT-
TTQKMDGTWDEDYFTGTGFPGHFYLKYHLYQQHFPLTALGRYQAMLQQKS

>seq_ID 186

MRTQDRVQVNSIAEAIAASQKYLLSLQNPAGYWWAELESNVTITAEVVLLH-
KIWGTDKTRPLHKVEAYLRSQQKQHGGWELFYGDGGELSTSVEAYMALKLL-
GVPATDPAMIQARDFILQRGGISKTRIFTKFHLALIGCYNWRGLPSLPAWVMLLPNQFPVNIY-
EMSSWARSSTVPLLIVFDQKPVYQVNPTITLDELYAEGVENVRYELPRSGDWTDLFLTLDEG-
FKLAESFNFIPFREEGIKAAEKWIIERQEATGDWGGIIPAMLNSMLALRSLGYDTNDPIVER-
GLQALDNFAIETVDCYRVQPCVSPVWDTAWVIRALIDSGIAPDHPAIVKAGEWLLQKQILDY-
GDWNVKNRQGKPGAWAFEFENRFYPDVDDTAVVVMALHAAKLPNEQLKQKACDRALQWVAS-
MQCKPGGWAAFDLDNDQDWLNSVPYGDLKAMIDPNTADVTARVIEMLGACNLSIDSHNLERAL-
TYLLNEQEAEGCWFGRWGVNYIYGTSGVLSALALINPQKYQRHIQQGATWLVGCQNPDGGW-
GETCFSYNDPSLKGQGDSTPSQTAWALIGLIAAGEATGNFAHDAIERGINHLVSTQQPDGS-
WFEAYFTGTGFPCHFYLKYHYYQQYFPLIALGRYQAIKSL

>seq ID 153

MQVQPRIEKKHLDSAIEASQAYLLARQYSPGYWWAELESNVSMTAEVVLLHKIWRTDTGRPLA-
KATAHLLAEQRAHGGWELFYGDGGDLNTSIEAYMALKLLGLTADHPALARARAFILAKGGIS-
RARIFTKIHLALIGCYDWRGVPSIPPWVMLLPEAFPVNIYEMSSWARGSTVPLLIVF-
DRKPVFAVEPAITLDELFVEGRAQARFDLPRSSSDWWANLFVDLDWGFKLAESLGAVPL-
REEGLKAAERWVLERQEATGDWGGIIPAMLNSLLALRCLDYDPHDPVVERGMAAVDRFAIE-
TESTYRLQPCVSPVWDTALTMRALVDSGLPPDHPALAAAGTWLLKKQILDYGDWAVKNRT-
GPPGGWAFEFDNRFYPDVDDTAVVVMALDAVRLADETAKGQAIARAVCWVASMQCRGGGWAAF-
DIDNDAHWLNSLPYADLKAMIDPNTADVTARVLEMYGRCRLIPAAAGAQRALDYLRRTQEP-
EGCWFGRWGVNYLYGTSGVLSALAAFAPAERTAIERAAAWLRGCQNTDGGWGETCG-
SYVDRTLMGQGPSTASQTAWALLGLIDASRVARFSDSSALERGLAYLVETQKADGS-
WDEPYFTGTGFPGHFYLKYHLYQQHFPLSALGRYRRLLS

>seq_ID 122

MQIQARNISTKVTEVFSKVKEAIAASQQYLLSIQYPEGYWWAELESNVTITAEAVLLH-
KIWGTDTTRPLHKVETYLRRQQREHGGWELFYGDGGDLSTSVEAYMALRLLGVSASDPALVRA-
KAFILSRGGISKSRIFTKMHLALIGCYDWRGVPSIPPWIMLLPENFPFTIYEMSS-
WARGSTVPLLIVFDKKPVYQCGITLDELYSEGINHVRYDLPRNGDWTDVFVWLDGVFKFA-
ETNNLIPFRNESLKAAERWVLERQEDTGDWGGIIPAMLNSLLALRALDYEVNDPIVHRGFKS-
VDNFAIETEETYHVQPCISPVWDTAWVLRALVESGLKPDEPVLVKGAQWLLDKQILDY-
GDWAVKNKEGTPGGWAFEFDNRWYPDLDDSAVVVMALEQVKMPDEQLKYGAM-
RRCVRWMATMQCKAGGWGAFDVNNDQNWLNYLPYADLKAMIDPNTADVTARVLEMLGTCELS-
MDHDRVKRAIAYLEQEQEADGSWFGRWGVNYIYGTSGALSA-
LAAIAPVTHQAQIEKGAAWLVGCQNPDGGWGETCFSYNNPALRGKGDSTASQTAWGLIGLLAA-
GEATGKFAKTALERGVNYLLATQRPDGTWDESYFTGTGFPCHFYLKYHLYLQYFPLIALSRYQRLLGFN

>seq_ID 129

MSLTSDPSPAAPTAEKSPKRPTIPVPATADAYGISRSSPPLPAATGRPQAAGPASAGVAT-
ARARDHLLALQSEEGWWKGDLETNVTMDAEDLFMKQFLGIRGDDETEQTARWIRSQQLAD-
GGWPTFYGGPADLSTTIEAYIALRLAGDAVDAPHMARAAELVRAQGGVAAS-
RVFTRIWLAALGQWSWDDVPVIPPELIFLPSWIPLNVYDFACWARQTIVALTIVGSLRP-
SHDLGFSIDELKVPAAARKPAALRSWEGAFERLDKLLHRYEKRPIKLLRTLALRRA-
TEWVVARQEADGCWGGIQPPWVYSVMALHLMGYPLNHPVIATAFRG-
MERYVIRRDTPQGPIRQIEACQSPVWDTALAVVALADAGVPGDHPAMVKAGRWLVDEEVR-
VAGDWAVRRPELAPGGWAFEFDNDFYPDVDDTAEVVLALRRLLGAGHVAPPASRQGRAEAPP-
VNTVEDADPRLAAAMRAAAARGVDWSVGMRSSNGAWGAFDADNVRTLTTKIPFCDFGE-
VVDPPSADVTAHIVEMLADLGRSDHPITQRAVQWLLDNQEPGGSWFGRWGVNHLYGTGAVVPA-
LIGAGVPTDHPAITAAVRWLLEHQSPEGGWGEDLRSYTDPAWIGRGELTASQTA-
WALLALLAVDPHSLAVKRGVRWLCETQRPDGTWDEPYFTGTGFPGDFSLNYHLYRLVF-
PLTALGRYVSLTGVATP

>seq ID 164

MHSGRVFLEKENREENRATFHSSPLILVEESLNLPKKVEETIKKAQRYLL-
SIQKEDGHWVGELFVDVTLACDCIHLMHWRGKIDYKKQLRLVKHIVDRQLPDGGW-
NIYPGGPSEVNATVKAYFALKLAGFSPDDPLMAKARSTILRLGGIPKCMTYTKLGLALL-
GVYPWDRLPVIPPEIILFPNWFPFNIYEISAWSRAMLVPLSVIHHFKPTRNLPEKYQL-
HELFPYGTEHGKFSWLKKGARYLSKQGLFLACDKFLQYWDKTSLKPFRKMALKKAEKWLLERI-
SAGSDGLGAIFPAMHYAIMALIAMGYTEDNPILKKAIADFEGLEVDDKKNDDL-
RIQPCLSPVWDTAVGLVALAESGVARNAKELKRAAYWLLDREIKIKGDWHVRNPHPEPSG-
WAFEYNNVYYPDVDDTLMVLLALRLIDIEDKIRKEEVMQRALRWVISFQCKNGG-
WAAFDKDVYKKWLEDIPFADHNAILDPPCSDITARALELFGKMGIKKTERFVQKAIAYL-
KETQENDGSWMGRWGVNYIYGTWQALRGLQAIGENMNQEWILRARDWLESCQNEDGGWGET-

PASYDNPQLKGKGPSTASQTAWAVSGIMACGDIFRPSVSRGIKYLCDRQLSDGSWAEEFLT-
GTGFPGVFYLKYDMYRNAWPLLVIGEYHRQYLKAKEQVSYWVDGTIGRKVKKERLPEI

>seq_ID 20

MRTQDRVQVNSIAEAIAASQKYLLSLQNPTGYWWAELESNVTITAEVVLLHKIWGTDKTRPLH-
KIEAYLRSQQKQHGGWELFYGDGGELSTSVEAYMALKLLGVPATDPAMIQARDFILQRG-
GISKTRIFTKFHLALIGCYNWRGLPSLPAWVMLLPNQFPVNIYEMSSWARSSTVPLLIV-
FDQKPVYQVNPAITLDELYAEGVENVRYELPRSGDWTDLFLTLDEGFKLAESFNFIPFREEGI-
KAAEKWIIERQEATGDWGGIIPAMLNSMLALRVLGYATNDPIVERGLQAIDN-
FAIETADCYRVQPCVSPVWDTAWVIRALIDSGMAPDHPAIVKAGEWLLQKQIFDY-
GDWNVKNRQGQPGAWAFEFDNRFYPDVDDTAVVVMALHAAKLPHEQLKQKACDRALQWVAS-
MQCKPGGWAAFDIDNDQDWLNAVPYGDLKAMIDPNTADVTARVIEMLGACNLSIDSHDLERAL-
TYLLNEQEAEGCWFGRWGVNYIYGTSGVLCALALINPQKYQRHIQQGATWLVGCQNPDGGW-
GETCFSYNDPSLKGQGDSTPSQTAWALIGLIAAGEATGNFAHDVIERGINHLVSTQQPDGS-
WFEAYFTGTGFPCHFYLKYHYYQQYFPLIALGRYQAINPL

>seq ID 185

MQTQDRVKVNQVAEAIAASQQYLLSIQNPAGYWWAELESNVTITAETVLLH-
KIWGTDQTRPLHKVEAYLRQEQRQHGGWELFYGDGGELSTSVEAYMALRLLGVPATDPAMIRA-
QAFILQRGGISKTRIFTKLHLALIGCYNWRGIPSLPPWIMLLPKAFPVNIYEMSSWARS-
STVPLLVVCDRKPVFITDPTINLDELYAEGIDRVRWELPQSGDWTDLFLTLDQGFK-
WAESLNLVPFREEGIKAAEKWILERQEATGDWGGIIPAMLNSMLALRCLDYDRSDPIVER-
GLQAIDNFAIETDNSYRVQPCVSPVWDTAWVMRALVESGFVPDHPAVVKAGEWLLQKQILDY-
GDWAVKNRQGKPGAWAFEFENRFYPDVDDSAVVVMALHLAKLPNEKIKQAAIARAVNWIAS-
MQCKPGGWAAFDLDNDQDWLNSIPYGDLKAMIDPNTADVTARVVEMLGACDLSIDSDNLERSL-
TYLLREQETEGCWFGRWGVNYIYGTSGVLSALALIDPQRHKLSIERGAAWLLGCQNLDGGW-
GETCRSYDDPSLKGKGDSTASQTAWALIGLLAAGEAT-
GKLAVKAIEQGIGYLMATQQPDGTWFEANFTGTGFPCYFYLKYHLYQQYFPLIALGRYQAAIKES

>seq ID 244

MVIAASPSVPCPSTEQVRQAIAASRDFLLSEQYADGYWWSELESNVTITAEVVILHKIWG-
TAAQRPLEKAKNYLLQQQRDHGGWELYYGDGGELSTSVEAYTALRILGVPATDPALVKAKN-
FIVGRGGISKSRIFTKMHLALIGCYDWRGTPSIPPWVMLLPNNFFFNIYEMSSWARSSTVPL-
MIVCDQKPVYDIAQGLRVDELYAEGMENVQYKLPESGTIWDIFIGLDSLFKLQEQAKVVPF-
REQGLALAEKWILERQEVSGDWGGIIPAMLNSLLALKVLGYDVNDLYVQRGLAAIDNFAVE-
TEDSYAIQACVSPVWDTAWVVRALAEADLGKDHPALVKAGQWLLDKQILTYGDWQIKNPHGE-
PGAWAFEFDNNFYPDIDDTCVVMMALQGITLPDEERKQGAINKALQWIATMQCKTGGWAAF-
DIDNDQDWLNQLPYGDLKAMIDPSTADITARVVEMLGACGLTMDSPRVERGL-
TYLLQEQEQDGSWFGRWGVNYLYGTSGALSALAIYDAQRFAPQIKTAIAWLLSCQNADGGW-
GETCESYKNKQLKGQGNSTASQTAWALIGLLDALKYLPSLGQDAKLTTAIEGGVAF-
LVQGQTPKGTWEEAEYTGTGFPCHFYIRYHYYRQYFPLIALARYSHLQAS

>seq ID 109

MDDRHIQSEITFGKIDGIRERIQQAMDAAKRYLFSKQDPEGFWCGELEADTTLQSDYIVMHT-
LLGTGDPVKMQKAGKQILQHQNPDGGWNIYPDGPSNISAAVKAYFSLKLIGHKPDEPEMTKA-
REWILAHGGVTACNTFSKMYLCFFGQYDYDTVPAIPPEIVLFPNWFWFNLYEISSWSRGIL-
VPLAICYAKKPFKKIPDEANIDELFVEGRHANLHLTWDKKPFSWRNFFLVLNNMVHF-
FERVHVRPLRKLAMKRAEKWMLERLEMSDGLGGIYPAILNSIIALRALGYSTDDPQVIRAMDE-
FEKLGIEEDDTFRMQPCMSPVWDTAYALYALGEAGVPGSD-
PRMQKAAEWMLKKQVTHKGDWAVKVRNVQPGGWYFEFNNEFYPDVDDTAQVILSLNHVRTS-
NERYQDDTVKRALDWQLAMQCKNGGWASFDKDNNKMVFQYIPFADHNAMLDPATVDITGRV-
LEALSHHGYSLKDKVVQRAVKFIQSEQEPDGSWFGRWGVNYIYGTMLCLRGLAAVGVDHHEP-
MVQQAAEWLRMVQNPDGGWGESVGSYDDPKLRGQGPSTASQTAWAVMGLLAANDLRSDS-
VTRGIAWLLENQKPNGSWWEKWITGTGFPRVFYLKYTMYAEYFPLIAFAEYLRRLNTPLDEKVKLGPQA

>seq ID 174

MQIQDKITEIAAKTAKAIELSQNYLLSTQYSEGYWWAELESNVTITSEAILLHKIWKTDK-
KRPLDKAATYLRQQQCPNGAWELFYGDGGDLSTTVEAYMGLRLLGIPANDPALEKAREFIL-
AKGGISKTRIFTKMHLALIGCYDWQGVPSIPAWIMLLPENFPFTIYEMSSWARGSTVPLLIV-
FDKKPVYKMGFNLDELYTEGVNNVKYELPKNNNWSDVFLWLDGLFK-
WAEKTDLVPFRQESLKAAEKWVIERQEDTGDWGGIIPAMLNSLLALKALDYDVYDPIVARG-
LKAVDNFAIETDNTYCVQPCVSPVWDTAWVIRSLIESGLNPAHPAMIKAGQWLIDQQILDY-
GDWAIKNKIGTPGGWAFEFDNRWYPDLDDSAVVVMALELIKMPDENIKTSVM-
KRAVNWMATMQCKAGGWGAFDIDNDQNWLNSLPYADLKAMIDPNTADVTARV-
LEMLGTCDVKMGENRVKKALDYLEKEQEADGSWFGRWGVNYIYGTSGALSALAF-

LEPNQYRQQLQKGANWLSSCQNVDGGWGETCFSYNNPKFKGQGNSTASQTAWALIG-
LLAVGKVTGNYQREVIEKGVNYLLVTQKENGTWDEDYFTGTGFPCHFYLKYHFYQQYFPLLALGRYRALI

>seq_ID 130

MSLTSDPSPAAPKAAKSSKRVNIPAPATPDAYGISRSSPPLSGGGVSGGGVSGGGAATAD-
GTPPTTQTSVDPDLAAAMTAANQARDHLLGLQSEEGWWKGDLETNVTIDAEHLFMKQFLGIR-
TEEETEPIARWVRSQQLADGGWATYYGGPAELSTTVEAYIALRLAG-
DEPDAPHMAAAAALIRSQGGVAAARVFTRIWLATFGEWSWDDVPVLPPELIFLPSWF-
PLNVYDFGCWARQTIVALTIVGSLRPVRDLGFSIDEIKVAAPVTPPKPAPLHSWEG-
AFERLDAILHRYERRPIKVLRTLALRRATEWVVARQEADGCWGGIQPPWIYSVMALHL-
MGYPLNHPVIATAFRGMERYIIRRETPEGPTAQIEACQSPVWDTALAVVALSDAGVPADH-
PAMVRAGRWLVDEEVRVAGDWAVRRPALAPGGWAFEFDNDFYPDTDDTAEVVLALRRLLGGS-
HVTPGGTVTPSGSVTPGGTAELSPAARDRASRGLAAVDPQLAGAMRAAAARGVDWSVGMRSSD-
GAWGAFDADNVRTLTAKIPFCDFGEVVDPPSADVTAHIVEMLADLGRSDHPITRRAVQWLLD-
NQEPGGSWFGRWGINHVYGTGAVVPALIAAGVPADHPAITAAVRWLLEHQSPDGGWGEDPR-
SYDDPAWIGRGELTASQTAWALLALLAVDPHSKAVKRGVRWLCETQRPDGTWDEPQFT-
GTGFPGDFYLNYHLYRLVFPLTALGRYVTLTGVATP

>seq ID 248

MPTSLATAIDPKQLQQAIRASQDFLFSQQYAEGYWWAELESNVTMTAEVILLHKIWGTEQRL-
PLAKAEQYLRNHQRDHGGWELFYGDGGDLSTSVEAYMGLRLLGVPETDPALVKARQFILARG-
GISKTRIFTKLHLALIGCYDWRGIPSLPPWIMLLPEGSPFTIYEMSSWARSSTVPLLIVM-
DRKPVYGMDPPITLDELYSEGRANVVWELPRQGDWRDVFIGLDRVFKLFETLNIHPLRE-
QGLKAAEEWVLERQEASGDWGGIIPAMLNSLLALRALDYAVDDPIVQRGMAAVDRFAIETE-
TEYRVQPCVSPVWDTALVMRAMVDSGVAPDHPALVKAGEWLLSKQILDYGDWHIKNKKGRPGG-
WAFEFENRFYPDVDDTAVVVMALHAVTLPNENLKRRAIERAVAWIASMQCRPGG-
WAAFDVDNDQDWLNGIPYGDLKAMIDPNTADVTARVLEMVGRCQLAFDRVALDRALAYLRNE-
QEPEGCWFGRWGVNYLYGTSGVLTALSLVAPRYDRWRIRRAAEWLMQCQNADGGWGE-
TCWSYHDPSLKGKGDSTASQTAWAIIGLLAAGDATGDYATEAIERGIAYL-
LETQRPDGTWHEDYFTGTGFPCHFYLKYHYYQQHFPLTALGRYARWRNLLAT

>seq ID 150

MAKGILNKFAVIAGTKKAGPPAGEERTVIAPIKEISGKAVHCSQAVKKAEEYLLALQN-
PEGYWVFELEADVTIPSEYIMLQRFLGREISPELGKRLENYLLDRQLPDGGWPLYAEDGFANI-
SATVKAYLALKVLGHSPQAPHMIRARLMVLSLG-
GAARCNVFTRILLALFGQIPWHTPPAMPVEIVLLPQWFFFHLSKVSYWSRTVIVPLLLLYAK-
QPVCRLRPEEGIPELFSTPPDKLRHLDGFQPGYWRKNAFIIFDRLLKRFNRFIPSALHR-
KAIAEAEQWTRSHMQGSGGIGAIFPAMAYAVMALRVLGCGEGDPDYIRGLQAIDDLLQHRT-
PQEADPPRTDGTCIDSGMSAAFALTPSAHAAADGTGSSSICQPCNSPIWDTCLSLSALMEAGM-
PASHPAATQAVEWLLSQQILSPGDWSLKVPDLEGGGWAFQFENTLYPDLDDTSKVIMSLL-
RAGALENERYRDRIARGVNWVLGMQSSDGGWAAFDIDNNYHYLNDIPFADHGALLDPSTS-
DLTGRCIELLSMVGFDRTFPPIARGIGFLRSEQEENGAWFGRWGVNYIYGTWSVLSGLRQA-
GEDMQQPYIRKAVGWLASCQNHDGGWGETCYSYDDPSLAGKGASTPSQTAWSLLGLMAAGE-
VNSLAVRRGVRYLLDHQNQWGTWEEKHFTGTGFPRVFYLRYHGYRHFFPLWALGVYSRLSS-
GQKACQDERRHASPGDLHLPWLERIKKR

>seq ID 128

MPDLELRDVDRADGRHHAPNLGRTDTLSPSAPTGEPAPASTPAAVATPTPTPTTAPAPAPAPE-
NALRETVQRAAEHLLRLQDPRGWWKFDLETNPTMDAEDLLLREYLGIRTVEQTEATAKHIRS-
RRLDDGSWPTYFGGPGELSTTVECYIALRLAGDSPDDEPLRRSAAWIRERGGI-
PATRVFTRIWLALFGWWRWEDLPVLPPEIMFLPPRAPLSIYSFASWARQTIVPLTIV-
SAARPQCPAPFDLAELDPDEVPAAQSHGAAQSPDTRSPAGGRTLRGAM-
RRLGGDRPNTAKVFFRGLDAALHRYHRHPIGPLRRHALRTAERWIIARQEADGCFGGIQPPA-
VYSIIALRLLGYDLDHPVLAAALRSLDAYTLHREDGSRMIE-
ASQSPIWDTALAVLALADAGIDAPADVDVAPALPTQRVATGAPAPSAPVPTALERAADWLL-
GQEIQHRRGDWAITHPGVAPGGWAFEFDNDTYPDTDDTAEVVLALHRLNRLRRLRHPT-
NTRIDAALERSTAWLFALQSRDGGWGAYDSDNASTLVYQIPFADFGALTDPSSADVTAH-
VVELLCETGRIRDPRTLRGVDWLLRNQEADGSWYGRWGVNYVYGTGSVLPALQAAGLPPTH-
PAMVAGARWLLSRQNSDGGWGEDIRSYGDPAWSGRGLSTPSQTAWAMLGLLATDHGGVHADA-
LAAAARWLTEQQRPDGGWDEEMFTGTGFPGFFYLNYHGYRLVWPVMALGRYLHSRQHPSD

>seq ID 131

MSLTSDQSSAAPTAAAQSPKIPNPSVARPSADAGSFETAGAVRTDSVSIDSVST-
GTPVDPVVGAMRRGRDHLLSLQAEEGWWKGELETNVTMDAEDLMLRQFLGILTPSTATETGR-
WIRSQQLSDGGWATFYGGPSDLSTTIEAYVALRLAGDDPDAPHMRSAAEWVRSAGGIAAS-

RVFTRIWLALFGEWSWDDVPVLPAEMTFLPPWFPLNIYDFACWARQTVVALTIVGS-
LRPVRSFGFTLDELRVQAPKATKAPLRSWAGAFERLDSVLHRYEKRPFQPLRRLAL-
RRAAEWVIARQEADGCWGGIQPPMVYSIMALHLMGYPLNHPVISMAFRALDRFTIREET-
PEGTVRRIEACQSPVWDTALAVVALADAGLGGDHPAMVRAGRWLADEEVRVAGDWAVRRPT-
LAPGGWAFEFDNDFYPDVDDTAEVVIAIRRLLGDGHGPVDHSDGS-
GPGSAAATAASAAAEAAVAAAGTIAAADPELAARLRAAAERGVDWSVGMRSSNGAWAAF-
DADNVRTLVRKIPFCDFGEVVDPPSADVTAHMVEMLALLGRSDHPITQRGVRWLLD-
NQEAGGSWFGRWGVNHVYGTGAVVPALISAGVDAEHPAIVSSMHWLVEHQTPEGGWGEDLR-
SYRDDEWIGRGEPTASQTAWALLALLAAEPASGTAEWEAVERG-
VRWLCDTQRPDGTWDEPQFTGTGFPWDFSINYHLYRLVFPVTALGRYVTLTGRSTS

>seq ID 242

MSISALQTDRLSQTLTQSVVAAQQHLLSIQNPEGYWWANLESNASITAEVVLLH-
KIWGTLDSQPLAKLENYLRAQQKTHGGWELYWNDGGELSTSVEAYMGLRLLGVPASDPAL-
VKAKQFILHRGGVSKTRIFTKFHLALIGCYRWQGLPSLPAWVMQLESPFPFSIYELSS-
WARGSTVPLLIVFDKKPVYPLQPSPTLDELFTESAENVRWELEEKGDWSDAFLWLDKAF-
KLAESVDLVPFREESIRKAEKWVLERQEPSGDWGGIIPAMLNSMLALRALGYSVSDPVVRRG-
FQAIDNFMVESETECWAQPCISPVWDTGLAVRSLTDSGLSPNHPALVKAGEWLLDKQIL-
SYGDWSVKNPQGQPGGWAFEFENSFYPDVDDTAVVAMALQDITLPNEPLKRRAIARA-
VRWIATMQCKTGGWAAFDINNDQDWLNDIPYGDLRAMIDPSTADITGRVLEMHGRFAADLD-
LANSYAADLSPYRLSRGLNYLIKEQELDGSWFGRWGVNYIYGTGQALSALALIAPER-
CRIQIERGIAWFVSVQNADGGWGETCESYKDKSLKGKGISTASQTAWALLGLLDVSFCLDPAA-
KIAVDRGIQYLVSTQSEGTWQEESFTGTGFPQHFYLRYRLYCHYFPLMALGRYQRVINSSAGI

>seq ID 143

MAKGILNKFAVIAGNKNAGLTAEEECTVVAPIKEVSGKAVHCRQAVKMAEEYLLALQN-
PEGYWVFELEADVTIPSEYIMLQRFLGREISPELRMRLENYLLDRQLPDGGWPLYAVDGFANI-
SATVKAYLALKVLGHSPQAPHMIRARIMVLSLG-
GAARCNVFTRILLALFGQLPWHTPPAMPVEIVLLPQRFFFHLSKVSYWSRTVIVPMLLLYAK-
QPVCRLRPEEGIPELFNTPPDKLRNLDGFQSGRWRKNAFIIIDRLLKRFNRFIPSAIHRKAMA-
EAEHWTRSRMQGSGGIGAIFPAMAYAVMALRVLGCREDDPDYVRGMQAIDDLLQHRTPQEAD-
SPRTGGPCIDSGTSAAFAFDPSPHAAADGRGNSSICQPCNSPIWDTCLSLSALMEAGMPASH-
PAAKQAVEWLLSQQIFSPGDWSLKAPDLEGGGWAFQFENTLYPDLDDTSKVIMSLLRAGAL-
ENGLYRDRVARGVNWVLGMQSSDGGWAAFDIDNNYHYLNDIPFADHGALLDPSTSDLTGRCI-
ELLSMVGFDRTFPPIAQGIGFLRSKQEGSGAWFGRWGVNYIYGTWSVLSGLRQA-
GEDMQQPYIRRAVGWLTSCQNHDGGWGETCYSYDDPSLAGQGESTPSQTAWSLL-
GLMAAGDVHSLAVRRGVRYLLDHQNQWGTWEEKHFTGTGFPRVFYLRYHGYRHYFPL-
WALGVYSRLSSGQKTRQEERRHSSPGDLHLPWLERIGRR

>seq_ID 71

MIKNFTALWPIRRVKGVSVTSQDGHSANGASKPDFEVRPHVDLETAIHRSQSFLL-
KEQKPEGYWVGELIVDSTLVSDTIAYHHWNGKVDMEWQRKAVNHIFSMQLPDGGWNIYYGG-
PAEINATVKAYLALKLAGVPVMDPRMLRARSVALSMGGVPRMNTFSKLYLALL-
GLFPWNYVPTIPCEVILIGKWFHVNFYEMSSWSRSMLVPLAIIN-
HFKPTRKLQNQVKLDELYPEGYHERDLALPPDPEFLTFRNFFLWLDKLHKFAEL-
WVQAGIHPFRRRALKKCEHWMLERFEGSNGLAAIFPAMLNSLIALKALGYPGDHPE-
VKRAEKELKNLEHETADTVRIEPCFSPVWDTAIVAICLHESGIPSDH-
PALKKSAEWLIDKEIRFRGDWYFKNPVDVEPSGWVFEFENKWNPDVDDTAMVLLALRKIPTS-
DVKRRDECFQRGLKWMMAFQCKDGGWAAFDKDCTKGILEKVPFADHNAMLDPECADITARIL-
ELLGYEGVGVDHPQIKKALQFIQEEQEDDGSWYGRWGVNYIYGTWQVLRGLRALNIN-
MNQPWLLKARDWLESVQHEDGGWGERCNTYDDPVFKGQGPSTASQTAWAVM-
GLCTFDDPQRPSLMRGIDYLIKTQNSDGSWTEHEITGTGFPRVFYLKYDMYRNSWPLLALA-
TYRNLYASSEKTANGHTNGHSVQLPEALKTPPAFK

>seq_ID 126

MNKKSAMKLKKKAKNHVVSLLQPTDALNRVMKRFRSLQSPEGYWVFALEADVTIPSEYIM-
FNRFLGRKMDKGLAERLGNYIRAKQMADGGWPLHDNDGPVNISASVKAYMALKMLGDNK-
DAEHMVRARQIILAKGGAETANVFTRICLATFGQIPWHCPPAMPIEIVLLPKWFFFHLD-
KVSYWSRSVIYPLLIIYAKQPVCRLRPEEAVPELFCKPAEEHIHIDKYRDKGWRKNLFILL-
DRVLKRTIHLVPKSINKKALNYAEKWTREHMAGRGGIGAIFPAMANAVMALSLLGYDESDPD-
FARGMQSVDDLMVDKFHVPEKSPWEHTVITGGAELSAAPELDISPDHGTAEN-
LEQAMCQPCNSPIWDTCLTLSAMMEAGENQDSKSTQQALNWLWDQQIFFRGDWIS-
KAPKLEGGGWAFQFENTFYPDLDDTAMVLMAMCRAGVLDQPEHRENFIKGVNWLIGMQSSNGG-
WAAFDIDNCAEYLNDIPFADHGALLDPPTSDLTARVIELLGVLGYDKSFRPIKDGIEFLK-
KEQEDDGSWFGRWGVNYIYGTWSVLCGLRQAGEDMNSSYVCKAVEWFENHQNKDGGWGESCL-

SYNDKNYAGLGDSTASQTAWALLGLMAAGRVHSKAVSRGVRYLLDTQKDDGSWDESLFT-
GTGFPRVFYLRYHGYSQYFPMWALGVYQRFSADEDTKQIMMRRKSPLDLGRKW

>seq ID 114

MIFTDTPTGSTQNRLDVAIRRAQQNLLRLQHNEGYWCGELFVDSTLCSDYVLFMHWA-
DEIDPVMEEKCVAHIRRRQLEDGGWNIYEGGPSDVNATVKAYFALKLAGHAPTQPWMQEARA-
CILRLGGIPKMNTYAKLYLALLGQFPWRYLPTVPVEIMFMPRWFFFDIYEVSSWSRAMLM-
PLAILNHYKPTKHLPADKQLHELYPIGSEESDLGLGMQKPRFSWPNFFLFCDRLIKIM-
HSLPWKPWKRAALARAEAWMTQRMGEGSDGLAAIFPAMLNSMIALRTLRYSREHPLYVKAKND-
FAGLFVDDPQDFRIQPCLSPVWDTAINLVALLESGLDPHDPKIEAAVNWLKEKEVRING-
DWYVKNHHVPPSGWAFEFNNVYYPDTDDTMMVLAALARAGAHEESAPVETKAMFERAL-
KWLLSFQCRDGGWAAFDKDVTQGWLEDVPFADHNAILDPTCSDLTGRVLELL-
GLIDYDRNCTPVRRALKFLRDTQEDDGSWYGRWGVNYIYGTWQVLRGLRSIGEDMRQQWIV-
RARDWLESCQNEDGGWGETCASYDDPTLKGKGPSTASQTAWALMGLIAAADPTEPGAF-
DRKSIRQGVDYLLSTQVADGSWVEPEVTGTGFPRVFYLRYDMYRNNFPLMALATYRKAREGKLPVRQRE

>seq ID 194

MKKATRSVFSLLDGGKISDSGSRGDSRHAGSRLDSVTKSAAALLASRQNPDGHWVFDLEAD-
VTIPAEYVMMRCFIGEPLDSDMASRLSAYLLERQLPDGGWPLYAVDGNANISAT-
VKAYFALKLLGHDKYAPHMVSARRMILAQGGAERSNVFTRITLALFGQVPWHTTPAMPIEIM-
LLPKWFFFHLSKVAYWSRTVIVPLLILYNKQPVCRLGYSEGIAELFSTSPDMLVHLDHFRY-
RAWRKNAFIVLDRLLKRTMHLVPGRIKRRALEEAERWTRERMKGDGSIGAIYPAMANAVMAL-
KTLGCGDSDPDYLRGLRAIDRLLIHGKPEAGALPADGAGTLFPVLD-
GASSAAVDLYPASLSDTAKSHAFSFCQPCNSPVWDTALSLTALSEAGGGGYSPE-
RAMEWLFNRQIATQGDWTERCPGLECGGWAFQYENALYPDVDDTAKVLMSLFRAGALER-
GEYPEKIAKAVRWVLGMQGADGGWGAFDVDNNHFYLNDIPFADHGALLDPSTADLTGR-
CIEMLGMLGHGPDYPPITRGIEFLREEQEPFGGWFGRWGVNYIYGTWSVLSGLSQAGEDM-
GRPYVRKAVEWLVSCQNDDGGWGETCASYDDPSLAGSGASTASQTAWALLGLMAAGEAD-
HAAVRAGIAYLADSFADGWDERHFTGTGFPRVFYLRYHGYSLFFPVWALGVYARHREGGKT-
VQEQVRERGVNGVFDFVMGGSA

>seq ID 154

MMANATDTIELPPSRAADRIVPMTDIDQAVDAAHAALGRRQQDDGHWVFELEADATI-
PAEYVLLEHYLDRIDPALEERIGVYLRRIQGDHGGWPLYHGGKFDVSATVKAYFALKAIGDDI-
DAPHMARARAAILDHGGAERSNVFTRFQLALFGEVPWHATPVMPVELMLLPRKALFS-
VWNMSYWSRTVIAPLLVLAALRPRAINPRDVHVPELFVTPPDQVRDWIRG-
PYRSQLGRLFKYVDIALRPAERLIPDATRQRAIKAAVDFIEPRLNGEDGLGAIYPAMANTVM-
MYRALGVPDSDPRAATAWEAVRRLLVELDGEAYCQPCVSPIWDTGLAGHAMIEAASGPEGIR-
PEDTKKKLAAAAEWLRERQILNVKGDWAINCPDVPPGGWAFQYNNDYYPDVDDTAVVGMLL-
HREGDPANDEALERARQWIIGMQSSNGGWGAFDIDNNLDFLNHIPFADHGALLDPPTADVTAR-
CISFLAQLGHPEDRPVIERGIAYLRTDQEREGCWFGRWGTNYIYGTWSVLCAY-
NAAGVAHDDPSVVRAVDWLRSVQREDGGWGEDCASYEGATPGIYTESLPSQTA-
WAVLGLMAVGLRDDPAVMRGMAYLTRTQKDDGEWDEEPYNAVGFPKVFYLRYHGYRQFF-
PLLALSRYRNLASSNSRHVAFGF

>seq ID 156

MLIYSDILEKEDRVSETLSRQSVEPDEINHAIEGAQAALGGKQKSDGHWVYELEADATI-
PAEYVLLEHYLDRIDPEKQAKIGVYLRRIQGHHGGWPLYHDGGFDLSATVKAYFALKAIGDDI-
NAPHMRIAREAILDHGGAARTNVFTRIQLALFGEVPWDATPVMPVELMLLPRKAFFS-
VWNMSYWSRAVIAPLLVLNALRPKAINPRGIHVQELFVKPPSEVKDWIRGPYRS-
VWGRFFKHLDSALRPVLPLIPRSVHKKALKAASDFIEPRLSRGGLGAIYPAMANVVMMYRA-
QGVPDSDPRAKTAWDAIQDLLVDHGDEIYCQPCVSPVWDTGLSGLAMIEAASGPAGTKTKET-
LAALKKSAEWLREHQILDVKGDWAINAPDLRPGGWAFQYENDYYPDVDDTAVVAMLLHR-
VDPENSREAISRAREWIIGMQSTNGGWGAFDIDNDHELLNHIPFSDHGALLDPPTADVSAR-
CISFLAQLGDPDDRPVILKAIEYLRSEQEPEGCWFGRWGTNYIYGTWSVLCALNI-
AGVPHDDPMVLRAVNWLESVQRPDGGWGEDCATYEGGTAGTYKKSLPSQTAWAVLALMAVGR-
RESEAVKRGVAYLVSQQNEKGEWQEEAYNAVGFPKVFYLRYHGYKQFFPLTALARYRN-
LGVSNSGKVEYGF

>seq ID 74

MEGASPTASNRISQYAVDLRAKARAAVASTCDWLLSHQHADGHWCAELEGDSILQSEYILL-
LAWLGKERTEIARRCAAHLLKQQEPNGAWTQFPGAPIDVGSSVKAYFALKLTGH-
DAAADYMVRARNAILEAGGADKVNSFTRFYLALLGQIPFELCPAVPPEMVLLPNWSPINIY-
RISSWSRTIFVPLSIVWAHRAARDIVEDVSIHELFIRKPEDWPELRCPGLEKPAGLFS-
WDRFFRTADSGLKLLEKYGLRPLRKRALRQAQQWMLDRFQQSDGPGAIFPPIVWSAIALRTLG-

YAEDSPEIQYCLDHLERLVLEDGETTKLQPCKSPVWDTSITLRALAAAGLGLAQEPTCRG-
VEWLLSKEVRVPGDWTNNVDCEPGGWFFEYENAFYPDNDDTSMGIMALADQLAAANITLEVHP-
GETLANTSVVVGGRGIAEQLAGSSAAMMEQAAAATRRAVAWMVAMQNKDGGWGAFDKNN-
DAEFLCHVPFADHNAMIDPSTPDLSARVIESFGRLGVTIESPGKLGDT-
VRRAVAYIRANQLSDGSWFGRWGVNYIYGTWQCLVGLRAVGVPANDPAIEQGKLWL-
LAHQQACGGWGESCETYEDPSLRGQGSPTASQTAWALLGIIAAGGANLAEVVHGVQYLM-
DTQREDGAWDEIEFTGTGFPRVFYLKYHYYPIYFPLLALAEWNRATARS

>seq_ID 326

MFDTISFDFDALDQAISRAHARLSAEQRADGHYVYELEADATIPAEYVLLEHFLDRIDPELEA-
RIGVFLRGIQGNSPQNPGGWPLFHDGAMDISASVKAYFALKAIGDDPDAPHMRRAREAILARG-
GAARTNVFTRIQLALFGAVPWRACPVMPVEIMLLPDWFPITIWKISYWSRTVIAPLLVLLTER-
PIARNPRNVRIDELFVTPPDQVTDYIRGPYRSNWGYLFKAIDSALRPLERHFPARSRKRAI-
QAAIDFITPRLNGEDGLGAIYPAMANTVMMYHTLGYSPDHPDYATAWASVRKLVTDASYRFE-
GASYVQPCLSPVWDTSLAAHALAEAGSPGDAQLAAACDWLIPRQILDVKGDWAYRKPDAPPGG-
WAFQYNNAHYPDVDDTAVVGMILDRNGDPAHREAVERARQWILGMQSRSGGWGAFDSD-
NEFHYLNHIPFADHGALLDPPTADVTARCISFLAQLGHAEDRPAIERGVAYLRREQEQDGS-
WFGRWGTNYIYGTWSSLCALNAAGVAQDDPMMVRAVEWLLARQRPDGGWGEDCETYAHAKPGE-
YHESLPSQTAWALLGLMAAGQAEHEAVARGIAWLQSVQEDDGSWTEQPYNAVGF-
PRVFYLRYHGYPRFFPLLAMARYRNLARGNSRQVQFGF

>seq_ID 192

MDKIKMKNINQPKFRVFRGGQKAATPCPGTTNERRGALDRGRLSASLKHSREWLLSLQADAG-
NWVFALEADTTIASEYVMLQRFLGRPLAPELQQRLANYLLSRQLPDGGWPLYAEDGFANIST-
TVKAYLALKLLGYPTHCDPLVRARQIVLALGGAEKCNVFTRIALALFGQIPWRTT-
PAMPVEIMLLPRWFYFHLSKISYWARTVVVPLLILYAKRPVCRLEPWEGIPELFVTPP-
DKLGYLDVCKPGQWRKNVFIWVDRLTRKMVRCVPRRLHNLALRAAETWTREHMQGAGGIGAIF-
PAMANAVMALRTLGCSPDDADYQRGLKALDDLLIDRCDVPPREDTPVSPCWCTGTSAAPMLD-
PSPAGSHAQGGDQGICQPCASPIWDTGLALTALLEGGLDARHPAVDRAVRWLL-
DQQVDVKGDWAQRVPNLEAGGWAFQFENALYPDLDDTSKVLMSLIRAGAMDNPGYRQELS-
RAINWVIGMQNSDGGWGAFDVDNNYLYLNDIPFADHGALLDPSTADVTGRCIEMLAMAGF-
GRDFLPIARGVDFLRREQEDFGGWYGRWGVNYIYGTWSALSGLIHAGEDLQAPYIRQAVGW-
LESVQNPDGGWGETCYSYDDPALAGRGVSTASQTAWALLGLMAAGEVDNLAVRRGIQYLVE-
EQNRAGGWDERHFTGTGFPRVFYLRYHGYSQYFPLWALGLYERLSSGNPSRQQMVRRAG-
PAGLHLPVLDRRKKLRRKRKA

>seq ID 72

MKSEEVTIKPAVGLEKDELNAAITRSQSFLLCEQKPEGYWVGELMVDSTIVSDTIAYHHWNG-
KVDPEWQRKAVNHILSMQLPEGGWNIYQNGPPEVNATIKAYLALKLAGIPITDPRM-
LKARQVALTLGGVPRMNTFSKLYLALLGLWPWKYVPTIPCEVLLLGKWFHVNIWDMSNWSRA-
MIVPLAIINHYKPTRPVKVDLSELFLEGFHERDLALPKDPQSFTWRNFFLGLDQLHKFAEL-
WVNAGIHPFRRLALKKCEQWMLERFEGSDGLAAIFPAMLNSLIALKSLGYPDDHPEVL-
RAERELKKLEHETKDTVRIEPCLSPGWDTAIAAMCLRESGVPAEHPRLKKAGDWLVNREVRF-
KADWHHKNPVDVEPSGWVFQFNNKWNPDLDDTAMVLLALRLIPTDHPRRRDEAFQRGLKWLL-
AFQCRDGGWAAYDKDCTKNILEKVPFADHNAMLDPECADITARVLELLGFEGYALD-
HPQVQEAVEYLREHQETDGSWYGRWGVNYIYGTWQTLRGLWALKMDMNQPWLLKARDWLES-
VQLPDGGWGERCNTYDDPVFKGQGPSTASQTAWAVMALCTFGDPKRPSLVRGIQYLIEN-
QNEDGSWTELETTGTGFPRVYYLKYDIYRNTWPLLAMATYRKMLDPKEVRVK

>seq_ID 145

MNKHKGTFSVIEGGKTTQARGSETCAIMDAADLEKVTSVAASQLAGQQQDDGHWVFDLEAD-
VTIPAEYVMLQRFIGREIDPEISERLAAYMQERQLPDGGWPLYAVDGNVNISAS-
VKAYFALKLLGHDKNAPHMVRARQLILSLGGAAKCNVFTRITLATFGQIPWHTAPAMPIEIM-
LLPRWFFFHLNKVAYWSRTVIVPLLILYATQPICRLQYNEGITELFTTPPDMLVHLDKFRH-
HAWRKNVFIALDRVLKRTMHLVPGRIKQHALAEAERWTRARMQGDGGIGAIYPAMANAVMAL-
KTLGCSDDDADYLRGLEAVDNLMVHRNLKTGTIPMDDDSGGIAIDNSSAAPELSPTYLT-
DTAGNTEFSFCQPCNSPIWDTCMSLSALCESGYAENNSGVTDRAIKWLFSQQIATPGDW-
SEKCPGLESGGWAFQYENSRYPDVDDTAKVLMSLFRAGALEKPEYREKIERAIRWVQGMQST-
DGGWGAFDVDNDYFYLNDIPFADHGALLDPSTADLTGRCIEMMGMLGHGPDYPPIARGIAYLK-
KEQEPFGGWFGRWGVNYIYGTWSVLSGLHQAGENMDAPYVRKAVEWLISCQNSDGGWGETCA-
SYDDPSLAGSGASTASQTSWALMALMAAGEWRHSAVRNGVRYLTESYCNGWNEKQFTGTGF-
PRVFYLRYHGYSLFFPVWALAVYSRYINGTATVQEKVREKQFRQCLMV

>seq_ID 127

MLPYNQDFYNEDEALKDDHCEGAGNVSNPPTLDEAIKRSQDFLLSQQYPEGYWWA-
ELEGNPTITSHTVILYKILGIEDEYPMDKMEKYLRRMQCIHGGWELFYGDGGQLSVTIE-
SYVALRLLNVPPTDPALKKALKFIIDKGGVXKSRMFTKICLALLGCFDWRGIPSLPPWVMLL-
PGWFLSSIYETACWARGCVVPLIVVFDKKPVFKVSPEVSFDELYAEGREHACKTL-
PFCGDWTSHFFIAVDRVFKMMERLGVVPFQQWGIREAEKWLLERQEDTGDFLGVYPP-
MFYSVVCMKTLGYEVTDPVVRRALLSFKKFSIERADECSVQSSLSPVWDTALVVRSLVES-
GLPPDHPALQRAGEWLLQKQITKHGDWSFKNQSGVAGGWAFQFFNRWYPDLD-
DSAVVVMALDCLKLPNEDVKNGAITRCLKWISSMQCKGGGWAAFDKDNHQHWINSTPFSDL-
KAMVDPSTTDISARVLEMVGRLKLHGTSFDEAHFLPPESIARGLVYLRREQENEGCWF-
GRWGVNYIYGTCGALVALSLVAPMTHEEEIARGARWLVQVQNMHGKKINGPQDGGWGE-
TCFSYNDPALKGQGDVSTASQTAWALQGLLAAGDALGKYEVESIGHGVQYLLSTQRKDGS-
WHESQFTGGGFPIHFYLRYHFYAQHFTLSSLARYRTRLQASKIKPPIP

>seq ID 166

MNTEPRFSAPETLRAIAGAGRALGRHQRRDGHWVFELEADATIPAEYVLLEHYMDRITPER-
QARIGAYLRRIQGEHGGWPMFHAGEFNISASVKAYCALKAIGDDPQAPHMVRARQAILGHGGA-
ERANVFTRIQLALFGAIPWRGVPVMPVEIMHLPKWFFFNIWAMSYWARTCVVPLLVLQARK-
PRARNPRQVSFDEIFRTEPDEVRDWIRGPYRSRWGVVFKHIDTVLRWTEPLFSKVARESAIF-
KAVDFVEERLNGEDGLGAIYPAMAYALMMYDVLGYPEDDPRCVTIWKAIDKLLIETDEE-
VYCQPCVSPVWDTSLSGHAMIEAARTGGIEAQAELDAACDWLVARQVKDVRG-
DWAETRPDAEPGGWAFQYRNDHYPDVDDTAVVAMLLHRNGRPEHAEAIEKARRWVVGVQSRN-
GGWGAFDADNDREFLNHIPFSDHGALLDPPTADVTGRCISFLSQLGHEEDRPVIERALAYL-
RAEQERDGSWYGRWGTNYVYGTWTVLCGLNAAGIPHDDPMVRRAVDWLVSIQRADGGWGEDER-
SYDVGHYVENAESLPSQTAWAMLGLMSVGQADHPAVLRGAAYLQRTQGPDGEWQERAYNA-
VGFPRVFYLKYHGYRLFFPLFALSRLHNLQRGNSREVSFGF

>seq_ID 21

MSGEVRVAGDALAEDAGRAAAAASQYLYRTQQRDHWRAELESNVTVTAEYVLLRQALGLD-
LEERRDALVRYLCSRQKADGSFGIASTLPGDVSTTAEAYLALRLLGLDREDERLRAAERFIR-
GAGGLARVRVFTRINLALFGLFPWEAVPTVPAELIFLPRWAPVNVYRLASWARST-
MVPLFVLFHHRPVFALPGGAGSDWLDHLWLGPGDKRVPYRTSVMETVRRHGPGWKAFF-
NAADAWLRVHDRLRHLPPLGRLRTEALRACEEWILARQEASGDWAGIFPPMLNGVLAL-
HVAGHGLDAAPVRRGLEAIERFAVSDREGFRIEACQSPVWDTILALIGLLDSGESPTD-
PRLVAARRWIEGMQLTNDWGDWKVYDPRGEPGGWAFEYANSWYPDVDDTAAVIVGLLKHDPAS-
RAGETVRRAAAWVASMQNRDGGWAAFDVNNDRLFLNEIPFSDMDSLCDPSSPDVTGRVLEAF-
GMLDAPHLRAACRRGVAYLRRAQEPEGSWYGRWGVNYVYGTSNVLNGLARQRVPASDPMVA-
RALGWLDSVQNADGGFGEGLESYADRAAMGRGPSTASQTAWGVMGLLAYRAADDAAVRR-
GIAWLVERQLADGEAQGSWEEEAFTGTGFPRHFYLRYHLYRHYFPLMALGRFCAQGRG

>seq ID 111

MSYEWTEPVRPGRRHAVSPVQNFCQSLAPAIQRACDALFSQQAADGFWCGELTADTTLES-
DYILLQLWLNQPDDHGWNPPTRPRIDRAGRSILERQLPDGGFNIYAGGPSEVSATI-
KAYCALKLAGLDPHSPPLRRARERILALGGLQAANSYVKINLSLFGLYPRKHVPSVPPEIV-
MLPGNVLYEMSSWTRSILVPLSIVQARGSNRRAPNGFNLDELLLPGVKLALPKRK-
GLAVLFHHLDRMFKVWEKRGSERIRGAAIREAERWLIARTHYTEGLGAIYPAMMYFI-
MALDALGYAEDHPDRSEAIRHFESLLIETDDRFLFQPCVSPVWDTAICAFALGEAGNTDD-
PRMTLAADWLISKEVRRKGDWSIKRPDTEPSGWAFEFANEFYPDIDDTAMVLLALMHANGSN-
PEAQAAAERRAVNWLLAMQSSDGGWAAFDVDNNWAMLNQVPFADHNAMLDPTCPDITGRV-
LECLCRRGMAGHDAARRGVAYLLQAQEKDGSWYGRWGVNYIYGSFLAMRGLTTSGAPGSQDA-
VDRAARWLRAIQNPDGGWGESCASYARDGYVAAPSSASQTAWALLGLCAAGDRDSAQFRRG-
VEYLLTLQAPDGKWPEGATTGTGFPNVFYLTYAMYRDYFPLLALSQV

>seq_ID 157

MPKDIPADLASEAISGDMLEQAVLRASMALHRKQQTDGHWVFELEADATIPAEYVLLEHFLD-
RIDDDLERKIGVYLRRIQGDHGGWPLFHEGAFNLSASVKAYYALKAIGDDPDAPHMRRA-
REAILAAGGAERSNVFTRIQLALFGQIPWRGVPVMPAELMIAPKWFPINMWKVSYWSRT-
VIAPLLVLMDRKPKARNPRNVHVRELFLHDPDRIRDWIRGPFRSGWGHFFKYLDSVLRVVEP-
VALKPMRPRSIRLAVDFVRERLNGEDGLGAIYPAMANSVMMYDVLGYSPDHPEAAIAWES-
VRKLLVIKEDEAYCQPCLSPIWDTGLSGHAMAEAEGAVSPGVAAACDWLRNRQIT-
DVVGDWAEIRPGVQPGGWAFQYNNAHYPDVDDTAVVAMLLHRQGDPAHEESIRKA-
REWIIGLQCRDGGWGAFDADNDKDYLNHIPFADHGALLDPPTADVTARCISFLAQLGN-
PEDKPVIDRAMAWLRKEQEADGSWFGRWGTNYIYGTWSVLCAMNVAG-
MPHDDPAIRRAVNFLVATQREDGGWGEDEETYDPASGAQPGRYKESTPSQTAWALIGLMAA-
GEAEHEATRRGIAYLQATQKPDGEWDEAAYTAVGFPRVFYLKYHGYRQFFPL-
MALSRYKNLRSSNMKKVSFGF

>seq ID 205

MNQAATITRPQDETLTTSARRPAQPALPDPLDAGIAHVVESLLAQQQSDGHWVYELEADATI-
PAEYILMVHYLGETPDLVLEGKIANYLRRIQNADGGWPLFHAGASDISASVKGYFALKMAGDN-
PEAEHMRRARAAIHAMGGAEASNVFTRTLLALYGVMPWQAVPMMPVEIMLLPEWFPFHLS-
KVSYWARTVIVPLLVLNSLRPQARNPRKIGIDELFVRPCQATRLPRRAPHQSPLWVGV-
FRTLDAVVRMAEPLFPRGLRQRAIERAREFTVERLNGEDGLGAIFPAMVNSVLM-
FDVLGVPESDPNRAIARRSIDKLLVIKDDEAYCQPCLSPVWDTSLAAHALLEVGE-
PRTIAAAARGLDWLLPLQELELRGDWTVRRPNVRPGGWAFQYANPHYPDVDDTAVVAAAM-
DRVDKGDRSNRYDEAVSRACEWIVGMQSSNGGWGAFEPENTHLYLNNIPFADHGALLDPP-
TADVSARCLAMLCQLGQMPANSEPAARALRYLLDEQEADGSWFGRWGTNYIYGTWSALCGL-
NAAGIGTDAPEMKRAAQWLLSIQNEDGGWGESGDSYKLEYRGYEKAPSTASQTAWAMLGLMAA-
GAGDHPALVRGVEYLLRTQASHGFWDEPYFTAVGFPRVFYLRYHGYSRFFPLWALARFRNLL-
RDGNRAISWGL

>seq ID 218

MKTDGNTTLDTTISMEELERTVKSAYEALAKDQQDDGHWIYELEADVTIPAQFILLEHTLDKI-
DEELEQKIANYLRRCQSREHWGWPVYYGGEFNISASVQAYFALKMTGEDINAPHMVRAREAI-
LAHGGPEYANVFTRIQLSLFGEASWLATPFMPVEIMLLPRWMYFSIWNMSYWSRTTVAPLLIV-
ADLKPKAINPRNVHIPELFPTPPDKVKTWIHGPFRSKWGHVFKFIDTAIRPFTRFVPSFLHK-
KAYKAALDFIEPRLNGVDGLGAIYPPMSYSAVMYRALGIPDDDPRAATNWEALKGLLVI-
KEREAYCQACVSPVWDTALSGHALMEASFGPDGINADRTEKLIDRAAHWL-
RAHQVLNVVGDWAINNPNLQPGGWAFQYGNDYYPDVDDTAVAAMLLHRQNLPENEEALDRARK-
WIIGMQSSNGGWGAFDIDNDKQILNDIPFADHGALLDPPTADVSARCISLLAELGH-
PEDRPVIERGIKYLRKEQEEDGSWFGRWGTNYIYGAWSVLCAFNAS-
GVPHDDPSVLKCVNFLKSVQREDGGWGESCETYEGSAHGVYTESLPSQTAWAVLGLMAS-
GRRTDPAVKRGIVWLIQHQQDNGEWAEEPFNAVGFPRMFYLHYLGYKQFFPLLALA-
RYRHMEKSGTNNVSFAF

>seq_ID 11

MLPYNQDHHFGKVAENATMPPTLDEAIERSQDFLLSLQYPEGYWWAELEANVTLTAQTIM-
LYKILGIDHKYPIHKMKTYILRTQRAHGGWEIFYGDGGCLSTTIGAYMAL-
RILGVPKTDPVLQKALKLIHSKGGVTKSRMFTKICLALLGCYDWKGIPSLPPWLVLLPSWF-
PFSLYDTASWVRGCVVPLTIIFDKKPVYKLNPLLCLDELYSEGKGKARVHLSFIPGDWTSNF-
FVGLDHVFKYMENLGVVPFRQWGIKEAERWTLERHEDSGDFHGIYPPMFYSIVSYSLL-
GYEITDPVVHRALESMRGFTVEREDECVVQSCISPMWDTAFVIRSLAESGLQPDHPALQKA-
GEWLLQKQATQHGNWFYKKRTGRAGGWAFQFFNRWYPDVDDSAAVSMAL-
NAIKLQDDDVKKGAIKRCAEWISVMQCKDGGWAAYDCDNDREWLNCTPFGDLKAMIDPNT-
VDVTARVLEMVGRVKEAGDASAILPPRAIARGLAYLRREQETEGCWYGRWGVNYIYGTSGAL-
MALALVAPSTHKEEIERGARWLVEVQNKRGTKGANGYSHTNGAREGGVAMNGNCK-
NMGAPEDGGWGETCFSYNDITLKGRNEVSTVSQTAWALQGLLAAGDALGKYEVESIEH-
GVQYLLSTQRKDGSWCEKHFTGGGFPRFFYIRYHLYAGHFPLSALARYRDRVRAGKMAK

>seq_ID 214

MDATAPLRDPGAPSAENCSVDRRELDDVIGESCRWLGERQNQDGHWVFELEADATI-
PAEYILLNHFLDEIDDAREARIASYLRAIQGKHGGWPLFHDGDFDMSATVKAYYALKLTGD-
GVDEPHMVRARQAILEHGGAERTNVFTRFTLAMFDQVPWRACPVTPVEALLLPRFAPF-
HWSKVSYWSRTVMTPLMILYSRRARAVNPRGIGVRELFRRDPEVIRDWLKNPTGHWIGDALI-
QIDKVLRVIEPAIHWAFRDRAEKWALDFIEERLNGRDGLGGIYPAIANTLMAYHTLGYA-
KDHPGYRIAREAVDGLCTPHAKGEYVQPCLSPVWDTCLASHAIQEAGQSAGDRAVDQSNAWL-
RERQVLDVVGDWKSNRGHLRPGGWAFQYNNPHYPDVDDTAVVVMALARSKEDEANREAI-
ARAEEWIIGMQSSNGGWGAFDAENEHDFLNHVPFADHGALLDPPTVDVSARCLGM-
LAQLGRPKTDPVVARGLDYLWREQEADGSWFGRWGTNYIYGTWSALNAFNAVEWDMTDPRICK-
AVDWLKSRQRDDGGWGEDCATYWKERRSVSKASTPSQTAWAVLGLMAAGEVDSPEVERGIRYL-
LEAPRDGGKWEEELYNAVGFPRIFYLRYHGYSAYFPLWALARYRNLTSGNCKRTIHGM

>seq ID 73

MPEEAILTETHPLDATTIETAITRARKALLGEQRADGHFVFELEADVSIPCEYILFYHFIGR-
PAPAELEAKIGHYLRARQSAEHDGWPLFQDGAFNISSSVKAYFALKAIGDTPDMPHMQR-
ARTAILAHGGAAAANVFTRSLLALFGLIPWHGIPVMPIEIMHLPEWFPFHIAKISYWGRT-
VLVPMMVVHALKPKPANTCTIRIDELFVIPPDQVRHWPGSPGKRFPWTAIFA-
GIDKVLQIAEPYFPRRSRQSAIDKAVAFVTKRLNGEDGLGAIYPAMAYSALMYL-
SIGRSLSDPHIQLVLKAIDKLVVVKDHEAYVQPCVSPVWDTALASHALMEAGDGDKPILDS-
LKKGLAWLKPLQVTDIAGDWAWKKPDVKPGGWAFQYGNAYYPDLDDTAVVVMAMDRARD-
RWPEIDEDNFRPSIARAREWIVGLQSENGGFGAFDADNDRDYLNAIPFADHGALLDPPTAD-

VTARCISMLTQLGEKPENSETLRRAIAYLFAEQEKDGSWFGRWGLNYIYGTWSVLCSL-
NAAGIAHDAPEVRRAVAWLRTIQNEDGGWGEDAESYALDYAGYQQAPSTSSQTAWAVLGLMAA-
GEKDDPAVARGIAYLTRTQGEDGFWTEKRFTATGFPRVFYLRYHGYSKFFPLWAMARYRN-
LHNGNHASVLTGM

>seq ID 103

MNDMTEMHTLDATAVPAAPAAADAPAPSAATTGLDAAVARATDALLAAQNADGHWVYELEAD-
STIPAEYVLLVHYLGEEPNAELEQKIARYLRRIQQPDGGWPLFTDGAPNISAS-
VKAYFALKVIGDDENAEHMQRARRAIHAMGGAEMSNVFTRIQLALYGVVPWYAVPMMPVEI-
ILLPQWFPFHLSKVSYWARTVIVPLLVLNAKRPVAKNPRGVRIDELFKSAPVNTGLL-
PKQPHQHAGWFAFFRAVDGVLRLADGLFPRYTRERAIRQAAAFVDERLNGED-
GLGAIYPAMANAVMMYAALGYPEDHPNRAIARQSIEKLLVVGEEEAYCQPCLSPVWDTS-
LAAHALLETGDERAREAAVRGLDWLVPRQILDVRGDWISRRPHVRPGG-
WAFQYANAHYPDVDDTAVVVMAMDRVAKHDQTDAYRESIARAREWVVGMQSSDGGWGAFE-
PENTQYYLNNIPFSDHGALLDPPTADVSGRCLSMLAQLGETSASSE-
PARRALDYMLKEQEPDGSWYGRWGMNYIYGTWTALCSLNAAGLGHDDPRVKRAAQWLL-
SIQNPDGGWGEDGDSYKLDYRGYERAPSTSSQTAWALLGLMAAGEVDNPAVARGIGHLL-
GTQREHGLWDETRFTATGFPRVFYLRYHGYRKFFPLWALARYRNLKRAGAARVTVGM

>seq_ID 95

MNDMTEMHTLDAAAAPAADAPAVTAVTAGLDAAVARATDALLAAQNADGHWVYELEADSTI-
PAEYVLLVHYLGEEPNAELEQKIARYLRRIQQPDGGWPLFTDGAPNVSASVKAYFALKVIGD-
DENAEHMQRARRAIHAMGGAETSNVFTRIQLALYGVVPWYAVPMMPVEVMLLPQWFPFHLS-
KVSYWARTVIVPLLVLNAKRPVAKNPRGVRIDELFKSAPVNTGLLPKQPHQSTGWFAF-
FRAVDGVLRLVDGLFPRYTRERAIRQAVAFVDERLNGEDGLGAIYPAMANAVMMY-
AALGYPEDHPNRAIARQSIEKLLVVGEEEAYCQPCLSPVWDTSLAAHALLETGDER-
ARDAAVRGLDWLIPRQILDVRGDWISRRPHVRPGGWAFQYANPHYPDVDDTAVVVMAMDRVA-
KLDQSDAYREQIARAREWVVGMQSSDGGWGAFEPENTQYYLNNIPFSDHGALLDPPTAD-
VSGRCLSMLAQLGETNASSEPARRAFDYMLKEQEPDGSWYGRWGMNYIYGTWTALCAL-
NAAGLGHDDPRVKRAAQWLLSIQNQDGGWGEDGESYKLDYRGYERAPSSSSQTAWALLGLMAA-
GEVDNPVVARGIDYLLGAQCEHGLWDETRFTATGFPRVFYLRYHGYRKFFPLWALARYRNL-
KRANTTRVTVGM

>seq ID 106

MNDLTDMPTLAADSAAADLDAAVARATDALLAAQQADGHWVYELEADSTIPAEYILLVHYL-
GETPNLELEQKIGRYLRRIQQPDGGWPLFTDGAPNISASVKAYFALKVIGD-
DENAEHMQRARRAIHAMGGAEMSNVFTRIQLALYGAIPWRAVPMMPVEIMLLPQWFPFHLS-
KVSYWARTVIVPLLVLNAKRPIAKNPRGVRIDELFIDPPVNAGLLPRQGHQSAGWFAF-
FRVVDHALRAVDGLFPSYTRERAIRQAVAFVDERLNGEDGLGAIYPAMANAVMMYDA-
LGYPEDHPNRAIARRSVEKLLVVHDDEAYCQPCLSPVWDTSLAAHALLETGDPRAEDA-
VVRGLEWLRPLQILDVRGDWISRRPNVRPGGWAFQYANPHYPDVDDTAVVVMAMDRVEKLRHS-
DAYREAISRAREWVVGMQSSDGGWGAFEPENTQYYLNNIPFSDHGALLDPPTADVSGRCLS-
MLSQLGETAANSEAARRSLDYMLKEQEPDGSWYGRWGMNYVYGTWTALCSLNAAGLGPDD-
PRVKRGAQWLLSVQNKDGGWGEDGDSYKLDYRGYEQAPSTSSQTAWALLGLMAAGEVNHPA-
VARGIDYLIAEQKEHGLWDETRFTATGFPRVFYLRYHGYRKFFPLWALARYRNLKRANATRVTVGM

>seq ID 87

MNDLTEMATLSAGAVPAGVDAAVARATDALLAAQQADGHWVYELEADSTIPAEYVLLVHYL-
GETPNLELEQKIGKYLRRIQQADGGWPLFTDGAPNISASVKAYFALKVIGD-
DENAEHMQRARRAIHAMGGAEMSNVFTRIQLALYGAIPWRAVPMMPVEIMLLPQWFPFHLS-
KVSYWARTVIVPLLVLNAKRPLAKNPRGVRIDELFIDPPVNAGLLPRQGHQSPGWFAF-
FRVVDHALRAVDGLFPSYTRERAIRQAVSFVDERLNGEDGLGAIYPAMANSVMMYAALGYA-
EDHPNRAIARKSVEKLLVVHDDEAYCQPCLSPVWDTSLAAHALLETGDARA-
QEAVLRGLEWLRPLQILDVRGDWISRRPNVRPGGWAFQYANAHYPDVDDTAVVVMAMDRA-
QKLTQSDTYRESMARAREWVVGMQSSDGGWGAFEPENTQYYLNNIPFSDHGALLDPPTAD-
VSGRCLSMLSQLGETPLNSEPARRALDYMLKEQEPDGSWYGRWGMNYVYGTWTALCSL-
NAAGLTPDDPRMKRGAQWLLSIQNKDGGWGEDGDSYKLNYRGYEQAPSTASQTAWALLGLMAA-
GEVNNPAVARGVDYLVAQQNEEGLWDETRFTATGFPRVFYLRYHGYRKFFPLWALARYRNL-
KRANATRVTVGM

>seq ID 107

MNDLTDMANLSAGTVPAGLDASVARATDALLAAQNADGHWVYELEADSTIPAEYVLLVHFL-
GETPNLELEQKIGRYLRRIQQADGGWPLFTDGAPNVSASVKAYFALKVIGD-
DENAEHMQRARRAIHAMGGAEMSNVFTRIQLALFGAIPWRAVPMMPVEIMLLPQWFPFHLS-
KVSYWARTVIVPLLVLNAKRPLAKNPRGVRIGELFIDPPVNAGLLPRQGHQSPGWFAF-

FRVVDHALRAADGLFPSYTRERAIRQAVSFVDERLNGEDGLGAIYPAMANAVM-
MYDVLGYPEDHPNRAIARKSIEKLLVVHDDEAYCQPCLSPVWDTSLVAHAL-
LETGDARAEQAVLRGLDWLRPLQILDVRGDWISRRPNVRPGG-
WAFQYANAHYPDVDDTAVVVMAMDRAQKLQNTDTYRESIARAREWVVGMQSSDGGWGAFE-
PENTQYYLNNIPFSDHGALLDPPTADVSGRCLSMLAQLGESALSSE-
PARRALDYMLKEQEPDGSWYGRWGMNYVYGTWTALCSLNAAGLGPEDPRVKRAAQWLL-
SIQNKDGGWGEDGDSYKLNYRGFEPAPSTASQTAWALLGLMAAGEVNHPAVERGIGYLIAQQN-
DEGLWDETRFTATGFPRVFYLRYHGYRKFFPLWALARYRNLKRANATRVTVGI

>seq ID 212

MESGNNKQPAAAIGALDASIESATNALLGYRQPDGHWVFELEADCTIPAEYVLLRHYLGEP-
VDAALEAKIANYLRRVQGAHGGWPLVHDGGFDMSASVKGYFALKMIGDDIDAPHMAKA-
REAIRSRGGAIHSNVFTRFLLSMFGITTWRSVPVLPVEIMLLPMWSPFHLNKISYWARTTIV-
PLMVLAALKPRAVNRLDIGLDELFLQDPKSIKMPAKAPHQSWALFKLFAGIDAVLR-
TIEPLFPKRLRDHAIKLAVDFVEERLNGEDGLGAIYPPMANTVMMYKVLGFPEDHPPRAITRR-
GIDKLLVIGEDEAYCQPCVSPVWDTALTCHALLEVGGEAAVPPAKRGMDWLLPKQVLD-
LKGDWAVKRPNLRPGGWAFQYNNAHYPDLDDTAVVVMAMDRSRRATGSREYDEAIARARE-
WIEGMQSDDGGWAAFDVNNLEYYLNNIPFSDHGAMLDPPTEDVTARCVSMLSQLGE-
TAASSKAVADGVEYLRRTQLPDGSWYGRWGLNYIYGTWSVLCAL-
NAAGVDHQDPVIRKAVTWLASVQNPDGGWGEGAESYRLNYTRYEQAPTTASQTSWALLGLMAA-
GEVDSPVVARGVEYLKSTQTGKGLWDEQRYTATGFPRVFYLRYHGYAKFFPLWALARYRN-
LRSTNSKVVGVGM

>seq ID 101

MNDLTEMATLSAGAVPAGVDTAVARATDALLAAQNADGHWVYELEADSTIPAEYVLLVHYL-
GETPNLELEQKIGKYLRRIQQADGGWPLFTDGAPNISASVKAYFALKVIGD-
DENAEHMQRARRAIHAMGGAEMSNVFTRIQLALYGAIPWRAVPMMPVEIMLLPQWFPFHLS-
KVSYWARTVIVPLLVLNAKRPLAKNPRGVRIDELFIDPPVNAGLLPRQGHQSAGWFAF-
FRVVDHALRAVDGLFPNYTRERAIRQAVSFVDERLNGEDGLGAIYPAMANSVMMYDVLGYA-
EDHPNRAIARKSIEKLLVVQEDEAYCQPCLSPVWDTSLAANALLETRDARAEDAAIRG-
LEWLRPLQILDVRGDWISRRPHVRPGGWAFQYANAHYPDVDDTAVVAVAMERA-
QQLKQNDAYRDSIARAREWVVGMQSSDGGWGAFEPENTQYYLNNIPFSDHGALLDPPTAD-
VSGRCLSMLSQLGETPLNSEPARRALDYMLKEQEPDGSWYGRWGMNYVYGTWTALCSL-
NAAGLTPDDPRVKRGAQWLLSIQNKDGGWGEDGDSYKLNYRGFEQAPSTASQTAWALLGLMAA-
GEVNNPAVARGIDYLIAEQNAEGLWDETRFTATGFPRVFYLRYHGYRKFFPLWALARYRN-
LKRDNTTRVTVGL

>seq_ID 112

MSAPSHVGNTLEHAAELATRKAMAYLTCLQERDGHWCAELTADTTLES-
DYILFQLWLYPPQDGKWEPETRPLIRKAVNSILERQLPDGGFNICVGGPSEVSASVKAYVAM-
KLAGLPPEDDRMARLRERILALGGIQAANSYVKVNLSLFDLYPREFSPSIPPEVALLPFDL-
LYQMSAWTRAIVISLGIVHAANPRRPAPAGFNLQELWLPGVSPEFRRDPSFFTWHNTFLTVD-
KALKLWERYGSKAVRRRAVEKAKTWMIERLHHSDGLGAIYPPMMYSVMALDVLGYA-
KDDPLRVEALRHFNNLMVDDGDRFFFQPCFSPVWDTAIGAYALVQADPSHEAIAPAADWLI-
AKEVRRKGDWSVKRPNTEPSGWAFEYSNEYYPDIDDTAMVMLALGETRASNTEAQAAACK-
RGLAWLLAMQSSDGGWAAFDADNNWEFLSQVPFADHNAMLDPTCADITGRV-
LEALASQGLDRNHKAVRRGAEWLIRHQENDGSWYGRWGVAYIYGTCFALRGLAASGENDREA-
HILRAGEWLRSIQNADGGWGESCKSYDNRIFTGGPSTPSQTAWAILGLIAGGDANSLS-
VQHGIEYLLETQRSDGSWDEQFATGTGFPRVFYLNYHMYKDYFPLLALASFVKARAGSNG

>seq ID 83

MNDLTEMATLSAGTVPAGLDAAVASATDALLAAQNADGHWVYELEADSTIPAEYVLLVHYL-
GETPNLELEQKIGRYLRRVQQADGGWPLFTDGAPNISASVKAYFALKVIGD-
DENAEHMQRARRAIHAMGGAEMSNVFTRIQLALYGAIPWRAVPMMPVEIMLLPQWFPFHLS-
KVSYWARTVIVPLLVLNAKRPIAKNPRGVRIDELFVDPPVNAGLLPRQGHQSPGWFAF-
FRVVDHVLRAADGLFPSYTRERAIRQAVSFVDERLNGEDGLGAIYPAMANAVMMYDVLGYA-
EDHPNRAIARKSIEKLLVVHEDEAYCQPCLSPVWDTSLAAHALLETGDARAEEAVIRG-
LEWLRPLQILDVRGDWISRRPHVRPGGWAFQYANAHYPDVDDTAVVAVAMDRVQKLKHNDT-
FRDSIALAREWVVGMQSSDGGWGAFEPENTQYYLNNIPFSDHGALLDPPTADVSGRCLSM-
LAQLGETPLNSEPARRALDYMLKEQEPDGSWYGRWGMNYVYGTWTALCALNAAGLTPDD-
PRVKRGAQWLLSIQNKDGGWGEDGDSYKLNYRGFEQAPSTASQTAWALLGLMAAGEVNNPA-
VARGVEYLIAEQKEHGLWDETRFTATGFPRVFYLRYHGYRKFFPLWALARYRNLKRDNATHVTFGL

>seq_ID 175

MLQTEAITTEGLRFRSLAPDDPLLPRVKQALKLSGQHSREEMHSDGHWCGEVKTNATTSAEH-
VLLCQALDINLDADREAFISWFRCTQGADGGWSTAPDQAGDISVTVEAYLALKILGLSED-
DAAMRSARDFAIAAGGVARVRIFTRIYLAMFGLFPWAAVPELPPELILLPSRVPVSIYHWSA-
WARATVVPLLIISHHRPIYALPGGKATCSDYLDELWCDPRNKMVPYNHDKPTAWRSDPFALI-
FTLADSILHRLDGLRSFNPLRRFALRKCVDWILEHQEDMGDIGDIMPPLHGAMLALR-
LEGYPLHSDPIHRGLEAIERFAYRDQQGKRIQTTVSAFWDTSLMLVALGDAG-
MASSPWLTRSLGWLQQHQRLGNYGDWKVNNPGLKAGGFSFGYFNTWYPDVDDTA-
SAVLAIIRQDERLVCSASVLDALNWLLGMQNTDGGWGAFDRDNNKLFLNKIPFSDMEAF-
CDPSTPDVTGHVLEAFGIFLAVSARQQSPTKADVLTDRIVSASRRAICYLSDTHVSSGGWYG-
RWGCNYIYGTSAVLCALAYFGSKSDTLSGVRSVKDAVNQAIRWLETVQNQDGGWGETVN-
SYKDPSRAGSGPSTASQTAWAIMALLPYLPPSTEVIQRGVEYLLRTQTKTASQGATWHEKA-
YTGTGFPKYFYMGYSFYCHYFPMMALGRYAYPCPEWHENWRPKKE

>seq ID 88

MNDLTDMATLSAGAAPAADLDAAVARATDALLAAQNADGHWVYELEADSTIPAEYVLLVHYL-
GETPNLELERKIGRYLRRIQQADGGWPLFTDGAPNVSASVKAYFALKVIGD-
DENAEHMQRARRAIHAMGGAEMSNVFTRIQLALYGAIPWRAVPMMPVEIMLLPQWFPFHLS-
KVSYWARTVIVPLLVLNAKRPLAKNPRGVRIDELFIDPPVNAGLLPRQGHQSAGWFAF-
FRVVDHVLRAVDGLFPKYTRERAIRQAVSFVDERLNGEDGLGAIYPAMANAVMMYDVLGYA-
EDHPNRAIARKSIEKLLVVHDDEAYCQPCLSPVWDTSLAAHALLETGDPRAEDAAL-
RGLEWLRPLQILDVRGDWISRRPNVRPGGWAFQYANAHYPDVDDTAVVAMAMDRA-
QKLRQSDTYRESIARAREWVVGMQSSDGGWGAFEPENTQYYLNNIPFSDHGALLDPPTAD-
VSGRCLSMLSQLGESALTSEPARRALDYMLKEQEPDGSWYGRWGMNYVYGTWTALCAL-
NAAGLGPDDPRVKRAAQWLLSIQNKDGGWGEDGDSYKLNYRGYEQAPSTASQTAWALLGLMAA-
GEVNNPAVARGIDYLLAEQKEHGLWDEVRFTATGFPRVFYLRYHGYRKFFPLWALARYRNL-
KRANATRVTVGM

>seq_ID 92

MNDMTEMHTLDATAAPAGLDAAVARATDALLAAQQADGHWVYELEADSTIPAEYVLLVHYLGE-
APNVELEQKIARYLRRIQQPDGGWPLFTDGAPNISASVKAYFALKVIGD-
DENAEHMQRARRAIHAMGGAEMSNVFTRIQLALYGVVPWYAVPMMPVEIMLLPQWFPFHLS-
KVSYWARTVIVPLLVLNAKRPVAKNPRGVRIDELFKGAPVSTGLLPKQPHQSAGWFAF-
FRAVDGVLRLVDGLFPRYTRERAIRQAVAFVDERLNGEDGLGAIYPAMANAVMMY-
AALGYPEDHPNRAIARRSIEKLLVVGEQEAYCQPCLSPVWDTSLAAHAL-
LETGDARAREAAVRGLDWLVPRQILDVRGDWISRRPHVRPGG-
WAFQYANAHYPDVDDTAVVAMAMDRVAKLDRTDAYRESIARAREWVVGMQSSDGGWGAFE-
PENTQYYLNNIPFSDHGALLDPPTADVSGRCLSMLAQFGETSASSE-
PARRALDYMLKEQEPDGSWYGRWGMNYIYGTWTALCSLNAAGLGHDDPRVKRAAQWLL-
SIQNADGGWGEDGDSYKLDYRGYERAPSTSSQTAWALLGLMAAGEVDNPAVARGVDYLL-
GTQREHGLWDETRFTATGFPRVFYLRYHGYRKFFPLWALARYRNLKRANAMRVTVGM

>seq ID 206

MTRKTIPASELDAAIVRARDALLDRQHPDGHWCFELECDATITAEYILMMHFVDEIDTALQAR-
MAKYLRAVQRLDGHGAWDLYFGGDLDISCSVKAYFALKAAGDPPDAPHMVRAREAILARG-
GAAKSNVFTRILLATFGEIPWRGTPFMPVEFVLFPRWAPIHMDKVAYWARTTMVPLL-
VLCSIRAAAKNPLGVHVQELFVTPPELEREYFPRKRGLQQAFLVADRVVRHLEPLIPRAL-
RRRAIQRAVEWSEARMNGEDGFGGIFPPMVYSYEMMVLLDYPEDH-
PLRVECKAALKKLVVHRDDGSSYCQPCLSPVWDTAWSVMALEQAPSDARTETAIARAYDWLT-
DRQVLDLRGDWENNAAPSTPPGGWAFQYENPYYPDIDDSAVVLAMLHARGKRTGQPGRY-
EMPVARCLDWIIGLQSRNGGFGAFDANCDRDFLNAIPFADHGALLDPPTEDVSGRVLLALGI-
TERPQDATARERCIQYLRDTQQPDGSWWGRWGTNYIYGTWSVLAGLGLAGVDRKLPMVRN-
GLQWLRGKQNADGGWGETNDSYARPELAGKHEDGSMAEQTAWAMLGQMAVGEGDADSVHR-
GAAYLLDAQNEDGFWMHPYHNAPGFPRIFHLKYHGYTAYFPLWALGRYRRLAAARASAMQTAKA-
ESAESMTAH

>seq_ID 96

MNDLSMTQTLGEVLPQTLIDDHAPVAAALATGAAPVDALDAAVTRATEAILAVQKDDGHWVYE-
LEADATIPAEYVLLVHFLGETPNLELEQKIARYLRRIQLPNGGWPLFTDGAMDVSAS-
VKAYFALKMIGDPEDAAHMVRARECILANGGAEAANVFTRILLALFGVVTWYAVPMMPVEIM-
LLPKWFPFHLSKVSYWARTVIVPLLVLNAKRPVARNPRGVRIDELFRGAPVTTGLL-
PRSGHQSKSWFAFFRAVDGVLRVTDGLFPKASRERAIKAAVSFVDERLNGVDGLGAIF-
PAMANSVMMYDVLGYPADHPNRAIARESIEKLLVVHEDEAYCQPCLSPVWDTSLAAHAL-
LETGDARAEEAAERGLAWLRPLQILDVRGDWISRRPDVRPGGWAFQYN-
NAHYPDVDDTAVVAMAMHRSAAVTNSNVDANAIARAREWVVGMQSSDGGWGAFEPENTQYYLN-
NIPFSDHGALLDPPTADVSGRCLSMLAQLGEMPATSEPARRAYDYLLKEQEDDGSWYG-

RWGMNYIYGTWTALCALNAAGISLEDARIKRAAQWLVSIQNADGGWGEDGTSYKLDYRGYE-
KAPSIPSQTAWALLGLMAAGYVDHPAVARGIDYLQREQRDHGLWDEERFSATGF-
PRVFYLRYHGYRKYFPLWALARYRNLKRTGEKRVTVGM

>seq ID 104

MNDMTEMHTLDATAAPAAPTVATGLDAAVARATDALLAAQNADGHWVYELEADSTI-
PAEYVLLVHYLGEAPNVELERKIARYLRRIQLPDGGWPLFTDGAPNISASVKAYFALKVIGD-
DENAEHMQRARRAIHAMGGAEMSNVFTRIQLALYGVVPWYAVPMMPVEIMLLPQWFPFHLS-
KVSYWARTVIVPLLVLNAKRPVAKNPRGVRIDELFKSAPVNTGLLPKQPHQSAGWFAF-
FRAVDGVLRLTDGLFPRYTRERAIRQAVAFVDERLNGEDGLGAIYPAMANAVMMY-
AALGYPEDHPNRAIARQSIEKLLVVGEDEAYCQPCLSPVWDTSLAAHALLETGDER-
AREAAVRGLDWLVPRQILDVRGDWISRRPHVRPGGWAFQYANAHYPDVDDTAVVAMAMDRVA-
KLDRTDAYRESIARAREWVVGMQSSDGGWGAFEPENTQYYLNNIPFSDHGALLDPPTAD-
VSGRCLSMLAQFGETSASSEPARRALDYMLKEQEPDGSWYGRWGMNYIYGTWTALCSL-
NAAGLGHDDPRVKRAAQWLLSIQNPDGGWGEDGDSYKLDYRGYERAPSTSSQTAWALLGLMAA-
GEVDHPAVARGIDHLLGTQREHGLWDETRFTATGFPRVFYLRYHGYRKFFPLWALARYRNL-
KRANATRVTVGM

>seq ID 27

MAHQETMASETSISLHTLACDATKLAGTYALRQVREDGHWYGEM-
KSNATITAEYVFLAQALGFSIEEDRDDLIKYFLSEQNTDGSWSLAYDFPGDVSVTAEAYFAL-
CLLGLDRSHPAMASAREFTLSKGGIAKVRVFTRMFFACFGLFPWSAVPELPAELILLPAAAP-
MSIYQLASWARATVVPMLVIRHHRPIYALPNGRSSSNEYLDELWVDPTDKMVPYSPS-
LWSLWNDDLTAFGFTLADNILKALGGLRWFPSRKIALRHCVAWILERQEPEGDIGGIFPPL-
HAALFALALEGYGLESSPVRRGIDALQNTYAWRDSTGLRIQGCISPILDTILMTIGLIDSSL-
PAESPLVARSSRYLKAHQQLGNEGDWRVYNGNVPSGGFNFEYFNS-
WYPDIDDTAAAILAMVKQDPNLLDLGPILSAVQWILGLQNDDGGWAAFDRENNY-
LFLNKIPFSDMDSFCDPSTADVTGRVIECFGLNGKNPIPRFFIDDMSSATERAID-
FLSTEQEADGSWYGRWGSNYIYGTSAVLCGLVYHLEGWDDTYPVMEKRHKVDTHAALDWLK-
RHQNPDGGWGERLESYYEPRLAGNGPSTASQTAWALMGLLAYLAPT-
DESITRGIQYLSRTQIKEGELAGSWKEDHYTGTGFPNHFYLCYTLYSQYFPMMALGRYTSLS-
GYRPLENLESTVEDHKGNSSDC

>seq_ID 28

MMTLREEGHKEGITPGKEQLTSDIEHSLKLATEYALSSIRSDGHWCGEL-
RSNVTITAEYIFLRHALGLDLRTDNAAYCRYILSQQNCDGSWGLAPEYPGDVSTT-
TEAYLALKLLGTSPDMPAMQQARAFVRKAGGAEKVRVFTRIFLATFGLFPWDAVPQLPVELI-
LLPSSCPINMYTLASWARGTIAPLLIICHHQPVYALPEDYLDELWLDPTDKNVPYGS-
SLRDLLSRGDITGLAFSVVDNLLYYLNGLRSVPLLRSYARRKCIQWILERQEPT-
GDWAGIFPPMHASIYAFVLEGYELNDPPVRLGIQALENFAWEDEKGKRIQACVSPVWDTALM-
SIGLCDAMSPDKQILQQAITWIRNRQLLKPCGDWRIYRSKLAPGGFSFEYENS-
HYPDVDDTAAIILAQLKQDPQSVASDSVIAAATWILGMQNPDGGWAAFDVEND-
KLFLNKIPFSDMDSLCDTSCADITGRILEAFGLMMKRELKRPVLSPMLRHACIRGITYLA-
STQESNGAWFGRWGCNYIYGTCHALGLVAPALQWLKSKQNDDGGWGEPLLSYRT-
PGTQLQQQSTPSQTAWALMGLLAHLPLTDPAIERGIRWLVCSQQPEKGNGASWPEA-
VYTGTGFPNHFYLGYDYYRHYFPMMALGRYLQASQAQA

>seq ID 94

MNDLTDMATLSAGTVPAELDAAVARATDALLAAQNADGHWVYELEADSTIPAEYVLLVHYL-
GETPNLELEQKIGRYLRRIQQADGGWPLFTDGAPNISASVKAYFALKVIGD-
DENAEHMQRARRAIHAMGGAEMSNVFTRIQLALYGAIPWRAVPMMPVEIMLLPQWFPFHLS-
KVSYWARTVIVPLLVLNAKRPLAKNPRGVRIDELFIDPPVNAGLLPRQGHQSAGWFAF-
FRAVDHVLRAVDGLFPAYTRERAIRQAVAFVDERLNGEDGLGAIYPAMANAVMMYDVLGYA-
EDHPNRAIARKSIEKLLVVHEDEAYCQPCLSPVWDTSLAAHALLETRDPRAEQAAVRGLD-
WLRPLQILDVRGDWISRRPHVRPGGWAFQYANPHYPDVDDTAVVAMAMDRAQKLNQSDTY-
RESIARAREWVVGMQSSDGGWGAFEPENTQYYLNNIPFSDHGALLDPPTADVSGRCLSMLSQL-
GETALNSDAARRALDYMLKEQEPDGSWYGRWGMNYVYGTWTALCALNAAGLGPD-
DARVKRAAQWLLSIQNKDGGWGEDGDSYKLNYRGYEPAPSTASQTAWALLGLMAAGEVNNPA-
VKRGIDYLIAEQKEHGLWDEARFTATGFPRVFYLRYHGYRKFFPLWALARYRNLKRDNTTRVTVGI

>seq ID 30

MERSSLLVPASIDSHSRESETTGLDQAIVRARAALLGRQGADGHWCFELESDC-
TITAEYILMMHFTDEIDEDLQERMARYLRATQVQETHGGWPQYVG-
GAIDLSCTVKAYYALKAAGDSPEAPHMRRAREAVLALGGAAKSNVFTRILLAM-
FEQVPWRAVPYLPVEIMLLPRWAPIHIEKMSYWARTTLVPLTILCSLKARAANPKRVDI-

RELFVTAPEQERHYFLRGGLLNRIFLGLDKFARTLDRWMPKSLRQHAIRKAEAWFLPRMNGE-
DGLGAIFPPMVNCYEAMILLGYPKDHPARKTCLRSIQKLIVHRDDGSAYCQPCV-
SPVWDTAWSAMALIHSGDDTATQTAIARAGDWLVQRQELDCRGDWEAQAPQAAPGG-
WAFQYANGYYPDIDDTALVAALLHISDRRRGQPGQHAFNIDRAVDWMLALQSRNGGFAAF-
DADNTHYYLNAIPFADHGALLDPPTEDVSGRVAACLGILKRDQDRDGLRRCIDYLRT-
TQQPDGSWWGRWGSNYIYGTWSALSGLALAGEDLRQPYLRKSVDWLRTRQHPDGGWGETND-
SYIDPHLAGTNAGISTPHSTAWAVLAQLAMGEVESDSVRRGIAFLLACQQTDGLWSHP-
SHNAPGFPRVYYLKYHGYAAYFPLYALARYRHLLNRSREQR

>seq ID 98

MNDMTEMHTLDATAAPAGLDAAVARATDALLAAQQADGHWVYELEADSTIPAEYVLLVHYLGE-APNVELEQKIARYLRRIQQPDGGWPLFTDGAPNISASVKAYFALKVIGD-DENAEHMQRARRAIHAMGGAEMSNVFTRIQLALYGVVPWYAVPMMPVEIMLLPQWFPFHLS-KVSYWARTVIVPLLVLNAKRPVAKNPRGVRIDELFKGAPVSTGLLPKQPHQSAGWFAF-FRAVDGVLRLVDGLFPRYTRERAIRQAVAFVDERLNGEDGLGAIYPAMANAVMMY-AALGYPEDHPNRAIARRSIEKLLVVGEQEAYCQPCLSPVWDTSLAAHAL-LETGDARAREAAVRGLDWLVPRQILDVRGDWISRRPHVRPGG-WAFQYANAHYPDVDDTAVVAMAMDRVAKLDRTDAYRESIARAREWVVGMQSSDGGWGAFE-PENTQYYLNNIPFSDHGALLDPPTADVSGRCLSMLAQFGETSASSE-PARRALDYMLKEQEPDGSWYGRWGMNYIYGTWTALCSLNAAGLGHDDPRVKRAAQWLL-SIQNADGGWGEDGDSYKLDYRGYERAPSTSSQTAWALLGLMAAGAVDNPAVARGVDYLL-GTQREHSLWDETRFTATGFPRVFYLRYHGYRKFFPLWALARYRNLKRANATRVTVGM

>seq ID 187

MTSDTASAAALDPRRLATSITRASRALHDVQQPDSHWVFELEADVTIPAEYVMMRHYFAEP-VDAEIEAKIAKYLRRMQNDNGGWSLFYGHEFDMSASVKAYYALKMIGDSPDAPHMKKAREAM-LARGGASRANVFTRIMLALFGQVSWKAVPMMPVEIMLLPRWFPFHLTKVSYWARTVIVPLL-VLMTLKPRAKNPRGIGVRELFLEDPQTVGPTPKAAHQSQLWFTSFDIIDRVLRITDPF-FPKGMRKRAIAKAEAFVTERLNGVDGLGAIFPAMVNSIMMYDVLGYPPNDPNRALARESVER-LLVIKDDEAYCQPCVSPVWDTALAAHSMLESGEAADIEAAKAGLDWLLPRQVLDLKGDWAD-KRPDVRPGGWAFQYNNAHYPDLDDTAVVVMAMDRVRRLDGTTKYDEAIARA-TEWILGLQSENGGWAAFDADNLEYYLNNIPFADHGALLDPPTEDVTARCLSMLAQLGDT-LETSEPMRRGVEYLRKTQLPDGSWFGRWGINYVYGTWSVLCALNAVGVPHDDPMIAKAADWLE-SIQNEDGGWGEDGNSYKLNYKGYERAATTASQTAWAT-LALMAAGRVDRDATQRGIDNLVQSQEADGFWGEPYYTGGGFPRVFYLRYHGYSKFFPLWAMA-RYRNLRSSNSRFVGAGM

>seq ID 207

MNKHSGNRTAIDPAALEMSIASATEALLAYRHADGHWAFELEADSTIPSEYILLRHYLAEPI-DVVLEAKIGNYLRRTQGAHGGWPLVHDGPFDMSASVKSYFALKMIGDSVDAAHMVKA-REAIRARGGAANSNVLTRFLLALYGVVSWRAVPVLPIEIVLLPIWSPFHLYKISYWARTTIV-PLMVLAVLKPRAKNPKGVGIEELFLQDTKSVGMNPKAPHQSWGWFLLFRGIDGILRVIEPHL-PKKLRERAIASALAFTEERLNGEDGMGAIYPSMANIVMMYDALGKDDHFPPRAIARRAIDKLL-VIGEEEAYCQPCLSPVWDTALTCHALQEVGGANAVAKAKQGLDWLKPRQVLD-VKGDWAVKAPNIRPGGWPFQYNNAHYPDLDDTAVVVMAMDRAQRHAGSKEYATAIARGRE-WIEGMQSRDGGWAAFDVNNLEYYLNNLPFADHGALLDPPTEDVTARCVSM-LAQVGEFTQRSKAVAEGIAYLRRTQHAEGSWYGRWGLNYIYGTWSVLCALNAAGIDHQDPMIR-KAVEWLVSIQSWDGGWGEDAISYRLDYSGYEQAPSTSSQTAWALLGLMAAGEVEHPAVARG-VNYLKNAQTENGLWDEQRYTATGFPRVFYLRYHGYSKFFPLWALARYRNLRSTNV

>seq_ID 29

MTTGHRQFDDGLSERERLIHEAGLTLQRSMDYAYNVVRSDGHWCGEMSS-NVTITAEYIFLRQALGLDLKTDGAAYCRHILSQQNSDGSWGLAPEYPGDVSTT-TEAYLALKMLGLSTDAPAMQQAKAFVLNAGGVAKVRVFTRIFLATFGLFPWKAVPQLPVELI-LLPSACPINIYKFASWARGTIAPLLIICHHQPVYALPNGVFAENEYLDELWQDST-NKSEPYSPSIWELLSQGDITGLTFSLLDKLLYQLNGLRSIPLLRSYALKQCMKWILERQEPT-GDWAGIFPPMHASVYAFVLEGYKLEDPPVRLGIEALENFAWEDAKGKRVQPCVSPVWDT-TLMSIALSDAATPNHQIVDRAIQWIRDRQLLEPRGDWRVYRPRLAPGGFSFEYTNS-HYPDIDDSAAIILAQVKHDPISANSSSVIAAATWILGMQNPDGGWAAFDVEND-KLFLNKIPFSDMDSLCDTSCADITGRILEAFGLLIRRVPDKDSSQLFQLLPAIRAACRR-GIRYLASTQEANGAWFGRWGCNYIYGTSHALCGLAYFLQEDQQVPAMVQPALQWLKSQQND-DGGWGESLLSYQSPERKEQRSTASQTAWALMGLLAHLPHTDIVIERGIRWLVSSQRPVET-LGSTWPEPVYTGTGFPNHFYLGYDYYRHYFPMMALGRYLRGVQG

>seq_ID 25

MLQTEAITTEGLRVRSLSPDDPLLPRIKQAIKLSGQHSRGEMHSDGHWCGEVKTNATTSAEH-
VLLCQALGINLDADREAFISWFRCTQGADGGWSTAPDQAGDISVTVEAYLALKILGLSED-
DAAMRRARDFAIAAGGVAKVRIFTRIYLALFGLFPWAAVPELPPELILLPSRVPVSIYHWSA-
WARATVVPLLIISHHRPIYALPGGGKGTSSDYLDELWCDPQNKMIPYNHDEPTAWRSDPF-
ASIFTLADSILHRLDGLRSFNPFRRFALQKCVDWILEHQEDMGDIGDIMPPLHGAMLALR-
LEGYPLHSGPIHRGLEAIERFAYRDKQGKRIQTTVSAFWDTSLMLIALGDAGMAS-
KPWLTRSLGWLQQHQRLGNYGDWKVNNHGLKAGGFSFGYFNTWYPDVDDTA-
SAVLAMIRQDERLVHSASVLDALNWLLGMQNTDGGWGAFDRDNDKHFLNKIPFSDMDAL-
CDPSTPDVTGHVLEAFGLFLALSKADALADRVVAASRRAIRYLSDTHVLSRGWYG-
RWGCNYIYGTSAVLCALAYFGSENDALSGVRVMKDAINQAIRWLETVQNPDGGWGETVD-
SYKDPSRAGSGPSTASQTAWAIMALLPYLPPSTEVIQRGMEYLLRTQTKTASQGATWHEKA-
YTATGFPKYFYMGYSLYAHYFPMMALGRYAYPCPAWHENWRLKRD

>seq ID 97

MNDLSQAQPLDAILPDFADAAPSAPAPAVTGEAP-
TASLDAAITRATEAILAAQKPDGHWVYELEADATIPAEYVLLVHYLGETPNLELEQKIA-
RYLRRIQLPDGGWPLFTDGALDISASVKAYFALKMIGDPADAEHMVRAREAILAHG-
GAETVNVFTRILLALFGVVSWRAVPMMPVEIMLLPMWFPFHLSKVSYWARTVIVPLLVL-
NAKRPVARNPRRVRIDELFRGAPVNTGPRDRAPHQHAGWFRFFSGVDVLLRAVDGLFPKSTRER-
AVRQAVAFVDERLNGEDGLGAIFPAMANSVMMYDVLGYPADHPNRAIARQSIDKLLVIKD-
DEAYCQPCLSPVWDTSLAAHALLETGEAHAEQAAERGLAWLRPLQILDVRGDWIS-
RRPNVRPGGWAFQYNNAHYPDVDDTAVVAMAMQRSATVTQSDVDRDAI-
ARAREWVVGMQSSDGGWGAFEPENTQYYLNNIPFSDHGALLDPPTADVSGRCLSMLAQLGEL-
PQNSEPAQRAFDYMLKEQESDGSWYGRWGLNYIYGTWTALCSLNAAGLPHDDPRMKRAAQWLL-
SIQNEDGGWGEGGESYKLDYHGYERAPSTASQTAWALMGLMAAGEVNHEAVARGVAYLERE-
QREHGLWDETRFTATGFPRVFYLRYHGYRKFFPLWALARFRHLKRNGLTRVAVGM

>seq_ID 176

MNSVNATVAPIDDAALGGSIGAATRGLLDLKQPDGHFVFELEADATIPSEYVLLRHYLGEP-
VDAALEAKIAVYLRRIQGAHGGWPLVHDGPFDMSASVKAYFALKMIGDSIDAPHMA-
RAREAILSRGGAANVNVFTRFLLSLFEVLTWRSAPVLPIEIMLLPMWSPFHINKISYWARTT-
MVPLMVLAALKPRARNPRGIGIRELFLQDPATVGTPKRAPHQSPAWFTLFNSLDWILR-
KIEPLFPKRLRARAIEKAIAFVEERLNGEDGLGAIFPPMVNTVMMYDALGFPPEHPPRAVARR-
GIDKLLVIGKDEAYCQPCVSPIWDTALTCHALLEAGGPEALSGAGKSLDWLLPKQEL-
VLKGDWAVKRPDVRPGGWAFQYANAHYPDLDDTAVVVMAMDRVRRNDRSDKYNEAIARGRE-
WIEGMQSRDGGFAAFDADNLEYYLNNIPFSDHAALLDPPTEDVTARCVSMLAQLGETVRS-
SPSMAAGVDYLRRTQLKEGSWYGRWGLNYIYGTWSVVCALNAAGVDHQDPAM-
RKAVDWLVSIQNADGGWGEDAVSYRLDYKGFEGAPTTASQTAWALLALMAAGEVENPA-
VARGMKYLIDTQTKKGLWDEQRFTATGFPRVFYLRYHGYSRFFPLWALARYRNLRSTNSKVVGVGM

>seq ID 210

MDSGTFNPGGERGNTLDASIDAARAALLGYRRDDGHWVFELEADCTIPAEYVLLRHYLGEPI-
DAALEAKIAVYLRRTQGAHGGWPLVYDGEFDMSATVKGYFALKMIGDSIDAPHMAKA-
REAILSRGGAVHANVFTRFLLAMFGILTWRAVPVLPVEIMLLPMWSPFHLNKISYWARTTIV-
PLMVLAALKPRAVNRLGVGLDELFLQDPKSIGMPARGPHQNRGLFALFGAIDAVLR-
VIEPLIPKKLRKHAIDRAVAFVEERLNGEDGLGAIYPPMANTVMMYKVLGYPEDHPPRAITRR-
GIDLLLVIGEEEAYCQPCVSPIWDTSLTCHALLEAGGAEAAQPVREGLDWLLPKQVLD-
LKGDWAVKAPNVRPGGWAFQYNNAHYPDLDDTAVVVMALDRARRDQPSAAYDNAIARGRE-
WIEGMQSDDGGWAAFDVNNTEYYLNNIPFSDHGAMLDPPTEDVTARCVSMLAQL-
GETEQTSKAVARGVAYLRKTQLPDGSWYGRWGMNYIYGTWAVLCAL-
NAAGVDHQDPAIRKAVAWLASIQNADGGWGEDGVSYRLDYRGYETAPSTASQTAWALLSIMAA-
GEVDHPAVARGIEYLKGTQTEKGLWDEQRHTATGFPRVFYLRYHGYSKFFPLWGLARYRNL-
RATNSKVVGVGM

>seq ID 23

MTTGHRQFDDGLSERERLIHEAGLTLQRSMDYAYNVVRSDGHWCGEMSS-
NVTITAEYIFLRQALGLDLKTDGAAYCRHILSQQNSDGSWGLAPEYPGDVSTT-
TEAYLALKMLGLSTDAPAMQQAKAFVLNAGGVAKVRVFTRIFLATFGLFPWKAVPQLPVELI-
LLPSACPINIYKFASWARGTIAPLLIICHHQPVYALPNGVFAENEYLDELWQDPT-
NKSEPYSPSIWELLSQGDITGLTFSLLDKLLYQLNGLRSIPLLRSYALKQCMKWILERQEPT-
GDWAGIFPPMHASVYAFVLEGYKLEDPPVRLGIEALENFAWEDAKGKRVQPCVSPVWDT-
TLMSIALSDAATPNHQIVDRAIQWIRDRQLLEPRGDWRVYRPRLAPGGFSFEYTNS-
HYPDIDDSAAIILAQVKHDPISANSSSVIAAATWILGMQNPDGGWAAFDVEND-
KLFLNKIPFSDMDSLCDTSCADITGRILEAFGLLIRRVPDKDSSQLFQLLPAIRAACRR-
GIRYLASTQEANGAWFGRWGCNYIYGTSHALCGLAYFLQEDQQVPAMVQPALQWLKSQQND-

DGGWGESLLSYQSPERKEQRSTASQTAWALMGLLAHLPHTDIVIERGIRWLVSSQRPVET-
LGSTWPEPVYTGTGFPNHFYLGYDYYRHYFPMMALGRYLRGVQG

>seq_ID 91

MNDLSQAHVLGAAMPETAGEAQNAQAAANSAAAAAEASAVLAPSLDAAITRATDAIL-
AAQKPDGHWVYELEADATIPAEYVLLVHYLGETPNVELEQKIARYLRRIQLPNGGWPLFTD-
GAIDISASVKAYFALKMIGDPVDAEHMVRAREAILAHG-
GAETVNVFTRILLALFGVVSWRAVPMMPVEITLLPMWFPFHLSKVSYWARTVIVPLLVL-
NAKRPLARNPRRVRIDELFRGAPVNTGMPARAPHQHVGWFGFFRVVDTVLRAVDGLFPKATRER-
AVREAVAFVDQRLNGEDGLGAIFPAMANSVMMYDVLGYPADHPNRAIARRSIEKLLVIKD-
DEAYCQPCLSPVWDTSLAAHALLETGDARAEQAAERGLAWLRPLQILDVRGDWIS-
RRPNVRPGGWAFQYNNAYYPDVDDTAVVAMAMHRSEALTHSGADREAI-
ARAREWVVGMQSSDGGWGAFEPENTQYYLNNIPFSDHGALLDPPTADVSGRCLSM-
LAQLGEFPQNSEPAQRALDYMLKEQEADGSWYGRWGLNYIYGTWTALCSLNAAGLPHDDPR-
IRRAAQWLLSIQNEDGGWGEGGESYKLDYRGYERAPSTASQTAWALMGLMAAGEVDHEAVAR-
GIEYLQREQREHGLWDETRFTATGFPRVFYLRYHGYRKFFPLWALARYRHLKRNGLTRVAVGM

>seq ID 213

MDSGSYTTGVERNALEASIDAARSALLNYRRDDGHWVFELEADCTIPAEYVLLRHYLGEPVDA-
ELEAKIAVYLRRIQGAHGGWPLVHDGDFDMSASVKGYFALKMIGDSIDAPHMVRAREAIRSRG-
GAIHSNVFTRFLLTLYGVTTWRAVPVLPVEIMLLPSWSPFTLTKISYWARTTMVPLL-
VLCALKPQAKNPKGVGIDELFLQDPKTIGMPVKAPHQNWALFKLFGSIDAVLRVIEP-
VMPKGIRKRAIDKALAFIEERLNGEDGMGAIFPPMANAVMMYEALGYPEDYPPRASQRR-
GIDLLLVDRGDEAYCQPCVSPVWDTALASHAVLEADGHEGAKSVRPALDWLLPRQVLD-
VKGDWAVKAPNVRPGGWAFQYNNAHYPDLDDTAVVVMALDRARKDQPNPAYDAAIARARE-
WIEGMQSDDGGWGAFDINNTEYYLNNIPFSDHGAMLDPPTEDVTARCVSMLAQLGETMDSSPA-
LARAVGYLRDTQLAEGSWYGRWGMNYIYGTWSVLCALNAAGVPHADPMIRKAVAWLES-
VQNRDGGWGEDAVSYRLDYRGYESAPSTASQTAWALLALMAAGEVDHPAVARGIEY-
LKSTQTEKGLWDEQRYTATGFPRVFYLRYHGYSKFFPLWALARYRNLQATNSKVVGVGM

>seq ID 196

MSMTSREDHDASSLISQVEHALKLSNDYALGLVHPDGHWYGEMNTNVTVTAEYVFLRQAL-
RLDLKTDIAAYCHYLLSQQNSDGSWGLAPEYPGDVSTSTEAYLALKILGTSPHTPAMRNA-
RAFVLKAGGIARVRIFTRIFLATFGLFPWSAVPELPVELMLLPSICPINIYKFAS-
WARGTIAPLLIICHHQPVYSLPNGKSTDNDYLDELWVDCTNKSVPYGLPLWDLMSQGEFAG-
LAFGVLDKVLYQLNGLRSIPLIRAYARKQCIQWILERQEKTGDWAGIFPPMHANMYAFT-
LEGYKLDDDPVRLGFQALERFAWEDEKGKRIQACVSPVWDTALMTIGLCDAMSPNKQTIDHA-
LAWIRARQLLEPRGDWRVYRPQLAPGGFSFEYENSWYPDVDDTAAIILAQVKHDNGSIGSNS-
VIAAATWILGMQNPDGGWAAFDVENDKLFLNKIPFSDMDSLCDTSCADITGRILEAYGLMMM-
KYFSAKSDADPLLHTLRAACMRGMHYLASTQEPNGSWYG-
RWGCNYIYGTSHVLCGLAYFVEKRLVCVMVKSALQWLKSRQNDDGGWGESLLSYQSPDRE-
QQASTPSQTAWALMGLLSHLPVTDDAIERGIRYLVSSQRPEKGIGSS-
WPQAEYTGTGFPNHFYLGYDYYRHYFPMMALGRYLQGSRGLN

>seq ID 99

MNDLSQTQPLAAVLPEAADAPAVADASATAAPEPVQAASPSALDASITRATD-
TILAAQKPDGHWVYELEADATIPAEYVLLVHYLGETPNLELEQKIARYLRRIQLPNG-
GWPLFTDGALDISASVKAYFALKMIGDPVDAEHMVRARDAILAHGGAERAN-
VFTRILLALFGVVSWRAVPMMPVEIMLLPVWFPFHLSKVSYWARTVIVPLLVLNAKRPLARN-
PRKVRIDELFRAAPVNTGMNERAPHQHAGWFGFFRCVDTVLRAVDGLLPKATRER-
AIRAAVAFVDERLNGEDGLGAIFPAMANSVMMYDVLGYPADHPHRAIARKSLDKLLVIKD-
DEAYCQPCLSPVWDTSLAAHALLETGEARAEQAAERGLAWLRPLQILDVRGDWIS-
RRPNVRPGGWAFQYNNAHYPDVDDTAVVAMAMHRSAALTQSDVDREAI-
ARAREWVVGMQSSDGGWGAFEPENTQYYLNNIPFSDHGALLDPPTADVSGRCLSMFAQIGEL-
PQSSEPARRAFDYMLQEQEPDGSWYGRWGLNYIYGTWTALSSLNAAGMPHDDPRM-
RRAAQWLVSIQNEDGGWGEGGESYKLDYHGYERAPSTASQTAWALLGLMAAGEVN-
HEAVARGIDYLQREQREHGLWDETRFSATGFPRVFYLRYHGYRKFFPLWALARFRHLKRHGLTRVTVGM

>seq ID 85

MIRRMNKSAPSPWSALDAAIARGRDALVRLQQPDGSWCFELESDATITAEYILMMHFMD-
RIDDVRQERMARYLRANQRLDTHGAWDLYVDGAPDVSCSVKAYFALKAAGDSEHAPHMI-
RARDAILKLGGAARSNVFTRILLATFGQVPWRAAPFMPIEFVLFPKWVPISMYKVAYWARTT-
MVPLLVLCSLKARARNPRNVSIRELFVTPPEQERHYFLPARGMRRLFLALDRTVRPIEPLL-
PKRLRQRAIRHAEAWCAERMNGEDGLGGIFPPIVYSYQMMQVLGYPDDHPLRRD-
CENALEKLLVTRPDGSMYCQPCLSPVWDTAWSTMALEQARGVAAPETGDTASGALRELDERIA-

RAYDWLATRQVNDLRGDWIENAPADVEPGGWAFQYANPYYPDIDDTALVTAMLDRRGRTHR-
GADGTHPYASRVARALDWMRGLQSRNGGFAAFDADCDRMYLNAIPFADHGALLDPP-
TEDVSGRVLLCFGVTKRAADRASLAHAIDYVKRTQQPDGSWWGRWGTNYLYGTWSVLAG-
LALAGEDKSQPYITRALDWLRARQHADGGWGETNDSYIDPKLAGTNDGESTSNCTA-
WALLAQMAFGDCESDSVKRGIAYLQSVQQEDGFWWHRSHNAPGFPRIFYLKYHGYTAYFPLWA-
LARYRRLAGAKDADATRSPASATPATDNALA

>seq ID 93

MIRAMNKSALSPWSALDTAIARGRDALARLQQPDGSWCFELESDATITAEYILMMHFMDRID-
DALQERMARYLRAIQRLDTHGAWDLYVDGAPDVSCSVKAYFALKAAGDSEHAPHMI-
RAREAILKLGGAARSNVFTRILLATFGQVPWRATPFMPIEFVLFPKWVPISMYKVAYWARTT-
MVPLLVLCSLKARARNPRNVAIPELFVTPPDQERHYFPPTRGMRRAFLILDRVVRHVEPLL-
PKRLRRRAIRHAEAWCAQRMNGEDGLGGIFPPIVYSYQMMDVLGYPEDHPLRRD-
CENALAKLLVTRPDGSVYCQPCLSPVWDTAWSTMALEQARSVAVPESDESARALDELDARI-
ARAYDWLATRQVNDLRGDWIENAPADTQPGGWAFQYANPYYPDIDDSAVVTAMLDRR-
GRTHRNADGSHPYAARVARALDWMRALQSRNGGFAAFDADCDRLYLNAIPFADHGALLDPP-
TEDVSGRVLLCFGVTRRAEDRASLARAIDYVKRTQQPDGSWWGRWGTNYLYGTWSVLAGLTLA-
GEDPSQPYIARALEWLRAHQHADGGWGETNDSYLDPALAGTNGGESTSNCTAWALLAQMAF-
GDCASDSVKRGIAYLQSVQQDDGFWWHRSHNAPGFPRIFYLKYHGYTAYFPLWALARYRR-
LAGAAEARARASSGRAPHAADTALA

>seq_ID 168

MGKVETLHRMSTQDITLDDVERRVSLASKALMRLAGPDGHWCFELEADATIPSEYILYHH-
FRGSIPSAELEGKIANYLRRTQSAQHDGWSLVHDGPFDMSATVKAYFALKMIGDSIEAPHMR-
RAREAILRRGGAAHANVFTRTLLALYGEVPWSAVPVMPVEVMLLPRWFPFHLDKVSYWART-
VMVPLFVLQAKKPRARNPRGIGIQELFVEPPERVKRWPAGPQESSPWRPVFAAIDKVLQK-
VEGSFPAGSRARAIDKAVAFVSERLNGEDGLGAIFPAMVNAVLMYEALGYPEDHPLVATARSS-
VEKLVTVKEHEAYVQPCLSPVWDTALSAHALMEAGGVEAERHAKRALDWLKPLQVLDIK-
GDWAASKPNVRPGGWAFQYANPHYPDLDDTAVVVMAMDRAQVRRSPGPDAADYGQSI-
ARAREWVEGLQSRDGGWAAFDADNTYHYLNYIPFSDHGALLDPPTADVTARCVSMLAQLGE-
TRESCPPLDRGVAYLLADQEADGSWYGRWGMNYIYGTWSVLCALNAAGVDPASEP-
VRRAVNWLTTIQNPDGGWGEDAASYKLEYRGYERAPSTASQTAWALLGLMAAGEADSPAVAR-
GINYLTRSQGADGLWTEDRYTATGFPRVFYLRYHGYAKFFPLWALARYRNLQQSNSRRVAVGM

>seq ID 184

MKKFGGMARTSLQAQSPGSNNTPSMDEKMLKAGLEAARGALLAQQREDGHWCFPLEADCTI-
PAEYILMMHFMDEVDLDLEVRIARFIREKQDVAHGGWPLYYGGEFDLSCSVKAYYALKIVGD-
SPDAPHMVRARAAILKHGGAARANVFTRLLLAMYDQLPWRGVPFVPVEIILFPKWFPFHTS-
KVAYWSRTVMVPLSILCSLKARAANPRKVAIRELFTVPPGEERNYFPVRTALNRVFLLIERT-
LSLLEPFIPQGVRRLALRRAESWIVERLNGDSGLGAIFPAMVNAGEALALLGYPYDHPARE-
QCRKALRLLLVEEGERTWCQPCVSPVWDTVLTCLAFQEDTEVDQKPIRKALD-
WLVPCQVLDAPADWQEDHPGLPGGGWAFQYANPHYPDLDDTAAVAWALYQADPKAYQESIS-
RAADWLAGMQSSNGGFAAFDSDNTYYYLNEIPFADHGALLDPPTSDVSARCAGFLALYGQS-
RHKQALERSLAYLFNEQEASGAWFGRWGSNYIYGTWSVLEAFRLAGIDAGH-
PAIRRAVHWLKSVQREDGGWGESNDSYLSPQQAGQFHTSTSFHTAWALLALMGAGEWRSHE-
VHRGIAYLLREQDSDGLWHEPWFTAPGFPRVFYLKYYGYTKYFPVWALTRFHALNRKFPG

>seq ID 12

MMYNNQWYFNQFNDIFCFPEQQKEYFPPTGTNISLNLKKRPDRQLLAHGASDLNGPFHLSQHN-
AFSAMLLAEVQKVLRLAVGHSLDLQRTDGAWCGEVHSNATFTAQYVFLQQQLGLPLDPTEI-
EGLSRWLFSQQNEDGSWGLGPGLGGDVSTTTETYLALKILGVSPEDPRMAAARSSIIKAGSL-
PATRMFTRVFLASFGLIPWSAVPPLPAELILLPTLFPVNIYNLSSWARATCVPLLLIRH-
HEPLHSLPNGRHAENDFLDELWTKDIPRDFCYTTPLSRMWRLGDYAGIFFTSADH-
GLRFLGQYFNSPLRNLSRRKIINWILDHQEQSGEWAGYWPPQHNNIWALSLEGYSLDHPVLRR-
GIAAVKSFVLHDVTGMRAQVTVSQVWDTALMSIALSDSAPSTGIISPTQAIDWLMHHE-
VASHRGDWRVLRPKLATGGFCFEEFNTLYPDVDDTAAVIMALIKSNPAHLIS-
GCVRRAAQWILGMQNRDGGWGAFDWNNDKFFLNKIPFSDMDSLCDPSTPDVTGRIIECFGMM-
MAGRHGYSLDGPLESRLRASSQLAIAYLLGCQENNGSWWGRWGVNYLYGTS-
NVLCGLAYYYDRSGLSKGDGKSNSHIVSAVDRASEWLKARQHSNGGWGEGPE-
SYDSAQLAGCGQPTASQSAWVTMALLNYLSPTDEVIQRGISYLVRSQVKYGDESRAT-
WPLERYTATGFPGHLYMEYDYYRHYFPIMALGRYVNKLSESHKLL

>seq ID 100

MIRRMTTPTPSPWSALDTAIARGRDALVRLQQPDGSWCFELESDATITAEYILMMHFMD-
KIDDLRQEKMARYLRANQRLDTHGGWALYVDGDPDVSCSVKAYFALKAAGD-

SEHAPHMVRARDAILKLGGAARANVFTRILLATFGQVPWRAAPFMPIEFVLFPKWVPISMY-
KVAYWARTTMVPLLVLCSLKARARNPRNISIRELFVTPPDEERQYFPPARGMRKLFLALDRT-
VRHVEPLMPKGLRQRAIRHAEAWCAERMNGEDGLGGIFPPIVYCYQMMEVLGYPDDHPLRRD-
CENALEKLLVTRPDGSMYCQPCLSPVWDTAWSTMALEQARGVAVAEDGEPGDARRALDERIT-
RAYDWLAERQVNDLRGDWIENAPADVQPGGWAFQYANPYYPDIDDTAVVTAMLDRRGRTHA-
NADGTNPYATRVARALDWMRGLQSRNGGFGAFDADCDRLYLNAIPFADHGALLDPP-
TEDVSGRVLLCFGVTKRADEHASLARCIDYVKRTQQPDGSWWGRWGTNYIYGTWSVLAG-
LALAGEDKSQPYIARAIEWLRARQHADGGWGETNDSYIDPKLGGTNGGESTSNFTA-
WALLAQMAFGDCESDSVKRGIAYLQSVQQEDGFWWHRSHNAPGFPRIFYLKYHGYTAYFPLWA-
LARYRRLAGVANKRVSTADKTADAMA

>seq_ID 84

MIRRMNQSAPSSWSALDAAIARGRDALVRLQQPDGSWCFELESDATITAEYILMMHFMD-
RIDDVRQEKMARYLRANQRLDTHGAWDLYVDGAPDVSCSVKAYFALKAAGDSEHAPHMI-
RARDAILKLGGAARSNVFTRILLATFGQVPWRAAPFMAVEFVLFPKWVPISMYKVAYWARTT-
MVPLLVLCSLKARARNPRNVSIRELFVTPPEQERHYFPPARGMRRLFLALDRTVRPIEPLL-
PKRLRQRAIRHAEAWCAERMNGEDGLGGIFPPIVYSYQMMQVLGYPDDHPLRRD-
CENALEKLLVTRPDGSMYCQPCLSPVWDTAWSTMALEQARGVAAPETGDTATGAPRDLDGRIA-
RAYDWLATRQVNDLRGDWIENAPADVEPGGWAFQYANPYYPDIDDTALVTAMLDRR-
GRTHRAADGTHPYASCVSRALDWMRGLQSRNGGFAAFDADCDRMYLNAIPFADHGALLDPP-
TEDVSGRVLLCFGVTKRAADRASLARAIDYVKRTQQPDGSWWGRWGTNYLYGTWSVLAG-
LALAGEDKSQPYIARALDWLRARQHADGGWGETNDSYLDPKLAGTNGGESTSNCTA-
WALLAQMAFGDCESDSVKRGIAYLQSVQQEDGFWWHRSHNAPGFPRIFYLKYHGYTAYFPLWA-
LARYRRLAGAKDAGATRSGASGASATSVTDDALA

>seq ID 86

MIRRMNKSAPSPWSTLDTAIARGRDALVRLQQPDGSWCFELESDATITAEYILMMHFMD-
RIDDVRQEKMARYLRANQRLDTHGAWDLYVDGAPDVSCSVKAYFALKAAGDSEQAPHMI-
RARDAILKLGGAARSNVFTRILLATFGQVPWRAAPFMPIEFVLFPKWVPISMYKVAYWARTT-
MVPLLVLCSLKARARNPRNVSIRELFVTPPEQERRYFPPARGMRRLFLALDRAVRHIEPLM-
PKRLRQRAIRHAQAWCAERMNGEDGLGGIFPPIVYSYQMMQVLGYPDDHPLRRD-
CENALEKLLVTRPDGSVYCQPCLSPVWDTAWSTMALEQARGVAAPETGETAAGTLRELDERIA-
RAYDWLAARQVNDLRGDWIENVPADVEPGGWAFQYANPYYPDIDDSALVTAMLDRRGRTHR-
HADGTNPYAPRVARALDWMRGLQSRNGGFAAFDADCDRMYLNAIPFADHGALLDPP-
TEDVSGRVLLCFGVTKRAEDRASLARCIDYVKRTQQPDGSWWGRWGTNYLYGTWSVLAG-
LALAGEDKSQPYIARALDWLRARQHADGGWGETNDSYLDPTLAGTNGGESTSNCTA-
WALLAQMAFGDCESDSVKRGIAYLQSVQQEDGFWWHRSHNAPGFPRIFYLKYHGYTAYFPLWA-
LARYRRLAGAAAAPPAALVAADTALA

>seq ID 80

MIRRMNKPAPSPWSALDTAIARGRDALMRLQQPDGSWCFELESDATITAEYILMMHFMDKID-
DARQEKMARYLRAIQRLDTHGGWDLYLDGDPDLSCSVKAYFALKAAGD-
SEHAPHMVRARDAILKLGGAARSNVFTRILLATFGQVPWRATPFMPIEFVLFPKWVPISMY-
KVAYWARTTMVPLLVLCSLKARARNPRNIAIPELFVTPPDQERQYFPPARGMRRAF-
LALDRVVRHVEPLLPKRLRQRAIRHAQAWCAERMNGEDGLGGIFPPIVYSYQMMDVLGYPD-
DHPLRRDCENALEKLLVTRPDGSMYCQPCLSPVWDTAWSTMALEQARGVAVPEAGAPA-
GALDELDARIARAYDWLAERQVNDLRGDWIENAPADTQPGGWAFQYANPYYPDIDDSAVI-
TAMLDRRGRTHRNADGSHPYAARVARALDWMRGLQSRNGGFAAFDADCDRMYLNAIPFADH-
GALLDPPTEDVSGRVLLCFGVTKRADDRASLARAIDYVKRTQQPDGSWWGRWGTNYLY-
GTWSVLAGLALAGEDPSQPYIARALAWLRARQHADGGWGETNDSYIDPALAGTNAGESTS-
NCTAWALLAQMAFGDGESESVKRGIAYLQSVQQDDGFWWHRSHNAPGFPRIFYL-
KYHGYTAYFPLWALARYRRLAGGASSAGAHTVPASTGADAALA

>seq_ID 82

MNKPAPSPWSALDTAIARGRDALMRLQQPDGSWCFELESDATITAEYILMMHFMD-KIDDVRQEKMARYLRAIQRLDTHGGWDLYVDGDPDVSCSVKAYFALKAAGD-SEHAPHMVRARDAILALGGAARSNVFTRILLATFGQVPWRATPFMPIEFVLFPKWVPISMY-KVAYWARTTMVPLLVLCSLKARARNPRNIAIPELFVTPPDEERHYFPPARGMRRAF-LALDRVVRHVEPLLPKRLRQRAIRHAQAWCAERMNGEDGLGGIFPPIVYSYQMMDVLGYPD-DHPRRRDCENALEKLLVTRTDGSMYCQPCLSPVWDTAWSTMALEQARAVAVPEAGARA-SALDELDARIARAYDWLAERQVNDLRGDWIENAPADTQPGGWAFQYAN-PYYPDIDDTAVVTAMLDRRGRTHRNADGSHPYAARVARALDWMRGLQSRNGGFAAF-DADCDRMYLNAIPFADHGALLDPPTEDVSGRVLLCFGVTKRAAD-RASLARAIDYVKRTQQPDGSWWGRWGTNYLYGTWSVLAGLALAGEDPSQPYIARALAWL-RARQHADGGWGETNDSYIDPTLAGTNAGESTSNCTAWALLAQMAFGDCESESVRR-

GIAYLQSVQQDDGFWWHRSHNAPGFPRIFYLKYHGYTAYFPLWALARYRRLASGVS-SAGVHAVPASTGADAALA

>seq_ID 108

MNDLSQTQPRDAVLPEAAGAVPPASAPAPAAASEAPAASLDTAITRATDAIL-AAQKPDGHWVYELEADATIPAEYVLLVHYLGETPNVELEQKIARYLRRIQLPDGGWPLFTD-GAPDVSASVKAYFALKMIGDPADAEHMVRAREAILANG-GAEAVNVFTRILLALFGVVSWRAVPMMPVEIMLLPMWFPFHLSKVSYWARTVIVPLLVL-NAKRPLARNPRRVRIDELFRGAPVNTGPRDRAPHQHAGWFRFFSGVDMLLRAVDGLFPKATRER-AVRAAVAFVDERLNGEDGLGAIFPAMANSVMMYDVLGYPADHPNRAIARQSIEKLLVIKD-DEAYCQPCLSPVWDTSLVAHALLETGEARAEQAAERGLAWLRPLQILDVRGDWIS-RRPNVRPGGWAFQYNNDYYPDVDDTAVVVMAMHRSAALTHSEVDREAI-ARAREWVVGMQSSDGGWGAFEPENTQYYLNNIPFSDHGALLDPPTADVSGRCLSMLAQLGEL-PQGSEPAQRAFAYMLKEQEPDGSWYGRWGLNYIYGTWTALCSLNAAGMPHDDPRMKRAAKWLL-SIQNEDGGWGEGGESYKLDYHGYERAPSTASQTAWALMGLMAAGEVNHEAVARGVAYLQRE-QREHGLWDETRFTATGFPRVFYLRYHGYRKFFPLWALARFRHLKRHGLTRVAVGM

>seq_ID 169

MREAAVSKVETLQRPKTRDVSLDDVERGVQNAARALTEMTQTDGHICFELEADATIP-SEYILFHQFRGTVPRDGLEAKIGNYLRRTQSKVHGGWALVHDGPFDMSATVKAYFALKMIGD-DIEAPHMRAARKAILQRGGAANANVFTRILLALYGEVPWAAVPVMPVEVMHLPKWFPFHLD-KVSYWARCTMVPLFVIQAKKPRAKNPRGIGVAELFVTPPDSVRTWPGSPHATWPWTPIF-GAIDRVLQKTQDHFPKVPRQRAIDKAVAWVSERLNGEDGLGAIFPSMVNSVLMYEVLGYPP-DHPQVKIALEAIEKLVAEKDDEAYVQPCLSPVWDTALTSHAMLETGGAAAEANARAGLDWLK-PLQILDIKGDWAETKPNVRPGGWAFQYANPHYPDLDDTAVVVMAMDRAQRQHGLVSGMPDYSA-SIARAREWVEGLQSADGGWAAFDADNNHHYLNHIPFSDHGALLDPPTADVTARVVSMLSQL-GETRETSRALDRGVTYLLNDQEKDGSWYGRWGMNFIYGTWSVLCAL-NAAGVDPQSPEIRKAVAWLIRIQNPDGGWGEDASSYKLNPEFEPGYSTASQTA-WALLALMAVGEVDDPAVARGVNYLMRTQGQDGLWNEERYTATGFPRVFYLRYHGYPKFFPLWA-MARFRNLKKGNSRQVQFGM

>seq_ID 163

MREAAVSKVETLQRPKTRDVSLDDVERGVQSAARALTDMTQADGHICFELEADATIP-
SEYILFHHFRGTEPRAGLEAKIGNYLRRTQSKVHGGWALVHDGPFDMSASVKAYFALKMIGD-
DIEAPHMRAVRKAILQRGGAANANVFTRILLALYGEVPWTAVPVMPVEVMHLPKWFPFHLD-
KVSYWARCTMVPLFVIQAKKPRAKNPRGVGVAELFVTPPDSVRTWPGSPHATWPWTPIF-
GAIDRVLQKTQDHFPKVPRQRAIDKAVAWVSERLNGEDGLGAIFPSMVNSVLMYEVLGYPP-
DHPQVKIALEAIEKLVAEKDDEAYVQPCLSPVWDTALTSHAMLEVGGTQAEANARAGLDWLK-
PLQILDIKGDWAETKPNVRPGGWAFQYANPHYPDLDDTAVVVMAMDRAQRQHGLVSGMPDYS-
TSIARAREWVEGLQSADGGWAAFDADNNHHYLNHIPFSDHGALLDPPTADVTARVVSMLAQL-
GETRETSRALDRGVTYLLNDQEKDGSWYGRWGMNFIYGTWSVLCAL-
NAAGVDPQSPEIRKAVAWLIRIQNPDGGWGEDASSYKLNPEFEPGYSTASQTA-
WALLALMAVGEVDDPAVARGVNYLMRTQGADGLWNEERYTATGFPRVFYLRYHGYPKFFPLWA-
MARFRNLKRGNSRQVQFGM

>seq_ID 105

MKPNHTFSPAALDAAILRGRDTLSGLQQPDGSWCFELESDATITAEYILMMHFMDKIDE-
VRQAQMARYLRAIQRVETHGAWDLYVDGAPDISCSVKAYFALKAAGDSEHAPHMIRAREAIL-
KLGGAARSNVFTRILLATFGQVPWRAAPFMPVEFVLFPKWVPISMYKVAYWARTTMVPLL-
VLCSLRARARNPRNVSIAELFVTPPDEERHYFPPAKGMRKLFLALDRTVRHLEPLL-
PRRLRQRAIRHAEAWCAERMNGEDGLGGIFPPIVYSYQMMEVLGYPEDHPLRRDCEDA-
LEKLLVTRADGSVYCQPCLSPVWDTAWSTMALEQARGATPAAPDTQVSERELDARI-
ARAYDWLATRQVNDLEGDWRENARPGTLPGGWAFQYANPYYPDIDDSAVVTAMLDRRGRAQA-
RASGENPYAERVTRALDWMRGLQSRNGGFGAFDADCDRLYLNAIPFADHGALLDPP-
TEDVSGRVLLCFGVTKRPADRAAAARAIEYVKRTQQPDGSWWGRWGTNYLYGTWSVLAGLALS-
GEDKSQPYIARALDWLRAHQHADGGWGETNDSYADPRLRATNYGESTSNCTAWALLAQMAF-
GDWQSDSVRRGIAYLLSVQQDDGFWWHRSHNAPGFPRIFYLKYHGYTAYFPLWALARYRR-
LAGAQAAPSSPGPGTAATIADPAVA

>seq_ID 211

MTSGTTILGAERGRTLDASIDAARAALLGYRRDDGHWVFELEADCTIPAEYVLLRHYLGEP-
VDAALEAKIAVYLRRTQGAHGGWPLVHDGEFDVSATVKAYFALKMIGDSIDAPHMAKA-
REAILARGGAIHVNVFTRFLLSMFGILTWRSVPVLPVEIMLLPMWAPFHLNKISYWARTTIV-
PLMVLAALKPRAVNKLDIGLDELFLQDPQSIGMPAKAPHQSWGLFTLFGSIDAVLR-
VIEPLIPKKLRSYAIGRAVAFIEERLNGEDGLGAIYPPMANTVMMYKVLGYGEDHPPRAITRR-

GIDLLLVVGEEEAYCQPCVSPIWDTSLTCHALLEAGGAEAALPVRKGLDWLIPKQVLD-
LKGDWAVKAPNVRPGGWAFQYNNAHYPDLDDTAVVVMALDRARRDQPSAAYDNAIARGRE-
WIEGMQSDDGGWAAFDVNNTEYYLNNIPFSDHGALLDPPTEDVTARCVSMLAQLGETAETSSA-
LARGVAYLRKTQLAEGSWYGRWGLNYIYGTWSVLCALNAAGVAHQDPAM-
RKAVAWLASIQNADGGWGEDAVSYRLDYRGYESAPSTASQTAWALLALMAAGEVDHPAVARG-
VEYLKGTQTEKGVWDEQRYTATGFPRVFYLRYHGYSKFFPLWALARYRNLRATNSKVVGVGM

>seq_ID 76

MDSVNATAREAKESKISESEILESSIASATQGVLGFQQSDGHWVFELEADCTIPAEYVLL-
RHYLAEPVDTVLEAKIGNYLRRVQGAHGGWPLVHDGEFDMSASVKAYFALKMIGD-
SIDAPHMVRAREAIHARGGAIHSNVFTRFMLAMFGIVTWRAVPVLPIEIMLLPFWSPFHINKI-
SYWARTTMVPLMVIAALKPRAKNPKGVGIDELFLQDPRSIGMTAKAPHQSMAWFLL-
FRSLDAILRVIEPLFPKSLRKRAIDTALAFSEERLNGEDGMGAIYPPMANLVMMYDALGKDE-
NYPPRAVTRRGIDKLLVIGDDEAYCQPCVSPVWDTTLTAHALLEAGGDKAGPAAKHGLD-
WLIPKQELEVKGDWAVKRPDVRPGGWAFQYNNAYYPDLDDTAVVVMSMDRMRREH-
GVTGYDSAIDRGREWIEGMQSDDGGWAAFDVNNLEYYLNNIPFSDHGALLDPP-
TEDVTARCVSMLAQLGETAKTSKHVADGVAYLRKTQHPEGSWYGRWGMNFIYGTWSVLCALN-
MAGVRHDDPMIRKAADWLASIQNKDGGWGEDTVSYRLDYKGWEAAPSTASQTAWALLALMAA-
GEVDHPAVARGVEYLIATQNEKGLWDEQRYTATGFPRVFYLRYHGYSKFFPLWGLARYRN-
LRNTNSRVVGVGM

>seq_ID 179

MEQQPELISGGVGGVAYPWDLGSQAIEEAILAARAALLAHLHPDGYWCFELEADCTI-PAEYIMMMHYTGELEAALELKLARYIRECQLQEGGWPLYYGGAMDIS-CSVKAYFALKLAGDDPEAAHMRRARKAVLERGGAVNANVFTHIALALFGEIPWRG-VPFMPPEILLLPRWFPFHLSKVSYWSRTVMVPLFILAAHKPRARNPRAIHI-SELFVTDPQLETGYFKARSRLNRLFITLDALGRRIEPFIPRAVRAKALRRAAEWFITRLN-GEHGLGAIFPAMVNSYEALELLGYAADHPLRQQVRKGLRDLVVEQADRAYCQPCL-SPIWDTALACLALQEADRGSSSAQVRHALDWLQARQLLDTPGDWSEQHPSLPGGGWPFQFRND-HYPDLDDTAIVAWAMQRASDPERYGAAIRRATVWLLGMQSANGGFAAFDSDNTRYYLNEI-PFADHGALLDPPTSDVTARVVALLGSLDGEVHDRSALNRAVAFLHREQEAEGCWYG-RWGTNYIYGTWSVLTALEQLGYDFNAPWVRKAVIWLKSVQRDDGGWGESNDTYLDHR-PQDRQADESTPFQTAWAVLALIAAGECRSPEVWRGVEYLLRHQRPDGLWYCPWFTAPGF-PRVFYLKYHGYDAYFPLMALARYRNCVLDNDA

>seq_ID 81

MIRRMNKPAPSPWSALDAAIARGRDALMRLQQPDGSWCFELESDATITAEYILMMHFMDKID-DARQEKMARYLRAIQRLDTHGGWDLYVDGDPDVSCSVKAYFALKAAGDSEHAPHMVRARDAIL-ALGGAARSNVFTRILLATFGQVPWRAAPFMPIEFVLFPKWVPISMYKVAYWTRTTMVPLL-VLCSLKAHARNPRNIAIPELFVTPPDQERHYFPPARGMRRAFLALDRVVRHAEPLL-PKRLRQRAIRHAQAWCAERMNGEDGLGGIFPPIVYSYQMMDVLGYPADHPLRRD-CENALEKLLVTRPDGSMYCQPCLSPVWDTAWSTMALEQARGVAVHEAGAPASALDELDARI-ARAYDWLAERQVNDLRGDWIENAPADTQPGGWAFQYANPYYPDIDDSAVVTAMLDRR-GRTHRNADGTHPYAARVARALDWMRGLQSRNGGFAAFDADCDRMYLNAIPFADHGALLDPP-TEDVSGRVLLCFGVTKRADDRASLARAIDYVKRTQQPDGSWWGRWGTNYLYGTWSVLAG-LALAGEDPSQPYIARALAWLRARQHADGGWGETNDSYIDPALAGTNAGESTSNCTA-WALLAQMAFGDGESESVKRGIAYLQSVQQDDGFWWHRSHNAPGFTRIFYLKYHGYTAYFPLWA-LARYRRLAGGASSAGAHAVPASTAADAALA

>seq_ID 22

MATLTTMATTATMATTEASQPLEAQARTALTKATSYAWEIISNRHWCGELESNVTVTCEHIFF-LYVLYQHIDPDEGSQYRQWLLSQQNADGSWGIAPNYPGDVSTSAEAYLALRIIGMSPDSPEL-FQARTFIRAAGGLSKMRMFTRIFFAEFGLVPWTAIPQLPAEFILVPAHFPISIYRLAS-WARSNVVPLLIIAHHRPLYPLPNGLHKQNPFLDELWLDPATKPLPYGSLDPTDPLSFVFTILD-KALSYLGGLRRCPTRGYARRRCIQWILQHQEKAGDWAGIIPPMHAGIKALW-LEGYKLHDEPIQLGLAAIERFTWTDNRGKRLQCCISPVWDTVLMIRALQDTPASLGIKSDPRI-ADALAWTAENQHRGPEGDWRVYQPNIPVGGWAFEYSNTWYPDIDDTAAAVLAFLTHDPA-TARSRLVRDAVLWIVGMQNADGGWAAFDHENNRLFLNKIPFSDMESLCDPSTPDVTGR-TIECLGMLRDLLMLPAEKAGKKGEKYGYPDGERDAAADSHLLKIINTACARAIPYLIRTQEAT-GAWYGRWAVNYVYGTCLVLCGLQYFKHDPTFAPEIDTMATRAVKWLRQIQNSDGGWGESVLSY-REPWRAGCGPSTPSQTAWALMGLLTVCGGEDRSVQRGVRHLVDTQDDILSKGEGGAAAW-TEREFTSTGFPNHFYISYTLYRVYFPITALGRYLSLVEGGKKENGGGA

>seq_ID 178

MNSINATAAPIDDNVLGDRIGAATRGLLSLKQSDGHFVFELEADATIPSEYILMRHYLGEP-
VDTVLEAKIAAYLRRIQGAHGGWPLVHDGPFDMSASVKAYFALKMAGDSIDAPHMA-
RAREAILSRGGAANVNVFTRFLLSFFGELTWRSVPVLPVEIMLLPMWSPFHLNKVSYWARTT-
MVPLMVLAALKPRARNPRGIGIRELFLEDPATVGTPKRAPHQSPGWFALFTGFDRV-
LRLIEPLSPKWLRARAMKKAIAFVEERLNGEDGLGAIFPPMVNTVMMYDALGFPPEHPPRA-
VTRRGIDKLLVVGENEAYCQPCVSPIWDTALSCHALLEAGGPEAVNSAGKCLDWLLLKQEL-
VLKGDWAVKRPDVRPGGWAFQYANGHYPDLDDTAVVVMAMDRVRRNGPNGRYDEAIARGRE-
WIEGMQSRDGGFAAFDADNLEYYLNNIPFSDHAALLDPPTEDVTARCVSMLAQLGETVDSSSS-
MAAGVEYLRRTQLAEGSWYGRWGLNYIYGTWSVLCALNVA-
GVDHQDPVIRRAVNWLVSIQNADGGWGEDAVSYRLDYKGFEGAPTTASQTAWALLALMAAGE-
VENPAVARGIKYLIDTQTKKGLWDEQRYTATGFPRVFYLRYHGYSKFFPLWALARYRNLRST-
NSKAVGVGM

>seq_ID 177

MNATVAQIGDAVLEDRIGSATRGLLNLKQSDGHFVFELEADATIPSEYILLRHYLGEPVDT-
VLEAKIAAYLRRIQGAHGGWPLVHDGPFDMSASVKAYFALKMIGDSVDAPHMARAREAILSRG-
GAANVNVFTRFLLSFFEVLTWRSVPVLPVEIMLLPMWSPFHLNKISYWARTT-
MVPLMVLAVLKPRARNPRDVGIRELFLQDPATVRTPKRAPHQSPAWFALFSSLDWILR-
RIEPLFPKRLRARAMEKAIAFVEERLNGEDGLGAIFPPMVNTVMMYDALGFPPEHPPRAVTRR-
GIDKLLVIGEDEAYCQPCVSPIWDTALSCHALLEAGAPEALNSAGKCLDWLLPKQEL-
VLKGDWAAKRPDVRPGGWAFQYANGHYPDLDDTAVVVMAMDRVRRNGRGDKYDEAIERGRE-
WIEGMQSRDGGFAAFDADNLEYYLNNIPFSDHAALLDPPTEDVTARCVSMLAQLGATVDGSSS-
MAAGVEYLRRTQLAEGSWYGRWGLNYIYGTWSVLCALNAAGVDHQDPAIRKAVDWLL-
SIQNEDGGWGEDAVSYRLDYKGFEGAPTTASQTAWALLALMAAGEVENPAVTRGIKYLID-
TQTKKGLWDEQRYTATGFPRVFYLRYHGYSKFFPLWALARYRNLRSTNSKVVGVGM

>seq_ID 170

MREAVSKVEALQRSKTQGISLEDVERGVAQATRALTALAHDDGHICFELEADATIP-
SEYILFHHFRGTQVPGDLEAKIGNYLRRTQGRHGGWALVHEGPFDMSCTVKAYFALKMIGDDIE-
APHMRRAREGILSRGGAANANVFTRFMLALYGEVPWRAVPVMPVEVMFLPKWFPFHLDKISY-
WARTTVVPLFVLQATKPRARNPRGISVQELFVTPPESVRSWPGSPHATWPWTPIFGFIDRV-
LQRVENHLPRKSRQRAMEMARAWVSERLNGEDGLGAIFPAMVNSVLMYEVMGYRPDHPQVR-
VACDAIEKLVVEKADEAYVQPCVSPVWDTALASHALLEAGGPEAEAQARAGLDWLK-
PRQVLDIVGDWAARKPKVRPGGWAFQYANAHYPDLDDTAVVVMAMDRAMHQHGLVAGMPDYKA-
SIARAREWVEGLQSEDGGWAAFDADNNHMYLNHIPFSDHGALLDPPTADVTARVVGMLSQL-
GETRETSRALDRGVNYLLNDQEEDGSWYGRWGMNFIYGTWSVLCALNAAGVDPAD-
PRIQKAVSWLIRIQNPDGGWGEDASSYKIDPAFEPGSSTASQTAWALLALMAAGAVDDPA-
VTRGINFLTRTQGADGFWKEERYTATGFPRVFYLRYHGYPKFFPLWAMARFRNLKRGNSRRVQFGM

>seq ID 14

MLLAEVQKALRLAVGHSLDLQRADGAWCGEVHSNATFTSQYVFLQQQIGLPLDPTEIEG-
LSRWLFSQQNEDGSWGLGPGLGGDVSTTTETYLALKILGVSPEDPRMAAARTSIIKAGSL-
PATRMFTRVFLASFGLIPWSAVPPLPAELILLPTLFPVNIYNLSSWARATCVPLLLIRH-
HEPLHSLPNGRHAENDFLDELWTKDIPRDFCYTTPLSRMWRLGDYAGIFFTSADH-
GLRFLGQYFHSPLRNLSRRKIINWILDHQEQSGEWAGYWPPQHNNIWALSLEGYSLDHPVLRR-
GIAAVKSFVLHDATGMRAQVTVSQVWDTALMSIALSDSAPSTGIISPTQAIDWLMHHE-
VASHRGDWRVLRPKLATGGFCFEEFNTLYPDVDDTAAVIMALIKSNPAHLIS-
GCVRRAAQWILGMQNRDGGWGAFDWNNDKFFLNKIPFSDMDSLCDPSTPDVTGRIIECFGMM-
MAGRHGYSLDCQLENRLRASSQLAIAYLLGCQENNGSWWGRWGVNYLYGTS-
NVLCGLAYYYDRSSLSKGDVKSNSNIVSAVDRASEWLKARQHSNGGWGEGPESYD-
NAQLAGCGQPTASQSAWVTMALLNYLSPTDEVIQRGVSYLVRNQVKYGDESRAT-
WLLERYTATGFPGHLYMEYDYYRHYFPIMALGRYVNKLSGSHKLL

>seq_ID 180

MTRALRQAPESAGAIGIAAASPATETSGQDTHPREISGAITAARDALLKLQQADGHWCFM-
LEADCTIPAEYILWTHFTGELEPEIERKLAARLRAK-
QASHGGWPLYEGGDLDISCSVKVYYALKLVGDDPNAPHMRRAREAILAQGGGARANVFTR-
LALAMFSQIPWRGVPFIPVEIMLLPRWFPFHLSKVSYWSRTVMVPLAILYSLKAQAQNPRN-
VHIQELFTVPPEQERHYFPVRSRLNKILLSVERTARLLEPLIPSMLRRRALKKAETWFTERLN-
GEDGLGGIFPAMVNAHESLILLGYSPDHPWRVQAKKALQNLVIEEKNSASCQPCLSPIWDT-
GLAALALQETEGGHTTAPVIRALDWLKERQILEQSGDWQVQHPNLKGGGWAFQYNN-
SYYPDLDDTALVAWSMDQAATPERYGEAIGRACDWLCGMQSRNGGFAAFESDNTHYYLNEI-
PFADHGALLDPPTADVTARCIVLLGRLNKPQYAETLQRALDYLRREQEPNGSWF-
GRWGTNYIYGTWSALTALEQANIDPQEGFIRKAVEWLKQVQRLDGGWGEDNYSYFDSSLAG-

RYQESTPVHTAWALLALMAVGEANSEAVKKGIAYLLQIQQEDGLWDHPAFNAPGFPRVFYL-
KYHGYDKFFPLWALARYRNHLNRQC

>seq_ID 155

MMANATDTIELPPSRAADRIVPMTDIDQAVDAAHAALGRRQQDDGHWVFELEADATI-
PAEYVLLEHYLDRIDPALEERIGVYLRRIQGDHGGWPLYHGGKFDVSATVKAYFALKAIGDDI-
DAPHMARARAAILDHGGAERSNVFTRFQLALFGEVPWHATPVMPVELMLLPRKALFS-
VWNMSYWSRTVIAPLLVLAALRPRAINPRDVHVPELFVTPPDQVRDWIRG-
PYRSQLGRLFKYVDIALRPAERLIPDATRQRAIKAAVDFIEPRLNGEDGLGAIYPAMANTVM-
MYRALGVPDSDPRAATAWEAVRRLLVELDGEAYCQPCVSPIWDTGLAGHAMIEAASGPKGIR-
PEDTKKKLAAAAEWLRERQILNGEGRLGDQLPRRAPRRLGLPVQQRLLPRRGRHGS-
GRHVLHREGDPANDEALERARQWIIGMQSSNGGWGAFDIDNNLDFLNHIPFADHGALLDPP-
TADVTARCISFLAQLGHPEDRPVIERGIAYLRTDQEREGCWFGRWGTNYIYGTWSVLCAY-
NAAGVAHDDPSVVRAVDWLRSVQREDGGWGEDCASYEGATPGIYTESLPSQTA-
WAVLGLMAVGLRDDPAVMRGMAYLTRTQKDDGEWDEEPYNAVGFPKVFYLRYHGYRQFF-
PLLALSRYRNLASSNSRHVAFGF

>seq_ID 8

MNRMLQPLHSGAGIFRSSLDRVIAQARQALGGRQAEDGHWCFEFEADCTI-
PAEYILMQHYMDERDEALEARIAVYLRGKQADHGGWPLYYGGHFDLSAS-
VKVYYALKLAGDDPELPHMRRAREAILAHGGAERSNVFTRITLALFAQVPWRAVPFIPVEIM-
LLPRWFPFHIYKVASWSRTVMVPLFILCSLKARAKNPLQVHIRELFRRPPDQITDYFSHARR-
GIVAYIFLSLDRFWRLMEGWIPHGIRRRALKKAEAWFTARINGEDGLNGIFPAMVNAHEAL-
ELLGYPPDHDYRRQTGAALRKLVVERANDAYCQPCVSPVWDTCLALHALLEEDGEVSPA-
VQNGIRWLKNRQIGAEPGDWRESRPHLAGGGWAFQYANPYYPDLDDTAAVGWALARAGRA-
EDRDSIEKAANWLAGMQSRNGGFGAYDVDNTHYYLNEIPFADHKALLDPPTADVTGRVVAF-
LAHLARPRDRDVLRRAVAYLLREQESSGAWFGRWGTNYIYGTWSVLMALAELNDPSLKPTMER-
AAYWLRAVQQGDGGWGESNDSYSDPGLAGMGQTSTAAQTAWACLGLMAAGDRDSVALHR-
GIAWLQAHQEGDGCWQAPFFNAPGFPKVFYLIYHGYAFYFPLWALARYRNLGCMAHE

>seq ID 203

MSMNĒAVLAAPRAAVATAAPALQAPIEALSPLDAGIGHAVDALLAQQNADGHWVYELEADATI-
PAEYVLMVHYLGETPDLSLEARIARYLRRIQNADGGWPLFHEGRSDISASVKAYFALK-
MAGDDPQAAHMARAREVILAMGGAETSNVFTRTLLALYGVMPWQAVPMMPVEIMLLPQWFPF-
HLSKVSYWARTVIVPLLVLNSLRPQARNPRKVGIDELFLGSRDAVRLPPRAPHQHKGWHALFH-
GADVLLRTAEHVMPRGLRRRAIDAAKAFVRERLNGEDGLGAIFPAMANSVMMFDVLGVPP-
DDPDRAIARRSIDKLLVVHGDEAYCQPCLSPVWDTALAAHALLEASEPRATAAVTRALD-
WLRPLQVLDVRGDWTVRRPDVRPGGWAFQYANPHYPDVDDTAVVVAAMHRAARTDHSGRADP-
NAEATARAIEWIVGMQSANGGWGAFEPENTHLYLNNIPFADHGALLDPPTADVSARCLS-
MLCQTGATPDKSEPAARALQYLLAEQLPDGSWFGRWGTNYIYGTWSALCAL-
NAAGLGPDAPPLRRAAEWLVAIQNPDGGWGEDGDSYKLEYRGYETAPSVASQTA-
WALLALMAAGQAAHPAVTRGIDYLLRTQQADGLWHEPRFTAVGFPRVFYLRYHGYARYFPLWA-
LARYRNLERSGNRQVAWGL

>seq ID 165

MREAAVSKVETLQRPKTRDVSLDDVERGVQSATRALTEMTQADGHICFELEADATIP-
SEYILFHQFRGTEPRPGLEAKIGNYLRRTQSKVHGGWALVHDGPFDMSASVKAYFALKMIGD-
DIEAPHMRAVRKAILQRGGAANANVFTRILLALYGEVPWAAVPVMPVEVMHLPKWFPFHLD-
KVSYWARCTMVPLFVIQAKKPRAKNPRGVGVAELFVTPPDSVRTWPGSPHATWPWTPIFG-
GIDRVLQKTQDHFPKVPRQRAIDKAVAWVSERLNGEDGLGAIFPAMVNSVLMYEV-
LGYPPEHPQVKIALEAIEKLVAEKEDEAYVQPCLSPVWDTALNSHAM-
LEAGGHQAEANARAGLDWLKPLQILDIKGDWAETKPNVRPGGWAFQYANPHYPDLD-
DTAVVVMAMDRAQRQHGLVSGMPDYSESIARAREWVEGLQSADGGWAAFDADNNHHYLN-
HIPFSDHGALLDPPTADVTARVVSMLSQLGETRATSRALDRGVTYLLNDQEKDGSWYGRWGMN-
FIYGTWSVLCALNTAGVDPQSPEIRKAVAWLIRIQNPDGGWGEDASSYKLNPEFE-
PGYSTASQTAWALLALMAAGEVDDPAVARGVNYLVRTQGQDGLWSEERYTATGF-
PRVFYLRYHGYPKFFPLWAMARFRNLKRGNSRQVQFGM

>seq ID 181

MSISPTFSGSSLQKSSLSDHSTISEPFTVVDRVNGISAVALDDAITRAR-
SALLAQQREDGHWCFSLEADCTIPAEYILMMHFMDEIDTALERRIANFLRN-
RQVTDGHGGWPLYYGGDFDMSCSVKVYYALKLAGDSPEAAHMVRARNAILERG-
GAARSNVFTRLLLAMYRQIPWRGVPFVPAEIMLLPRWFPFHLSKVAYWSRTVMVPL-
SILCTLKAKAANPRNIHVRELFTVDPEMEKNYFPVRTPLNHLLLYLERLGSKLEPLIPS-
FIRRRALKKAEQWTIERLNGRDGLGAIFPAMVNAYEALTLLGYDHDHPLLQQCRLALRELL-

VNEGEDITWCQPCVSPVWDTVLASLALQEDERADNGPVRHALDWLVPLQALDQPGDWRNS-
RPDLPGGGWAFQYANPHYPDLDDTAAAAWALCQADTEDYRTSITRAADWLAGMQSSNGGFAAF-
DIDNVHYYLNEIPFADHGALLDPPSSDVTARCIGLLALNGEARHQETVKRGLTFLFNEQEPS-
GAWFGRWGTNYVYGTWSVLEALKLARVDHDHQAVKRAVQWLKSVQRADGGWGETNDSYLDSE-
LAGQLETSTSFQTAWAVLGLMAAGEVGSTAVRNGIDYLIRTQSAAGL-
WEEPWFTAPGFPKVFYLKYHGYSKYFPLWALNRYRAMNSRSVV

>seq_ID 110

MILFPAGFYFSIYEISYWSRCIVVPLSIAIARKPHVTVGDDLLKELYLVPREDVVYRIER-
DQDGFCWYNFFIDADSIFRRYEQHPIKFIRRIAKKMAEKWLLEHMEKSGGLGAIWPA-
MINSIFAMKCLDYPDDHPALTAQMKEVEALVIYEGDMLYLQPCVSPVWDTAWSIIAMNDS-
GIPGSHPVLQKAGKWLLSKEVRDFGDWKLKCKVEEPSGWYFQYANEFYPDTDDTGAVL-
MALQRVSLPEDMHKEKTLLRALRWLQAMQCDDGGWGAFDRNNNKTILNNIPFADFNALLD-
PSTSDVTGRCIEFFGRIGFNKTYLNIKKAVEFLKKEQDEDGSWFGRWGSNYIYGTWSVISGLI-
AVGEDINKAYIKKAIAWLKSVQNSDGGWGETIKSYEDSALKGIGKSTPSQTAWALLTLITA-
GEIKSSSTERGIDFLLSTQKEDGSWDEREFTATGFPKVFYLKYHMYRNYFPLMALGRYRHFTHKLATSQ

>seq_ID 182

MSISQAFFRTLIQKSSLSDSSLVSENFPADDVAGNEANEISAVTLDEAITRA-
YTALLAQQREDGHWCFPLEADCTIPAEYILMMHFMDEVDTVLERKI-
ANFLRTRQVTDGHGGWPLYYGGDFDMSCSVKTYYALKLAGDSPEAAHMVHARNAILERG-
GAARSNVFTRLLLAMYRQIPWRGVPFVPAEIMLLPRWFPFHLSKVAYWSRTVMVPL-
SILCTLKAKAINPRNVHVQELFVVDPVKEKNYFPVRTSLNRLLLYVERLASKLEPFIPS-
FIRRRAVKKAEQWVIERLNGNDGLGAIFPAMVNAYEALTLLGHDRDHPLLQQCRQSLRELL-
VDEGEEITWCQPCVSPVWDTVLATLALQEDKQADSEPIRRALDWIV-
PLQILDEPGDWRDSRPNLLGGGWAFQYANPHYPDLDDTAAVAWALIQTGAEDYRVSITRAAD-
WLAGMQSSNGGFAAFDIDNAYYYLNEIPFADHGALLDPPTSDVSARCVGLLALNGE-
VRHQEAVKRGLDFLFNEQESSGAWFGRWGSNYIYGTWSVLEAFRLARVDKGHQAVQRAIQW-
LESVQRADGGWGETNDSYLDPQLAGQLEASTSFQTAWAVLGLMAAGEVENTAVRKGIDYLL-
RTQIATGLWEEPWFTAPGFPRVFYLKYHGYSKYFPLWALNRYRTLSSKSAV

>seq ID 162

MSPFLQASDDNNPLFKESCQALDHATEFARDTLVNKEHWCGWVLS-
NVTVTAEWIFLQYILGLEMSNEDRRGFLKHFTSSQRPDGSWSLATQTTTGGELSCTIEAYLAL-
KILGVSPEEDYMVRARDYVRSHGGAEKMRMLSRFHLAMFGLIPWAAVPQMP-
PELIFMPSWSLVNIYKFSSWARCNIVGLCMLRVHEPLYALPNGKQLDNDYLDELWLDPYH-
KAIPYTVPYLQLMQTSPLGVLFQLGDLFLWLLSFLGFWFLRRWAVSSSIQWTLDHQEPSGDWG-
GIYPPMHHNILALMLEGWSQDDPVIQRGIGACQRFLAEDPAHGKWMQPSVSPVWDTFLMI-
RAVADAKTTDDADKLLVKPVDWVLAQQIDDDHIGDWRIYRPDIPAGG-
FAFEYFNKWYPDVDDTAVGVVALMRHDPSLVNDDRILKAAAWTLGMQNRDFGWAAFDADNNAF-
YLHATPFSDMDSLTDSSTPDVTGHVLEMLGLMYRLERQGRVKSPEMLAFLSQSH-
GACDRGLGYLLGSQEAFGGWYGRWGVNYIFGTSAALCALAYFADRKGVRGKMAAGADWLRS-
RQNPDGGWGELLESYDNKALAGRGRSTPSQTAWALQGLLELEDPRGEVVEAGVNWLL-
RHQVTSPSRNSGRVSATWPEDDYTATGFPGHFYLKYELYCHYFPMMALARYRSCIQDGA

>seq ID 172

MDDRVGAATFEAQPRAGFGSVEAAISRAREALLAVQKPDGHFVFELEADVSIPAEYILFRH-
FLGDPAKTEIERKIGVYLRRRQTAAGGWPLFAEGVFNVSSSVKAYFALKIIGDDP-
NAPHMAKARNAILAHGGAAQSNVFTRSLLALYGEVPWRAVPAMPVEIMHLPRWFPFHLS-
KVSYWGRTVIAPLIVVHALKPRAKNPRKISVSELFVAPAETVSRWPGAPHKSFPWTTIF-
GAIDRVLHKTEPLLPARSHQTAIDKAVAFVTARLNGEDGLGAIYPAMAY-
SAMMFFALGAPLSDPRIVQIRKAIDRLLVIKDGEAYCQPCVSPVWDTALASHALMESAGQR-
PEARTAPAAAAVFEALDWLKPLQVLDVKGDWATQNPDVRPGGWAFQYANPHYPDLD-
DTAVVVLAMDRAVKTSPLIAGEEETAYVEAISRAREWILGLQSANGGFGAF-
DADNDRDYLNYIPFADHGALLDPPTADVTARCVSMLGQLGERPETSPALARAIDYLLSEQEE-
EGSWFGRWGMNYIYGTWSVLSAFNAVERPADCAATRKAAAWLKRIQNPDGGWGEDGE-
SYALGYKGYNPAPSTASQTAWALLALMAAGEVDAPEVALGLDYLVSTQADDGFWDEARFTAT-
GFPRVFYLRYHGYAKFFPLWAMARYRNLKSGNRLKTQFGM

>seq ID 24

MLGAIREPPIDVQIALHSRDDNQTGLVLRGTRRTVDRVLKGLCSSPCFFCSVSLTMATLTTT-
MATTATMATTEASKPLEAQARTALTKATNYAWEIFSNRHWCGELESNVTVTCEHIFFLY-
VLYQHIDPGEGSQYRQWLLSQQNSDGSWGIAPNYPGDISTSAEAYLALRIIGMSTDSPELYR-
ARTFIRAAGGLSKMRMFTRIFFAEFGLVPWTAIPQLPAEFILVPAHFPISIYRLAS-
WARSNVVPLLIIAHHRPLYPLPNGLHKQNPFLDELWLDPATKPLPYGSSDPTDPVAFVFTILD-

KALSYLGGLRRSPTRGYARRRCVQWILQHQEKAGDWAGIIPPMHAGIKALL-
LEGYKLHDEPIQLGLAAIERFTWADNRGKRLQCCISPVWDTVLMIRALQDTPASLGIKLDPRI-
ADALAWTAENQHRGPEGDWRVYKPNIPVGGWAFEYHNTWYPDIDDTAAAVLAFLTHDPA-
TARSRLVRDAVLWIVGMQNADGGWAAFDHENNQLFLNKIPFSDMESLCDPSTPDVTGR-
TIECLGMLRDLLMRPAENAENGEKYGYPDGEGDAAADAHLLQIINTACARAIPYLIRSQEAT-
GTWYGRWAVNYVYGTCLVLCGLQYFKHDPKFAPEIQAMAARAVKWLKQVQNSDGGWGESLLSY-
REPWRAGCGPSTPSQTAWALMGILTVCGGEDRSVQRGVRHLVDTQDDTLSQGDGGAAAW-
TEREFTIREPLHEASQRIGSD

>seq_ID 26

MATLTTTMATTATMATTEASKPLEAQARTALTKATNYA-
WEIFSNRHWCGELESNVTVTCEHIFFLYVLYQHIDPGEGSQYRQWLLLQQNSDGS-
WGIAPNYPGDISTSAEAYLALRIIGMSTDSPELYRARTFIRAAGGLSKMRMFTRIFFA-
EFGLVPWTAIPQLPAEFILVPAHFPISIYRLASWARSNVVPLLIIAHHRPLYPLPNGLHKQN-
PFLDELWLDPATKPLPYGSSDPTDPVAFVFTILDKALSYLGGLRRSPTRGYA-
RRRCVQWILQHQEKAGDWAGIIPPMHAGIKALLLEGYKLHDEPIQLGLAAIERFTWADNRG-
KRLQCCISPVWDTRVYKPNIPVGGWAFEYHNTWYPDIDDTAAAVLAFLTHDPATARSRLV-
RDAVLWIVGMQNADGGWAAFDHENNQLFLNKIPFSDMESLCDPSTPDVTGR-
TIECLGMLRDLLMRPAENAENGEKYGYPDGEGDAAADAHLLQIINTACARAIPYLIRSQEAT-
GTWYGRWAVNYVYGTCLVLCGLQYFKHDPKFAPEIQAMAARAVKWLKQVQNSDGGWGESLLSY-
REPWRAGCGPSTPSQTAWALMGILTVCGGEDRSVQRGVRHLVDTQDDTLSQGDGGAAAW-
TEREFTSTGFPNHFYISYTLYRVYFPITALGRYLSLIEGGQEKKKKGGGT

>seq_ID 171

MGKVETLHRTSTQDITLDDVERRVTLASKALMRLANADGHWCFELEADATIPSEYILYHH-
FRGSIPTAELEGKIAAYLRRTQSAQHDGWALIHDGPFDMSATVKAYFALKMVGDPIDAPHMR-
RARDAILRRGGAAHANVFTRIMLALYGEVPWTAVPVMPVEVMLLPRWFPFHLDKVSYWART-
VMVPLFVLQAKKPRARNPRGIGIRELFVEAPERVKRWPAGPQESSPWRPVFAAIDKVLQK-
VEGFFPAGSRARAIDKAVAFVSERLNGEDGLGAIFPAMVNTVLMFEALGYPDDHPFAVTARSS-
VEKLVTVKEHEAYVQPCLSPVWDTALAAHALMEAGGTEAERHAKRAMDWLKPLQVLDIK-
GDWAASKPDVRPGGWAFQYANPHYPDLDDTAVVVMAMDRVQSRRSPGPDAADYGLSI-
ARAREWVEGLQSRDGGWAAFDADNTYHYLNYIPFSDHGALLDPPTADVTARCVSMLSQLGET-
RETCPPLDRGVAYLLADQEADGSWYGRWGMNYIYGTWSVLCALNAAGIDPACEPVRRAVTWLTA-
IQNPDGGWGEDASSYKLEYRGYERAPSTASQTAWALLALMAAGEADNPAVARGI-
NYLTRTQGADGLWAEDRYTATGFPRVFYLRYHGYAKFFPLWALARYRNLQRGNSLKVAVGM

>seq_ID 173

MLREATAISNLEPPLTASYVESPLDAAIRQAKDRLLSLQHLEGYWVFELEADCTI-
PAEYILMMHFMDEIDAALQAKIANYLRHHQSADGSYPLFRGGAGDISCTVKVYYALKLAGDSI-
DAPHMKKAREWILAQGGAARSNVFTRIMLAMFEQIPWRGIPFTPVEIMLLPKWFPFHLD-
KVSYWSRTVMVPLFILCSHKVTARNPSRIHVRELFTVEPQKERHYFDHVKTPLGKAILALER-
FGRMLEPLIPKAVRKKATQKAFDWFTARLNGVDGLGAIFPAMVNAYEALDFLGVPPDDERRR-
LARESIDRLLVFQGDSVYCQPCVSPIWDTALTSLTLQEVARHTADLRLDAALSKGLKWLAS-
KQIDKDAPGDWRVNRAGLEGGGWAFQFGNDYYPDVDDSAVVAHALLGSEDPSFDDNLRRAAN-
WIAGMQSRNGGFGAFDADNTYYYLNSIPFADHGALLDPPTADVSARCAMFLARWVNRQPEL-
RPVLERTIDYLRREQEADGSWFGRWGTNYIYGPGAVLLAYEGRRVPND-
DPSVRRAVAWLKSIQREDGGWGEDNFSYHDPSYRGRFHTSTAFQTGFALIALMAAGEXGSPE-
VQAGVDYLLRQQRPDGFWNDECFTAPGFPRVFYLKYHGYDKFFPLWALARYRNERYALA

>seq_ID 117

MNETAFANPAPQVGPAQRQPAAPQEAPAARLPAPALDRGIDRALDALL-
HQQRPDGHWVYELEADATIPAEYVLMVHYLGEDPDRDLEARIA-
RYLRRIQNPDGGWPLFHQGRSDISASVKAYFALKMAGDDPQSAPMQRARQAIHAMG-
GAEATNVFTRTLLALYGVLPWKAVPMMPVEIMLLPRWFPFHLSKVSYWARTVIVPLL-
VLNSLRPQARNPRGVGINELFVGNCHTVGLPPRAAHQHAGWYTVFRGLDALLRLAEPLF-
PRTLRRRAIAAAQRFVRERLNGEDGLGAIFPAMANSVMMFDVLGVPPEDPARAVARRSIERLL-
VEHGDEAYCQPCLSPVWDTALATHALLETGEARAAQAAGRALDWLRPLQVLDLRG-
DWAVRRPLVRPGGWAFQYANAYYPDVDDTAVVAAAMDRFMRAHHAPGRYGEAVARATEWIV-
GMQSGNGGWGAFEPENTHLYLNNIPFADHGALLDPPTADVSARCLSMLCQTGATPANSEPAAR-
ALRYLLAEQMPDGSWFGRWGTNYIYGTWSALCALNAAGLPPEAPELCRAVAWLARIQNADGGW-
GEDGSSYRLDYSGYEPAPSVASQTAWALLALMAAGAAQHPAVARGIDYLL-
RTQQPGGLWHEPRFTAVGFPRVFYLRYHGYARYFPLWALARYRNLQRGLGDHGGNSGQVAWGL

>seq_ID 204

MSMNETAFATAVPRIAPASAGDSPAPRDAAQALDQGIGRAIDALLHQQRPDGHWVYELEADA-
TIPAEYVLMVHYLGEAPDLELEARLARYLRRIQNPDGGWPLFHEGRSDVSASVKAYFALK-
MAGDDPQAAHMQRARRAVHALGGAEASNVFTRTLLALYGVMPWLAVPMMPVEIMLLPQWFPF-
HLSKVSYWARTVIVPLLVLNSLRPQARNPRGVGINELFVGNCHTVGLPPRAAHQHAGWYTV-
FRGLDALLRVAEPLVPRTLRRRAIAAAQAFVRERLNGEDGLGAIFPAMANSVMMFDVLGVPP-
DDPARALARQSVERLLVEHGDEAYCQPCLSPVWDTALAAHALLETGEARATAAAGRGLD-
WLRPLQVLDVRGDWAVRRPLVRPGGWAFQYANAYYPDVDDTAVVAAAMNRYMRAHDVPGRY-
DEAVARAAEWIVGMQGGDGGWGAFEPENTHLYLNNIPFADHGALLDPPTADVSARCLSMLCQI-
GATPGKSEPAARALRYLLAEQMPDGSWFGRWGTNYIYGTWSALCALNATGLAPEA-
PEMRRAVAWLEQIQNADGGWGEDGSSYRLDYRGYEPAPSVASQTA-
WALLALMAAGAAQHAAVARGIDYLLRTQQSGGLWHEPRFTAVGFPRVFYLRYHGYARYFPLWA-
LARYRNLQRGGAHQVPWGL

>seq_ID 79

MRIGTTTNPSMPFPLSSSGAVFYREVNELREVQQEINRIQAF-
LLQRQQEDGTWRFCLESSPMTDSHMIILLRTLGIHDERLMEKLTA-
HITALQHDNGAWKLYPDEQEGHLSTTIDSYYALLLSGKYTKNEPRMALARS-
FILEKGGLTQANMLTKFATALTGQYQWPSHFLVPVEIALLPPSFPVSFYDFVGYA-
RVHLAPMMIVADRNYVKKPDNAPDLSDLYADTPISRGLYPHRFLENFLKEGQSFLATIHDS-
LQQLPFLPGQLHKLALRRLEQYILARIEPDGTLYNYSTSTFFMIFALLARGFSPKDPLI-
QKAMQGLTGSVYDYENGAHLQLATSAVWDTALLTFSLQKSGLSPTHPAIQKANRYLL-
RKQQHTYGDWKIRNPNGKPGGWGFSDYNTMNPDIDDTTAAL-
RSLRLLARTDVTAATAWKRGLEWLLSMQNDDGGWPAFERNTDADFIRHLPIEGADTVSTDPS-
SADLTGRTLEFLGNYAGRTLTDLHVEKGVRWLLKHQESDGSWYGRWGIAYLYGTWAAIT-
GLMAVGFSPTEPAIQKAVAWLVANQNPDGGWGESCQSDLKKTYVPLGASTPSQTAWAIDALI-
AVSSKPTAELQRGIRYLLTHNQANDWTTRYPTGGGRPGGTYFAYHSYRWIWPLLALSHYQVKYANT

>seq_ID 70

MLLYDKVHEEIERRTTALQTMQRQDGTWQFCFEGALLTDCHMIFLLKLLGRNDEIEPF-
VKRLVSLQTNEGTWKLYEDEKGGNLSATIQAYAALLASEKYSKEDMNMRRAEMFIKEHGGVS-
RAHFMTKFLLAIHGEYEFPALFHFPTPILFLQDDSPLSIFGLSSSA-
RIHLIPMMICMNKRFRVEKKLLPNLNHIAGGGGQWFREERSPLIQSFLGDVKKVISY-
PLSLHHKGYEEVERFMKERIDENGTLYSYASATFYMIYALLALGHSIQSPIIEKAVTGLK-
SYIWKMDRGSHLQNSPSTVWDTALLSYSLQEAKVTNENKMIQRATEY-
LLQKQQTKKVDWSVHASSLVAGGWGFSDVNTTIPDIDDTTAALRALARSRGNDRVDDAWGRG-
VEWVKGLQNNDGGWGAFERGVTSKLLSNLPIENASDMITDPSTPDITGRVLELFGTYAPNEL-
LEEQKKKAIKWLMDVQEQNGSWYGKWGICYIYGTWATMTGLRALGVPSTHPALKKAASW-
LEHLQHEDGGWGESCQSSVEKKFISLPFSTPSQTAWALDALISYYDQETPIIRKGISY-
LLAQSTMNEKYPTGTGLPGGFYIRYHSYGHIYPLLALAHYVKKYRK

>seq_ID 140

MAGERSALITALKRSQAADGSWRFPFETGISTDAYMIILLRTLDINDEPLIQALVERIES-
RQEANGAWKLFADEGDGNVTATVEAYYALLYSGYRQPTDRHMQKAKRRILDMGGLDRVHLFT-
KVMLALTGQYPWPGRFPLPLEFFLLPPSFPLNMYDLSVYGRANMIPLLI-
AADSRYSRKTDKSPDLSDLFASRGDWGMPESRSLLTYVKRSLIGLPAQLHQAAK-
QRAVRYLFEHIEPDGTLYSYFSSTFLFIFALLALGYRNDDPRIRQAVRGLRSLRT-
TIDGHVHLQYTTASVWNTALASYTLQEAGVPMTDRAIEKAN-
RYLLSRQNVRYGDWAVHNPYSTPGGWGFSDVNTMNPDVDDTTAALRAIRQAAA-
KETAFRHAWDRANQWLFSMQNDDGGFAAFEKNVSSRFWRYLPIEGAEFLLMDPSTADLTGRT-
LEYFGTFAGLTKDQRAVSRAVDWLLSHQERNGSWYGRWGICYIYGTWAAITGLTAVGVPAHH-
PALQKAVRWLLSIQNDDGGWGESCKSDGAKTYVPLGDSTPVHTAWALDALVAAAERPTLEM-
KAGFRALFRLLHHPDWTASYPVGQGMAGAFYIHYHSYRYIFPLLALAHYEQKFGPLDD

>seq ID 137

MAGERSALITALKRSQAADGSWRFPFETGISTDAYMIILLRTLDINDEPLIQALVERIES-
RQEANGAWKLFADEGDGNVTATVEAYYALLYSGYRQPTDRHMQKAKRRILDMGGLDRVHLFT-
KVMLALTGQYPWPGRFPLPLEFFLLPPSFPLNMYDLSVYGRANMIPLLI-
AADSRYSRKTDKSPDLSDLFASRGDWGMPESRSLLTYVKRSLIGLPAQLHQAAK-
QRAVRYLFEHIEPDGTLYSYFSSTFLFIFALLALGYRNDDPRIRQAVRGLRSLRT-
TIDGHVHLQYTTASVWNTALASYTLQEAGVPMTDRAIEKAN-
RYLLSRQNVRYGDWAVHNPYSTPGGWGFSDVNTMNPDVDDTTAALRAIRQAAA-
KETAFRHAWDRANQWLFSMQNDDGGFAAFEKNVSSRFWRYLPIEGAEFLLMDPSTADLTGRT-
LEYFGTFAGLTKDQRAVSRAVDWLLSHQERNGSWYGRWGICYIYGTWAAITGLTAVGVPAHH-
PALQKAVRWLLSIQNDDGGWGESCKSDGAKTYVPLGDSTPVHTAWALDALVAAAERPTLEM-
KAGFRALFRLLHHPDWTASYPVGQGMAGAFYIHYHSYRYIFPLLALAHYEQKFGPLDD

>seq_ID 136

MVADERSALIDALKRSQSVDGSWRFPFETGISTDAYMIILLRTLGIHDEPLIQALVERIES-
RQDANGAWKLFADEGDGNVTATVEAYYALLYSGYRKKTDSHMQKAKARILEVGGLERVHLFT-
KVMLALTGQHSWPRRFPLPLVFFLLPPSFPLNMYDLSVYGRANMVPLLVVAER-
RYSRKTDNSPDLSDLAASRNDWRLPDTEALWSYVKRSLTGLPAWLHRAAEQRAVRYMLEH-
IEPDGTLYSYFSSTFLLIFALLALGYPKDDPHIARAVRGLRSLRTEIDGHTHMQYTTASVWN-
TALASYALQEAGVPPTDRTIEKANRYLLSRQHIRYGDWAVHNPYGVPGGWGFSDVNT-
MNPDVDDTTAALRAIRRAAAKETAFRHAWDRANRWLFSMQNDDGGFAAFEKNVGKRFWRYL-
PIEGAEFLLMDPSTADLTGRTLEYFGTFAGLTKDHSAIARAIDWLLDHQEADGSWYG-
RWGICYVYGTWAAVTGLSAVGVPIDHPAMQKAVRWLLSIQNDDGGWGESCKSDGAKTYVPL-
GASTPVHTAWALDALIAAAERPTPEMKAGVRALVRMLHHPDWTASYPVGQG-
MAGAFYIHYHGYRYIFPLLALAHYEQKFGPFVD

>seq_ID 49

MLLYEKVYEEIARRTTALQTMQRQDGTWRFCFEGAPLTDCHMIFLLKLLGRDKEIEPFVKR-
LASLQTNEGTWKLYEDEVGGNLSATIQSYAALLASEKYTKEDANMKRAEMFINERGGVARAH-
FMTKFLLAIHGEYEYPSLFHLPTPIMFLQNDSPLSIFELSSSA-
RIHLIPMMLCLNKRFRVGKKLLPNLNHIAGGGGEWFREDRSPVFQTLLSEVKKII-
TYPLSLHHKGYEEVERFMKERIDENGTLYSYATASFYMIYALLALGHSIQSPIIQKAITGIAS-
YIWKMERGSHLQNSPSTVWDTALLSYALQEAQVPKASKVIQNASAYLL-
RKQQTKKVDWSVHAPNLFPGGWGFSDVNTMIPDIDDTTAVLRALARSRGDENVD-
NAWKRAVNWVKGLQNNDGGWGAFEKGVTSRILANLPIENASDMITDPSTPDITGRVLEF-
FGTYAQNELPEKQKQSAINWLMNVQEENGSWYGKWGICYIYGTWAVLTGLRSLGIPSSDPS-
LKRAALWLEHIQHEDGGWGESCQSSVEKRFVTLPFSTPSQTAWALDALISYYEKETPIIRKGI-
SYLLSNPYVNEKYPTGTGLPGGFYIRYHSYAHIYPLLTLAHYTKKYRK

>seq_ID 62

MNIVIRISKGWVSNLLLDEKAHEEIVRRATALQTMQWQDGTWRFCFEGAPLTDCHTIFLL-
KLLGRDKEIEPFVERVASLQTNEGTWKLYEDEVGGNLSATIQSYAALLASKKYTKEDANMKRA-
ENFIQERGGVARAHFMTKFLLAIHGEYEYPSLFHVPTPIMFLQNDSPFSIFELSSSA-
RIHLIPMMLCLNKRFRVGKKLLPNLNHIAGGGGEWFREDRSPVFQTLLSDVKQIISY-
PLSLHHKGYKEIERFMKERIDENGTLYSYATASFYMIYALLALGHSLQSSMIQKAIAGIT-
SYIWKMERGNHLQNSPSTVWDTALLSYALQEAQVSKDNKMIQNATAYLLKKQHTKKAD-
WSVHAQALTPGGWGFSDVNTTIPDIDDTTAVLRALARSRGNKNIDNAWKKGVNWIKGLQNN-
DGGWGAFEKGVTSKLLAKLPIENASDMITDPSTPDITGRVLEFFGTYAQNELPEKQIQRAIN-
WLMNVQEENGSWYGKWGICYIYGTWAVMTGLRSLGIPSSNPSLTRAASWLEHIQHEDGGWGE-
SCHSSVEKRFVTLPFSTPSQTAWALDALISYYDTETPAIRKGVSYLLSNPYVNERYPTGT-
GLPGAFYIRYHSYAHIYPLLTLAHYIKKYRK

>seq_ID 59

METLIDPEISRLTQRLLEDQEEDGAWRYCFENSLMTDAYMIVLIRSLGIK-
KERLVQELADRLLSQQEEKGFWKIYRDEVEGNLSATVEAYFALLWSGAVKEKDENMVKARD-
CILSGGGLDKVHSMTKFMLAAHGQYPWDRFFPVPVEVILLPTYFPVSFT-
DFSAYARVHLAPLLLLKSERYIRKTSTTPDLSYLLKDQEDFSFFREEERSFIEYVTSG-
VEAIAAFPANLNDLAKKTALNYMLARLEPDGSLYSYFSSSFYMIIALLSQGYSRKDPLVVNAI-
KALISYQCKGDGYPHIQNSPSTIWDTALISHALQSSGVDSRNAQILKAS-
HYLYRHQHTQKGDWASEAPQTAPGGWGFSESNTINPDVDDTTAALRALKLDA-
YTDPVKRMAWNRGVKWALSMQNKDGGWPAFEKNKNKDILSWVPMDGAEDAALDRSCADLTGRT-
LEFLGNDAGMGRENSQVLKGIEWLMNNQENDGSWYGKWGICYIYGTWAALTGMMAAGMSAD-
HQSIIKAIKWLYQIQNSDGGWGESCRSDKERKYISLGASTPSQTAWALDALISINDHPT-
KEIDRGIESLVRLLNTDDWRKEYPTGAGLPGRFYIHYHSYPYIWPLLALSNYKTKFLEVR

>seq ID 51

MVLYGRVCAEIERTITALHTMQQQDGAWRFCFEGSPLTDCHMIFLLRLLEKEEEIEPF-
VARLTSIQTNEGTWKLYEDERAGNVSTTIQAYAALLASGMYTKEDVNMKRAEAFIQERGGI-
ARSHFMTKFLLALHGGYEYPRMFYFPTPILFLPEDSPLSIFELSSSA-
RIHLIPMMICMNKRFTVSKTILPNLDHISGSSKSEWFREDRSSLFETILGE-
VKKFVTYPLSLHHKGDKEAERFMIERIDRNGTLYSYASATFYMIYALLALGHHIQSPLI-
QQAVAGLRTYKWHMEAGIHLQNSPSTVWDTALLSYALQEANVNESTPMIQTATEYIWQRQH-
HEKKDWSLHAPTLSPGGWGFSDVNTTIPDVDDTTAALRALARSRKRNRRIEEAWKKGVNWVK-
GLQNKDGGWAAFEKGVTNRFLTHLPLENSGDMMTDPSTADITGRVLEFFGTYAPNELQDHQKN-
RAITWLMDVQENNGSWYGKWGVSYIYGTWAALTGLRAVGVANTHPALKKAVMWLERIQHRD-
GGWGESCRSSIEKRFVPLSFSTPSQTAWAIDALISYYDEETPVIRKGISYLLEHAASHQEYP-
TGTGLPNGFYIRYHSYSYMYPLLTFAHYINKYRK

>seq ID 32

MLLYEKAHEEIVRRATALQTMQWQDGTWRFCFEGAPLTDCHMIFLLKLLGRDKEIEPFVER-
VASLQTNEGTWKLHEDEVGGNLSATIQSYAALLASKKYTKEDANMKRAENFIQERGGVARAH-
FMTKFLLAIHGEYEYPSLFHLPTPIMFLQNDSPFSIFELSSSA-
RIHLIPMMLCLNKRFRVGKKLLPNLNHIAGGGGEWFREDRSPVFQTLLSDVKQIISY-
PLSLHHKGYEEIERFMKERIDENGTLYSYATASFYMIYALLALGHSLQSSMIQKAIAGIT-
SYIWKMERGNHLQNSPSTVWDTALLSYALQEAQVSKDNKMIQNATAYLLKKQHTKKAD-
WSVHAPALTPGGWGFSDVNTTIPDIDDTTAVLRALARSRGNKNIDNAWKKGGNWIKGLQNN-
DGGWGAFEKGVTSKLLAKLPIENASDMITDPSTPDITGRVLEFFGTYAQNELPEKQIQRAIN-
WLMNVQEENGSWYGKWGICYLYGTWAVMTGLRSLGIPSSNPSLTRAASWLEHIQHEDGGWGE-
SCHSSVEKRFVTLPFSTPSQTAWALDALISYYDTETPAIRKGVSYLLSNPYVNERYPTGT-
GLPGAFYIRYHSYAHIYPLLTLAHYIKKYRK

>seq ID 31

MSTIHENVRSRQKKTISLLRETQNADGSWSFCFEGPILTNAFLILLLTSLGDNDKELIA-
ELAEGIRAKQRPDGTFANYPDDRKGNVTATVQGYAGLLASGLYSRSEAHMIQAERFIISNG-
GLRNVHFMTKWMLAANGLYPWPALHLPLSFLVIPPTFPLHFYQFSTYARIHFVP-
MAVTLNKRFSLKNPNVSSLAHLDRHMTKNPFTWLRSDQDENRDLSSLFAHWKRLLQI-
PAAFHQLGLRTAKTYMLDRIEEDGTLYSYASATIFMVYGLLALGVS-
RHSPVLRKALAGTKALLTSCGNIPYLENSTSTVWDTALLNYALMKSGISDNDQMITSAARFL-
RERQQKKVADWAVHNPHAEPGGWGFSNINTNNPDCDDTAAVLKAIPRKLYPASWERGLS-
WLLSMQNSDGGFSAFEKNVNHPLVRLLPLESAEEAAIDPSTSDLTGRVLHCLGEAGLS-
SDHPQIEKAVQWLIRHQEEDGSWYGRWGVCYIYGTWAALTGMKACGVSQNHPAVK-
KAIRWLKSIQNEDGSWGESCKSAEEKTYVPLSYGTLVQTAWAAEALLQYEKTHHQAVTKGIS-
FLIENRHYEGAAFSYPTGIGLPKQFYIRYHSYPYVFSLLALSTFMKMSEKEEEK

>seq ID 48

MLLYEKAHEEIARRATALQTMQRQDGTWRFCFEGAPLTDCHMIFLLKLLGRDKEIEPFVKR-LASLQTNEGTWKLYEDEVGGNLSATIQSYAALLASKKYTKEDANMKRAENFIKERGGVARAH-FMTKFLLAIHGEYEYPSLFHLPTPIMFLQNDSPLSIFELSSSA-RIHLIPMMVCLNKRFRVGKKLLPNLNHIAGGGGEWFREDRSPLFQTLLSDVKQIISY-PLSLHHKGYEEVERFMKERIDENGTLYSYATASFYMIYALLALGHSLQSSLIQKAIAGIT-SYIWKMERGSHLQNSPSTVWDTALLSYALQEAHVPKDHKMIQQTITYLLKKQHTKKADWSVHA-LALTPGGWGFSDVNTTIPDVDDTTAVLRALARSRGNENIDNAWKKGVNWIKGLQNN-DGGWGAFEKGVTSKLLANLPIENASDMITDPSTPDITGRVLELFGTYTQNELPKKQKQSAIN-WLMNVQERNGSWYGKWGICYIYGTWAVMTGLRSLGIPSNNPSLKRAALWLEHIQHEDGGWGE-SCQSSVEKRFVTLPFSTPSQTAWALDALISYYDKETPTIRKGVSYLLANPYVNEKYPTGT-GLPGGFYIRYHSYAQIYPLLTLAHYTKKYQK

>seq_ID 34

MNIVIRISKGWVSNLLLYEKVHEEIARRTTALQSMQRQDGTWRFCFEGAPLTDCHMIFLL-KLLGRDKEIEPFVKRLASLQTNEGTWKLYEDEVGGNLSATIQSYAALLASEKYTKEDANMKRA-EMFINERGGVARAHFMTKFLLAIHGEYEYPSLFHLPTPIMFLQNDSPLSIFELSSSA-RIHLIPMMLCLNKRFRVGKKLLPNLNHIAGGGGEWFREDRSPVFQTLVSDVKKII-TYPLSLHHKGYEEVERFMKERIDENGTLYSYATASFYMIYALLALGHSLQSSMIQKAIAGIT-SYMWKMESGNHVQNSPSTVWDTALLSYALQEAHVLKDNKMLQNATAYLLKKQHTKKAD-WSVHAPALTPGGWGFSDVNTTVPDVDDTTAVLRVLARSRGNEKVDHAWQKGINWVKGLQNN-DGGWGAFEKGVTSHILANLPIENASDMITDPSTPDITGRVLEFFGTYAQNELPEKQKQSAIN-WLMNVQEENGSWYGKWGICYIYGTWAVLTGLRSLGIPSSDPSLKRAALWLEHIQHEDGGWGE-SCQSSVEKRFVTLPFSTPSQTAWALDALISYYDKETSVIRKGISYLLSNPYINETYPTGT-GLPGGFYIRYHSYAHIYPLLTLAHYAKKYRK

>seq_ID 47

MLLYEKVHEEIVRRATALQTMQWQDGTWRFCFEGAPLTDCHMIFLLKLLGREKEIEPFVE-RIASLQTNEGTWKLYEDEVGGNLSATIQSYAALLASKKYTKEDANMKRAENFIKERGGVA-RAHFMTKFLLAIHGGYEYPSLFHLPTPIMFLQNDSPLSIFELSSSA-RIHLIPMMLCLNKRFRVGKKLLPNLNHIAGGGGEWFREDRSPVFQTLISDVKQIISY-PLSLHHKGYEEIERFMKERIDENGTLYSYATASFYMIYALLALGHSPQSSMIQKAIAGLT-SYIWKMGRGSHLQNSPSTVWDTALLSYALQEARVSKDNKMIQNATAYLLKKQHTKKAD-WSVHAPALIPGGWGFSDVNTTIPDIDDTTAVLRALARSRGNKNIDNAWQKGVNWIKGLQNN-DGGWGAFEKGVTSKLLANLPIENASDMITDPSTPDITGRVLEFFGTYAQNGLPEKQKQSAIN-WLMNAQEENGSWYGKWGICYIYGTWAVMTGLRSLGIPSSNPSLKRAASWLEYIQHEDGGWGE-SCHSSVEKRFVTLPFSTPSQTAWALDALISYYDTETPAIRKGVSYLLSNPYVNERYPTGT-GLPGAFYIRYHSYAHIYPLLTLAHYLKKYRK

>seq ID 52

MRSILEDVKAFRQKTLAELQNRQRSDGSWRFCFEGPVMTDSFFILMLTSLGDQDSS-LIASLAERIRSRQSEDGAFRNHPDERAGNLTATVQGYTGMLASGLYDRKAPHMQKAEAFIK-DAGGLKGVHFMTKWMLAANGLYPWPRAYIPLSFLLIPSYFPLHFYHFSTYARIHFVPMAIT-FNRRFSLKNNQIGSLRHLDEAMSKNPLEWLNIRAFDERTFYSFNLQWKQLFQWPAYVHQLG-FEAGKKYMLDRIEEDGTLYSYASATMFMIYSLLAMGISKNAPVVKKAVSGIKSLISSCGKE-GAHLENSTSTVWDTALISYAMQESGVPEQHSSTSSAADYLLKRQHVKKAD-WAVSNPQAVPGGWGFSHINTNNPDLDDTAAALKAIPFQRRPDAWNRGLAWLLSMQNKDGGFAA-FEKDVDHPLIRNLPLESAAEAAVDPSTADLTGRVLHLLGLKGRFTDNHPAVRRALRWLD-HHQKADGSWYGRWGVCFIYGTWAALTGMKAVGVSANQTSVKKAISWLKSIQREDGSWGESCK-SCEAKRFVPLHFGTVVQSSWALEALLQYERPDDPQIIKGIRFLIDEHESSRERLEYPT-GIGLPNQFYIRYHSYPFVFSLLASSAFIKKAEMRETY

>seq_ID 188

MRSELLQLQSADGSWRLCFDSGTMPDSYFIIILRMLGYSQDEALIRQIAS-
RILSRQLPNGTWKIYPDEEDGNLDATAEAYFALLYSGFLTKLDPRMQLAKQFILSKGGLS-
KIRSLLTQAIFAAAGQASWPKSMRIPLEVFFSDNGIGIDLFSLSGHARVHIVPIIM-
LANAQFVQHSASMPDLSDLFAGSSKRFENDSPWIAALATLIGSLSLSELLPFESPTPQE-
KAVQFLFDRLEPDGTLLTYTTATMFMILVLLMLGYSSSSPLIHRMVSGIHSVICANSHVQIAS-
SEVWDTAMLVHALRKAGVNPTSTALENAGAYLRQRQQTQLGDWAIRNPGT-
PAGGWGFSNVNTLYPDVDDTTAALRAIQPYSSRTPELQADWQRGLNWVLTMRNDNG-
GWPAFERQGSRLPITFFNFEGAKDIAVDPSTVDLTSRTLQFLGQELGMNAGNSWIEST-
LRWVLSQQESNGSWYGRWGITYVHGTSAALQGLTAVGIAEDHPAVKKGVDWLLQVQNEDGGW-
GESCISDKVRRYVPLNFSTPSQTAWALDGLTAALPKPTPALERGVDALLQSLDRHDWTYTYP-
TGGALPGSVYAHYASNNYIWPLLALSNIWQKYS

>seq_ID 60

MGTLQEKVRRFQKKTITELRDRQNADGSWTFCFEGPIMTNSFFILLLTSLDEGENEKE-
LISSLAAGIHAKQQPDGTFINYPDETRGNLTATVQGYVGMLASGCFHRTEPHMKKA-
EQFIISHGGLRHVHFMTKWMLAANGLYPWPALYLPLSLMALPPTLPIHFYQFSSYARIHFAP-
MAVTLNQRFVLINRNISSLHHLDPHMTKNPFTWLRSDAFEERDLTSILLHWKRVFHAP-
FAFQQLGLQTAKTYMLDRIEKDGTLYSYASATIYMVYSLLSLGVSRYSPIIRRAIT-
GIKSLVTKCNGIPYLENSTSTVWDTALISYALQKNGVTETDGSVTKAADFLLERQHTKIAD-
WSVKNPNSVPGGWGFSNINTNNPDCDDTTAVLKAIPRNHSPAAWERGVSWLLSMQNNDGGFSA-
FEKNVNHPLIRLLPLESAEDAAVDPSTADLTGRVLHFLGE-
KVGFTEKHQHIQRAVKWLFEHQEQNGSWYGRWGVCYIYGTWAALTGMHACGVDRK-
HPGIQKALRWLKSIQNDDGSWGESCKSAEIKTYVPLHRGTIVQTAWALDALLTYEN-
SEHPSVVKGMQYLTDSSSHSADSLAYPAGIGLPKQFYIRYHSYPYVFSLLAVGKYLDSIEKETANET

>seq_ID 56

MQDFKTKVNVYMDELHMQMQHRQREDGAFVFCFEGSMMTNAFLIMLLKAVGDTDQAL-
VHQLAEAIREKQNEDGSFSLYHDQAGHVTATVQGYCGMLVSGRYQQDEPHMEKAA-
RYIRSKGGLKDVHFMTKWMLAVNGMHPWPYFYAPLSFLLIPTYFPLHFYHLSAYARIHFVPMMI-
ALNKRYTSHEQFPSLSHLDANMSKNPFDWFMAREERSTHHFLAYMR-
SYTALDSRFDFFGYEAAKRYMFDRLEKDGTLYSYLSASIFMVYALMSLGYSPGHHLIL-
KAVKGMKQLVTDCGGKKYAENSTSTVWDTALVSYASQRAGRTQDDPVIKKSFTYLLNRQQMK-
KADWAIHNRHAAPGGFGFSDLNTNNPDCDDTQIVLKAIPQTYAPVQWKRGFDWLLSMQNRD-
GGFSAFEKNQDHFLLRHLPLESAEDAAIDPSTPDITGRVLHLIASEEND-
KSPLMQRQKDHCVKWLLDHQEKDGSWYGRWGVCYIYGTWAALTGLKASGIPSSHPA-
VQKACRFLKTIQLEDGSFGESCKSSEVKRYVPLPFGTVVQTAWAAEALLQYVQPDDKSIL-
KAISFLIQHQHSSKALHYPVGIGLPKQFYITYHSYPFVFPMMACSTFLEEMRRKNE

>seq_ID 58

MKNRNKGAGCMQLVKSEIERLKQQLLSEQTPDGSWNHPFDTGCMTDIYMIVLLRTLEEEDEEE-
LIKELAKGILSRQGKDGAWRLFHDHHEGSLSLTIEAYYALLYSGYYEKNHPALVKARRVIT-
KGGGLKKAGMYTKIMLALTGQYPWPLLFPVPMEVILLPRSFPLNMYDISVFGRSNLIPVILL-
GNKKFSRKTALSPDLGDLSVRDDDDPWPELRSAEWRSLTSFLAAGVKALVGIPRQIRAWSIE-
KAREYMQSHTEPDGTLYNYFSSTFYMIFALLALGGGPEEPAIRNAVAGLKRMTVKADGRT-
HIQYTTAAVWNTALISHALQEAGVPPKENAIQKANQYLAGQQHRRFGDWIVHNTKA-
EPGGWGFSRFNTINPDVDDTTAALRSLYQPAREKPHYDDIWKKGLLWTLSMQNRD-
GGWPAFERNVDKKLLHLLPIQGAEFILTDPSTADLTGRTLEFLGKAGYADASLPPIK-
KAVKWLKKHQEPNGSWYGRWGICYIYGTWAAVTGMAAVGVTLEDKSMKKGIDWLL-
SIQNEDGGWGESCRSDMEKKYIPLKESTLTQTAWAVDALAAAGMADSTPSRKGAAFLVREG-
KRKDWTADYPMGQGMANFFYIHYHSYRCIWPLLALSHYIEKSEAPD

>seq_ID 57

MQDFKTKVNEYIDELHMQLQRRQREDGAFVFCFEGPMMTNAFLIMLLKAVGDSDQAL-
VHQLAEAIREKQNEDGSFSLYHDQAGHVTATVQGYCGMLVSGRYQQDEPHMEKAAHFIRSNG-
GLKDVHFMTKWMLAVNGMHPWPYFYAPLSFLLIPTYFPLHFYHLSAYARIHFVPMMIAL-
NKRYTSHEQFPSLAHLDANMSKNPFDWFMAREERSTHHFLAYMRSYTALDSRLD-
FFGYEAAKRYMFDRLEKDGTLYSYLSASIFMVYALMSLGYSPGHHLIL-
KAVKGMKQLVTDCGGRKYAENSTSNVWDTALVSYASQQAGRTQDDPVIKKSFTYLLNRQQMK-
KADWAIHNRHAAPGGFGFSDLNTNNPDCDDTQIVLKAVPQTYAPVQWKRGFDWLLS-
MQNQDGGFSAFEKNQNHFLLRHLPLESAEDAAIDPSTPDIAGRVLHLIALEENS-
MSPLMQRQKDHCVKWLLDHQEKNGSWFGRWGVCYIYGTWAALTGLKTA-
GISSSHSAVQKACRFLKTIQLEDGSFGESCKSAEVKRYVPLPFGTVVQTAWAAEAL-
LQYVQPDDKVILKAISFLIQHQHSSEALHYPVGIGLPKQFYITYHSYPFVFPMMACSTFLEEMRRKNE

>seq_ID 61

MGTLQEKVRRFQKKTITELRDRQNADGSWTFCFEGPIMTNSFFILLLTSLDEGENEKE-
LISSLAAGIHAKQQPDGTFINYPDETRGNLTATVQGYVGMLASGCFHRTEPHMKKA-
EQFIISHGGLRHVHFMTKWMLAANGLYPWPALYLPLSLMALPPTLPIHFYQFSSYARIHFAP-
MAVTLNQRFVLINRNISSLHHLDPHMTKNPFTWLRSDAFEERDLTSILLHWKRVFHAP-
FAFQQLGLQTAKTYMLDRIEKDGTLYSYASATIYMVYSLLSLGVSRYSPIIRRAIT-
GIKSLVTKCNGIPYLENSTSTVWDTALISYALQKNGVTETDGSVTKAADFLLERQHTKIAD-
WSVKNPNSVPGGWGFSNINTNNPDCDDTTAVLKAIPRNHSPAAWERGVSWLLSMQNNDGGFSA-
FEKNVNHPLIRLLPLESAEDAAVDPSTADLTGRVLHFLGE-
KVGFTEKHQHIQRAVKWLFEHQEQNGSWYGRWGVCYIYGTWAALTGMHACGLTE-
SIPVYKRLCVGSNPYKMMTEAGENPAKAPKSKHMYRFIEEPLYKRPGL

>seq_ID 50

MAEAISYPRRVHIITTKFPVNFYDFSVFGRSNIAPILLLADSKFQIPKTTETPDISHLYV-
RELYWWSEDRGWNGFTKAINKGVNNLIGLPNELHTLGRKQAENYMLDRLEDDGTLLSYYSST-
FFMIYALLSVGYTKDHKVIKKAARGLLSMNTTVKDTIHIQYTTAHIWNTSLISHALQTAG-
ASPDDTMVMRANHYLLQRQHTKFGDWAIYQPNLGPGGWGFSHSNTFNPDVDDT-
TASLRSIQNSLHSHPNYQSSWYRGLSFTLGMQNQDGGFPAFEKGVDKTFLHLL-
PVQGAEFLLTDPSTPDLTGRTLEFLGESAHLYKDSGAIKRGVNWLIENQRRDGSWYG-
RWGICYIYGTWAALTGLQAVGVSKEHPSVQEGIDWLKSIQQDDGGWGESCESDSQKTY-
IPLSKSTVTQTAWAVDALIAYEKEETVEIKKGMEYLLENWNHEDWTMDYPMGQGMAKA-
FYIHYHSYRYVFPLLTMGHYMRKFM

>seq ID 199

MSETISCQRIQAAYQRSRAELLSLRNSTGHWTGELSTSALSTATAIMALEMIRRKRL-
PADLSLNTYIDNGIRWLAEHQNSDGGWGDTVKSFSNISTTMLCHAVFHAT-
KSTEQYVSHVVNARQYIDRVGGVEAVVARYGKDKTFSVPILTHCALAGLVKWKTIPALPFEL-
ACLPARFYKTVRLPVVSYALPALIAIGQVRHHFCKPRNPITRLIRKLAVKRSLKKLISIQPS-
NGGFLEAAPLTSFVTMSLAGMGLTDHPVVQKGLQFLLDSVRPDGSWPIDTNLATWTTTLSVNA-
LEGTLAEFEKTPIREWLLQQQYKELHPYTSAEPGG-
WAWTDLPGGVPDADDTPGAILALLNLQPDEPDTQQPADLQVALRNGVKWLLDLQNSNGGWPT-
FCRGWGALPFDQSAADISAHVIRALQAWLQTEPESAEAELRLRAERAVRKCFKYLAT-
VQRPDGSWLPLWFGNQHVENDENPVYGTARVLAAYAQGEQCGSIQAEQGILFLKSVQNLD-
GGWGGATSAPSSVEETALAVDTLLALGLEPADPVVAQGLNWLSGRVENGTYTETTPIG-
FYFAKLWYFEQLYPIIFTVSALHRAETVLKKSADDNLRLSLEEEDYPIMSVKEK

>seq ID 75

MDQDRLQRCYAIARDDLLAQRNGQGHWTGELSTSALSTATAVSALQLVVRHDPAQSERLM-
PLIEGGVRYLTEHQNPDGGWGDTDRSYSNIATTMLAVAALTIAERREALFEQLAFAENYIE-
AQGGIPGLRRRYGKDKTFAVPILTNYALAGLVDWREVSPLPFEL-
ACLPQKFYKLVKLPVVSYAIPALVAIGQARYFHRPPFNPLM-
RGLRGAAVKKSLAVLERMQPASGGYLEAAPLTSFVVMSLASIGNASHPVAQNGVQFLVDSV-
REDGSWPIDSNLANWVTTLSISALATGGDDIAELDCLPWVLANQYQETHPFT-
GADPGGWGWTDLSGSVPDADDTPGAMLAIAHFFHSPRADNETRRQIASAAISGARWLLD-
LQNSDGGWPTFCAGWGTQPFDRSGSDLTAHAIRALHAWRSELGDLPVERAIER-
GLRYLQKQQRDDGSWLPLWFGNQDIHDDENPIYGTVKVLLAYRDLGKMS-
SETAQRGAAWLAARQNEDGGFGGGPSISTLCGGPGESSVEETALAIEALFAAENSNISAEIV-
PPAVGWLCQRVEEGSYVNCTPIGFYFSKLWYYEKLYPRVMTVTSLGAALQANASVPPAPETVTTSSDH

>seq_ID 325

MATSDPSLAEAIQNTRAHLLSLRNARGHWEGHLSNSALSTATAIVALHLVDAPLHSA-
RIAQGVRWLVLHQNKDGGWGDTTLSKSNLSTTLLCWSALSLCEPDRTEPIQHCEAWIKERTGS-
LEPEVICRAVVARYGKDKTFSVPILMLCAIGGRLGPEKEAWSRVLALPFELAAMPREWFGAI-
GLPVVSYALPALIAIGYARFYHAPPSLLNPLHALRKALWPRISPMLKLLQPSTGGYLEAT-
PLTSFVTMALASAGEKFHPCVPEAVRFLEDSQRPDGSWPIDTNLATWGTTLSTKALTAT-
SEGREALDIPALKSWLLEQQYQEIHPFTNAAPGGWAWTDLPGGVPDADDTSGALVALWHLCE-
DEAERQALAPAVAKGVQWLMDLQNRDGGIPTFCRGWGTLPFDRSTPEITAHALHAWGLWQVVL-
PEELQQEVSLRIPRAIAFIARPPSRGAPGFNHVPLWFGNEHAKEEENHVYGTAQIMNHLLSS-
GLNTPEIKVILETGHRNLLAWQQLDGGWSGSETGPASLEETAVSVAALALHTLHAGNRTRSSA-
EDAVAKGTQWLVQHTATGTTFPSAPIGLYFARLWYHEQLYPVIWTLGALHAVETLSAAALPL-
RARASAPPQHPGVVRTKPIHIAPPSDP

>seq_ID 135

MIPAERLRTAYRTARAALLAERVPEGHWVGELSTSALSTATAVMALHLVNPFTHRELI-
DAGRKWLAEHQNADGGWGDTVKSFSNISTTMLCRAAFKLAGEKEYPETVQRVEEYLSR-
NAGALPTARAAAIRARYGKDHTFSVPILMTCAVAKLVPWDEVPRLPFEL-
ACLPQSWYRFAKLPVVSYALPALIAIGQCIHHHRRSQNPIRNTVRRLARG-
LSLKVLRRIQPTSGGYLEATPLTSFVVMALSSIRRRRAAAEQQVIDEGVRFLVASVRPDGS-
WPIDTNLATWVTTLSVNALATAGDLEALDTKEQILAWLLKQQYKERHPYTGADPGG-
WAWTDLPGGVPDCDDTPGALIALAHLDPKSDPQAVLSGLRWVLRLQNGDGGAPTFCRGWGTL-
PFDRSGADLTAHSVRSLASWYRVWGAGPPPIEHLRHRLKDLEFPLSGLFWDVARRNPRF-
VRYLKKQQRSDGSWLPLWFGNQHAPDDINPVYGTARVLAAYRDLELKDAPECRRGIEFLLS-
VQNADGGWGGAKGCPSSVEETALAVEVLLDLADGDAVQKGVAWLAEAVESDRFRDASPIG-
FYFAKLWYFEKLYPIIFTVAALGRAVKITSPAPAAESA

>seq_ID 115

METLSRSRLEAALAKATQALLTELNPAGHWSGELSSSALSTATAIVALGAVDREQQRELI-
AGGMRWLAQHQNADGGWGDTVKSRSNISTTALCWAAVSTSTEHAESAAKAEAWLTRAAGS-
MAQLVPAIEARYGKDRTFSVPILMHLAICGRVSWSQIPALPFELAALPHQLF-
GALQLPVVSYALPALIAIGQAIHHHAPPTNPLLNGLRKSARARTLEVLESIQPQNGGFLEAT-
PLTSFVTMALASAGEAQHPVARRGVSFLQASVQRDGSWAIDTNLATWVTTLSI-
KALAHQPGALSPERALTLREWLLGQQYVVEHPYTHAAPGG-
WAWTDLPGGVPDADDTPGALLALLHLGVVDAPTRQAGQIGVRWLLDLQNRDGGIPTFCRG-
WGALPFDRSSPDLTAHTLRAWTAWLPQLDESLKRRTLRAVTKAIHFLATHQRTDGS-
WLPLWFGNEHAPDDENPLYGTAKVVIALRELLNRDFTLPNGMLERALCWLVERQDISGGWSG-
AKNGPVSVEETALAVEALAGTGHVSATDRGAAWLTEQIEADTWREPAPIG-
FYFAKLWYYERLYPQIWTVGALGRVAALRVGESESDTPAGLHRATSET

>seq_ID 208

MMAVVENSVSEVLDRRELRGTLDLLRGELLAQRTKDGHWTGELSASALSTATAI-
SAMSAAVRSGKLAGADKAALLEQIQSGRRWLADQQNDDGGFGDTDRSHSNIAT-
SYLVLAAWTLSDQVTGETTDANAISRLRNWIQLAGELDGLRRRYGKDKTFVVPILTNMAI-
AGLVPWKKVSALPFEAAVVPQSMYRFVGMPVVSYAVPALVAIGQVKFLEGGGCLPPWSLV-
RRAAIEPSMKVLRSMQPSSGGYLEATPLTAFVVMSLSASGRADHEVTQNGLRFLRDS-
MLPDGSWPIDTNLANWATSLATTALTMDPDDDRSWSTNELIQWQRGCQYQERHPFT-
GADPGGWGWTDLTGSVPDADDTPGAIISLRMQATTRPDPLCDDYSRDWPASDSSGSVSANAL-
DTWKACDRGVDWLLGLQNRDGGWPTFCRGWGKLPFDRSSNDLTAHALRAIACLPKRESAKRS-
RAVQRGLRFLRKNQQADGSWLPLWFGNQDRPEEDNPIYGTSRVLVDVSPALGHDAISRGLYY-
LINSQNSDGGWGGGESVRETFGLPEGFISSVEETALAVEALVSWWGRIPGNEGGQAA-
ENDIPDGSPWDASMRSALRAAILSGTRWLIDAVQRERHQVAWPIGFYFAKLWYYERLYPLVYT-
TAALGRVMQRDELLR

>seq_ID 247

MEIQDEVDLLEPQESLTASADSAVDRALFWLLDAQYEDGYWAGILESNACMEAEWLLCFHVL-
GIANHPMSRGLVQGLLQRQRADGSWDVYYGARAGDINTTVEVYAALRCQGYAADHPDIKRARD-
WIQLQGGVKQVRVFTRFWLALIGEWPWEETPNLPPEILFFPRWFPFNIYHFAAWARAT-
LVPLCILSARRMVVPLNKKSCLQELFPEDRSAVVALGKKAGAWSTFFYHADRALKKYQRTF-
KRPPGRQQAIKMCLEWILRRQDADGAWGGIQPPWIYSLMALKAEGYPVTHPVMAK-
GLAALDAHWSYERPGGARFVQACESPVWDTLLSSFALLDCGFSCTSSSELRKAVD-
WILDQQVLLPGDWQQKLPTVSPGGWAFERANVHYPDVDDTAVALIV-
LAKVRPDYPDTARVNLAIERGLNWLFAMQCRNGGWGAFDKDNDKDLLTKIPFSDFGE-
TIDPASVDVTAHVLEALGLLGYRTTHPAVAKALEFIRSEQENDGCWFGRWGVNYIYGTAAVL-
PALASLNMNMNQEFIRRAANWILGKQNNDGGWGESCASYMDDTQRGRGPSTASQTAWAMMS-
LLAVDGGTYAESLLRAEAYLKTTQTPEGTWDEPYYTGTGFPGYGIGRREIKRQRSLQQHA-
ELSRGFMINYNLYRHYFPLMALGRLAALRGA

>seq_ID 148

MTSPFKHPISHALTSFNGIVTEPEQSVEQKAGAKVHQFPASLWKSKPG-
KAKSPLDIAIEGCRDFFFREQLPKGYWWAELESNVTITAEYIMLFNFLSLVDHERQRK-
MSNYLLSKQTEEGFWTIYYGGPGDLSTTVEAYFALKLTGYPADHPAMVKARAF-
ILEKGGVIKSRVFTKIFLALFGEFDWLGVPSMPVELNLLPNWAYVNVYEFSSWARATIIPL-
SIVMLKRPVHKLPPSQRVQELFVRPPRAIDYTFTKEDGIFTWKNFFIGLDHMLKVY-
ERSPVRPFKKRAMGKAEEWVLEHQEETGDWGGIQPAMLNAVLALSALGYDNGHPAVAHGL-
KALENFCIESDEQIVLQSCISPVWDTALALKALVDAGVPSDHPSLVKGAQWL-
LEREVRRPGDWRVKSPDLEPGGWAFEFLNDWYPDVDDSGFVMIALKGVEVKDRKAM-
NAAVKRGIDWCLGMQSKNGGWGAFDKDNTRHILNKIPFADLEALIDPPTADLTGRM-
LELMGTFGYAKTYPAAQRALKFLKENQEPEGPWWGRWGVNYLYGTWSVLCGLAAIGED-
LEQPYIKKAVNWIKSRQNMDGGWGETCESYHDPTLAGMGESTASQTGWALLGLMAAGEVH-
SATVVRGVQYLISTQSQDGTWDETQYTGTGFPKYFMIKYHIYRNCFPLMALGTYRTLTGGTA

>seq_ID 149

MTSPFKHPISNALTSFNGNFAEPEQCVEQQTGAKVHHLPASIWKRKMGKAKSPLDVAIEGS-
RDFFFQEQLPKGYWWAELESNVTITAEYIMLFHFLGLVDRERQRKMSNYLLSKQTEEG-
FWPIYYGGPGDLSTTIEAYFALKLSGYPADHPALAKARAFILEQGGVVKSRVFTKIFLALFGE-
FEWQGVPSMPVELNLLPDWAYINIYEFSSWARATIVPLS-
VVMHSRPVRRVPPSARVQELFVRQPTAADYSFAKNDGIFTWENFFLGLDRV-
LKVYEKSPLRPFKNMALAKAEEWVLEHQEPTGDWGGIQPAMLNAVLALNVLGYQNDHPA-
VEQGLRALANFCIETEDQLVLQSCVSPVWDTALALKALLDAGVPPDHPSLVKGAQWLLD-
KEVTRPGDWRVKSPALEPGGWAFEFLNDWYPDVDDSGFVMIALKGIQVKDRKS-
MDAAIKRGINWCLGMQSKNGGWGAFDKDNTRHVLNKIPFADLEALIDPPTADLTGRM-
LELMGTFNYPITLPAAQRAIEFLKKNQEPEGPWWGRWGVNYLYGTWSVLCGLAAIGE-
DMDQPYIRKAVNWIKSRQNIDGGWGETCQSYHDRTLAGVGESTPSQTGWALLGLLAAGEMH-
SATVVRGVQYLISTQNSDGTWDEQQYTGTGFPKYFMIKYHIYRNCFPLMALGTYRTLTRTQP

>seq_ID 216

MTDVLTRELSPNSTRDRVRSCVSSARQYLLSLQHEEGWWKGELDTNVTMEAEDLLLRQFL-
GISDEQVTQETARWIRSCQREDGTWATFHGGPPDLSTTVEAYVALRLAGDAMDAAHLRKA-
REYILDSGGIESTRVFTRIWLALFGEWPWSRLPVLPPEMMLLPDWFPLNIYDWAS-
WARQTVVPLTIVGSLRPTRDLGFSVRELRTGIQRRDLESPLSWAGVFHGLDSVLHRLE-
KLPLKPLRKVALARAEQWILDRQESDGGWGGIQPPWVYSILALHLRGYPLDHPVLRKALD-
GLDGFTIRHRTENGWIRKLEACQSPVWDTALAMTALLDSGTPPNDPALVRAAD-
WILRQEIRVSGDWRVRRPALEPSGWAFEFANDHYPDTDDTAEVVLGLQRVRHPEPHRVNAAVE-
RATAWLVGMQSSDGGWGAFDADNTRTLCEKLPFCDFGAVIDPPSADVTAHIVEMLAARG-
MADSESARRGVRWLLEHQEVDGSWFGRWGANHVYGTGAVVPAL-
VACGISPQHEAVRAAVQWLVAHQNADGGWGEDLRSYVDRTWVGRGTSTPSQTAWALLALLAA-
GERGEVVRRGVEWLMAAQRPDGGWDEPQYTGTGFPGDFYISYHMYRIVFPLTALGRYLGRGGDVGTG

>seq_ID 229

MTATTDGSTGASLRPLAASASDTDITIPAAAAGVPEAAARATRRATDFLLAKQDAEGWWKGD-
LETNVTMDAEDLLLRQFLGIQDEETTRAAALFIRGEQREDGTWATFYGGPGELSTTIE-
AYVALRLAGDSPEAPHMARAAEWIRSRGGIASARVFTRIWLALFGWWKWDDLPELP-
PELIYFPTWVPLNIYDFGCWARQTIVPLTIVSAKRPVRPAPFPLDELHTDPARPNPPR-
PLAPVASWDGAFQRIDKALHAYRKVAPRRLRRAAMNSAARWIIERQENDGCWGGIQPPA-
VYSVIALYLLGYDLEHPVMRAGLESLDRFAVWREDGARMIEACQSPVWDTCLATIAL-
ADAGVPEDHPQLVKASDWMLGEQIVRPGDWSVKRPGLPPGGWAFEFHND-
NYPDIDDTAEVVLALRRVRHHDPERVEKAIGRGVRWNLGMQSKNGAWGAFDVDNTSAFPNRL-
PFCDFGEVIDPPSADVTAHVVEMLAVEGLAHDPRTRRGIQWLLDAQEADGSWF-
GRWGVNYVYGTGSVIPALTAAGLPTSHPAIRRAVRWLESVQNEDGGWGEDLRSYRYV-
REWSGRGASTASQTGWALMALLAAGERDSKAVERGVAWLAATQREDGSWDEPYFT-
GTGFPWDFSINYNLYRQVFPLTALGRYVHGEPFAKKSRAADAPAEAAPAEVKGS

>seq_ID 113

MTDVIDKAVAATGPADPSQGAAATLQAAADHLLGLQDDAGWWKGELETNVTMDAEDLLLRQFL-
GIRTEEVTREAGDWIRSQQRADGTWANFFDGPADLSTTIEAYTALRMAGDAKDAEHMRAAR-
TYILDSGGIEASRVFTRIWLALFGEWQWSDLPVMPPELIYLPKWF-
PLNVYDWACWARQTVVPLTIVNALRPVRPLGFDLKELRTGRRAPAQRGLFSTLDRALHVY-
ERKPLRSVRDAALRRSADWIIARQEADGSWGGIQPPWVYSLMALNLLGYGVDHPVMRKGIEG-
LDRFTIRDERGRRLEACQSPVWDTVLAMTALRDAELPENHPALVKAADWVLGEEIT-
NPGDWSVRRPRVAPGGWAFEFDNDGYPDVDDTAEVVLALNRVAHPDAPAAIRRGVDWLEG-
MACKDGGYGAFDADNTRTLALKLPFCDFGAVIDPPTADVTAHTLEAYAALGLANS-

RASQRALEWLVKAQERDGSWFGRWGANHVYGTGAVVPAMVAVGVDPEDEMIRRAVRW-
LEEHQNDDGGWGEDLRSYRDKSWIGRGVSTASQTAWALLALLAAGEERGTA-
VEQGVRFLIRTQRADGTWDEDHYTGTGFPGDFYLNYHLYRLVFPISALGRYVRAVGAAGD-
GGDAGHAGHAGTVS

>seq_ID 236

MTATTDGGGAITGGADPRHDSTAAPAAAAAGPSGGGTGLPEGVREAVDRATAELLARQDPAG-
WWKGDLQTNVTMDAEDLLLRQFLGIRDEAVTRAAALFIRGEQQGDGTWATFHGGPPELSATIE-
AYVALRLAGDPPDAPHMTRASAWIRAHGGIAAARVFTRIWLALFGWWSWDRLPELPPEL-
VFLPPWVPLNIYDFGCWARQTIVPLTVVSALRPVRSAPFALDELHTDARDPVPAKPLPPLAS-
WDGAFQRMDKALHLYRRVAPRRLRKAAMAAAGRWIVERQENDGCWGGIQPPAVYSVIALHLL-
GYDLGHPVMRAGLESLDRFAVWREDGARMVEACQSPVWDTCLAAIALADAGLPPDHPALV-
RAADWMLGEEIRRPGDWAVRRPGLAPGGWAFEFHNDNYPDIDDTAEVVLALRRIRHPQPGG-
VEAAIARGVSWTLGMQSKNGAWGAFDADNTSPFPNRLPFCDFGEVIDPPSADVTAHVVEM-
LAAEGRAADPRARRGIAWLLAEQEPDGPWFGRWGTNYVYGTGSVVPALTAAGIAPSHPA-
VRRAVRWLESVQNEDGGWGEDQRSYRDRSWAGKGASTASQTAWALMALLSAGERDGDAVARG-
LAYLVETQRPDGTWDEPYFTGTGFPWDFSINYHLYRQVFPLTALGRYLHGEPFGPERRNVPPAGES

>seq_ID 134

MSLTSDPSPATPATQPTSARPGSLSDRRSRSGGSAVAGPVLVTTRPVAPVAKSGAVTPTATS-
GAVTSTATSGPALLPDLATDLADPTGPLAGAASATVRAAGGAG-
TRTQQTGQLGSTELAGPQADQVADRAAAVLGRARDHLLGLQSEAGWWKGELETNVTMDAED-
LMLRQFLGILPPELAAETGRWIRSKQQDDGGWPTFHGGPSDLSTTFEAYVGLR-
LAGDLPDAPHMLAAASFVRAHGGLAATRVFTRIWMALFGEWPWDEVPVLPPELVLL-
PSWVPLNVYDFGCWARQTVVALTIVGHFRPVRSLGFSIDELRVAAVRPDRA-
PLVSWTGVFQRLDAGLRRYQRHPVKTLRELALRRATEWVLARQEADGGWGGIQPPWVYSI-
MALHLMGYSMDHPVLVAALDGLETFTVREQVREGDE-
VVTVRRLEACQSPVWDTALAVVALADAGLDARHPAMRKAGEWLVREEVTVPGDWRVRRPN-
LEPGGWAFEFANDIYPDVDDTAEVVLAVRRLLGSGWDDVDPTFAKQARAS-
VERAVNWSVGMRSANGAWGAFDADNVRELATKIPFCDFGEVIDPPSADVTAHMVEMLADL-
GRADHPVTQRAVRWLLDDQEPGGSWFGRWGVNHVYGTGAVVPALISAGVAADH-
PAIRSAVRWLVAHQHPDGGWGEDLRSYQDDAWVGRGEPTASQTA-
WALLALLAADPMNEAVGRGVRWLCDTQLPNGTWDEPYYTGTGFPWDFSINYHLYRLVF-
PLTALGRYVTLTGRSAA

>seq_ID 225

MTATTDGSTGAALPPRVTAASDTDTDIPVAAGVPDIAARAMRRATDFLLSRQSDQGWWKGD-
LETNVTMDAEDLLLRQFLGIRDEGTTRAAALFIRGEQREDGTWATFHGGPGDLSATIE-
AYVALRLAGDPPDAPHLARASAWIREQGGIAASRVFTRIWLALFGWWKWEDLPELPPELIWF-
PAWVPLNIYDFGCWARQTIVPLTIVSAERPVRPAPFPLDELHTDPARPNPPRALAPVTGWD-
GAFQRLDKALHVLRGAVPRRLRRAAMNTAARWIIERQENDGCWGGIQPPAVYSIIALHLL-
GYDLNHPVMRAGLESLDRFAVWREDGARMIEACQSPVWDTCLATIALADAGLPAD-
HPQLVKAADWMLGEQIVRPGDWSVRRPHLPPGGWAFEFHNDNYPDIDDTAEVVLALRR-
VAHHDPERVDNAIGRGVRWNLGMQSRNGAWGAFDVDNTSPFPNRLPFCDFGEVIDPPSAD-
VTAHVVEMLAAEGLAHDPRTRRGVQWLLAEQEPNGSWFGRWGVNYLYGTGSVVPALTAAGIS-
GSHPAIRRAVAWLESVQNDDGGWGEDLRSYRDARGWSGRGASTASQTAWALMALLAAGERES-
RAVERGVEWLAATQHEDGSWDEPYFTGTGFPWDFSINYHLYRQVFPLTALGRYVNGEPLAGKP-
RAAGAATAREDTGQEQSLAEAKGS

>seq_ID 223

MTATTDGSTGAANITGAPADDPTDTRTAANDVTDIARRAAERSVEHLLGRQDEQGWWKGD-
LATNVTMDAEDLLLRQFLGIQDPATTRAAALFIRGEQLGDGTWNTFYGGPGDLSATIE-
AYVALRLAGDRPDEPHMARASGWIRDQGGIAAARVFTRIWLALFGWWKWDDLPELPPELM-
FFPKWVPLNIYDFGCWARQTIVPLTIVSAKRPVRPAPFALDELHTDPDHPNPPRKLAPPTS-
WDGLFQRLDKGLHLYHKVAPRPLRRIAMNVAARWIIERQENDGCWGGIQPPAVYSVIALHLL-
GYDLDHPVMKAGLASLDRFAVHREDGARMIEACQSPVWDTCLATIALADAGLRPDHPAL-
VKAADWMLAEEITRPGDWSVRKPELAPGGWAFEFHNDNYPDIDDTAEVVLAL-
RRVRHPDPARLEAAIARGVRWNLGMQSRNGAWGAFDADNTSPFPNRLPFCDFGEVIDPPSAD-
VTGHVVEMLAVEGLANHPRTREGIEWLLAEQEACGAWFGRWGVNYVYGTGSVVPALITAGL-
PAGHPAIRRAVDWLESVQNDDGGWGEDLRSYQEEKWIGHGESTASQTAWALLALLAAGRRD-
TASVTRGVTWLTEAQQADGSWDEPYFTGTGFPWDFSINYHLYRQVFPLTALGRYVHGDPFADRTDAAEGV

>seq_ID 226

MTATTDGSTGAALPPRVTAASENDTDIPEAAGVPDIAAHAMRRATDFLLSRQDDQGWWKGD-
LETNVTMDAEDLLLRQFLGIRDEDTTRAAALFIRGEQREDGTWATFHGGPGELSTTIE-

AYVALRLAGDPPEAPHMARASAWIRERGGIAAARVFTRIWLALFGWWKWEDLPELP-
PELIWFPSWVPLNIYDFGCWARQTIVPLTIVSAKRPVRPAPFPLDELHTDPRRPRPPRPHA-
PPNTWDGAFQRLDRALHALRRAVPRRVRQAAMNAAARWIIERQENDGCWGGIQPPAVYSVI-
ALHLLGYDLRHPVMRAGLESLDRFAVWREDGARMIEACQSPVWDTCLAAIALADAGLPAD-
HPSLVKAADWMLGEQIVRPGDWSVRRPHLPPGGWAFEFHNDNYPDIDDTAEVVLAL-
RRVRHHDPERMDSAIGRGVRWSLGMQSKNGAWGAFDVDNTSPFPNRLPFCDFGEVIDPPSAD-
VTAHVVEMLAVEGLAHDPRTRRGIQWLLAEQEPDGSWFGRWGVNYLYGTGSVVPA-
LAAAGIPGSHPAIRRAVAWLEKVQNDDGGWGEDLRSYRHVREWSGRGASTASQTAWAL-
MALLAAGERDSGAVERGVAWLAATQREDGSWDEPYFTGTGFPWDFSINYHLYRQVF-
PLTALGRYVHGEPFSKKQTAARNGSAQPLAGVKGSR

>seq_ID 219

MDPALSRAVDWLLEHQDPAGWWCGEFETNVTITAEHILLLRFLGLDPSPLRDAVTRYLL-
GQQREDGSWALYYEGPADLSTSIEAYAALKVLGLDPTSEP-
MRRALQVIHDLGGVAQARVFTRIWLAMFGQYPWDGVPSMPPELIWLPPSAPFNLYDFACWA-
RATITPLLIILARRPVRPLGCDLGELVLPGSEHLLTRVPGSGPFWWGDKVLKRYDHLVRHP-
GRDRACQRIVEWIIARQEADGSWGGIQSAWVMSLIALHLEGLPLDHPVMRAGLAGFDRVALE-
DERGWRLQASTSPVWDTAWAVLALRRAGLPREHPRLALAVDWLLQEQIPGGGDWQVRT-
GTIPGGGWAFEFDNDHYPDIDDTAVVVLALLEAGHEDRVRNAVERAARWILAMRST-
DGGWGAFDRDNAREVIHRLPIADFGTLIDPPSEDVTAHVLEMLARLSFPSTDPVVARG-
LEFLQQTQRPDGAWFGRWGVNYIYGTWCAVSALTAFADTDATARAMVPRAVAWLLDRQNAD-
GGWGETCGSYEDPNLAGVGRSTPSQTAWAVLALQAAGLGQHPACRRGLDFLRERQVGGTWEER-
EHTGTGFPGDFFINYHLYRHVFPTMALAGAATGMDSPR

>seq_ID 220

FLGIRDEATTRSAALFIRGEQREDGTWATFHGGPPDLSTTVEAYVALRLAGDSPDAPHMTRA-
AHWVRSQGGIAEARVFTRIWLALFGWWPWDRLPELPPELIFLPPWAPLNIYDFGCWARQTIV-
PLTVVSAKRPVRPAPFPLDELHTDPADPAPRARFAPLASWNGAFQRLDRALHAYRKVAPRAL-
RRAAMATAGRWIVERQENDGCWGGIQPPAVYSMIALHLLGYDLGHPVMRAGLESLDRFTLT-
REDGSRMVEACQSPVWDTCLATIALADAGVPADHPQLVRAAD-
WMLDEQIERPGDWSVRRPHLAPGGWAFEFHNDNYPDIDDTAEVVLALRRVRHPDTAR-
MERAISLGVRWNLGMQSKNGAWGAFDVDNTSSLPNRLPFCDFGEVVDPPSADVTAHVVEM-
LAAEGLAADPRTRRAVDWLLAEQEPSGAWFGRWGVNYLYGTGSAVPALVDAGLPTTH-
PAIRRAVAWLESVQNDDGGWGEDLRSYREQGRMARGASTASQTGWALMALLAAGERES-
RAARRGVTFLAETQHEDGSWEEPYYTGTGFPWDFSINYHLYRQVFPLTALGRYTRGAAPEGA

>seq_ID 125

MQTQNRVTSTQKVELSNLTQAIIASQNYILSRQYPEGYWWGELESNITLTAETVLLH-
KIWKTDKTRPFHKVETYLRRQQNEQGGWELFYGDGGELSTSVEAYMALRLL-
GVTPEDPALIRAKDFILSKGGISKTRIFTKFHLALIGCYDWKGIPSIPPWIMLFPDNFPFTIY-
EMSSWARESTVPLLIVFDKKPIFEIEPAFNLDELYAEGVENVKYALPRNHNWSDIFLGLD-
KLFKWTEKNNLVPFHKKSLQAAEKWMLNHQQESGDWGGIMPPMVNSLIA-
FKVLNYDVADPSVQRGFEAIDRFSIEEEDTYRVQACVSPVWDTAWVIRALVDSGLKPDHPS-
LVKAGEWLLDKQILEYGDWAIKNKQGKPGGWAFEFINRFYPDLDDSAVVVMALNGIKLPDEN-
RKKAAINRCLEWMATMQCKPGGWAAFDVDNDQAWINEIPYGDLKAMIDPNTADVTARV-
LEMVGSCGLKMDENRVQKALFYLEKEQESDGSWFGRWGVNYIYGTSGVLSALA-
VIAPNTHKPQMEKAVNWLISCQNEDGGWGETCWSYNDSSLKGTGISTASQTAWAIIGLLDA-
GEALETLATDAIKRGIDYLLATQTPDGTWEEAEFTGTGFPCHFYIRYHLYRHYFPLIALGRY-
WKIGLKTPSVIPLN

>seq ID 228

MLARRATDRAVRHLLSRQDEQGWWKGDLETNVTMDAEDLMLRHFL-
GIQNPDVLDAAGRYIRSQQAADGTWATFHGGPPELSATVEAYVALRLAGDPP-
DAPHMAAASAWVRNNGGVASSRVFTRIWLALFGWWRWEDLPELPPEIIYFPP-
WLPLNLYDFGCWARQTIVPLTVVSAKRPVRPAPFSLDELHADPRRPNPPRPAAPLASWD-
GAFQRLDRALHLYRKVALRPLRRAALRSCARWIVERQENDGCWGGIQPPAVYSVIALHLL-
GYDLDHPVMRAGLESLDRFAVWREDGSRMIEACQSPVWDTCLAVIALADAGLAPDHPAL-
VKSADWMLAEEIDRPGDWSVKRPRLAPGGWAFEFDNDNYPDIDDTAEVILALRRVDHPRPE-
RIAAAVRRGVRWTLGMQSRNGAWGAFDVDNTSPLPNRLPFCDFGEVIDPPSADVTAHVVEMLA-
HEGGARDPRTRRAVGWLLAEQEPSGAWFGRWGTNYVYGTGSVVPALVAAGLPATH-
PAIRRAVRWLESVQNEDGGWGEDQRSYPDPEWIGHGASTASQTAWALLALLAAGERESKA-
VERGVGWLAATQDQDGSWDEPYFTGTGFPWDFSINYHLYRLVFPLTALGRYVSGEATGARPRRT

>seq_ID 241

MTATTDGSTGALPPRADAASEHDIETPEAAGVREAAVRAARRATDFLLSRQDAQGWWKGD-
LETNVTMDAEDLMLRQFLGVLDEKTAQAAALFIRGEQREDGTWASFYGGPGELSTTIE-
AYVALRLAGDAPDSPHLAKASAWIREQGGIAAARVFTRIWLALFGWWKWEDLPELP-
PELIWFPKWVPLNIYDFGCWARQTIVPLTIVSAKRPVRPAPFPLDELHTDPARPNPPRPLA-
PAFSWDGAFQRMDKGLHALRKVAPRGLRRAAMNAAARWIIERQENDGCWGGIQPPAVYSIIA-
LHLLGYDLQHPVMREGLASLDRFAVWREDGARMVEACQSPVWDTCLAAIALVDAGLPAD-
HPQLVKAADWMLGEEIVRPGDWSVRRPGLPPGGWAFEFHNDNYPDIDDTAEVILALR-
RITHHDPVRVDKAVGRGVRWTLGMQSKNGAWAAFDVDNTSPFPNRLPFCDFGEVIDPPSAD-
VTAHVIEMLAVEGLAHDPRTRRGIEWLLAEQEPDGSWFGRWGVNYVYGTGSVVPAL-
VAAGLPGAHPAIRRAVSWLESVQNDDGGWGEDLRSYKYVKEWSGRGASTASQTAWALMALLAA-
GERDSKAVERGVEWLAATQREDGSWDEPYFTGTGFPWDFSINYHLYRQVFPLTALGRYVHGE-
PFADRLKGS

>seq ID 238

MHEGEAMTATTDGSTGAATPPATTASAPLHLSPEARETHEATARATRRAVDFLLARQSDEG-
WWKGDLATNVTMDAEDLLLRQFLGIRDEATTRAAALFIRGEQQEDGTWNTFYGGPGDLSA-
TIEGYVALRLAGDSPEAPHMRKASAFVRAQGGVARARVFTRIWLALFGWWKWEDLPEMP-
PELMFFPKWAPLNIYDFGCWARQTIVPLTVVCAQRPVRPAPFALEELHTDPADPDPAQPAPP-
VVSWDNVFHKLDKLLHGYRRIAPRRVREAAMRAAATWIVERQENDGCWGGIQPPAVYSI-
MALNLLGYDLDHPVLRAGLASLDRFAVWREDGARMIEACQSPVWDTCLATVALADAGVPAD-
HPQMIKAADWMLAEQIVRPGDWVVRRPDLPPGGWAFEFHNDNYPDIDDTAEVVLALRR-
VAHPDATRVDKAVRRAVDWNVGMQSKNGAWGAFDADNTSPFPNRLPFSDFGEVIDPPSAD-
VTAHVVEMLAEEGLAHHPRTRRGIEWLLKNQEGNGSWFGRWGVNYVYGTGAVVPALVAAGL-
PASHPAIRRSVSWLGQVQNEDGGWGEDLRSYQDSAWHGRGHSTASQTAWALLALLAAGE-
RETEQVRRGIAYLVETQTEDGTWDEPWFTGTGFPWDFTINYHLYRQVFPVTALGRYLNGTGPGEN

>seq_ID 237

MRRRRSPRGPGAGPEADYGPARASAPDRLRGDAARGDAARRVQDATARAIRNLLGRQDPAG-
WWKGDLETNVTMDAEDLLLRQFLGIRDEAVTQAAALFIRREQREDGTWATFHGGPPELSATIE-
AYVALRLAGDAPDAPHMATASAWIRAHGGLAAARVFTRIWLALFGWWDWENLPELPPELVLL-
PPWVPLNIYDFGCWARQTIVPLTVVSAMRPVRPAPFALDELHTDARVPVPPRRMAPPTT-
WNGAFQWMDRALHVYRRFAPRRLREAAMASAGRWIIERQENDGCWGGIQPPAVYSVIALHLL-
GYDLGHPVMRAGLESLDRFAVWREDGSRMIEACQSPVWDTCLAAIALADAGVRPDHPAL-
VKAADWMLGEEIVRTGDWAVRRPGLAPGGWAFEFHNDTYPDIDDTAEVVLALRRIRHPDPAR-
VEAAIARGVSWNLGMQSRGGAWGAFDADNTSPFPNRLPFCDFGEVIDPPSADVTAHVVEM-
LAAEGRAADPRTRRGIAWLLAEQEPEGPWFGRWGTNYVYGTGSVVPALTAAGLSPGH-
PAIRRAVLWLESVQNPDGGWGEDQRSYQDRAWAGKGESTPSQTAWALMALLSAGERDAKTVER-
GIAYLVETQLADGGWDEPHFTGTGFPWDFSINYHLYRHVFPLTALGRYLYGEPFGHDGRHI-
GAHLGDRTGVPAEGV

>seq_ID 239

MDFLLDRQSDEGWWKGDLATNVTMDAEDLLLRQFLGIRDEATTQAAALFIRGE-
QQEDGTWNTFYGGPGDLSATIEGYVALRLAGDSPEAPHMRKASAFVRARG-
GVARARVFTRIWLALFGWWKWEDLPEMPPELMFFPKWAPLNIYDFGCWARQTIVPLT-
VVCAQRPVRPAPFALEELHTDPADPNPAQPAPPVASWDNVFHKLDKMLHGYRKVAPRRV-
REAAMRAAATWIVERQENDGCWGGIQPPAVYSIIALHLLGYDLDHPVLRAGLESLDRFAVW-
REDGARMIEACQSPVWDTCLATVALADAGVPADHPQMIRAADWMLAEQIV-
RPGDWVVRRPDLPPGGWAFEFHNDNYPDIDDTAEVVLALRRVAHPDATRVD-
KAVRRAVDWNAGMQSKNGAWGAFDADNTSPFPNRLPFSDFGEVIDPPSADVTAHVVEM-
LAEEGLAHHPRTRRGIEWLLENQEANGSWFGRWGVNYVYGTGAVVPALVAAGIPAAH-
PAIRRSVSWLGQVQNEDGGWGEDLRSYQDTAWHGRGHSTASQTAWALLALLAAGERDSEQVRR-
GIAYLVETQTEDGTWDEPWFTGTGFPWDFTINYHLYRQVFPVTALGR

>seq_ID 235

MTQTVPRTAASAPAARTAADTVAAAVQFLRREQDRAGWWKGELATNVTMDAEDLLLRHFL-
GILTPQIAEESARWIRSQQRADGTWANFPDGPADLSTTVEAWVALRLAGDPADAPWLATAAE-
WIREHGGIEATRVFTRIWLAMVGQWSWDDLPSLPPELIFLPSWFPLNVYDFACWARQTIV-
PLTIVGTLRPARKLPFDVAELRTGKRPPKPRAPWTWDGVFQNLDTAL-
HAYAKLPLNPVRKLALKQAAEWILARQEADGSWGGIQPPWVYSILALHLLGYSLDHPALKAGI-
AGLDGFTIREKTDQGWVRRLEACQSPVWDTALAMTALLDAGVSPGDESLVRAAEWMLGE-
EIRVPGDWAVRRPSLKPGGFAFEFANDGYPDTDDTAEVVLALRRMGKPDHLRIREAVDRSVAW-
LEGMQSSDGGWGAFDADNTQVLTTRLPFCDFGAVIDPPSADVTAHVVEMLAAEGKADTRECRR-
GIRWLWDNQEADGSWFGRWGANYVYGTGAVVPALVAAGVPGTDPRIRRAVRWLAEHQNDDGGW-
GEDLRSYDDRSWAGRGDSTPSQTAWALLALLAAGERESTVVARGVEWLCERQRPDGGWDE-
DKHTGTGFPGDFYLSYHLYRVVFPLSALGRYVRGGS

>seq_ID 159

MSGQSNFTGGKKMTPAEGSSSPAPALLEKAAPSIELDERSDPLSRTLARAVSWLVAAQD-
GAGHWVAPLEADATIPSEYVFLHEVLGRPLDPVRRDKIVRAILS-
VQGKEGAWPLFHDGDPDISATVKAYQALKLCGFDPSHPALVRAREWVLSQGGAGKVNVFTRIAL-
AIFGQYSWTKIPALPAEMVLLPSWFPFSIYSVSYWSRTVIVPLLFIYHHKPLVRLSPERGI-
SELFDPARPDGESFAPSPDFFSLRNLFLLLDKVLQVWNRHPPGFLRKKALSFAMEWMVPRLK-
GEGGLGAIYPAMANSAVALSLEGYELDHPLMQRVLASIDDLLIEGEKEVLVQPCV-
SPVWDTALAMGALIEAGISPDSPTVDRAMEWFCAREVRTRGDWAIRAPDCEPGGWAFQFEN-
DYYPDVDDTAMVLMGMAKILPARPDLAARMEGVFRRATLWVMAMQGTDGGWGAFDRDNDLL-
FLNHIPFADHGALLDPSTADLTGRVLELLGALGYGPDFPPAARAIRYLRREQEEDGSWF-
GRWGVNYIYGTWSVVAGLKSIGVPMSEPWVMRSMEFLLARQNPDGGWGEDCLSYASRDFAGR-
GASTPSQTAWALIALLHGGHAGHMAVRQGVDYLIQQMTPEGTWNEELFTGTGF-
PRVFYLRYHMYRHYFPLWALALYRNMTERGRALGHERVDFWKTAPYAPIARSV

>seq ID 232

MTATTDGSTGALPPRAPSASDTDHGTPVAAGVQEAALHAVGRATDFLLSRQDAQGWWKGD-
LETNVTMDAEDLLLRQFLGIRDDATTRAAALFIRGEQRPDGTWATFYGGPPDLSAT-
VEAYVALRLAGDDPAAPHMAKASAWIRARGGIAAARVFTRIWLALFGWWKWDDLPEMPPEI-
VYFPTWMPLNIYDFGCWARQTIVPLTVVSAKRPVRPAPFPLDELHTDPGRPNPPRPLDRLGS-
WEGAFQRLDRALHGYHKVALKRLRRAAMNRAARWIVERQENDGCWGGIQPPAVYSVIALHLL-
GYDLGHPVMRAGLESLDRFAVWREDGARMIEACQSPVWDTCLATIALADAGLPP-
DHPQLVKAADWMLGEEIVRPGDWSVKRPQLPPGGWAFEFHNDNYPDIDDTAEVVLAL-
RRVRHPDPERVERAVRRGVRWTLGMQSGNGAWAAFDADNTSPFPNRLPFCDFGEVIDPPSAD-
VTAHVVEMLAAEGLSHDPRTRRGIEWLLAEQEPGGAWFGRWGVNYVYGTGSVVPALVTAGL-
PAAHPAIRRAVAWLETVQNDDGGWGEDLRSYPDPAEWGGKGASTASQTAWALLALLAAGERDG-
KATERGVAWLARTQREDGSWDEPYFTGTGFPWDFSINYHLYRQVFPLTALGRYVHGEPAVLKPGTR

>seq_ID 224

MTATTDGSTGAANLRAAAASDPTESTSAAPDMMAVARHAAERSVEHLLGRQDEQGWWKGD-
LATNVTMDAEDLLLRQFLGIQDPETVKAAARFIRGEQLGDGTWNTFYEGPPDLSAT-
VEAYVALRLAGDRPDDPHMIRAAGWVREQGGIAESRVFTRIWLALFGWWKWDDLPELPPELM-
FFPKWVPLNIYDFGCWARQTIVPLTIVSAKRPVRPAPFALDELHTDPACPNPSRPTAPAAS-
WDGVFQRLDKALHLYHKVAPRRLRRIAMNEAARWIIERQENDGCWGGIQPPAVYSVIALHLL-
GYDLDHPVMRAGLESLDRFAVWREDGARMIEACQSPVWDTCLATIALADAGVSPDHPALV-
RAADWMLGEEIVRPGDWAVRKPGLAPGGWAFEFHNVNYPDIDDTAEVALAL-
RRVRHPDPARVDAAIERGVRWNLGMQSRNGAWGAFDADNTSPFPNRLPFCDFGEVIDPPSAD-
VTGHVVEMLAVEGRAHDPRTRRGVEWLLAEQEASGAWFGRWGVNYIYGTGSVVPALIAAGL-
PAAHPSVRRAVDWLRSVQNDDGGWGEDLRSYREEKWIGHGSSTASQTGWALLALLAAGERET-
RSVERGVAWLAATQQADGSWDEPHFTGTGFPWDFSINYHLYRQVFPLTALGRYVYGDPFATA-
TAIGAGTGKGA

>seq_ID 243

MSISALQTDRLSQTLTQSVVAAQQHLLSIQNPEGYWWANLESNASITAEVVLLH-
KIWGTLDSQPLAKLENYLRAQQKTHGGWELYWNDGGELSTSVEAYMGLRLLGVPASDPAL-
VKAKQFILHRGGVSKTRIFTKFHLALIGCYRWQGLPSLPAWVMQLESPFPFSIYELSS-
WARGSTVPLLIVFDKKPVYPLQPSPTLDELFTESAENVRWELEEKGDWSDAFLWLDKAF-
KLAESVDLVPFREESIRKAEKWVLERQEPSGDWGGIIPAMLNSMLALRALGYSVSDPVVRRG-
FQAIDNFMVESETECWAQPCISPVWDTGLAVRSLTDSGLSPNHPALVKAGEWLLDKQIL-
SYGDWSVKNPQGQPGGWAFEFENSFYPDVDDTAVVAMALQDITLPNEPLKRRAIARA-
VRWIATMQCKTGGWAAFDINNDQDWLNDIPYGDLRAMIDPSTADITGRVLEMHGRFAADLD-
LANSYAADLSPYRLSRGLNYLIKEQELDGSWFGRWGVNYIYGTGQALSALALIAPER-
CRIQIERGIAWFVSVQNADGGWGETCESYKDKSLKGKGISTASQTAWALLGLLDVSFCLDPAA-
KIAVDRGIQYLVSTQSEGTWQEESFTGTGFPQHFYLRYRLYCHYFPLMALGRYQRVINSSAGI

>seq_ID 197

MTSGTFGAKRVDLLAAFEHSAPAEKTRETCVGLQTAIARTRQYLLDQQHSEGFFVAELEGDTI-
LESEYILLLAFLNEGQSPDAQAAARYLLTKQNTDGSWSNFPGGPIDVSCAVKAYLALRIT-
GHAADEPALIRAREAILQAGGVERVNSFTRFYLAMLGLIPYSLCPAVPPEVVLLPDWFPIN-
LSQMSAWSRTIVVPLSLLWAFQPAVELNDADGHQITIEELYASPEKQLPRFIRGVN-
HESNSNGWMNWSRFFFRVDQCLKSIESYGIKPLRSRAVRKCVQWILDRQEMSDGLGAIFP-
PIVWTLIGLKCAGFDDQHPMVQKQRDELNRLMLREQDALRLQPCLSPVWDTAISIIALRESG-
VEPDHPALSKARNWLLSKEVRHAGDWSKAHPETPVSGWYFEFNNEFYPDVDDTAMVLIAL-
ASTLPEEATPLAISHGVLPVQTGWSAESTSRVQALKQLENHRPVLEAMGRGVQWL-
KALQSKDGGWGAFDSDINKELLTKVPFADHNAMLDETNADISARVLEAYAAVGISFND-

PSVQRALEFIWNDQEDDHAWYGRWGVNYIYGTWQVLVGLTAIGISAHDPRLVRAAGWLKS-
KQQACGGWGETPATYDNPTLRGQGTPTASQTAWAVLGLIAAGEQNSIECQRGVEFLL-
KTQKHNGTWDEEEFTGTGFPRVFYLRYHYYPLYFPLMALGRFARAGGRVNFAG

>seq_ID 158

MTTNAAATSARSGEDAIRQVSGQQLETAIASARNSLLALQRPDGHFVFELEADATI-
PAEYVLMRHYLAEPVDAVLEEKIARYLRRIQSDDGGWPLFRDGASNISASVKAYYALK-
MIGDAPNAPHMQKARAWILAQGGASHSNVFTRNLLALFGAIPWSGVPVMPVEIMLLPKWFPF-
HIDKISYWARTVLIPLTVLNALKPVARNPKGVGIAELFVTPPDQVRNWPKGPHQKFPWSQVFG-
GIDRVLRLFEPAFPKSLRKKSIDKAVAFATERLNGEDGLGGIFPAMVNALLVYDALGY-
PHDHPDYVTARGSIEKLLVIKDDEAYCQPCLSPVWDTALAVHALMES-
GVAQADQNVDRALAWLKPLQVLDTVGDWAASRPGVRPGGWAFQYANAYYPDVDDTAVVVMAM-
DRAAGGDAAKRDHYRESMARGREWVAGVQSKNGGWGAFDADNTYEYLNQIPFSDHGALLDPP-
TADVSARCVSMLAQLGERRETSPVLDKAMRYLESTQEKDGSWYGRWGMNYIYGTWSVLCAL-
NAAGVAPSAPSMRKAADWLLSIQNSDGGWGEDGESYSLDYKGYEPAPSTASQTAWALMGLMAA-
GEVDHPAVQRGVAYLAAKQGSDGFWGEERFTATGFPRVFYLRYHGYSKFFPLWALARYRNL-
NAANSKSVLVGM

>seq ID 77

MAADGSALSESRLSSEALDRAVLSAHTALSQAQQDDGHWVYELEADATIPAEYILLEHFMD-
RIDDALEQKIAIYLRRIQSEEHGGWPLYHNGKFDLSATVKAYFALKAVGDDI-
NAPHMQRAREAILDHGGAERSNVFTRSQLALFGEVPWRATPVMPVELMLLPAKAFFS-
VWNMSYWSRTVIAPLLVLAALRPVAANPRQVHVRELFVTPPEKVQDWIRGPYRSAWGYVFK-
GLDSVLRPVVPFIPEKTHKKAIQAALDFIEPRLNGKDGLGAIYPAMANVVMMYRAMGVPDEDP-
RAKTAWEAVQALIVEKDDEAYCQPCVSPIWDTGLSGHAMIEAASGPNGIAPEKTVAELK-
KASAWLRSKQILNVKGDWAVRNPNLAPGGWAFQYGNDYYPDVDDTAVVGMLLHREGDPT-
NAEAIERARTWIVGMQSTDGGWGAFDIDNNKDVLNHIPFADHGALLDPPTADVTARCIS-
FLAQLRNPEDEPVIQRGLEYLRKEQEKDGSWFGRWGTNYIYGTWSALCALNAAGVSHDDPA-
VVKAVEWLRSVQRADGGWGEGCESYEGGPHGTYGESLPSQTAWAVLGLMAAGRRDDPA-
VTRGIAWLADQQDANGEWHEDPYNAVGFPKVFYLRYHGYKQFFPLMALARYRNLESSNTRRVSFGF

>seq ID 6

MTVSTSSAFHHSSLSDDVEPIIQKATRALLEKQHQDGHWVFELEADATIPAEYILLKHYLGE-
PEDLEIEAKIGRYLRRIQGEHGGWSLFYGGDLDLSATVKAYFALKMIGDSPDAPHML-
RARNEILARGGAMRANVFTRIQLALFGAMSWEHVPQMPVELMLMPEWFPVHINKMAYWART-
VLVPLLVLQALKPVARNRRGILVDELFVPDVLPTLQESGDPIWRRFFSALDKVLHK-
VEPYWPKNMRAKAIHSCVHFVTERLNGEDGLGAIYPAIANSVMMYDALGYPENHPERAIAR-
RAVEKLMVLDGTEDQGDKEVYCQPCLSPIWDTALVAHAMLEVGGDEAEKSAISALS-
WLKPQQILDVKGDWAWRRPDLRPGGWAFQYRNDYYPDVDDTAVVTMAMDRAAKLSDLHDD-
FEESKARAMEWTIGMQSDNGGWGAFDANNSYTYLNNIPFADHGALLDPPTVDV-
SARCVSMMAQAGISITDPKMKAAVDYLLKEQEEDGSWFGRWGVNYIYGTWSALCALNVA-
ALPHDHLAIQKAVAWLKNIQNEDGGWGENCDSYALDYSGYEPMDSTASQTAWALL-
GLMAVGEANSEAVTKGINWLAQNQDEEGLWKEDYYSGGGFPRVFYLRYHGYSKYFPLWALA-
RYRNLKKANQPIVHYGM

>seq_ID 89

MNDLTNSSAPGARPDDATPSAAGPTPAEAAGGAVAPSRAVQPADTQTAATGAAGAAAAVGAT-
PAELAATAPASSGTPAGASAAPAPSGTPSVDAPAELASAAPAPSGATPAATATAATAPAPA-
RAASIDAPALAAADLDAAITRATDALLAAQQADGHWIYELEADSTIPAEYVLLVHYLGETPN-
LELERKIARYLRRVQLPGGGWPLFTDGAPDVSASVKAYFALKMIGDDA-
NAEHMVRARNAIHAMGGAEMSNVFTRIQLALFGVVPWFAVPMMPVEIMLLPQWFPFHLS-
KVSYWARTVTVPLLVLSAKRPLARNPRGVRVDELFVAPPVNAGLLPRAGHQSPAWFACFRLL-
DGLLRLTDGLFPRYTRERAIRQALQFVDERLNGEDGLGAIYPAMANSVMMYAALGYPEDHPN-
RATARRAIEKLLVIHDDEAYCQPCLSPVWDTSLAAHALLETGEPRAEAAAIRGLD-
WLRPLQILDVRGDWISRRPDVRPGGWAFQYANPHYPDVDDTAVVTLAMDRVAKLAQT-
DAYRDAIARAREWVVGMQSSDGGWGAFEPENTHQYLNSIPFSDHGALLDPPTADVSGRCLSM-
LAQLGETAANSAPARRALDYLLAEQGADGSWYGRWGMNYIYGTWSALGALNAAGLPFDD-
PRVKRAAQWLLSIQNPDGGWGEDGDSYKLDYRGYERAASTASQTAWALLGLMAAGEVEHPA-
VARGIAWLAAQQREHGLWDEARFTATGFPRVFYLRYHGYRKFFPLWALARYRNLRRTGTRRVTVGM

>seq_ID 201

MLPYNQNSYKEALHGGHAAHNPPTLEEAIKRSQEFLLAHQHPEGFWWGDLECNVTSASHTLI-
LYKILGIADRYPLHKFEKYLRRMQCSHGGWEMSFGDGGYLSATIEAYIC-
LRLLNVPQSDPALQRALKNILARGGVTKARVFTKVCLALLGGFDWAALPSLPPWLMLF-
PAWFPWNIYEAASWARGCVVPLIVLLEKKPVFQVKPEVSFDELYVEGRAHACKAL-

PFSAHDWVSNIFVAADRAFKLMERFGAVPFRQWSIKEAKKWVLDRQEEMGDFIGYNPP-
MLYFAVCLKLWGYEVTDPLLQRALLAHKKLTVETEDECWLQSSQSPVWDTALVIPALVES-
GLPPDHPALQKAGQWLLEKQILKHGDWALKTGGGRMQDDIGGGWAFQFVNS-
WYPDVDDSAAVVIALNCIKMPDEDVKNGAIARCLKWIAFMQGRNGGWAAFDRDSNQRWMDAT-
PFSDIEAMLDVSTADVTARVLEMVGLMRLKHAAQPANNSLGKAHRHISTESIARGVDYLT-
KEQEKEGCWWGRWGVNYIYGTRGALMGLSQVAAKTHKKEIARGAAWLVKVQNKKNEK-
KQGAQDGGWGEACFSYDDPATKGQNSRSTASQTGWAMQGLLAAGEVLGRKYEMEAVE-
EGVQFLLDTQRKDGSWSEAEFTGGGFPKHYYLKYHYFAQHFPLSALARYRARLLQLSRPKNQA

>seq_ID 183

MDGSQRISDMSQQPEGIAVSDEISSAYSVSSLNQDEINVDELENKLTQARSAM-
LSLQKPDGHWCFPLEADCTIPAEYILMMHFMDEIDVILENKIARFIREKQDLTHGGWPLYYG-
GAFDISCTIKSYYALKLVGDSPDAAHMVRAREAILERGGAAKANVFTRLL-
LAMYEQIPWSGVPVVPTELMLLPSWFPFHISKVSYWSRTVMIPLSILCTIKARAINPRNVDI-
RELFIVPPEQEKNYFPQADTWLKRAFMLVERVLSRVEPKLPQAIRQYSIRKAENWTLER-
LNGECGIGAIFPAMVNAHESLALLGYAYDHPSRVQCRNALRGLLVDEGER-
AWCQPCTSPVWDTVLTCLALQEDPAADQGPVLKALDWLVDQQVLDEPGDWRDKRPDLLGGG-
WAFQYANPHYPDLDDTAAVAWALDQSDAQRYQKPLDRAANWLAGMQSRNGGFAAFDIDN-
TYHYLNEIPFADHGALIDPPTSDVTARCVGLLGKYGKHQREVWRGISFLLREQEKNGSWF-
GRWGTNYIYGTWSVLEAFQLANFDMQHTSVRRAVKWLESVQRVDGGWGETND-
SYLDIQLAGQFPQTSTTFQTAWAVLGLMAAGEVNSKSVRRGINYLLHNQADDHL-
WEDPWFTAPGFPRVFYLRYHGYSKFFPIWALVRYRALTKERVS

>seq_ID 102

MNDLSQTQPLDAVLPEAADAASNLAEAAVVANAPAVADALATATPSPMQTAGASPLD-
VSITRATDAILAAQQPDGHWIYELEADATIPAEYVLLVHYLGETPNLELEQKIA-
RYLRRIQLPNGGWPLFTDGALDISASVKAYFALKMIGDPVDAEHMVRARDAILAHGGAEHAN-
VFTRILLALFGVVSWRAVPMMPVEIMLLPMWFPFHLSKVSYWARTVIVPLLVLNAKRPLARN-
PRKVRIDELFRGAPVNTGMNERAPHQHAGWFGFFRCVDTVLRAVDGLLPKASRER-
AIRAAVAFVDERLNGEDGLGAIFPAMANSVMMYDVLGYPADHPNRAIARKSLDKLL-
VIKEDEAYCQPCLSPVWDTSLVAHALLETREARAEQAAERGLAWLRPLQILDVRGDWIS-
RRPNVRPGGWAFQYNNAHYPDVDDTAVVAMAMHRSAALTKSDVDREAI-
ARAREWVVGMQSSEGGWGAFEPENTQYYLNNIPFSDHAALLDPPTADVSGRCLSMFAQIGEL-
PQNSEPAQRAFDYMLQEQESDGSWYGRWGLNYIYGTWTALCSLNAAGMSHDDPRM-
RRAVQWLVSIQNEDGGWGEGGESYKLDYRGYERAPSTASQTAWALLGLMAAGEVDHDA-
VARGIDYLQREQREHGLWDETRFTATGFPRVFYLRYHGYRKFFPLWALARFRHLKRNGLTRVTVGM

>seq_ID 90

MIRPMKNSDLPLPSLLDAAILRGRDALAQRQSADGSWCFELESDATITAEYILMMHFMGKIDE-
ARQARMARYLRGIQRLATHGAWDLYVDGAPDVSCSVKAYFALKAAGDSEDAPHMARARETIL-
KLGGAAKSNVFTRILLATFGQVPWRATPFMPVEFVLFPKWVPISMYKVAYWARTTMVPLL-
VLCSLKARAKNPRNVSIRELFVTAPEAERHYFARGGFVRNLFLGIDRALRPLDALIPKAL-
RRRAIRHAEAWCAERMNGEDGMGGIFPPIVYSYQMMDVLGYPEDHPLRRDCENALDKLLVER-
PDGSVYCQPCLSPVWDTAWSTMALEQARAVPDPRDAPPVSDAQLQRCIAASYEWLAG-
KQVTQVRGDWVENAPAATPAGGWAFQYENPYYPDIDDSAVVAAMLHRRGRLLARST-
GTDPYAQVVARGLDWMRGLQSRNGGFGAFDADCDRLYLNLIPFADHGALLDPPTEDVSGRV-
LLCLGVTGRDEDKPALARAIEYVKRMQRADGCWWGRWGTNYIYGTWSVLAGLALAGEN-
PSQPYIARAIAWLRACQNADGGWGETNDSYLDPALAGTNGGESASNVTAWALLAQMAF-
GDWQSESVQRGIRYLLSVQQADGFWWHRSHNAPGFPRIYYLKYHGYTAYFPLWALARYR-
RLSQAGAARDVTDGAALAAS

>seq_ID 167

MREAAVSKVETLQRPKTRDVSLDDVERGVQSATRALTEMTQADGHICFELEADATIP-
SEYILFHQFRGTEPRPGLEAKIGNYLRRTQSKVHGGWALVHDGPFDMSASVKAYFALKMIGD-
DIEAPHMRAVRKAILQRGGAANANVFTRILLALYGEVPWVAVPVMPVEVMHLPKWFPFHLD-
KVSYWARCTMVPLFVIQAKKPRAKNPRGVGVAELFVTPPDSVRTWPGSPHATWPWTPIFG-
GIDRVLQKTQDHFPKVPRQRAIDKAVAWVSERLNGEDGLGAIFPAMVNSVLMYEV-
LGYPPEHPQVKIALEAIEKLVAEKEDEAYVQPCLSPVWDTALNSHAM-
LEAGGHQAEANARAGLDWLKPLQILDIKGDWAETKPNVRPGGWAFQYANPHYPDLD-
DTAVVVMAMDRAQRQHGLVSGMPDYSESIARAREWVEGLQSADGGWAAFDADNNHHYLN-
HIPFSDHGALLDPPTADVTARVVSMLSQLGETRATSRALDRGVTYLLNDQEKDGSWYGRWGMN-
FIYGTWSVLCALNAAGVDPQSPEIRKAVAWLIRIQNPDGGWGEDASSYKLNPEFE-
PGYSTASQTAWALLALMAAGEVDDPAVARGVNYLVRTQGQDGLWSEERYTATGF-
PRVFYLRYHGYPKFFPLWAMARFRNLKRGNSRQVQFGM

>seq_ID 133

MTTTDETALAAGTPKAAFAPAPRGAADDLVARTVAVEAPPSPAPASDDTLARAVAHL-
KSLQDEAGWWKGDLETNTTMDSEDLMLRHWLGIWNPEQAERTARFIRSKQYADGS-
WPIYHAGPGDLNATVESYVALRMVGDSPQDPHMRAAAAWARARG-
GVPATRIFTRIWLALFGWWRWEDLPVLPPELIFVPAKMPLSIYKFASWGRQTIVAIMVLMAHR-
PAGTPPFPIAELFPPPATKKKAAAQRKAQKKAGHAGGPTAWRDSSIDDMFTE-
PAPGTDTLRQPAALAIGPARPAPAKGRRGKGQPAAPDVMGRAKDGGGPGLPL-
PARLVSRVGFRTRRALRQAALDHVNWNLLFGGIDRFLHVYHRHPIRPVRSLALGLAERWIV-
VRQEADGCFGGIQPPTVYSIMALRVLGYPMDHPVMTAALRSLDEYSVTLPD-
GARMQEACQSPVWDTCLATIALADAGVPRDDPSLVRAADWMLAEEVRERRGDWSVPIPDVPT-
GGWSFEFDNDTYPDVDDSAEVMLALMRVAHPRPEKVVAATYRGLQWVFGMQCADGGWGAFDVD-
NAGELVYKIPFADFGMLTDPPSADVTAHVVELLGELGLGDDPRTKRGVEWLLHSQEADGS-
WYGRWGVNHLYGTGGVVPALRAAGLPASHPAIQRAADWLVAKQNPDGGWGESCYSY-
DEMSTAGVGVSTASQTAWALLALIAAGRVGDGVTGEAAARGVAWLAETQTAEGTWDEDYFT-
GTGFAGYFYINYHLYRLVWPVMALGRYQAALAGKGH

>seq ID 7

MNPVVHNLTRPHRSAEPRPSALQRSIAAAQAALLQHQAADGHWCFEFEADCTIPAEYILMM-
HYMDERDAALEAKMAAYLRRKQENHGGWSLYHGGHFDMSASVKAYFALKLAGDDPEAAHMR-
RARSAILAHGGAERANVFTRITLALFGQVPWRAVPFIPVEILLFPRWFPMHIYKVASWSRT-
VMVPLFILCSLKPQAKNPLGVHIRELFTRPPEDIDDYFAHALQGWVSRIFLWFDRLGRALES-
WIPQALRRRAIARAEAWFIERLNGEDGLNGIFPAMVNAHEALALLGYAAEHPYRQQTRAALT-
KLVVERAGEAYCQPCVSPVWDTCLALHALLEADGDVSEAARRSMQWLLDRQITDAPGDW-
RERRPHLAGGGWAFQYANPYYPDLDDTAAVAWALARARRPEDRPAVERAANWLAGMQSRNGGF-
GAYDVDNTYYYLNEIPFADHKALLDPPTADVSGRVLAFLAILDREQDAPVRARLIQYLLRE-
QEPSGAWFGRWGTNYIYGTWSVLMGMAELRDPGAEVRDAMARAAHWLRSVQQDDGGWGESND-
SYADPGLAGLGQESTAAQTAWACLALMAAGDSDSESLRRGIQWLQRHQEQPGDWQDPYF-
NAPGFPRVFYLTYHGYKIYFPLWALARYRNITERHCA

>seq ID 190

MALSNGEIREEIQRLSEELIQRQEPDGSWRFCFENGITIDACTIILLRTLNVDKEELIRQLH-
DRIVAAQQPEGCWRWYHDDKEGHLSATVEAYYALLCSGYSRPEDEPIQRAKRYILDRG-
GIGQARSLFTKAILAATGQRKWPASLSLIPIEILLLPESLPLNFYDFSGYSRVHLVPLLIMA-
ERNFRTRSVRTPDLSELFLDARNGEEDPLTLTPESREPLKLIQSGLAHLVGTPRRIRQAAVN-
RAEQYMLDRIEGDGTLYTYASCTVLMVFALLALGYEPQHPVIQRAVEGLSQMKFTVDST-
GQGGTRYVTIQNSPSTVWDTALISYALQEAGVSSSHPAIQRAADYLRNRQHR-
RPGDWQIHNPGIVPGGWGFSETNTFVPDVDDTTAALRALSALHGSEPA-
VLGAWNRGLNWVWSMQNNDGGWPAFEKNTNKEMLTWLAIEGAKSAATDPSEADLTGRTLEY-
LGNFAKLSVRQDWVARGADWLLSHQEADGSWYGRWGICYIYGTWAALTGLMAVGMPADHPGIA-
KAANWLIRIQNADGGWGESCRSDQVRRYVPLHASTPSQTAWALDALIAVHDRRAPEIERGVAR-
LIALLHEDDWPSTYPTGAGLPGYFYVHYHSYRYIWPLLALSHYVNKYGDSSP

>seq_ID 45

MSGVLLYDKVREEIERRTTALQTMQRQDGTWSFCFEGALLTDCHMIFLLKLLGRNDEIEPF-
VKRLASLQTNEGTWKLYEDENGGNLSATIQAYAALLASEKYSKEDINMRRAEMFIKEHGGVS-
RAHFMTKFLLAIHGEYEFPTLFHFPTPILFLQDDSPLSIFELSSSA-
RIHLIPMMICMNKRFRVEKKLLPNLNHIAGEGGQWFRE-
ERSPLFQSFVGDVKKVIAYPLSLHHKGYEEVERFIGERIDENGTLYSYASATFY-
MIYALLALGHSIQSPIIEKAVIGLKSYIWKMDRGSHLQNSPSTVWDTALLSYSLQEANVM-
KENKMIQKATEYLLQRQQTKRMDWSVHAPSIMAGGWGFSDVNTTIPDVDDTTAALRALARS-
RGSSRVDSAWERGVEWLKGLQNNDGGWGAFERGVTSRILANLPIENASDMITDPSTPDITGRV-
LEFFGTYAPNELPEEQKKKAVKWLMDVQELNGSWYGKWGICYIYGTWAAMTGLRAL-
GVPSSHPSLKKAASWLEHLQYEDGGWGESCQSSVEKKFISLPFSTPSQTAWALDALISY-
YDQETPIIRKGISYLLAQPTMNEKYPTGTGLPGGFYIRYHSYGHIYPLLALAHYIKKYKK

>seq_ID 53

MSGVLLYDKVHEEIERRTTALQTMQRQDGTWQFCFEGALLTDCHMIFLLKLLGRNDEIEPF-
VKRLVSLQTNEGTWKLYEDEKGGNLSATIQAYAALLASERYSKEAMNMRRAEMFIKEHGGVS-
RAHFMTKFLLAIHGEYEFPALFHFPTPILFLQDDSPLSIFGLSSSA-
RIHLIPMMICMNKRFRVEKKLLPNLNHIAGGGGQWFREERSPLFQSFLGDVKKVISY-
PLSLHHKGYEEVERFMKERIDENGTLYSYASATFYMIYALLALGHSIQSPIIEKAVTGLK-
SYIWKMDRGSHLQNSPSTVWDTALLSYSLQEAKVTNENKMIQRATEY-
LLQKQQTKKVDWSVHASSLVAGGWGFSDVNTTIPDIDDTTAALRALARSRGNDRVDDAWGRG-
VEWVKGLQNNDGGWGAFERGVTSKLLSNLPIENASDMITDPSTPDITGRVLELFGTYAPNEL-
LEEQKKKAIKWLMDVQEQNGSWYGKWGICYIYGTWATMTGLRALGVPSTHPALKKAASW-

LEHLQHEDGGWGESCQSSVEKKFISLPFSTPSQTAWALDALISYYDQETPIIRKGISY-
LLAQSTMNEKYPTGTGLPGGFYIRYHSYGHIYPLLALAHYVKKYRK

>seq ID 44

MSGVLLYDKVHEEIERRTTALQTMQRQDGTWQFCFEGALLTDCHMIFLLKLLGRNDEIEPF-
VKRLASLQTNEGTWKLYEDEKGGNLSATIQAYAALLASEKYSKEDMNMRRAEMFIKEHGGVS-
RAHFMTKFLLAIHGEYEFPALFHFPTPILFLQDDSPLSIFGLSSSA-
RIHLIPMMICMNKRFRVEKKLLPNLNHIAGGGGQWFREERSPLFQSLLGDVKKVISY-
PLSLHHKGYEEVERFMKERIDENGTLYSYASATFYMIYALLALGHSIQSPIIEKAVTGLK-
SYIWKMDRGSHLQNSPSTVWDTALLSYSLQEAKVTNENKMIQRATEY-
LLQKQQTKKVDWSVHASSLVAGGWGFSDVNTTIPDIDDTTAALRALARSRGNDRVDDAWGRG-
VEWVKGLQNNDGGWGAFERGVTSKLLSNLPIENASDMITDPSTPDITGRVLELFGTYAPNEL-
LEEQKKKAIKWLMDVQEQNGSWYGKWGICYIYGTWATMTGLRALGVPSTHPSLKKAASW-
LEHLQHEDGGWGESCQSSVEKKFISLPFSTPSQTAWALDALISYYDQETPIIRKGI-
TYLLAQSTMNEKYPTGTGLPGGFYIRYHSYGHIYPLLALAHYVKKYRK

>seq_ID 64

MSNLLLYEKVHEEIARRTTALQTMQRQDGTWRFCFEGAPLTDCHMIFLLKLLGRDKEIEPF-
VKRLASLQTNEGTWKLYEDEAGGNLSATIQSYAALLASEKYTKEDANMKRAEMFINERGGVA-
RAHFMTKFLLAIHGEYEYPSLFHLPTPIMFLQNDSPLSIFELSSSA-
RIHLIPMMLCLNKRFRVGKKLLPNLNHIAGGGGEWFREDRSPVFQTLLSDVKKII-
TYPLSLHHKGYEEVERFMKERIDENGTLYSYATASFYMIYALLALGHSIQSPIIEKAITGIT-
SYIWKMERGSHLQNSPSTIWDTALLSYALQEAQVPKASKVIHNASAYLL-
RKQQTKKVDWSVHAPDLFPGGWGFSDVNTTIPDIDDTTAALRALARSRGNENVD-
NAWKRAVNWVKGLQNNDGGWGAFEKGVTSRILANLPIENASDMITDPSTPDITGRVLEF-
FGTYTQNELPEKQKQSAINWLMNVQEENGSWYGKWGICYIYGTWAVMTGLRSFGIPSSNPS-
LKRAALWLEHIQHEDGGWGESCQSSVEKRFVTLPFSTPSQTAWALDALISYYDKETPVIRKGI-
SYLLSNSYINEKYPTGTGLPGGFYIRYHSYAHIYPLLTLAHYAKKYKK

>seq ID 68

MLLYEKVHEEIARRTTALQTMQRQDGTWRFCFEGAPLTDCHMIFLLKLLGKDKEIEPFVKR-
LASLQTNEGTWKLYEDEVGGNLSATIQSYAALLASEKYTKEDVNMKRAEMFINEHGGVARAH-
FMTKFLLAIHGEYEYPSLFHLPTPIMFLQNDSPLSIFELSSSA-
RIHLIPMMLCLNKRFRVGKKLLPNLNHIAGGGGEWFREDRSPVFQTLVSDVKKII-
TYPLSLHHKGYEEVERFMKERIDENGTLYSYATASFYMIYALLALGHSIQSPIIQKAITGIT-
SYIWKMERGSHLQNSPSTVWDTALLSYALQEAQVPKASKVIHNASAYLL-
RKQQTKKVDWSVHAPDLFPGGWGFSDVNTTIPDIDDTTAALRALARSRGNEN-
VDTAWKRAVNWVKGLQNNDGGWGAFEKGVTSRILANLPIENASDMITDPSTPDITGRVLEF-
FGTYTQNELPEKQKQSAINWLMNVQEENGSWYGKWGICYIYGTWAVLTGLRSL-
GIPSSDPSVKRAALWLEHIQHEDGGWGESCQSSVEKRFVTLPFSTPSQTAWALDALISY-
YDKETPVIRKGISYLLSNSYINEKYPTGTGLPGGFYIRYHSYAHIYPLLTLAHYAKKYRK

>seq_ID 41

MSNLLLYEKVHEEIARRTTALQTMQRQDGTWQFCFEGAPLTDCHMIFLLKLLGRDKEIEPF-
VKRLASLQTNEGTWKLYEDEMGGNLSATIQSYAALLASEKYTKEDANMKRAEMFINERGGVA-
RAHFMTKFLLAIHGEYEYPSLFHLPTPIMFLQNDSPLSIFELSSSA-
RIHLIPMMLCLNKRFRVGKKLLPNLNHIAGGGGEWFREDRSPVFQTLVSDVKKII-
TYPLSLHHKGYEEVERFMKERIDENGTLYSYATASFYMIYALLALGHSIQSPIIQKAITGIT-
SYIWKMERGSHLQNSPSTVWDTALLSYVLQEAQVPKASKVIHNASAYLL-
RKQQTKKVDWSVHAPDLFPGGWGFSDVNTTIPDIDDTTAALRALARSRGNEN-
VDTAWKRAVNWVKGLQNNDGGWGTFEKGVTSRILANLPIENASDMITDPSTPDITGRVLEF-
FGTYTQNELPEKQKQSAINWLMNVQEENGSWYGKWGICYIYGTWAVLTGLRSL-
GIPSSDPSVKRAALWLEHIQHEDGGWGESCQSSVEKRFVTLPFSTPSQTAWALDALISY-
YDKETPVIRKGISYLLSNSYINEKYPTGTGLPGGFYIRYHSYAHIYPLLTLAHYAKKYRK

>seq_ID 66

MSNLLLYEKVHEEIARRTTALQTMQRQDGTWQFCFEGAPLTDCHMIFLLKLLGRDKEIEPF-
VKRLASLQTNEGTWKLYEDEMGGNLSATIQSYAALLASEKYTKEDANMKRAENFIKERGGVA-
RAHFMTKFLLAIHGEYEYPSLFHLPTPIMFLQNDSPLSIFELSSSA-
RIHLIPMMLCLNKRFRVGKKLLPNLNHIAGGGGEWFREDRSPVFQTLASDVKKII-
TYPLSLHHKGYEEVERFMKERIDENGTLYSYATASFYMIYALLALGHSIQSPIIEKAIMGIT-
SYIWKMERGSHLQNSPSTIWDTALLSYALQEAQVPKASKVIQNASAYLL-
RKQQTKKVDWSVHAPDLFPGGWGFSDVNTTIPDIDDTTAVLRALARSRGNENVD-
NAWKRAVNWVKGLQNNDGGWGAFEKGVTSRILANLPIENASDMITDPSTPDITGRVLEF-
FGTYGQNELPEKQKQSAINWLTNAQEENGSWYGKWGICYIYGTWAVLTGLRSLGIPSSDPS-

LKRAALWLEHIQHEDGGWGESCHSSVEKRFVTLPFSTPSQTAWALDALISYYDKETPVIRKGI-
SYLLSNPYINEKYPTGTGLPGGFYICYHSYAHIYPLLTLAHYAKKYRK

>seq_ID 138

MVADERSALIDALKRSQSVDGSWRFPFETGISTDAYMIILLRTLGIHDEPLIQALVERIES-
RQDANGAWKLFADEGDGNVTATVEAYYALLYSGYRKKTDSHMQKAKARILEVGGLERVHLFT-
KVMLALTGQHSWPRRFPLPLVFFLLPPSFPLNMYDLSVYGRANMVPLLVVAER-
RYSRKTDNSPDLSDLAASRNDWRLPDTEALWSYVKRSLTGLPAWLHRAAEQRAVRYMLEH-
IEPDGTLYSYFSSTFLLIFALLALGYPKDDPHIARAVRGLRSLRTEIDGHTHMQYTTASVWN-
TALASYALQEAGVPPTDRTIEKANRYLLSRQHIRYGDWAVHNPYGVPGGWGFSDVNT-
MNPDVDDTTAALRAIRRAAAKETAFRHAWDRANRWLFSMQNDDGGFAAFEKNVGKRFWRYL-
PIEGAEFLLMDPSTADLTGRTLEYFGTFAGLTKDHSAIARAIDWLLDHQEADGSWYG-
RWGICYVYGTWAAVTGLSAVGVPIDHPAMQKAVRWLLSIQNDDGGWGESCKSDGAKTYVPL-
GASTPVHTAWALDALIAAAERPTPEMKAGVRALVRMLHHPDWTASYPVGQG-
MAGAFYIHYHGYRYIFPLLALAHYEQKFGPFVD

>seq ID 69

MLLYEKVHEEIARRTTALQTMQRQDGTWRFCFEGAPLTDCHMIFLLKLLGRDKEIEPFVKR-
LASLQTNEGTWKLYEDEVGGNLSATIQSYAALLASEKYTKEDANMKRAEMFINERGGVARAH-
FMTKFLLAVHGEYEYPSLFHLPTPIMFLQNDSPLSIFELSSSA-
RIHLIPMMLCLNKRFRVGKKLLPNLNHIAGGGGEWFREDRSPVFQTLLSEVKKII-
TYPLSLHHKGYEAVERFMKERIDENGTLYSYATASFYMIYALLALGHSIQSPIIQKAITGIT-
SYIWKMERGSHLQNSPSTVWDTALLSYALQEAQVPKASKGIQNASAYLL-
RKQQTKKVDWSVHAPDLFPGGWGFSDVNTTIPDIDDTTAVLRALARSRGNENVDNS-
WKRAVNWVKGLQNNDGGWGAFEKGVTSRILANLPIENASDMIPDPSTPDITGRVLEF-
FGTYAQNELPEKQKQSAINWLMNIQEENGSWYGKWGICYIYGTWAVLTGLRSLGIPSSDPS-
LKRAALWLEHIQHEDGGWGESCQSSVEKRFVTLPFSTPSQTAWALDALISYYEKETPVIRKGI-
SYLLSNPYVNEKYPTGTGLPGGFYIRYHSYTHIYPLLTLAHYAKKYRK

>seq_ID 67

MSNLLLYEKVHEEIARRTTALQTMQRQDGTWRFCFEGAPLTDCHMIFLLKLLGRDKEIEPF-
VKRLASLQTNEGTWKLYEDEVGGNLSATIQSYAALLASEKYTKEDANMKRAEMFINERGGVA-
RAHFMTKFLLAIHGEYEYPSLFHLPTPIMFLQNDSPLSIFELSSSA-
RIHLIPMMLCLNKRFRVGKKLLPNLNHIAGGGGEWFREDRSPVFQTLLSEVKKII-
TYPLSLHHKGYEEVERFMKERIDENGTLYSYATASFYMIYALLALGHSIQSPIIQKAITGIAS-
YIWKMERGSHLQNSPSTVWDTALLSYALQEAQVPKASKVIQNASAYLL-
RKQQTKKVDWSVHAPNLFPGGWGFSDVNTMIPDIDDTTAVLRALARSRGDENVD-
NAWKRAVNWVKGLQNNDGGWGAFEKGVTSRILANLPIENASDMITDPSTPDITGRVLEF-
FGTYAQNELPEKQKQSAINWLMNVQEENGSWYGKWGICYIYGTWAVLTGLRSLGIPSSDPS-
LKRAALWLEHIQHEDGGWGESCQSSVEKRFVTLPFSTPSQTAWALDALISYYEKETPIIRKGI-
SYLLSNPYVNEKYPTGTGLPGGFYIRYHSYAHIYPLLTLAHYTKKYRK

>seq_ID 35

MSNLLLYEKAHEEIVRRATALQTMQWQDGTWRFCFEGAPLTDCHMIFLLKLLGRDKEIEPF-
VERVASLQTNEGTWKLYEDEVGGNLSATIQSYAALLASKKYTKEDANMKRAENFIQERGGVA-
RAHFMTKFLLAIHGEYEYPSLFHLPTPIMFLQDDAPFSIFELSSSA-
RIHLIPMMLCLNKRFRVGKKLLPNLNHIAGGGGEWFREDRSPVFQTLLSDVKQIISY-
PLSLHHKGYEEIERFMKERIDENGTLYSYATASFYMIYALLALGHSLQSSMIQKAIAGIT-
SYIWKMERGNHLQNSPSTVWDTALLSYALQEAQVSKDNKMIQNATAYLLKKQHTKKAD-
WSVHAPALTPGGWGFSDVNTTIPDIDDTTAVLRALARSRGNKNIDNAWKKGGNWIKGLQNN-
DGGWGAFEKGVTSKLLAKLPIENASDMITDPSTPDITGRVLEFFGTYAQNELPEKQIQRAIN-
WLMNVQEENGSWYGKWGICYIYGTWAVMTGLRSLGIPSSNPSLKRAASWLEHIQHEDGGWGE-
SCHSSVEKRFVTLPFSTPSQTAWALDALISYYDTETPAIRKGVSYLLLNPYVNERYPTGT-
GLPGAFYIRYHSYAHIYPLLTLAHYLKKYRK

>seq_ID 43

MNALLLYEKVHEEIARRTTALQTMQRQDGTWRFCFEGAPLTDCHMIFLLKLLGRDKEVEPF-
VKRLASLQTNEGTWKLYDDEMGGNLSATIQSYAALLASKKYTKEDANMKRAEMFITERGGVA-
RAHFMTKFLLAIHGEYEYPSLFHLPTPIMFLQNDSPLSIFELSSSA-
RIHLIPMMLCLNKRFRVGKKLLPNLNHIAGGGGEWFREDQSPMFQTLLGNVKQIISY-
PLSLHHKGNEEVERFMKERIDENGTLYSYASASFYMIYALLALGHSIQSPMIQKAITGIT-
SYIWKMERGNHLQNSPSTVWDTALLSYALQEARVSKESKMIQNASAYLLKKQHKKKAD-
WSVHAPVLIPGGWGFSDVNTTVPDVDDTTAVLRALAQSRGNGNVDDAWKKGTNWIKGLQNN-
DGGWGAFEKGVTSKLLANLPIENASDMITDPSTPDITGRVLEFFGTYTQNELPEKQKQSAIN-
WLMNEQEENGSWYGKWGICYIYGTWAVMTGLRALGITSAHPSLKRATLWLEHIQHEDGGWGE-

SCQSSVEKRFATLPFSTPSQTAWALDALISYYDKETPAIRKGISYLLANPYVNEKYPTG-
TALPGGFYIHYHSYAHIYPLLTLAHYAKKYKK

>seq_ID 33

MNIVIRISKGWVSNLLLYEKVHEEIARRTTALQTMQRQDGTWQFCFEGAPLTDCHMIFLL-
KLLGRDKEIEPFVKRLASLQTNEGTWKLYEDEVGGNLSATIQSYAALLASKKYTKEDANMKRA-
EMFINERGGVARAHFMTKFLLAIHGEYEYPSLFHLPTPIMFLQNDSPLSIFELSSSA-
RIHLIPMMVCLNKRFQVGKKLLPNLNHIAGGGGEWFREDRSPMFQTLLSDVKQIISY-
PLSLHHKGYEEVERFMKERIDENGTLYSYATASFYMIYALLALGHSLQSSMIQKAIAGIT-
SYIWKMEKGNHLQNSPSTVWDTALLSYTLQEAHASKDNKMIQHAAAYVLKKQHTKKAD-
WSVHAPGLIPGGWGFSDVNTTIPDVDDTTAVLRALARSRGNENVDNAWKKGVNWVKGLQNN-
DGGWGAFEKGVTSNLLANLPIENASDMITDPSTPDITGRVLELFGTYAQNELPEKQKQSAIN-
WLMNVQEENGSWYGKWGICYIYGTWAVMTGLRSLGIPSSNPSMKRAALWLEHIQHEDGGWGE-
SCQSSVEKRFITLPFSTPSQTAWALDALISYHDEETPAIRKGISYLLANPYVNEKYPTGT-
GLPGGFYIHYHSYAYIYPLLTLAHYIKKYRK

>seq_ID 36

MSNLLLYEKVHEEIARRATALQTMQRQDGTWRFCFEGAPLTDCHMIFLLKLLGRDKEIEPF-
VKRLASLQTNEGTWKLYEDEVGGNLSATIQSYAALLASQKYTKEDANMKRAENFIKERGGVA-
RAHFMTKFLLAIHGEYEYPSLFHVPTPIMFLQNDSPLSIFELSSSA-
RIHLIPMMVCLNKRFRVGKKLLPNLNHIAGGGGEWFREDRSPLFQTLLSDVKQIISY-
PLSLHHKGYEEVERFMKERIDENGTLYSYATASFYMIYALLALGHSLQSSMIQKAIAGIT-
SYIWKMERGSHLQNSPSTVWDTALLSYALQEAQVPKDHKMIQQTITYLLKKQHTKKAD-
WSVHAPALTPGGWGFSDVNTTVPDVDDTTAVLRVLARSRENEKVNNAWQKGIDWVKGLQNN-
DGGWGAFEKGVTSKLLANLPIENASDMITDPSTPDITGRVLELFGTYTQNELPEKQKQSAIN-
WLMNAQEENGSWYGKWGICYIYGTWAVMTGLRSLGIPSNNPSLKRAALWLEHIQHEDGGWGE-
SCQSSMEKRFITLPFSTPSQTAWALDALISYYDTETPAIRKGISYLLANPYVNEKYPTGT-
GLPGGFYIRYHSYAQIYPLLTLAHYTKKYRK

>seq_ID 42

MSNLLLYEKVHEEIARRTTALQTMQRQDGTWRFCFEGAPLTDCHMIFLLKLLGRDKEIEPF-
VKRLASLQTNEGTWKLYEDEVGGNLSATIQSYAALLASEKYTKEDANMKRAEMFINERGGVA-
RAHFMTKFLLAIHGEYEYPSLFHLPTPIMFLQNDSPLSIFELSSSA-
RIHLIPMMLCLNKRFRVGKKLLPNLNHIAGGGGEWFREDRSPVFQTLVSDVKKII-
TYPLSLHHKGYEEVERFMKERIDENGTLYSYATASFYMIYALLALGHSIQSPIIEKAIMGIT-
SYIWKVERGSHLQNSPSTIWDTALLSYALQEAQVPKASKVIQNASAYLL-
RKQQTKKVDWSVHAPDLFPGGWGFSDVNTTIPDIDDTTAVLRALARSRGNEHVD-
NAWKRAVNWVKGLQNNDGGWGAFEKGVTSRILANLPIENASDMITDPSTPDITGRVLEF-
FGTYTQNELPEKQKQSAINWLMNVQEENGSWYGKWGICYIYGTWAVLTGLRSLGIPSSDS-
SLKRAVLWLEHIQHEDGGWGESCQSSVEKRFVTLPFSTPSQTAWALDALISY-
YDKETPVIRKGISYLLSNPYINEKYPTGTGLPGGFYIRYHSYAHIYPLLTLAHYAKKYRK

>seq ID 65

MSNLLLYEKVYEEIARRTTALQTMQRQDGTWRFCFEGAPLTDCHMIFLLKLLGRDKEIEPF-
VKRLASLQTNEGTWKLYEDEVGGNLSATIQSYAALLASEKYTKEDANMKRAEMFINERGGVA-
RAHFMTKFLLAIHGEYEYPSLFHLPTPIMFLQNDSPLSIFELSSSA-
RIHLIPMMLCLNKRFRVGKKLLPNLNHIAGGGGEWFREDRSPVFQTLVSDVKKII-
TYPLSLHHKGYEEVERFMKGRIDENGTLYSYATASFYMIYALLALGHSIQSPIIEKAIMGIT-
SYIWKMERGSHLQNSPSTIWDTALLSYALQEAQVPKVSKVIQNASAYLL-
RKQQTKKVDWSVHAPDLFPGGWGFSDVNTTIPDIDDTTAVLRALARSRGNENVD-
NAWKRAVNWVKGLQNNDGGWGAFEKGVTSRILANLPIENASDMITDPSTPDITGRVLEF-
FGTYTQNELPEKQKQSAINWLMNVQEENGSWYGKWGICYIYGTWAVLTGLRSLGIPSSDS-
SLKRAVLWLEHIQHEDGGWGESCQSSVEKRFVTLPFSTPSQTAWALDALISY-
YDKETPVIRKGISYLLSNPYINEKYPTGTGLPGGFYIRYHSYAHIYPLLTLAHYAKKYRK

>seq ID 39

MNNLLLYEKVHEEIARRATALQTMQQQDGTWRFCFEGAPLTDCHMIFLLKLLGRDKEIEPF-
VKRLASLQTNEGTWKLYEDEVGGNLSATIQSYAALLASKKYTKEDANMKRAENFIKERGGVA-
RAHFMTKFLLAIHGEYEYPSLFHLPTPIMFLQNDSHLSIFELSSSA-
RIHLIPMMLCLNKRFRVGKKLLPSLNHIAGGGGEWFREDRSPLFQTLVSDVKQIISY-
PLSLHHKGYEEVERFMKERIDENGTLYSYATASFYMIYALLALGHSLQSTMIQKAITGIT-
SYIWKMESGNHLQNSPSTVWDTALLSYALQEAHVPKDNKMIQHAATYLLKKQHTQKAD-
WSVHAPALTPGGWGFSDVNTTIPDVDDTTAVLRALARSRGNEKVDNAWPKGINWVKGLQNN-
DGGWGAFEKGVTSNILANLPIENASDMITDPSTPDITGRVLEFFGKYAQNELPEKQKQSAIN-
WLMNVQEENGSWYGKWGICYIYGTWAVMTGLRSLGIPSSNPSMKRAALWLEHIQHEDGGWGE-

SCHSSVEKRFVTLPFSTPSQTAWALDALISYYDKETSIIRKGISYLLANPYVNEKYPTGT-
GLPGGFYIRYHSYAHIYPLLTLAHYIKKYRK

>seq ID 63

MSNLLLYEKAHEEIARRATALQTMQREDGTWRFCFEGAPLTDCHMIFLLKLLGRDKEIEPF-
VKRLATLQTNEGTWKLYEDEVGGNLSATIQSYAALLASGKYTKEDANMKRAENFIKERGGVA-
RAHFMTKFLLAIHGEYEYPSLFHVPTPIMFLQNDSPLSIFELSSSA-
RIHLIPMMLCLNKRFRVGKKLLPNLNHIAGGGGEWFREERSPLFQTLLSDVKQIISY-
PLSLHHKGYEEVERFMKERIDENGTLYSYATASFYMIYALLALGHSLQSSMIQKAIAGIT-
SYIWKMESGNHVQNSPSTVWDTALLSYALQEAHVPKDNKMLQNATAYLLKKQHTKKAD-
WSVHAPALTPGGWGFSDVNTTVPDVDDTTAVLRVLARSKGNEKLDHAWQKGINWVKGLQNN-
DGGWGAFEKGVTSRILANLPIENASDMITDPSTPDITGRVLEFFGTYAQNELPEKQKQSAIN-
WLMNAQEENGSWYGKWGICYIYGTWAVMTGLRSFGIPSSNPSLKRAALWLEHIQHKDGGWGE-
SCHSSVEKRFVTLPFSTPSQTAWALDALISYYDTETPVIRKGISYLLANPYVNEKYPTGT-
GLPGGFYIRYHSYAHIYPLLTLTHYIKNIENKPRDISRFIFLGSRSLLKRIRLCFPYFSVDWRF

>seq ID 37

MSNLLLYEKAHEEIARRATALQTMQREDGTWRFCFEGAPLTDCHMIFLLKLLGRDKEIEPF-
VKRLASLQTNEGTWKLYEDEVGGNLSATIQSYAALLASGKYTKEDANMKRAENFIKERGGVA-
RAHFMTKFLLAIHGEYEYPSLFHVPTPIMFLQNDSPLSIFELSSSA-
RIHLIPMMLCLNKRFRVGKKLLPNLNHIAGGGGEWFREERSPLFQTLLSDVKQIISY-
PLSLHHKGYEEVERFMKERIDENGTLYSYATASFYMIYALLALGHSLQSSMIQKAIAGIT-
SYIWKMESGNHVQNSPSTVWDTALLSYALQEAHVPKDNKMLQNATAYLLKKQHTKKAD-
WSVHAPALTPGGWGFSDVNTTVPDVDDTTAVLRVLARSKGNEKLDHAWQKGINWVKGLQNN-
DGGWGAFEKGVTSRILANLPIENASDMITDPSTPDITGRVLEFFGTYAQNELPEKQKQSAIN-
WLMNAQEENGSWYGKWGICYIYGTWAVMTGLRSFGIPSSNPSLKRAALWLEHIQHKDGGWGE-
SCHSSVEKRFVTLPFSTPSQTAWALDALISYYDTETPVIRKGISYLLANPYVNEKYPTGT-
GLPGGFYIRYHSYAHIYPLLTLTHYIKKYRK

>seq_ID 46

MLLYEKVHEEVKEKMAALQAMQQQDGTWRFCFEGSPLTDCYMIFLLTLLGQDQEIEPFVAR-
LAALQTNEGTWKLYEDEPDGNLSATIQAYAALLVSKMYKKEDINMKRAEVFIRKQGGIT-
KAHFMTKFLLALHGGYEYPPLFHFPTPILFLSEDSPLSIFELSSSARIHLIPMMLCMNKRFT-
VSKKMLPNLDYISGGSKEQWFREERSPLFQTLLRDVTKFLSYPLSLHYKGDKAAERFMIERI-
DTNGTLYSYASATFYMIYALLALGHSIQSPLISNAVLGLKTYVWNMDR-
WAHLQNSPSTVWDTALLSYSLQEARVPHDNEMIQKAINYLLQKQHKEKKDWSVHAP-
TLDAGGWGFSDVNTTIPDVDDTTAVLRALAGSRQGNPKVESAWRKGIEWVKGLQNSDGG-
WAAFEKGVTSKVLTHLPLDNSGDMITDPSTVDITGRVLEFFGTYAPNELQGDQK-
DRAIRWLIYTQEKNGSWHGKWGVCYIYGTWAALTGLRAVGVPSNHIALQKAATWLE-
SIQHSDGGWGESCRSSVEKKFISLPFSTPSQTAWALDALIACYDSETPTIRKGISYLLKHST-
KHQEYPTGTALANGFYIRYHSYHHIFPLLTFAHYIKKYRK

>seq_ID 40

MSNLLLYEKVHEEIARRTTALQTMQRRDGTWRFCFEGAPLTDCHMIFLLKLLGRDKEIEPF-
VKRLASLQTNEGTWKLYEDEVGGNLSATIQSYAALLASKKYTKEDANMKRAEMFINERGGVA-
RAHFMTKFLLAVHGEYEYPSLFHLPTPIMFLQSDSPLSIFELSSSARIHLIPMMLCLNKKFR-
IRKKLLPNLNHISGGGGEWFRGNRSPLFQTLVSDVKQIISYPLSLHHKGNEEVERFMKERI-
DENGTLYSYATASFYMIYALLALGHSLQSTMIQKAITGITSYIWNMES-
GNHLQNSPSTVWDTALLSYALQEAHVPKDTNMLQHATAYLLKKQHTKKADWSVHAPAL-
APGGWGFSDVNTTIPDVDDTTAVLRALARSRGSEKVDYVWEKGINWVKGLQNN-
DGGWGAFEKGVTSNLLANLPIENASDMITDPSTPDITGRVLELFGTYAQNELPEKQTQSAIN-
WLMNVQEKNGSWYGKWGICYIYGTWAVMTGLRSLGIPSSNPSLKRAALWLEHIQHEDGGWGE-
SCHSSVEKRFVTLPFSTPSQTAWALDALISYYDKETPAIRKGISYLLANRYVNEKYPTGT-
GLPGGFYICYHSYAHIYPLLTLAHYIKKYRK

>seq_ID 38

MSNLLLYEKAHEEIARRATALQSMQWQDGTWRFCFEGAPLTDCHMIFLLKLLGRDKEIEPF-
VKRLASLQTNEGTWKLYEDEVGGNLSATIQSYAALLASGKYTKEDANMKRAENFIKERGGVA-
RAHFMTKFLLAVHGEYEYPSLFHLPTPIMFLQNDSPLSIFELSSSA-
RIHLIPMMLCLNKRFRVGKKLLPNLNHIAGGGGEWFREERSPLFQTLVSDVKQIISY-
PLSLHHKGYEEVERFMKERIDENGTLYSYATASFYMIYALLALGHSLQSSIIQNAITGIT-
SYIWKMESGNHLQNSPSTVWDTALLSYALQEAHVPKDNKMLQNATAYLLKKQHTKKADWSVHA-
SALTPGGWGFSDVNTTVPDVDDTTAVLRVLARSRGNEKVDHAWQKGINWVKGLQNN-
DGGWGAFEKGVTSNILAKLPIENASDMITDPSTPDITGRVLEFFGTYAQNELPEKQKQSAIN-
WLMNVQEENGSWYGKWGICYIYGTWAVMTGLRSFGIPSSNPSLKRAALWLEHIQHKDGGWGE-

SCHSSVEKRFVTLPFSTPSQTAWALDALISYYDTETPIIRKGISYLLANPYVNEKYPTGT-
GLPGGFYIRYHSYAHIYPLLTLAHYIKKYRK

>seq_ID 55

MLLYEKVRQEVERKVTALRTMQYQDGAWRFCFEGSPLTDCHMIFLLRLLGQNGEMEPFVTR-
VASLQTNEGTWKLYEDESVGNLSTTINAYVALLASGRYTKEDINMKRAEAFIRRQGGIT-
KAHFMTKFLLALHGGYEYPSLFHFPTPMLFLPEDSPLSIFELSSSARIHLIPMMICMNKRFT-
VSKTILPNLDYISGGSKKQWFREERSSLFQRLLGDVKKFLSYPLSLQHKGYKEAERF-
MIERIETNGTLYSYASATFYMIYALLALGHSIQSPLISNAVLGLKSYIWNMNKGTH-
LQNSPSTVWDTALLSYSLQEAGVPNDNQMIQKATDYLLQKQHKEKKDWSVHAPS-
LDAGGWGFSDVNTTIPDIDDTTAALRAIARSREGNQRIEEAWRKGIEWVKGLQNIDGG-
WAAFERGVTSHFLTHLPLDNAGDMTTDPSTSDITGRVLEFFGTYAPHQLKDDQKDRAIK-
WLMQAQEKNGSWYGKWGVCYIYGTWAALTGLRAVGVPSNHTALQKAATWLERIQHNDGGWGE-
SCRSSIEKHFISLPFSTPSQTAWALDALITFYDTETPVIRKGISYLLAHLNQNQDYPT-
GIGLPDGFYIRYHSYHHIFPILTFAHYIKKYMK

>seq ID 54

MLLYĒKVRQEVERKVTALRTTQYQDGAWRFCFEGSPLTDCHMIFLLRLLGQNGEMEPFVTR-
VASLQTNEGTWKLYEDESVGNLSTTINAYVALLASGRYTKEDINMKRAEAFIRRQGGIT-
KAHFMTKFLLALHGGYEYPSLFHFPTPMLFLPEDSPLSIFELSSSARIHLIPMMICMNKRFT-
VSKTIFPNLDYISGGSKKQWFREERSPLFQTLLGDVKKFLSYPLSLQHKGYKEAERF-
MIERIETNGTLYSYASATFYMIYALLALGHSIQSPLISNAVLGLKSYIWNMNKGTH-
LQNSPSTVWDTALLSYSLQEAGVPNDNQMIQKATDYLLQKQHKEKKDWSVHAPS-
LDAGGWGFSDVNTTIPDIDDTTAALRAIARSREGNQRIEEDWRKGIEWVKGLQNIDGG-
WAAFERGVTSHFLTHLPLDNAGDMTTDPSTSDITGRVLEFFGTYAPHQLKDDQKDRAIK-
WLMQAQEKNGSWYGKWGVCYIYGTWAVLTGLRAVGVPSNHTALQKAATWLERIQHNDGGWGE-
SCRSSIEKHFISLPFSTPSQTAWALDALITFYDTETPVIRKGISYLLAHLNQNQDYPT-
GIGLPDGFYIRYHSYHHIFPILTFAHYIKKYMK

>seq ID 189

MRSELLQLQSADGSWRLCFDSGTMPDSYFIIILRMLGYSQDEALIRQIAS-
RILSRQLPNGTWKIYPDEEDGNLDATAEAYFALLYSGFLTKLDPRMQLAKQFILSKGGLS-
KIRSLLTQAIFAAAGQASWPKSMRIPLEVFFSDNGIGIDLFSLSGHARVHIVPIIM-
LANAQFVQHSASMPDLSDLFAGSSKRFENDSPWIAALATLIGSLSLSELLPFESPTPQE-
KAVQFLFDRLEPDGTLLTYTTATMFMILVLLMLGYSSSSPLIHRMVSGIHSVICANSHVQIAS-
SEVWDTAMLVHALRKAGVNPTSTALENAGAYLRQRQQTQLGDWAIRNPGT-
PAGGWGFSNVNTLYPDVDDTTAALRAIQPYSSRTPELQADWQRGLNWVLTMRNDNG-
GWPAFERQGSRLPITFFNFEGAKDIAVDPSTVDLTSRTLQFLGQELGMNAGNSWIEST-
LRWVLSQQESNGSWYGRWGITYVHGTSAALQGLTAVGIAEDHPAVKKGVDWLLQVQNEDGGW-
GESCISDKVRRYVPLNFSTPSQTAWALDGLTAALPKPTPALERGVDALLQSLDRHDWTYTYP-
TGGALPGSVYAHYASNNYIWPLLALSNIWQKYS

>seq_ID 200

MALPFNQDSYKGDDEADVSKGAAKSPPSLEEAIQRSQEFLLAQQFPEGFWFGELEANVTI-
ISHTVILYKLLGIEENFPMYKFERYLRRMQCSHGGWEIAYGIGSYLSATIEAYIAL-
RLLNVPQSDPALQKALRVILDSGGVTKARIFTKICLALLGSFDWRGIPSLPPWLILCPTWFPL-
SIYEVSSWARGCIVPLLVILDKKPVFKVSPEVSFDELYAEGREHACKIIPISGDWTSKFFIT-
VDRVFKMMERLRVVPFRQWGIREAEKWILERQEESGDYVNIF-
PAMFYSVMCMKVLGYETTDPVVQRALLGFKGFTIETADECKVQSTVSPIWDTAFIVRALVDS-
GIPPDHPALQKAGQWLLQKQILKHGDWAFKDRQNPVNQRGFACLQRDSQIETADECRVQST-
LSPVWDTAFVVKALVDSGIPPNHPALQKAGQWLLQNQTLTHGDWAFKTQSGHLAAGG-
WAFQSHNRWYPDADDSAAVMMALDCIELPDEDVKNGAIARGLKWISALQSRNGG-
WAGYDKNCDQQWINKVPFNDLNGILDVPTADVTARVLEMVGRLSRLGAVGTPYSPRHCTLVES-
IPHLLLPETIARGLAYLRREQEGEGCWWGKWGVNYIYGTCGALLALSQVAPTTHQEEIARG-
AKWLAQVQNRCDKQKAAQGPRDGGWGESCFSYDDPALKGQNDASTASQTA-
WAVQGLLAAGDALGKYEVEAIEQGVQYLLATQRKDGTWHEAHFT-
GSCFAQHFYVRYHYYAQHFPLSALGLYRTRILQHQ

>seq_ID 139

MVADERSALIDALKRSQSVDGSWRFPFETGISTDAYMIILLRTLGIHDEPLIQALVERIES-
RQDANGAWKLFADEGDGNVTATVEAYYALLYSGYRKKTDSHMQKAKARILEVGGLERVHLFT-
KVMLALTGQHSWPRRFPLPLVFFLLPPSFPLNMYDLSVYGRANMVPLLVVAER-
RYSRKTDNSPDLSDLAASRNDWRLPDTEALWSYVKRSLTGLPAWLHRAAEQRAVRYMLEH-
IEPDGTLYSYFSSTFLLIFALLALGYPKDDPHIARAVRGLRSLRTEIDGHTHMQYTTASVWN-
TALASYALQEAGVPPTDRTIEKANRYLLSRQHIRYGDWAVHNPYGVPGGWGFSDVNT-

MNPDVDDTTAALRAIRRAAAKETAFRHAWDRANRWLFSMQNDDGGFAAFEKNVGKRFWRYL-
PIEGAEFLLMDPSTADLTGRTLEYFGTFAGLTKDHSAIARAIDWLLDHQEADGSWYG-
RWGICYVYGTWAAVTGLSAVGVPIDHPAMQKAVRWLLSIQNDDGGWGESCKSDGAKTYVPL-
GASTPVHTAWALDALIAAAERPTPEMKAGVRALVRMLHHPDWTASYPVGQG-
MAGAFYIHYHGYRYIFPLLALAHYEQKFGPFVD

>seq_ID 13

MAQMASSLGSPRLLLRMGREAAQQQHLASGTEVQKALRLAVGHSLDLQRTDGAWCGE-
VHSNATFTAQYVFLQQQIGLPLDPTEIEGLSRWLFSQQNEDGSWGLGPGLGGDVSTTTETY-
LALKILGVSPEDPRMAAARTSIIKAGSLPATRMFTRVFLASFGLIPWSAVPPLPAELILL-
PTLFPVNIYNLSSWARATCVPLLLIRHHEPLHSLPNGRHAENDFLDELWTKDIPRDFCYTT-
PLSRMWRLGDYAGIFFTSADHGFRFLGQYFNSPLRNLSRRKIINWILDHQEQSGE-
WAGYWPPQHNNIWALSLEGYSLDHPVLRRGIAAVKSFVLHDATGMRA-
QVTVSQVWDTALMSIALSDSAPSTGIISPTQAIDWLMHHEVASHRGDWRVLRPKLAT-
GGFCFEEFNTLYPDVDDTAAVIMALIKSNPAHLISGCVRQCFGMMMAGRHGYSLDCQLETRL-
RASSQLAIAYLLGCQENNGSWWGRWGVNYLYGTSNVLCGLAYYYDRSSLSKGDGKSNSNIV-
SAVDRASEWLKARQHSNGGWGEGLESYDNAQLAGCGQPTASQSAWVTMALLNYLSPTDE-
VIQRGVSYLVRNQVKYGDESRATWPLERYTATGFPGHLYMEYDYYRHYFPIMALGRYVNKLSGSHKLL

>seq_ID 198

MEDLTQKLQQALQLASRALLNERVRPGLAHWEGELSTSALSTATAVMALFQYAKCQQAS-
GRLQKVFDGKSEGDWRLIEQGLAWLLQHQLADGGWGDTDKSISNISTTMLAHAT-
LVACREAVRQKSLVLNASDIDAAIERSGRLIEELGGIQAIRDRYGKDHTFSVPILTHAALAG-
LVSWNEIPALPYELALLPHRFFEVIQLPVVSYALPALIAIGQTLHLRQRT-
WNPWWWVRRAAIPGTLQKLQSIQPESGGFLEATPLTSFVTMCLASVGRVDHPVTQAGLKFIR-
DSVRPDGSWPIDTNLATWVTTLSINHLGAEAFSSDEREALMRWLLQQQYRTMHPYTNAAPGG-
WAWTNLSGGVPDADDTPGAMLALMELDRVSVSSQESLSIEQALYQAALWLIKLQNRDGGWPT-
FCRGWGALPFDRSSNDITAHCLRALIQYERRLNDVTVDATGDTTSRPLAVEVPSPKLRE-
QMQRSIQQGFEYLEKTQREDGSWLPLWFGNQHSPDDENPLYGTARVLLAYADAGLEGSSAAL-
RGCDWLVRHQHADGAWGPGTSIETADTSDAESDVEGEPASIEETALALMALCRFDATHNVLHR-
GASWLITKVENETWREPTPIGFYFAKLWYYEKLYPQVFTVGALKALALRLGSALTTVSENE-
PAPSSAEPPIPPIATDRVADSMHLQRTSPSINLANGGITLA

>seq_ID 252

SPVWDTVLTLLALDDCGYNDCYSEEVDKAVQWVLDQQVLSKGDWSVKLPNVEPGGWAFEYANT-
RYPDTDDTAVALIVLSQFKDDPKWKERGINQAIERGVNWLFEMQCKNGGWGAFDKDNDKTLLT-
KIPFCDFGEALDPPSVDVTAHIVEAFGKLGYSKDHPKIAHAIEYLKEEQEADGAWF-
GRWGVNYVYGTGAVLPALEAIGEDMSQPYIRKAANWLVLHQNEDGGWGE

>seq_ID 253

SPVWDTVLTLLAFDDCDKNEAYQASVEKAVQWTLDNQVLRKGDWSVKLPDVEPGG-
WAFEYANTFYPDTDDTAVALIVLSQFRDVEKWQEAGIEKAIERGVNWLFAMQSKNG-
GWGAFDKDNDNNFITKIPFCDFGEALDPPSVDVTAHCIEAFGKLGLSRARPEIARGLDYLKSE-
QEADGAWFGRWGVNYVYGTGAVLPALEAIGEDMSQPYIRKAANWLILRQNEDGGWGE

>seq_ID 257

SPVWDTXLTLLALDDCDLNERQSKEVEKAVQWVLNQQVLRPGDWCVKVPKVQPGGWAFEYKNY-
FYPDTDDTAVALIVLSQFRDDPKWQEKNIEQAIDRGLNWLIGMQCKGGGWGAFDKDNDKTYLT-
KIPFCDFGEALDSPSVDVTAHIVEAFGKLGLGKSHPAMIRAIDYLKAEQEQDGAWF-
GRWGVNYIYGTGAVLPALEAIGEDMRAPYIAKACDWLIAVQQEDGGWGE

>seq_ID 254

SPVWDTLLTLLAYDDSGQNERKADEVEKAVDWVLAXQVLRPGDWKVKAPNLEPGGWAFEYANY-
FYPDTDDTAVALIVLSQFRNDAAWKEKGIEQAIEKGVNWLFGMQCKGGGWGAFDKDNDKQFLT-
KIPFCDFGEALDPPSVDVTAHIVEAFGKLKFSKDHPNIRRAIDYMKDEQEADGAWF-
GRWGVNYIYGTGAVLPALEAIGEDMFAPCIGRACDWLVSRQNDDGGWGE

>seq_ID 255

SPVWDTLLTLLAYDNSGHNARKASEVEKAVDWVLAQQVLRPGDWNVKAPNLEPGGWAFEYANY-
FYPDTDDTAVALIVLSQFRNDAAWKDKGIEQAIEKGVNWLFGMQCKGGGWGAFDKDNDRQFLT-
KIPFCDFGEALDPPSVDVTAHIVEAFGKLKFSKDHPNIRRAIDYTKDEQEDDGAWF-
GRWGVNYIYGTGAVLLALEAIGEDMSAPYIGRACDWLVSRQNDDGGWGE

>seq_ID 256

SPVWDTLLTLLAIEDSGQSVKRAQEVEKAVDWVLSQQVLRPGDWKVRAPHLEPGG-
WAFEYANYFFPDTDDTAVALIVLSQFRNDAAWKAKGIETAIEKGVNWLLGMQCK-
GGGWGAFDKDNDKTYLTKIPFCDFGEALDPPSVDVTAHIVEAFGKLGFSKDHPNIARAIEYLKS-
EQESDGXWFGRWGVNYVYGVGAVLPALEAIGEDMSAPYIGRACDWLVSKQNSDGGWGE

>seq ID 258

SPVWDTVLTMLAIHDCGADKQYAPQMDKAIDWLLANEVRHKGDWAVKLPDVEPGG-
WAFEYSNACYPDLDDTAVALIVLAPYRNDPKWQARDIEGAVERAVDWTLAMQCKNGGWGAF-
GKDNDKAILTKIPFCDFGEALDPPSVDVTAHVLEALAALGYDNSHPA-
VARAIRYLRDEQEPDGSWWGRWGVNYIYGTAAVLPALKAMGVDMNEPFVHKAADWIGSVQNEDGGWGE

>seq ID 302

SPVWDTSLVLVAMQEAGVPVDHPALVKAAQWLLDREVRLKGDWRVKSPDLEPGGWAFEFLND-
WYPDVDDSGFVMLALKDIKVRDKKQKSQAIKRGIAWCLGMQSANG-
GWGAFDKDNTKYLLNKIPFADLEALIDPPTADLTGRMLELMGTFNYPKSHVAVVRALGFLKS-
VQEPEGPWWGRWGVNYIYGTWSVLGGLDAIGEDMSQPYIRKAVNWLKSKQNLDGGWGEVCETY-
EDRSLMGCGPSTPSQTSWALLSLFSAGEINAKAVLRGIKYLVETQNQDGSWDEDAYTGTGFP

>seq_ID 271

SPVWDTAISVISLAXSGMERGHPALVRAAXWLMSKEIKTAGDWKVTNPAGPVGGWAFEFNNAF-
YPDIDDSAMVMMALRHVHLDEHTAHRREKACLRGLNWLLSMQSRTGGWAAFDKDNTKVIMT-
KIPFADHNAMIDPPWADITGRVLEFLGYIGYDQSYPAVARAARFLREEQEEDGSWF-
GRWGVNYIYGTWQVLRGLAAIDEDMSQPYIRRAAEWLRSVQPPDGGWGETCATYHDPSLKGKG-
PATPAQTAWAVMGLMAAGIYDESVSRGIDYLVRTQRPDGTWDETEYTGTGFP

>seq_ID 299

SPVWDTALVLVAMQEAGVPVDHPALIKSAQWLLDLEVRRKGDWHVKSPDLEPGGWAFESLND-
WYPDVDDSGFVMLFIKDIKVRDKKLKDQAIKCGIAWCLGMQSENGGWGAFDKDNTKHL-
LNKIPFADLEALIDPPTADLTGRMLELMGNFNYPKSHQAAVKALDFLKVEQEPEG-
PWWGRWGVNYIYGTWSVLCGLEAIGEDMSQPYIKKAVNWLKSKQNLDGGWGEVCD-
SYADRSLMGCGPSTASQTSWALLSLFAAGEVSSKAALRGVEYLLSTQKLDGTWDEDAFTGTGFP

>seq ID 314

SPVWDTALAVRALAAAGVPPEHPAMVKASEWLLTQQIFKPGDWSIKCPDLPPGGWAFEFVNN-
WYPDVDDSSMVLVALKDGLADAAKHQAALQRGINWCLGMQSKNGGFASFDKDNTKEWLNSL-
PFGDLKALVDPPTEDITARILEMMGAFGHGLDHPVAARALAYLHQTQRPEG-
PWWGRWGVNYIYGTWSVLVALKRIGEDMSRPYVRRAVDWVKAHQNPDGGWGEFCESYRN-
PELMGKGPSTASQTAWALLGLFAAGEVHAPEVTAGVDYLVKTQDSLGRWDEEQFTGTGFP

>seq_ID 251

SPVWDTVLTMLSVQDCDADENSENAPAIEKAIEWLLANEVRTGGDWQEKVKGVEPGG-
WAFEYKNASYPDTDDTAVAMMALAPYRTEEKWKKKGLPEALKRAAEWNIAMQCSNG-
GWGAFDKDNDKTILCKIPFCDFGEALDPPSVDVTAHVLEGLAALDYPPEHPAIQRAVQFIK-
DEQEPDGSWWGRWGVNFIYGTAAALPALKAVGEDMRAPYIDRAAKWIVDHQNEDGGWGE

>seq_ID 312

SPVWDTALAVRALAAAGVPPEHPAMVQASEWLLTQQIFKPGDWSVKCPDLPPGGWAFEFVNN-
WYPDVDDSSMVLVALKDGLADAAKHQAALQRGINWCLGMQSKNGGFASFDKDNTKEWL-
NAIPFGDLKALVDPPTEDITARILEMMGAFGHGLDHPVAVRAMAYLHETQRPEG-
PWWGRWGVNYIYGTWSVLVALKRIGEDMSRPYVRRAVDWVKAHQNLDGGWGECCESYRN-
PELMGRGPSTASQTAWALLGLFASGEVHTPEVKAGVDYLVKTQNSLGRWDEEQFTGTGFP

>seq_ID 250

SPMWDTVLTTLAVQDAGVDQEPEFKPAMERTLEWLLKNEVRTGGDWQQKTRGVEPGGWAFEYA-
NASYPDNDDTAVALIVLAPFRHDPKWQARGIQHVIDRAVNWMFAMQCDNGGWAAFDLDND-
KAILTRIPFCDFGEALDPPSVDVTAHVLEALAALGYSREHPAVRRAIAFLKEDQEPDGSWF-
GRWGVNFIYGTAAALPALKAMDEDMTQDWITRAADWMRSRQNDDGGWGE

>seq_ID 260

SPVWDTVLTLLAIQDADKQDDMAAEVDRAIGWLLSKEVRTNGDWSVKLPDVEPGGWAFEHENA-
RYPDTDDTAVAVMVLAPYRHHPKWRKRGLPEALDRAISWMRAMQCRNGGWGAFDKDNDNAF-
LCVIPFCDXGEALDPPSIDVTAHALEAFAAMGFGPEDTTVARALDYMSKEQEADGS-
WWGRWGVNYIYGTAAALPAYKAFGQDMRDPKLMKAADYLRAKQNADGGWGE

>seq_ID 259

SPVWDTVLTLLAMEDCEATEEHAAAIEQAIEWLLENEVRTPGDWQMKVPDADPGG-
WAFEYANAAYPDVDDTAVAILVLARYRDDPKWQAKGLPQAIDRAVAWVLAMQCSNGG-
WAAFDKDNDKSILCKIPFCDFGEALDPATVDVTAHVLEALAAVGYGPDHPAVRRGLDFLY-
AEQEADGSWWGRWGVNYVYGTGAALPAFKAIGADMRDPRMLKAADWILRCQNKDGGWGE

>seq ID 261

SPVWDTVLTLLAIQDADKQEEMAGEIDKAIGWLLSKEVRTKGDWSVKLPRVEPGGWAFEHENA-
RYPDIDDTAVAIMVLAPYRDHPKWKKRGLPEALDRAIAWMRAMQCRGGGWGAFDKDNDKQILC-
TIPFCDFGEALDPPSIDVTAYALEAFAAMGYGPDDKTVARALKYMSKEQEADGS-
WWGRWGVNYIYGTAAALPAYKALGQDMRDPGLMKAADYLRDKQNADGGWGE

>seq_ID 262

SPVWDTVLTLLAMQDADRTDKHKAAVDKAIQWVLDQEVRTPGDWCVQTPDVEPGGWAFEYENA-
RYPDVDDTAVAIMVLAPYQDDPKWRKRGLPDALARAIAWIRAMQCKNGGWGAFDRDNDNSMLT-
VIPFCDFGEALDPPSVDVTAHALEAFHMMGYGPEDPTVARALAYLDAEQEQDGSWWGRWGVN-
FIYGTSAALPALKAMGRDMRDPRYTKAADYLRAVQNDDGGWGE

>seq_ID 275

SPVWDTLLALLALQDCDRELTAEMSRALDWVLANEVRYHGDWTKKVKGVEPSGWAFERAN-
LNYPDIDDTAVALIVLARLPRAWLDEPRIRATIDRVLGWTLAMQSSNGGWAAFDKDNDRPIIT-
KIPFCDFGEALDPPSADVTAHVLEALGLLGFDRRHPAVERGLRFLRSEQEADGSWF-
GRWGVNYVYGTAAVLPGLAAIGEDMTQDYIRRANDWLIAHQNPDGGWGE

>seq_ID 280

SPVWDTLLSLVALQDCGKELTPARERALEWILGREIRTRGDWAKKVKNVEASGWAFERAN-
LHYPDIDDTAVALIMLARLPRAWLDQPRIRAVIDRALGWTLAMQSSSGGWAAFDKDNDRLIIT-
KIPFCDFGEALDPPSADVTAHVLEALGILGFDRQHAAVRHGLKFLRSEQEADGSWF-
GRWGVNHVYGTGAVLPALAAIGEDMAQDYVRRAADWLVAHQNADGGWGE

>seq_ID 277

SPVWDTLLALLAMQDCERELTPQMERALDWVLANEVRYYGDWSKKVRGVEPSGWAFERAN-
LNYPDIDDTVVALIVLARLPRALLDQPRIRAVIDRALGWTLAMQSSNGGWAAFDKDNDHLIIT-
KIPFCGFGEALDPPSADVTAHVLEALGLLGFDRHHPAVARGYQFLRKEQEADGSWFGRWGVN-
HIYGTAAVLPALAAIGEDMSQPYIRAAAEWIIAHQNADGGWGE

>seq_ID 300

SPVWDTALVLVAMQXAGVPVXHPALVKSAQWLLDLEVXXKGDWQVKSPELEPGGWAFXFLND-
WYPDVDDSGFVMLSIKXIKVRDKKHKEQAIKRGISWCLGMQSDNGG-
WAAFDKNNTKYLLNKIPFAXLEALIDPPTAXLTGRMLELMGNFNYPKTH-
KAAVQALEFLXMEXEPXGPWWGRWGVNYIYGTWSVLCGLEAIGEDMAQPYIKKSINWLKS-
KQNMDGGWGEVCESYGDRSLMGCGPSTASQTSWALLSLFAAGEVHSKAATRGIEY-
LLATQKLDGTWDEDAYTGTGFP

>seq_ID 279

SPVWDTLLXLLAMQDCERESTPSMERALDWXXANEVRYYGDWSKKVRGVEPSGWAFXRAN-
LNYPDIDDTDVALIVLARLPRALLDQSRVHAVIDRALGWTLXMQSSNGGWAAFDKDNNHLIIT-
KIPFCDFXEALDPPSADVTAHVLEALGLLGFNRNHPAVERGYRFLRSEQETDGSWF-
GRWGVNHVYGTXAVLPALAAIGEDMTQPYIRSAAEWIIAHQNADGGWGE

>seq_ID 264

SPVWDTLLTLEALLDCNLSPKTFTGMQAAVDWILSKQIVTPGDWQIKVPGVSCGGWAFE-
RANTFYPDMDDTAVAMIVLARIRRYYNDSSRIDRALACATDWILSMQCSNGGWAAFDLDNT-
NDLVTRIPFSDFGEMLDPPSVDVTAHVVEALGCLGRTRNDPAVARAVAYILDEQEPEGSWF-
GRWGVNHIYGTGAVLPALAAVGTDMSAGYITRAADWVATHQNADGGWGE

>seq_ID 19

GGWMFQASISPIWDTGLTVLALRSAGLPPDHPALIKAGEWLVSKQILKDGDWKVRRRKAKPGG-
WAFEFHCENYPDVDDTAMVVLALNGIQLPDEGKRRDALTRGFRWLREMQSSNG-
GWGAYDVDNTRQLTNRIPFCNFGEVIDPPSEDVTAHVLECFGSFGYDEAWKVIRKAVEY-
LKAQQRPDGSWFGRWGVNYVYGIGAVVPGLKAVGVDMREPWVQKSLDWLVEHQNEDGGWGE

>seq ID 278

SPVWDTLLSLLAMQDCERGFTPSMERALDWVLANEVRYYGDWSKKVRGVEPSGWAFERAN-
LNYPDIDDTAVALIVLARLPRAQLDQPRIREVIDRALGWTLAMQSSNGGWAAFDKDNDHLIIT-

KIPFCDFGEALDPPSADVAAHVLEALGLLGFERKHPAVERGLKFIRSEQEADGSWFGRWGVN-
HIYGTAAVLPALXAIGEDM

>seq ID 315

SPVWDTALAVRALAAAGLPPDHPFMTQATSWLLTQQIFKPGDWCIKCPDLPPGGWAFXFHNN-
WYPDVDDSSMVLVALKDGLPDTARHQAALQRGINWCLGMQSKNGGFASFDKDNTKEWLNAL-
PFGDLKALVDPPTEDITARILEMMGAFGHGLDHPTADRALAFLRRTQHPEG-
PWWGRWGVNYLYGTWSVLVALKRIGXDMSRPYVQRAVNWIKSHQNPDGGWGEVCESYRH-
PELMGQGPSTASQTAWALLGLLAAGEIQAAEVKAGVDYLVKTQNAQGRWDEKYFTGNWLP

>seq ID 297

SPVWDTALVLQAMQEASIPLDHPALVKAAQWLLDREVRIKGDWKIKSPGLEPGGWAFEFQND-
WYPDVDDSAAVLIAIKDIQVKNNKAKQGAVRRGIDWCLGMQSKNGGWGAFDKDNTKHL-
LNKIPFADLEALIDPPTADLTGRMLELMGNFGYDKHHPQAVHALEFLKKEQEPEGPWF-
GRWGVNYIYGTWYVLIGLEAIGEDMNQPYIKKAANWIKSRQNIDGGWGE

>seq_ID 17

QASISPVWDTGLAVLALRAAGLPADHDRLVKAGEWLLDRQITVPGDWVVKRPNLNPGG-
FALQFDNVYYPDVDDTAVVIWALNTLRLPDERRRRDAMTKGFRWIVGMQSSNG-
GWGAYDVDNTSDLPNHIPFCDFGEVTDPPSEDVTAHVLECFGSFGYD-
DAWKVIQRAVAYLKREQKPDGSWFGRWGVNYIYGTGAVVSALKAVGIDMREPYIQKALDWVEQHQNPDG

>seq ID 303

SPVWDTALVLVAMQEAGVPLDHPALVKAAQWLLDREVRIKGDWRIKSPDIEPGGWAFEFLND-
WYPDVDDSGFVMLAIKDVKVRDKKKKEQAIKRGINWCLGMQSANG-
GWGAFDKDNTKYLLNKIPFADLEALIDPPTADLTGRMLELLGTFNFPKDHHAI-
ERALEFIQLEQEPEGPWWGRWGVNYIYGTWSVISGLEAIGEDMSQPYIRKTVNWLKSKQNMDGGWGE

>seq_ID 298

SPVWDTTLVLVAMQEAGVPVDHPALVKSAQWLLDLEVRRKGDWQVKSPDVEPGGWAFEFMND-
WYPDVDDSGFVMLAIXNIRVRDKKHQEQAIKRGIAWCLEMQSENG-
GWGAFDKDNTKYLLNKIPFADLEALIDPPTADLTGRMLELMGNFDYSASYPAAVRALEFLK-
KEQEPEGPWWGRWGVNYIYGTWSVLCGLEAIGEDMSQPYIRKAVNWLKSKQNLDGGWGE

>seq ID 301

SPVWDTALALVAMQEAGVPKDHPALVKAAQWLLDLEVRRKGDWQIKSPELEPGGWAFEFLND-
WYPDVDDSGFVIMAIRDIKAPDKKHKEQAIKRGIAWCLGMQSKNGGWGAFDKDNTKHL-
LNKIPFADLEALIDPPTADLTGRMLELMGSFDYPMDHPAAARALEFLKKEQEPEG-
PWWGRWGVNYIYGTWSVLCGLESIGEDMSQPYIKKAVNWLKSKQNMDGGWGE

>seq_ID 276

SPVWDTLLTLLAMEDCDRGLTPSMQRALEWVLAQEVRYAGDWSKKVKGVEPSGWAFERAN-
LNYPDIDDTAVALIVLARLPRAWLDEPRIRATIDRVLGWTLAMQSSNGGWAAFDKDNDRPIIT-
KIPFCDFGEALDPPSADVTAHVLEALGLPGFDRRHPAVERGYKFLRSEQEADGSWFGRWGVN-
HIYGTAAVLPALASIXEDM

>seq ID 283

SPVWDTCLTSNALVESGGDTSAPHVHRSVQWLLNQEIRNHGDWSVKAPKVGPSGWAFEFAN-
KVYPDVDDAAEVIIALANYSNDSGTAPPDAIARGVRWISGMQSSNGGWGSFDKNNTS-
FFVTRLPFFDFGEVIDPPSVDVTAHVIEALAVAGWQEKASKQIQKALDYIWSEQEADGPWF-
GRWGINYIYGTCAVLSALEAIGYDMADARVVKALKWIEECQNADGGWGE

>seq_ID 307

SPVWDTPWMIEALLETGVPPGDPALLRAGRWLMSKQITGVRGDWAMKSPKGKPGGWAFEFEN-
DYYPDVDDTIQVLTALCKLSIPWREKEKAVMQGIDWLISMQNDDGGWGAFDRNQTRWIVN-
RIPFSDHKACLDPSSPDITGRMVEFLMRRNYSTSHPSVKKALKYIRETQEDFGAWFARWGI-
NYIYGTWCVLTALAAMGIGHTDSRVAKAVAWLSSVQRPDGGFSEAADTYHPHKPFESYSES-
VPSQSAWALMGLVAGGAVHSPAAARAACYLINNRNLNNGWDERHYTGTGFP

>seq_ID 267

SPVWDTAISVIALAESGLHRGHPSLVQATEWLVANEIRRGGDWQVKNPTAPISGWAFEFKND-
FYPDVDDTAMVLLALRHVHLYNDDVSQDREKSYLRGLNWMLSMQCKNGGWAAFDRDNVKTIFE-
KIPFADHNAMIDPPSVDITGRVLELLGYVGYDKSYPCVTKALEYIKKDQEADGSWYG-
RWGVNYIYGTWQVLRGLAAIGEDMQSEYVQKAVRWMKSVQNPDGGWGE

>seq ID 309

SPVWDTVLSITALADADLPRTHPAMRRAVAWVLGKQVLCEGDWRVKNRRGEPGGWSFEFNNN-FYQDNDDTAAVLIALHKARLPDEAKGEAMQRGLRWLLSMQCDDGGWSAFDVNNN-KRLLNKIPFADLESMLDPSTCDLTGRTLEALGSIGFPFTHRIVQHAVRFIRQHQEADGAWYG-RWGVNYIYGTCHVLCGLLSVGEDMHQPYVQRAVQWLIEHQNADGGWGE

>seq ID 202

MVYSYEMMVLLDYPEDHPLRVECKAALKKLVVHRDDGSSYCQPCLSPVWDTAWSVMALEQAPS-DARTETAIARAYDWLTDRQVLDLRGDWENNAAPSTPPGGWAFQYENPYYPDIDDSAVVLAML-HARGKRTGQPGRYEMPVARCLDWIIGLQSRNGGFGAFDANCDRDFLNAIPFADHGALLDPP-TEDVSGRVLLALGITERPQDATARERCIQYLRDTQQPDGSWWGRWGTNYIYGTWSVLAGL-GLAGVDRKLPMVRNGLQWLRGKQNADGGWGETNDSYARPELAGKHEDGSMAEQTAWAM-LGQMAVGEGDADSVHRGAAYLLDAQNEDGFWMHPYHNAPGFPRIFHLKYHG

>seq_ID 306

SPVWDTPWTVMALLEAGVPSNDPALLRSGRWLLAKQITDTKGDWAIKNKNTAPGGWSFEFEN-KYFPDVDDTIEVLHCLHKLAIPWREKEKPCRLGIDWLLSMQNDDGGWGAFDKNQKRQVVN-RIPFSDHGACLDPSSPDITGRMIEFLATQKFNSEYESVKRALKYIWKTQEDFGGWHARWGI-NYIYGTWCVLTGLRAIGFNMTDRRVQKALNWLESIQNKDGGFGESPASYEECRYIPWKES-VPSQTAWALMALVAGGGAGSAPAENAATFLINYRNSNGVWDEECYTGTGFP

>seq_ID 281

SPVWDTLLTLLAYQDCELEMNDSAGRALDWILSQENSYRGDWAHRNKKLEPSGWAFERAN-LHYPDIDDTSVALIVLARLPQAVRSRPDIKSAIDRALAWTLGMQCRNGGWAAFDRDNDKLIIT-MIPFCDFSEALDPPSADVTAHVVEAMAHLGFDRSHKAVEKAYQYLLAEQEDDGSWFGRWGVN-HIYGTAAVLPALAALGEDATVPHVKRAADWISAHQNTDGGWGE

>seq_ID 310

SPVWDTALAVRALAAAGLPPEHPAMVKASEWLLTQQIFKPGDWSVKCPDLPPGGWAFEFVNN-WYPDVDDSSMVLVALKEGLADAAKHQAALQRGINWCLGMQSKNGGFASFDKDNTKEWL-NAIPFGDLKALVDPPTEDITARILEMMGAFGHGLDHPVAVRGLAYLHQTQRPEG-PWWGRWGVNYIYGTWSVLVALKRIGEDMSRPYVRRAVDWVKAHQNPDGGWGE

>seq_ID 311

SPVWDTALAVRALAAAGLPPEHPAMVKASEWLLTQQIFKPGDWSVKCPDLPPGGWAFEFVNN-WYPDVDDSSMVLVALKDGLVDAAKHQAALQRGINWCLGMQSKNGGFASFDKDNTKEWL-NAIPFGDLKALVDPPTEDITARILEMMGAFGHGLDHPVAVRALAYLHQTQRPEG-PWWGRWGVNYIYGTWSVLVALKRIGEDMNRPYVRRAVDWVKAHQNLDGGWGE

>seq_ID 290

SPIWDTAKAVNALHESGLPSDHPQLKAAARWL-VEKEVRKPGDWKMRVPHVDVGGWPFQFRNEFYPDVDDTAAVVMALGRVDERDVPGIKDSITR-GINWVTQMQCSCGGWAAFDVDVKREFLTKVPYADHNAMLDPPCPDITGRCLEMYGRFPGVRK-DADVQRVIEKGIEYLKKTQEPDGSWYGRWGVNYIYGTWQSLKGLAAVGEDPSQPYIQKAAH-FLKTHQNSDGGWGE

>seq ID 292

SPVWDTAKAVNALHESGLPSDHPQLKAAARWL-
VEKEVRKPGDWKMRVPHVDVGGWPFQFRNEFYPDVDDTAAVVMALGRVDERDVPGIKDSITR-
GINWVTQMQCSCGGWAAFDVDVKREFLTKVPYADHNAMLDPPCPDITGRCLEMYGRFPEVRK-
DANVQNVIAKGIEYLKKTQEPDGSWYGRWGVNYIYGTWQSLKGLAAVGEDPSQPYIQKAAH-
FLKTHQNSDGGWGE

>seq_ID 293

SPVWDTCLSLAALTEAGAQNDHPAVKQAVEWLLDHQIFVEGDWCAQASGLEPGGWAFQYEND-
KYPDVDDTGMVLMSLLRAGVHDKEHKRKRVNQALNWVLGMQNPDGSWGAFDIENNYEY-
LNKIPFADHGALVDPGTADLTARCVELLAMLGYDATFPPVKRALEFLEHDQEEDGSWYG-
RWGVNYIYGTWSVLCALGAIGEDVAKPYVRKSVQWLQDTQNEDGGWGE

>seq ID 313

SPIWDTALAVRALTAAGMPPEHPAMVKASEWLLTQQIFKPGDWSVKCPDLPPGGWAFEFVNN-
WYPDVDDSSMVLVALKEGLADTAKHQAALQRGINWCLGMQSKNGGFASFDKDNTKEWL-
NAIPFGDLKALVDPPTEDITARILEMMGAFGHGLDHPVAVRALAYL-
HETQRPGGPWWGRWGVNYLYGTWSVLVALKRIGEDMSRPYVRRAVDWVKDHQNLDGGWGE

>seq ID 304

SPVWDTPWMVMALLEAGVPTDXPGLLRAGRWLISKQITGVHGDWAVKNRHALPGGWSFEFEN-
DYFPDVDDTIEVLHVIHRLAIPWEEKSECCRLGLDWLLSMQNDDGGWGAFDRNQTLVMVN-
RIPFSDHAACLDPSSPDIVGRVLEFLASRSFSREHPAVKRALDYIWREQSPFGGWWAR-
WGIDYLYGTWCVLTGLRAIGWDMEDPRVRKAVAWLESVARPDGGYGESPESYRDH-
SYVEWKRSVPSQTAWALMGLVAGGVGHGKAARGAADYLLTSRNAQGGWDEMDYTGTGFP

>seq ID 291

SPMWDTAKAVNALHESGLPSDHPQLKAAARWL-
VEKEVQKPGDWKMRVPYVDVGGWPFQFRNEFYPDVDDTAAVVMALGRVDERDVPGIKDSITR-
GINWVTQMQCSCGGWAAFDVDVKREFLTKVPYADHNAMLDPPCPDITGRCLEMYGRFPE-
VRKDVDVQRVIEKGIEYLKKTQEPDGSWYGRWGVNYIYGTWQSLK-
GLAAVGEDPSQPYIQKAAHFLKTHQNSDGGWGE

>seq ID 318

SPVWDTGLALHALLESGMDPDDPAIAKAMHWLDEREITDVAGDWAEQRPGLAPGGWAFQYRND-
HYPDVDDTAVVGMAMHRANPQARPETLERTRAWIEGMQSQNGGWGAFDADNTHYHLN-
HIPFADHGAMLDPPTADVSARCLGMLSQMGYDRDHPSIQRAIAYLKNDQEEDGSWF-
GRWGTNYIYGTWSVLSALNAAGEDMSQPYIRKAVDYLTNFQREDGGWGE

>seq_ID 294

SPVWDTCLSLAALTEAGAQNDHPAVKQAVEWLLDHQIFVEGDWCDQAPGLEPGGWAFQYENN-
KYPDVDDTGMVLMSLLRAGVHDKEHKRKRVNQALNWVLGMQNPDGSWGAFDIENNYEYLN-
RIPFADHGALVDPGTADLTARCVELLAMLGYDATFPPVKRALEFLEQDQEEDGSWYG-
RWGVNYIYGTWSVLCALGATGEDVAKPYVRKSVQWLQDTQNEDGGWGE

>seq_ID 320

SPVWDTCLGLHALLEAGEPREAPSVKKAVDWLLEREITETYGDWVWRRPHLKPSGWAFQYWNNY-
YPDVDDTAVVVMALDRVGDPRCRPAIERACEWIIGMQSTSGGWGSFDPENEFTYLNHIPFADH-
GALLDPPTVDVTARCISMLAQVGYRHDHPAIRKSVXFILREQEKDGSWYG-
RWGTNYVYGTWSALSALNAVGEDMSSPVVRKGVAWLEAFQQPDGGWGE

>seq_ID 295

SPVWDTCLSLTAMTESGAHPEHPAVKQAVEWLLDQQIFVKGDWADQAKNLEPGG-
WAFQFENDRCPDVDDTGMVLMALLRAGVQDKEHKIKRINQAVNWVLGMQNPDGSWGAFDIGND-
HEYLNNIPFADHGALVDPGTADLTARCVELLAMLGYGPDFPPIQRAVAFLERDQEEFGAWYG-
RWGVNYIYGTWSVLSAIGILGEDYAKPYVRKAVEWLKEIQNDDGGWGE

>seq ID 324

SPVWDTSLAAHALLEAGEPNDPEVIGLLDWLKDKQILTTVGDWSARRPNLRPGGWAFQYEN-
PHYPDVDDTAVVAMAMHRQGDPKYAEAIARACEWLAGMQSSS-
GGWGAFDPENEHFYLNSIPFADHGALLDPPTVDVTARCVGCLAQVDAERFA-
SEIQAGIDYIKREQEEDGSWFGRWGANYVYGTWSALVALNKAGEDMNTPYIRRAVDWLKARQRPDGGWGE

>seq_ID 296

SPVWDTCLSLNALTEADMPANDPRVRAAVQWLFDRQIFVRGDWSENAPELEPGGWAFQYEND-
KYPDVDDTGMVLMSLLRANAHEHDAQRKRMNQALNWVLGMQNSDGSWGAFDIDNHYTYLN-
NIPFADHGALVDPGTADLTGRCIELFGMLGYDKNFTPARRGIEFLKRDQHPCGGWYG-
RWGVNYLYGTWSVLTALGAIGEARDAPYLRRAVEWLYSVQNDDGGWGE

>seq ID 305

SPVWDTPWMVMALLEAGCPANDPXLIRAGRWLKAKXITEVRGDWAVKNRKALPGGWSFEFEN-
DYFPDVDDTIEVLSVIHRLSIPWNEKAKSCRLGLEWXLSMXNRDGGWGAFDREQXFKVVN-
RIPFSDHAACLDPSSPDITGRMVEFLASXNFSKGHVAVRRALDYIWKQQAXFGGWWAR-
WGIDYLYGTWCVLTGLASLGFXMDDPRARKAADWLESIQHADGGFGESPESY-
REDSFVDWKRSVPSQTAWALMGLVAAGRASGAAAQRAAAWLLDNRNTNGSWDEQDYTGTGFP

>seq_ID 282

SPMWDTSLAAHALMEADGRGDPKDNPRLISAMDWLADKQILDHVGDWAVRRPDVRPGG-
WAFQYENPDYPDVDDTAVVVMAMHRADPERYEMSIDRACEWLVGMQSKNGGWGAFEPENE-
HYYLNSIPFADHGALLDPPTVDVTARCVGALAQVDRDRYAAEIANGIRSIRREQEDDGSWF-
GRWGANYVYGTWSALVALKGAGEDMQQPYIRRAVDWLKARQRSDGGWGE

>seq_ID 316

SPVWDTAWAVIGLCESGMERTHPAVRSAIRWLYSMQILRPGDWAVKNPLTEPGGWAFEFHND-
FYPDNDDTAAVLMGLLFSDLNDEENHRAFERGVRWLLSMQNNDSGWGAFERN-
VDNKIFDQIPFNDQKNMLDPSTADVTGRVVELLGRIGRRLGGSFSDEPYVRQAIEFLKNEQEP-
EGCWFGRWGVNYIYGTWSVLVALEAIGESMRAPYIRKAVNWVKKVQNPDGGWGE

>seq_ID 266

SPIWDTGIVLHSLVESGVSPDHEALLRSVSWLLAKEVTHEG-
DWKVKCPDAPVGGWYFEYANEFNPDCDDTAKVLMATSRFSSVDFPDAGRLRDARNRGLQWLL-
HMQNKDGGWAAFDKGCDNELLTYIPFADHNAMIDPSTEDITGRVLETLAREGFDNTHPVVKRA-
IQYLHKTQDAEGPWYGRWGSNFIYGTWLVLQGLKAVGEDMTXPRYQRAANWLLNVQNXNGSWGE

>seq_ID 323

SPMWDTSLAAHAFLESGDREDPRLIRALDWLVDKQILDHVGDWAVRRPGLRPGGWAFQYEN-
PDYPDVDDTAVVAMAMHRTDPERYAENIDRACEWLAGMQSKNGGWGAFDPENE-
HYYLNSIPFADHGALLDPPTVDVTARCIGCLAQVDAEAFADNIKRGIGFIKREQEPDGSWF-
GRWGANYIYGTWSALVALKGAGEDMSQPYIRKSVAWLKGRQGPDGGWGE

>seq_ID 274

SPVWDTILSMQALLDTKEVFQPSPTLKKAMEWLLEQQVRAWGDWKVYVSDARGGGWAFQRANS-
FYPDVDDTIMVMMALRNVSPRGESKVVDEAIERALFWVLGMQCEDGGWAAFDRDNAKAFLT-
KVPFADHNAMIDPSTADLTSRTFEMFAMIAPEVFTIHHPVVRRGLEFLKKDQCKDGSWF-
GRWGVNYMYGTWQVLRGLRLIGEDMSKGYVRKGVEWFKSVQLEDGGWGE

>seq_ID 284

SPVWDTVAQLHALIASGLARRDEALRRAASWLLTRQSRTHGDWSGRN-
PAEPGGFYFEFRNEFYPDVDDTAMALMVLTQAEANVATDVQHAAIARALAWMLGMQNRDGG-
WAAFDRDNDKHFLTQVPFADHNAMIDPSTADITGRVLGALSHVPSYGPDHPSVRRAIAF-
LQRDQEPDGSWYGRWGVNYLYGTGQVLRGLRAIGFDMQQPFVRRAARFLSAHQNDDGGWGE

>seq_ID 285

SPVWDTAITIIALAESGLPKNHPAFEQAATWLEKKEIRFKGDWAVRMPGVEPSG-
WAFEHENKYYPDTDDTMMVLMALRHVQSRNSAERCEQFDRALKWLLAFQCQDGG-
WAAFDKDVTASWLEHVPFADHNAILDPTCSDLTARVLELLGSISFDRQSAIVRRAVAMM-
RRTQETDGSWYGRWGVNYIYGTWQALRGLAAIGENMDQEWIRRGRDWLESCQNDDGGWGE

>seq_ID 308

SPVWDTAIAGYALGESGCAPQSALRRMADWLLTKEVRRKDDWSVKRPD-
VEPSGWYFEFANEFYPDTDDTAMVLLSLLHGRATNPAAQEACAKRAVNWLLAMQSKDGG-
WAAFDVDNDWKPLSYVPFADHNAMLDPSCPDITGRVLEALCKYGVSQEHPAVLRAIDYLI-
QTQEQDGSWHGRWGVNYVYGTFLALRGLKAAGVSDREAYVLRAGEWLDLIQNPDGGWGE

>seq_ID 288

SPVWDTAITAVSLAESGLEPDHPALQKSAEWLLDKEVRIQGDWAIKNRHGEASG-
WAFEFNNEFYPDVDDTLKVLLALRLIKTRDEETKREAMERALGWVMSFQCSDGG-
WAAFDKDVTQRWLEDVPFADHNAILDPTCSDITARCLELLGKMGCTSDHPAVRRALRMV-
RETQEPDGTWWGRWGVNYIYGTWQILRGLSALKIDMNQDWIVRAKEWLESCQNPDGGWGE

>seq_ID 287

SPVWDTAITSVALTSSGVKPDHPQIQKAADWLLDREVVMRGDWKVKNPYPHASGWAFEFNND-
FYPDADDTFKVLLALMKMKSSDPERQRKIMDRALDWARSFQCKDGGFAAFDKDVTKKWLEH-
VPFADHNAILDPSCSDITARGLECMGKLGWPRTDRVIRRAIRYLKKTQEEDGS-
WWGRWGVNYIYGTWQSLRGLEAIGEDMNQDWVVRARNWLESCQNPDGGWGE

>seq_ID 289

SPIWDTAIVTMAIAESGQDPNDPRLQKAADWLLEREIGFRGDWRENCDFPEATGWAFEFNND-
WYPDVDDTFQVILGLKPLSASDSRRQEQTLDRAIRWCRAMQCREGGFAAFDKDINDAWLNE-
VPFADHNAILDPPCSDITGRALETLSLMGFDREDPVVRRARQYLMETQLEDGSWF-
GRWGVNYIYGTGHALRGLHAIGEDINGSAMQRARNWLENCQNDDGGWGE

>seq_ID 286

SPVWDTAINVISLAESGLLSDHPALQKAADWLVNKEVRFRGDWSVNNSYPQVSG-
WAFEYNNVYYPDTDDTAMVLMALRLIRPKDPQALNELFRRALDWQLSFQCRDGG-
WAAFDKNVTTPWLEDMPFADHNAILDPTCSDLTARTLELLGYTGFDPKAQSVRDALQYLID-
TQDEDGSWYGRWGVNYIYGTWQVLRGLRAMGQDMTQDWILRGRDWLESCQNSDGGWGE

>seq_ID 270

SPVWDTALAMSALLEGDTAPDDEALQRGCRWLLGKEVRHRG-
DWQVNVGAEPGGWFFEYENEFYPDCDDTAEV-
LAVLERVRLSDPEEDQRRRDALDRALAWQLGMQSTNGGWGAFDKDCDHRILELVPFADHNA-
MIDPPTVDVTSRSIEAALAMGVPASDAAIRRAVRFLYSEQEADGSWYGRWGSNYLYGTWLAL-
CALRSAGEDLTSPAVQRAVEWLLSVQQEDGGWGE

>seq ID 322

SPVWDTGIAAHALGEAGHASAMQSTADWLLTKEVRRKGDWSVKRPD-
VEPSGWYFEFANEFYPDIDDTAQVLLGLAHAKASDPAKQKACMDRAVAWLLAMQGSDGG-
WAAFDVDNNWEFLSSVPFADHNAMLDPTCPDITGRVLEALAACGVPNSHPAVKRGVEFLRNS-
VEKDGSWYGRWGVNYIYGTYLALRGLRASGEDDREAHILRAGEWLRAIQNADGGWGE

>seq_ID 263

SPVWDTSLILNALLAGSEKTETDPKILKAGQWLLDREVREIGDWKIKNNRG-
PVGGWYFEYANEFYPDCDDTAEVITVLNQMQFSDPEKEKAKQVAQQRGLDWLLSMQNK-
DGGWPAFDKNCDKQSLTYMPFADHNAMIDPSYEDITGRTLEALASLGFSEDDPIVRRAVD-
FLKSKQLPDGTWYGRWGCNFLYGTWLAISGLYHAGEDLNEERYQSLLSWLEQCQNEDGGWGE

>seq ID 268

SPVWDTCLILNSMLEHLEPDHPRVQKAAEWLLSKEVTEPGDWQVKCPE-
APVGGWYFEYANEFYPDCDDTAEVLAALQRVQFTDA-
DREAQKRGAIQRGLGWLLAMQNQDGGXAAFDRECTREALTYVPFADHNAMIDPSNGDITGRV-
LKALDYAGYSPDDPIVRGGVDFLLANQEPDGTWYGRWGCNHLYGS-
WLVVWGLKHAGVNLQQTQFTQVMSWLESCQNADGGWGE

>seq_ID 265

SPVWDTTNAMTAVLDAGLPGNHPAVLRAARWLLSKEVRMPGDWRLWYKNGE-
PGGWFFEYNNEFYPDADDTAEALHCLCRVVFDCEDEMDR-
CRAAIKRGLNWQFACQNPDGGWPAFDKECDDEYLTFIPFADHNAMIDPSCCDIT-
GRSLQALSKLGYTTNDVDVKRAIDYLLDAQEDDGTWYGRWGINYIYGTWLAVQGLRAIGVDL-
SEKRFQKVTKWLRKKQNPDGGWGE

>seq_ID 269

SPVWDTCLILNSLLEHLEPDHPRLQHAAEWLLSKEVTEPGDWQVKCPE-
APIGGWYFEYANEFYPDCDDTAEVLAALQRVRFSDADREAQKHAAIERGLGWLLAMQNGDGG-
WAAFDRECTREALTYVPFADHNAMIDPSNGDITGRVLKALDYSGRSPQDPVVQGGVH-
FLLANQEPDGTWYGRWGCNHLYGSWLAIWGLKHAGVDSQQSQFMRLLSWLESCQNPDGGWGE

>seq_ID 319

SPVWDTSLSAHALMEAGLEENDKRLEGLLDWLKDLQILDVKGDWVARRPDVRPGGWAFQYRND-
HYPDVDDTAVVAMAMHRQGDEKYKEAIDRAAEWIVGMQSSS-
GGWGAFDPENEHFYLNSIPFADHGALLDPPTEDVTARCVGFLAQLDPDAYAEPIKRG-
VEFLKRTQQEDGSWWGRWGANFVYGTWSVLCALNAAGEDPKSPYIQKAVAWLKSRQREDGGWGE

>seq ID 321

SPVWDTGIACQALQEVGGPAADAGVQRALDWLVERQLRDEPGDWRRDRPDLEGGGWAFQYNN-
PHYPDLDDTSMVAWVMQVADHGRYREEIRRAAKWVVGMRSEGGGFASFEVDNTYYYLN-
HIPFADHGXLLDPPTXDVTARCIAVLAITDRAQHETVIREAIDFLFVDQEEDGSWF-
GRWGTDYIYGTWSVLSXLDVVGFDMRDARVRXSVEWLFXQQNPDGGWGE

>seq ID 272

SPVWDTGLVALALQEVDKHNSQDALQRNLKQAYSWLLSKQLKDEPGDWRISKPTLTGGG-
WAFQFNNPHYPDVDDTAVVAFALAQAEHTELDESIHLATRWIEGMQSQNG-
GYGAFDVDNTFYYLNEIPFADHGALLDPPTADVSARCAMLMARVAKDHEEYLPALERTIQYLR-
SEQEADGSWFGRWGTNYVYGTWSVLLGLEQTNVPKTDPLFTKAAQWLKSVQRPDGGWGE

>seq_ID 273

SPVWDTGLVALALPEVDKHNSQDALQPNLKQAYSWLLSKQLKDQPGDWRISKPTLTGGG-
WAFQFNNPHYPDVHDTAVLAFALAQAEHTELDESIHLATRWIEGMQSQNG-
GYGAFDVDNTFYYLNEIPFADHGALLDPPTADVSARCAMLMARVAKGHEEYLPALERTIQYLR-
SEQEADGSWFGRWGTNYVYGTWSVLLGLEQTNVPKTDPLFTKAAQWLKSVQRPDGGWGE

>seq_ID 317

SPVWDTILGMIGLVDCGHDGKDPLLVTARDWIVKRQLLVNYGDWKVYNPNGPSGGWSFEYDNS-
WYPDVDDTAAIVIGFLKQDYEFRHSEVVKRACDWIASMQNQXGGWAAFDINNDKTFLNEI-
PFSDMESLCDPSSPDVVGRVLEAFGILNDPKYAEVCRRGIEYLRRTQESEGSWF-
GRWGVNYVYGTSNVLCSLKRQDVAXKDPMVTRALTWLKKVQNKDGGWGE

>seq_ID 215

MGRQTRNLTRREPAAEAEERGFRLLDAHRRADSSWVGELSSSALATAMSALALRLLGHPAES-
GPVAGGLAWLAATRNPDGGWGDAPGEPSNMNATSIAAAALARCAPRRYREEVAGGRRWVEEH-
GGFAALNDPRTTTLSGPGRTLWALAGLVPPERVRKLPTEMILLPRRIRRTVSTTFPAF-
LSLSLLHERFRPSPRWRRPLRRRAEREALAWLRRAQGPNGSYEESAFLTS-
LIAAALTAAGAEGGDIVRRALPFVLRSRRPDGSWPIDRDLENFDTTQAILAHHEAGRPLREA-
GRVREWLLDNQFRRPFFPTSSPPGGWAWAYPAGWPDTDDTACALRSLRLL-
GVPAGHPSIRLGLRWLYRMQNRDGSWPTFVRGSRMPFDHGCPYITSQVLSALALMGPEARR-
GAPLRRALAYLRRAQRPDGSLGSLWFRPHTRGTAAAVEAFSDLGLSGDPLVGRAAR-
WLAEHQNPDGGWGDGHGAPSTAEETAWASAALLRLGGGEAARKGVRWLVEHQDPGGWKPA-
VIGLYYASLSYSDTFYALSYPLVALARHRRLSR

>seq ID 191

```
MIKKĪLVLILLMVVVTSKVDIERVQTVIRDAREICWNELTDNEWVYPTYLGTLFLSEYYFEL-
KALGIQNSQFEESKFTQILLGSQLPDGSWVQVEDAYIQTGQLDATIFNYWYLKAVGIDIHT-
DTMKKAQEWIKANGGIEKAQTMTKFKLAMFGQYPWKKLFKIPLILFYKKFNPLYIK-
DITAQWVYPHMTALAYLQNQRIIFNVAVSISELYKNKAPKIKNHQKKGRPSFFINN-
LVQEMLKLRQPMGSFGGYTVSTLLSMLALNDYTGRTNKHKSEISDALKKGLDFVEFNY-
FNFRQAYHGSLDDGRWWDTILISWAMLESGEDKEKVRPIVENMLQKGVQPNGGIEYGYDFG-
YAPDADDTGLLLQVLSYYGTDYADAMDKGAEFVYSVQNTDGGFPAFDKGKMGKNPLYKYAFKI-
AGIADSAEIFDPSSPDVTAHILEGLISSDRSNYDVVVKSLKYFMDTQENFGSWEGRWGI-
NYIYAAGAVLPALKKMNNGWAKAVNWLVSKQNADGGFGETTLSYRDPKKYNGIGVSTVTQTS-
WGLLGLLAVEDHYDVKEAIEKARDGEFKDISVVGTGHRGLLYLQYPSYARSFPVISLGRFLDQQR
```

**Patentansprüche**

1. Enzymmutante mit Cyclase-Aktivität, ausgewählt unter Mutanten eines Wildtyp-Enzyms, das eine Aminosäuresequenz, ausgewählt unter SEQ ID NO: 3 bis 326 oder eine Teilsequenz davon umfasst; wobei die Mutante wenigstens die Cyclisierung eines Citronellal-Isomers zu wenigstens einem Isopulegol-Isomer katalysiert.

2. Enzymmutante nach Anspruch 1, umfassend eine Mutation in einer unter SEQ ID NO: 3 bis 326 ausgewählten Sequenzen, wobei die mutierte Position der Position F486 von SEQ ID NO: 2 entspricht.

3. Enzymmutante nach einem der vorhergehenden Ansprüche bei der bis zu 25% der Aminosäurereste jeweils gegenüber der SEQ ID NO: 3 bis 326 durch Deletion, Insertion, Substitution, Addition, Inversion oder einer Kombination davon verändert sind.

4. Enzymmutante nach einem der vorhergehenden Ansprüche, bei der die Mutation in der der Position F486 von SEQ ID NO:2 entsprechenden Position in einer der Sequenzen gemäß SEQ ID NO: 3 bis 326, eine Substitution ist, die einer Substitution von SEQ ID NO:2, ausgewählt unter F486N, F486Q, F486L, F486M, F486E, F486G, F486S, F486V, F486T, F486C, F486I und F486A entspricht.

5. Enzymmutante nach einem der vorhergehenden Ansprüche, bei der zusätzlich wenigstens eine Mutation in einer der Positionen W374, D437, D440, F428, W555, Y561, Y702, Y705 der SEQ ID NO: 2 , entsprechenden Position in einer der Sequenzen gemäß SEQ ID NO: 3 bis 326, vorliegt.

6. Enzymmutante nach einem der vorhergehenden Ansprüche, bei der keine Mutation in einer der Position D437 und/oder D439 und/oder D440 von SEQ ID NO: 2 jeweils entsprechenden Position in einer der Sequenzen gemäß SEQ ID NO: 3 bis 326, vorliegt.

7. Enzymmutante nach einem der vorhergehenden Ansprüche, bei der keine Mutation in einer der Position Y702 von SEQ ID NO: 2 entsprechenden Position in einer der Sequenzen gemäß SEQ ID NO: 3 bis 326, vorliegt oder falls eine Mutation vorliegt, diese einer Substitution Y702F in SEQ ID NO:2 entspricht.

8. Enzymmutante nach einem der vorhergehenden Ansprüche, welche gegebenenfalls weiterhin mutiert ist in wenigstens einer der Positionen P229, D439, D508, E601, G553, G556, N432, P436, P499, R224, S371, T376, T563, W414 oder W624 von SEQ ID NO: 2, entsprechenden Position in einer der Sequenzen gemäß SEQ ID NO: 3 bis 326.

9. Enzymmutante nach einem der vorhergehenden Ansprüche, ausgewählt unter von einer der SEQ ID NO: 3 bis 325 abgeleiteten Mutanten korrespondierend zu

   a) den Einfachmutanten

      F486X mit X = N, Q, L, M, E, G, S, V, T, C, I oder A gemäß SEQ ID NO: 2
      Y702X mit X = F, A, C oder S gemäß SEQ ID NO: 2
      Y561X mit X= A oder S gemäß SEQ ID NO: 2

   b) den Mehrfachmutanten F486A / Y702A, F486A / Y561A oder F486A / Y705A gemäß SEQ ID NO: 2.

10. Enzymmutante nach einem der vorhergehenden Ansprüche, die mindestens 50% der Citronellal-Isopulegol-Cyclase

Aktivität eines Enzyms zeigt, das eine Aminosäuresequenz korrespondierend zu SEQ ID NO: 2 von Position 1 bis 725, 2 bis 725 oder 16 bis 725, ggf. N-terminal verlängert durch einen Methioninrest, umfasst.

**11.** Enzymmutante nach Anspruch 10, wobei die Citronellal-Isopulegol-Cyclase Aktivität unter Verwendung eines Citronellals als Referenzsubstrat unter Standardbedingungen bestimmt wird.

**12.** Enzymmutante nach einem der vorhergehenden Ansprüche, wobei die Mutation in einem Enzym erfolgt, die eine Aminosäuresequenz korrespondierend zu SEQ ID NO: 2 von Position 1 bis 725, 2 bis 725 oder 16 bis 725, ggf. N-terminal verlängert durch einen Methioninrest, umfasst.

**13.** Nukleinsäuresequenz, kodierend für eine Mutante nach einem der vorhergehenden Ansprüche.

**14.** Expressionskassette, umfassend eine Nukleinsäuresequenz nach Anspruch 13.

**15.** Rekombinanter Vektor, umfassend unter der Kontrolle wenigstens eines regulativen Elements wenigstens eine Nukleinsäuresequenz nach Anspruch 13 oder wenigstens eine Expressionskassette nach Anspruch 14.

**16.** Rekombinanter Mikroorganismus, enthaltend wenigstens eine Nukleinsäuresequenz nach Anspruch 13 oder wenigstens eine Expressionskassette nach Anspruch 14 oder wenigstens einen Vektor nach Anspruch 15.

**17.** Verfahren zur Herstellung von Isopulegol der allgemeinen Formel (I)

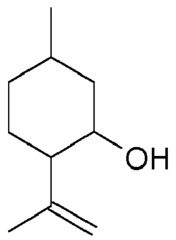

(I)

wobei man Citronellal der allgemeinen Formel (II)

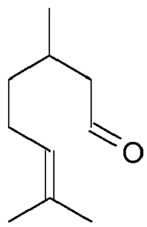

(II)

zu Isopulegol der Formel (I) mittels eines Enzyms der EC Klasse EC 5.4.99, insbesondere der EC Klasse EC 5.4.99.17, oder in Gegenwart eines dieses Enzym exprimieren Miroorganismus cyclisiert.

**18.** Verfahren zur Herstellung von Isopulegol der allgemeinen Formel (I)

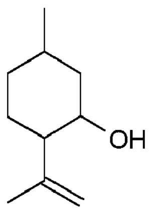

(I)

wobei man Citronellal der allgemeinen Formel (II)

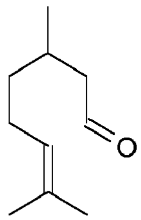

(II)

zu Isopulegol der Formel (I) mittels einer Enzymmutante nach einem der Ansprüche 1 bis 12, oder in Gegenwart eines diese Enzymmutante exprimieren Miroorganismus nach Anspruch 16 cyclisiert.

**19.** Verfahren zur Herstellung von Menthol der Formel III

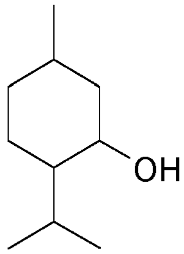

(III)

wobei man

a) Citronellal zu Isopulegol nach einem Verfahren gemäß Anspruch 17 oder 18 cyclisiert und
b) Isopulegol katalytisch zu Menthol hydriert.

**20.** Verfahren nach Anspruch 19, wobei die Hydrierung in Gegenwart von Wasserstoff und einem Katalysator erfolgt, der

- 30 bis 70 Gew.-% sauerstoffhaltige Verbindungen des Nickels, berechnet als NiO,
- 15 bis 45 Gew.-% sauerstoffhaltige Verbindungen des Zirkons, berechnet als $ZrO_2$,
- 5 bis 30 Gew.-% sauerstoffhaltige Verbindungen des Kupfers berechnet als CuO und
- 0,1 bis 10 Gew.-% sauerstoffhaltige Verbindungen des Molybdäns, berechnet als $MoO_3$,

umfasst, wobei sich die Gew.-%-Angaben auf den trockenen, nicht reduzierten Katalysator beziehen.

**21.** Verfahren zu enzymatischen oder biokatalytischen Umsetzungen von Verbindungen der allgemeinen Formel IV

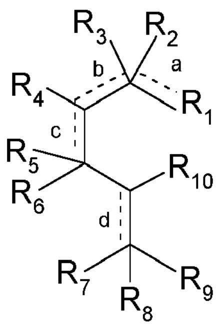

(IV)

worin
"a", "b", "c" und "d", jeweils unabhängig voneinander, eine C-C-Einfach- oder Doppelbindung darstellen, mit der Maßgabe, dass kumulierte Doppelbindungen ausgeschlossen sind; und den folgenden Maßgaben:

$R_1$ folgende Bedeutungen besitzt:

(1) wenn "a" eine Doppelbindung ist:
$R_1$ ausgewählt ist unter

Oxo (=O), oder
$CH-(CH_2)_n-Z$,

worin n für 0, 1 oder 2 steht und
Z für OH, CHO, $C(O)C_1-C_4$-Alkyl; COOH, $C(CH_2)-CH=CH_2$; $C(OH)(CH_3)-CH=CH_2$; $C(CH_3)=CH-CH=CH_2$; oder einen Rest der Formel $C(CH_3)=CH-CH_2Y$ steht
worin
Y für OH, $CH_2OH$, COOH, oder $CH_2C(O)CH_3$ steht; oder

(2) wenn "a" eine Einfachbindung ist:
$R_1$ ausgewählt ist unter
$CH_3$; CHO; $CH_2CH_2OH$; $CH=CH_2$; $CH_2C(O)OH$; $CH_2CHO$ oder $C_3H_6CH(CH_3)CHO$ steht;

wobei, wenn "a" eine Doppelbindung ist, diese E- oder Z- Konfiguration aufweist;
$R_2$ und $R_3$ folgende Bedeutungen besitzen:

(1) wenn "a" und "b" jeweils eine Einfachbindung sind:
$R_2$ und $R_3$ unabhängig voneinander für H, $C_1-C_4$-Alkyl oder OH stehen oder $R_2$ und $R_3$ zusammen für eine Methylen (=$CH_2$)- oder Oxo (=O)- Gruppe stehen; oder
(2) wenn "a" oder "b" eine Doppelbindung ist, einer der Reste $R_2$ und $R_3$ fehlt und der andere der beiden Reste für H, $C_1-C_4$-Alkyl oder OH steht;

$R_4$ für H oder Hydroxy-$C_1-C_4$-alkyl steht;
$R_5$ und $R_6$ folgende Bedeutungen besitzen:

(1) wenn "c" eine Einfachbindung ist:
$R_5$ und $R_6$ jeweils für H stehen oder $R_5$ und $R_6$ zusammen für eine Oxo (=O)-Gruppe stehen; oder
(2) wenn "c" eine Doppelbindung ist, einer der Reste $R_5$ und $R_6$ fehlt und der andere der beiden Reste für H steht;

$R_7$, $R_8$ und $R_9$ folgende Bedeutungen besitzen:

(1) wenn "d" eine Einfachbindung ist:
zwei der Reste $R_7$, $R_8$ und $R_9$ jeweils unabhängig voneinander für H oder $C_1-C_4$-Alkyl stehen und der andere der Reste für OH steht; oder
(2) wenn "d" eine Doppelbindung ist, einer der Reste $R_7$, $R_8$ und $R_9$ fehlt und die andere der beiden Reste jeweils unabhängig voneinander für H oder $C_1-C_4$-Alkyl stehen;

$R_{10}$ für H oder Hydroxy-$C_1-C_6$-Alkyl oder ein oder mehrfach ungesättigtes $C_2-C_6$-Alkenyl steht;
wobei man eine Verbindung der Formel IV in stereoisomerenreiner Form oder ein Stereoisomerengemisch davon unter Verwendung eines Enzyms der Klasse EC 5.4.99, insbesondere der Klasse EC 5.4.99.17, oder eine Enzymmutante nach einem der Ansprüche 1 bis 12 oder in Gegenwart einen diese Enzyme oder Enzymmutanten exprimierenden Mikroorganismus nach Anspruch 16 umsetzt.

**22.** Verfahren nach Anspruch 21, worin eine Verbindung umgesetzt wird, die ausgewählt ist unter Verbindungen der Formel IVa

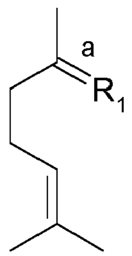

(IVa)

worin $R_1$ die oben angegebenen Bedeutungen besitzt und insbesondere für den Rest $CH-(CH_2)_n-Z$ steht worin

n = 0 und Z = CHO, oder COOH ist; oder
n = 1 und Z = OH ist; oder
n = 2 und Z = $C(O)CH_3$; COOH, $C(CH_2)-CH=CH_2$; $C(CH_3)=CH-CH=CH_2$; oder ein Rest der Formel $C(CH_3)=CH-CH_2Y$ ist
worin Y für OH, $CH_2OH$, COOH, oder $CH_2C(O)CH_3$ steht;

und "a" ggf. E- oder Z-Konfiguration aufweist; oder der Formel IVb

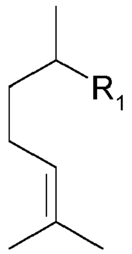

(IVb)

worin $R_1$ die oben angegebenen Bedeutungen besitzt; und insbesondere für $CH_2CHO$ steht; order der Formel IVc

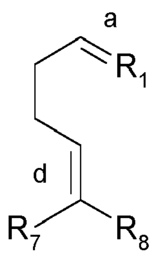

(IVc)

worin $R_1$ die oben angegebenen Bedeutungen besitzt, und insbesondere für CH-CHO steht; und einer der Reste $R_7$ und $R_8$ für H und der andere für $C_1$-$C_4$-Alkyl,wobei insbesondere $R_7$ für Ethyl steht und die Doppelbindung "a" und "d" Z-Konfiguration aufweisen.

**23.** Verfahren nach einem der Ansprüche 20 bis 22, worin die Verbindung der Formel IV ausgewählt ist unter Citronellal; Citral; Farnesol; Homofarnesol; Homofarnesolderivaten, wie Homofarnesylsäure; Geranylaceton, Melonal; Nonadienal; und Trimethyldecatetraen.

**24.** Verwendung eines Enzyms aus der EC Klasse EC 5.4.99, insbesondere der EC Klasse EC 5.4.99.17 zur Cyclisierung von Terpenen, insbesondere zur Umsetzung von Citronellal zu Isopulegol, sowie zur Umsetzung von Verbindungen der allgemeinen Formel IV.

**25.** Verwendung einer Enzymmutante nach einem der Ansprüche 1 bis 12, einer Nukleinsäure nach Anspruch 13, eines Expressionskonstruktes nach Anspruch 14, eines rekombinanten Vektors nach Anspruch 15 oder eines rekombinanten Mikroorganismus nach Anspruch 1 zur Cyclisierung von Terpenen und/oder Terpenoiden, sowie zur Umsetzung von Verbindungen der allgemeinen Formel IV gemäß der Definition in einem der Ansprüche 20 bis 23.

**26.** Verwendung nach Anspruch 25 zur Umsetzung von Citronellal zu Isopulegol; oder zur Umsetzung von Squalen zu Hopen.

1 MGIDRMNSLS RLLMKKIFGA EKTSYKPASD TIIGTDTLKR PNRRPEPTAK
51 VDKTIFKTMG NSLNNTLVSA CDWLIGQQKP DGHWVGAVES NASMEAEWCL
101 ALWFLGLEDH PLRPRLGNAL LEMQREDGSW GVYFGAGNGD INATVEAYAA
151 LRSLGYSADN PVLKKAAAWI AEKGGLKNIR VFTRYWLALI GEWPWEKTPN
201 LPPEIIWFPD NFVFSIYNFA QWARATMVPI AILSARRPSR PLRPQDRLDE
251 LFPEGRARFD YELPKKEGID LWSQFFRTTD RGLHWVQSNL LKRNSLREAA
301 IRHVLEWIIR HQDADGGWGG IQPPWVYGLM ALHGEGYQLY HPVMAKALSA
351 LDDPGWRHDR GESSWIQATN SPVWDTMLAL MALKDAKAED RFTPEMDKAA
401 DWLLARQVKV KGDWSIKLPD VEPGGWAFEY ANDRYPDTDD TAVALIALSS
451 YRDKEEWQKK GVEDAITRGV NWLIAMQSEC GGWGA**F**DKDN NRSILSKIPF
501 CDFGESIDPP SVDVTAHVLE AFGTLGLSRD MPVIQKAIDY VRSEQEAEGA
551 WFGRWGVNYI YGTGAVLPAL AAIGEDMTQP YITKACDWLV AHQQEDGGWG
601 ESCSSYMEID SIGKGPTTPS QTAWALMGLI AANRPEDYEA IAKGCHYLID
651 RQEQDGSWKE EEFTGTGFPG YGVGQTIKLD DPALSKRLLQ GAELSRAFML
701 RYDFYRQFFP IMALSRAERL IDLNN

**Fig. 1a**

1 atgggtattg acagaatgaa tagcttaagt cgcttgttaa tgaagaagat
51 tttcggggct gaaaaaacct cgtataaacc ggcttccgat accataatcg
101 gaacggatac cctgaaaaga ccgaaccggc ggcctgaacc gacggcaaaa
151 gtcgacaaaa cgatattcaa gactatgggg aatagtctga ataataccct
201 tgtttcagcc tgtgactggt tgatcggaca acaaaagccc gatggtcatt
251 gggtcggtgc cgtggaatcc aatgcttcga tggaagcaga atggtgtctg
301 gccttgtggt tttgggtct ggaagatcat ccgcttcgtc caagattggg
351 caatgctctt ttggaaatgc agcgggaaga tggctcttgg ggagtctatt
401 tcggcgctgg aaatggcgat atcaatgcca cggttgaagc ctatgcggcc
451 ttgcggtctt tggggtattc tgccgataat cctgttttga aaaaagcggc
501 agcatggatt gctgaaaaag gcggattaaa aaatatccgt gtctttaccc
551 gttattggct ggcgttgatc ggggaatggc cttgggaaaa gacccctaac
601 cttcccctg aaattatctg gttccctgat aattttgtct tttcgattta
651 taattttgcc caatgggcgc gggcaaccat ggtgccgatt gctattctgt
701 ccgcgagacg accaagccgc ccgctgcgcc ctcaagaccg attggatgaa
751 ctgtttccag aaggccgcgc tcgctttgat tatgaattgc cgaaaaaaga
801 aggcatcgat ctttggtcgc aatttttccg aaccactgac cgtggattac
851 attgggttca gtccaatctg ttaaagcgca atagcttgcg tgaagccgct
901 atccgtcatg ttttggaatg gattatccgg catcaggatg ccgatggcgg
951 ttggggtgga attcagccac cttgggtcta tggtttgatg gcgttacatg
1001 gtgaaggcta tcagctttat catccggtga tggccaaggc tttgtcggct
1051 ttggatgatc ccggttggcg acatgacaga ggcgagtctt cttggataca
1101 ggccaccaat agtccggtat gggatacaat gttggccttg atggcgttaa
1151 aagacgccaa ggccgaggat cgttttacgc cggaaatgga taaggccgcc
1201 gattggcttt tggctcgaca ggtcaaagtc aaaggcgatt ggtcaatcaa
1251 actgcccgat gttgaacccg gtggatgggc atttgaatat gccaatgatc
1301 gctatcccga taccgatgat accgccgtcg ctttgatcgc cctttcctct
1351 tatcgtgata aggaggagtg gcaaaagaaa ggcgttgagg acgccattac
1401 ccgtggggtt aattggttga tcgccatgca aagcgaatgt ggcggttggg
1451 gagcc**ttt**ga taaggataat aacagaagta tcctttccaa aattcctttt
1501 tgtgatttcg gagaatctat tgatccgcct tcagtcgatg taacggcgca
1551 tgttttagag gcctttggca ccttgggact gtcccgcgat atgccggtca
1601 tccaaaaagc gatcgactat gtccgttccg aacaggaagc cgaaggcgcg
1651 tggtttggtc gttggggcgt taattatatc tatggcaccg gtgcggttct
1701 gcctgctttg gcggcgatcg gtgaagatat gacccagcct tacatcacca
1751 aggcttgcga ttggctggtc gcacatcagc aggaagacgg cggttggggc
1801 gaaagctgct cttcctatat ggagattgat tccattggga agggcccaac
1851 cacgccgtcc cagactgctt gggctttgat ggggttgatc gcggccaatc
1901 gtcccgaaga ttatgaagcc attgccaagg gatgccatta tctgattgat
1951 cgccaagagc aggatggtag ctggaaagaa gaagaattca ccggcaccgg
2001 attccccggt tatggcgtgg gtcagacgat caagttggat gatccggctt
2051 tatcgaaacg attgcttcaa ggcgctgaac tgtcacgggc gtttatgctg
2101 cgttatgatt tttatcggca attcttcccg attatggcgt taagtcgggc
2151 agagagactg attgatttga ataattga

**Fig. 1b**

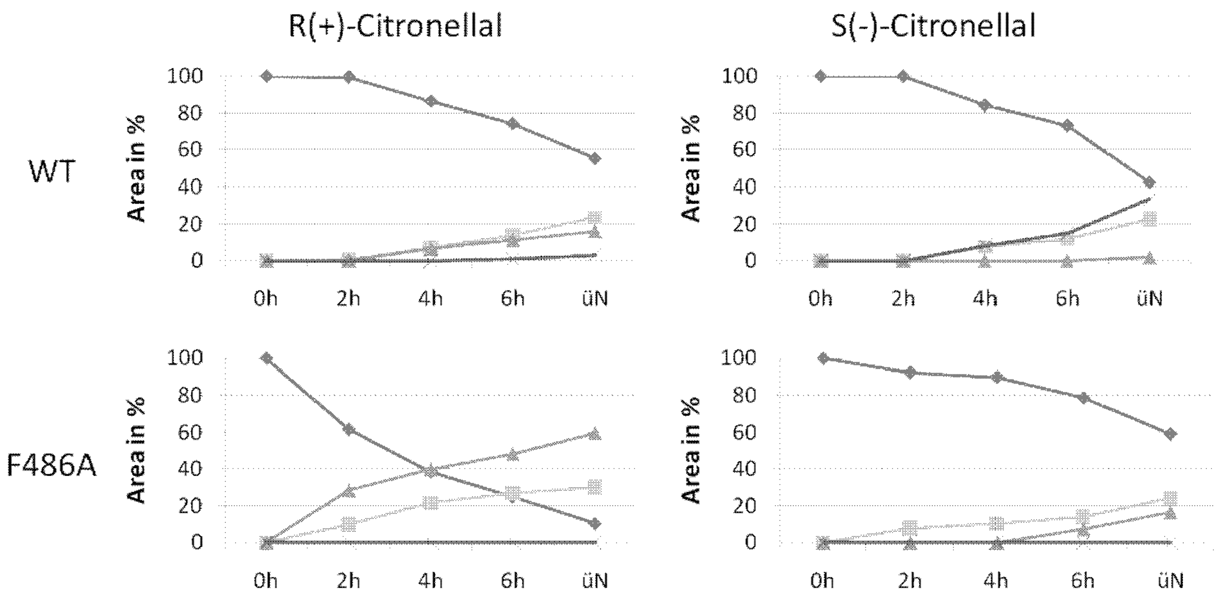
R(+)-Citronellal
S(-)-Citronellal
WT
F486A
Area in %
100
80
60
40
20
0
0h
2h
4h
6h
üN

**Fig. 2**

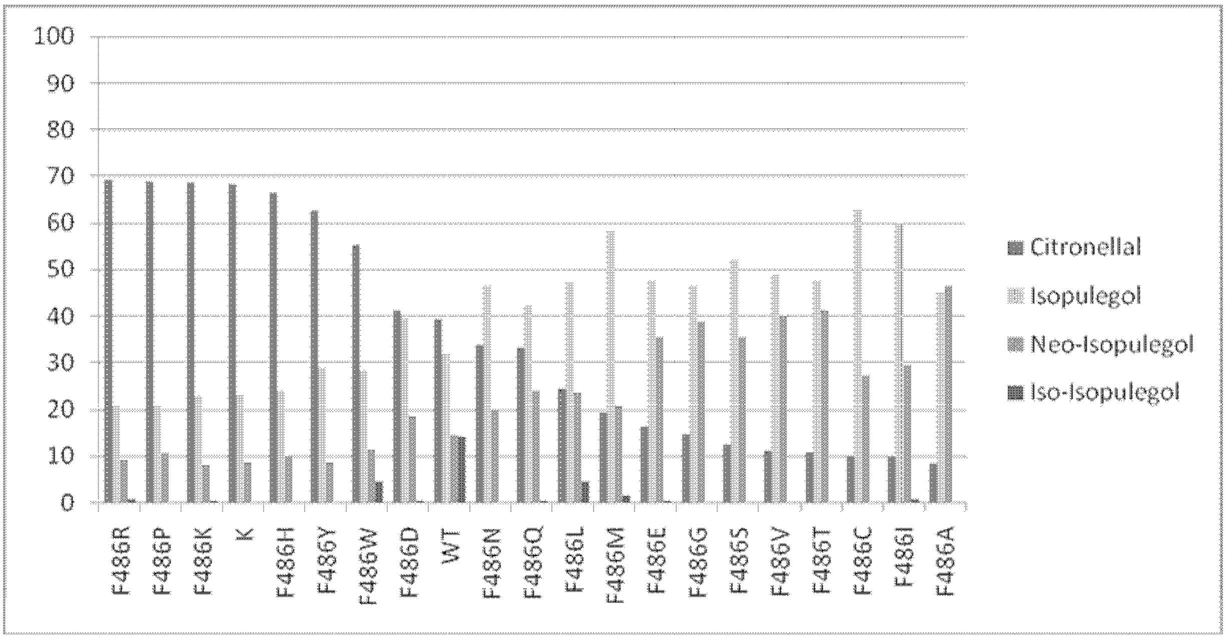
100
90
80
70
60
50
40
30
20
10
0
F486R
F486P
F486K
K
F486H
F486Y
F486W
F486D
WT
F486N
F486Q
F486L
F486M
F486E
F486G
F486S
F486V
F486T
F486C
F486I
F486A
Citronellal
Isopulegol
Neo-Isopulegol
Iso-Isopulegol

**Fig.3**

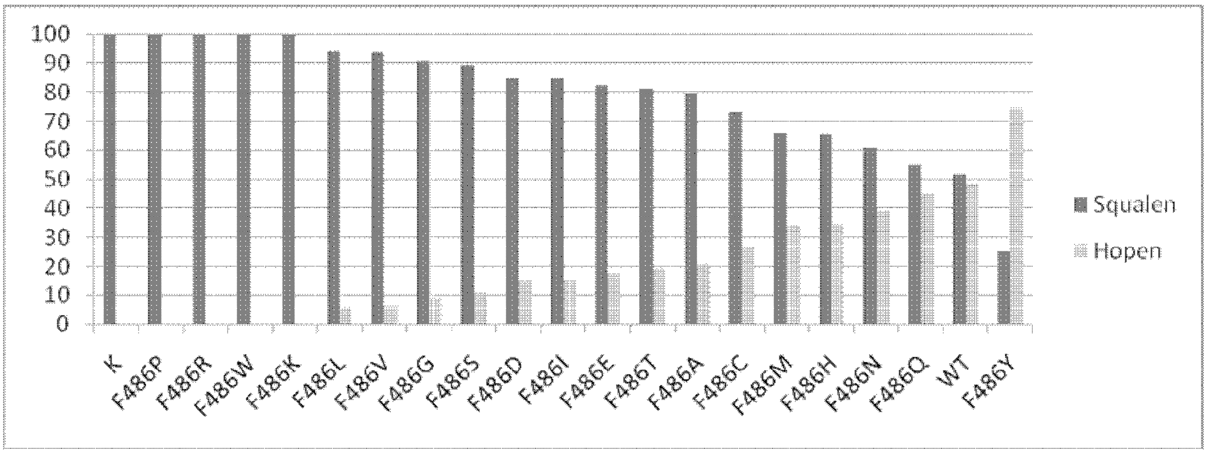


**Fig. 4**

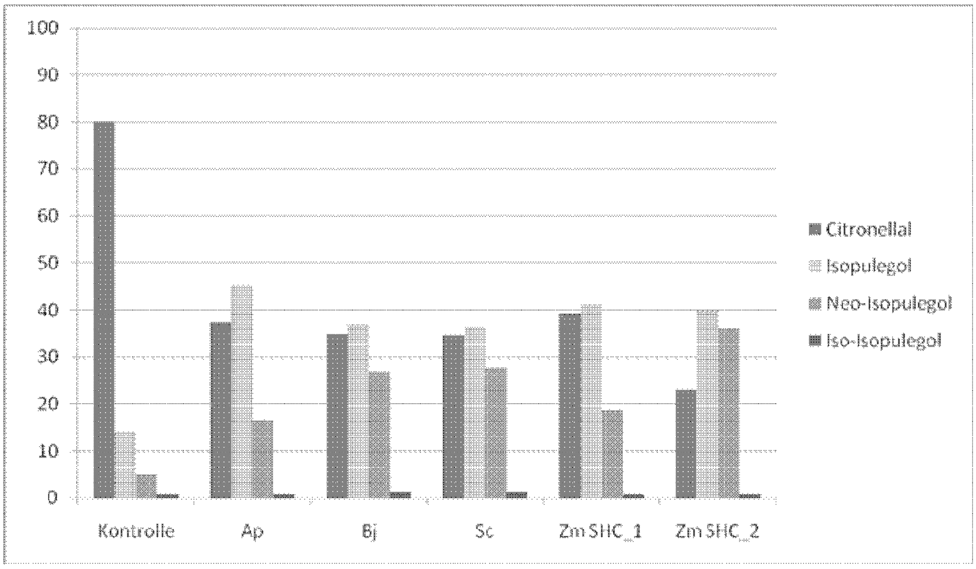


**Fig.5**

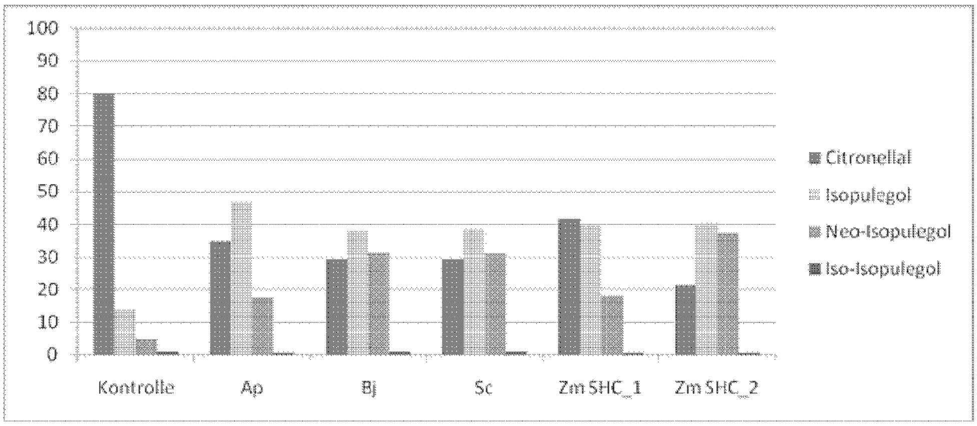


**Fig.6**

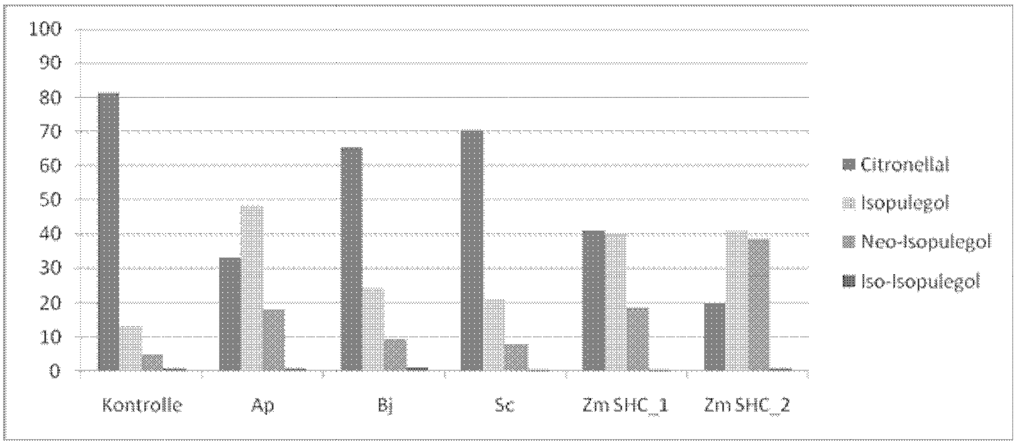
100
90
80
70
60
50
40
30
20
10
0
Kontrolle
Ap
Bj
Sc
Zm SHC_1
Zm SHC_2
Citronellal
Isopulegol
Neo-Isopulegol
Iso-Isopulegol

Fig.7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2010057696 W
- WO 2010139719 A2
- WO 2005108590 A
- WO 2006094945 A
- EP 1149849 A
- EP 1069183 A
- DE 100193773 A
- WO 2009013192 A
- EP 0696572 A


### In der Beschreibung aufgeführte Nicht-Patentliteratur


- *Genpept Accession No AAV90172 2004 und Nat Biotechnol,* 2005, vol. 23, 63-68
- *Curr. Opin. Chem. Biol.,* 2009, vol. 13, 180-188
- *Eur. J. Biochem.,* 1999, vol. 264, 610-650
- **SOBOLEV V ; SOROKINE A ; PRILUSKY J ; ABOLA EE ; EDELMAN M.** Automated analysis of interatomic contacts in proteins. *Bioinformatics,* 1999, vol. 15 (4), 327-332
- **NARANG, S.A.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- **HIGGINS DG ; SHARP PM.** Fast and sensitive multiple sequence alignments on a microcomputer. *Comput Appl. Biosci.,* April 1989, vol. 5 (2), 151-1
- **CHENNA ; RAMU ; SUGAWARA ; HIDEAKI ; KOIKE ; TADASHI ; LOPEZ ; RODRIGO ; GIBSON ; TOBY J.** Multiple sequence alignment with the Clustal series of programs. *Nucleic Acids Res,* 2003, vol. 31 (13), 3497-500
- Phosphoamiditmethode. Wiley Press New York, 896-897
- **SAMBROOK et al.** Molecular Cloning: A laboratory manual. Cold Spring Harbor Laboratory Press, 1989
- **SAMBROOK, J. ; FRITSCH, E.F. ; MANIATIS, T.** Molecular Cloning: A Laboratory Manual. Cold Spring Harbor Laboratory, 1989
- **SAMBROOK et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1991
- Essential Molecular Biology: A Practical Approach. IRL Press at Oxford University Press
- **SAMBROOK, J. ; FRITSCH, E.F. ; MANIATIS, T.** Molecular Cloning (A Laboratory Manual). Cold Spring Harbor Laboratory Press, 1989
- Current Protocols in Molecular Biology. John Wiley & Sons, 1989, 6.3.1-6.3.6
- **SAMBROOK ; RUSSELL.** Molecular Cloning. Cold Spring Harbor Laboratory Press, 2001
- In vitro mutagenesis protocols. Humana Press, 1996
- **KEGLER-EBO DM ; DOCKTOR CM ; DIMAIO D.** *Nucleic Acids Res,* 1994, vol. 22, 1593
- **BARETTINO D ; FEIGENBUTZ M ; VALCÄREL R ; STUNNENBERG HG.** *Nucleic Acids Res,* 1994, vol. 22, 541
- **BARIK S.** *Mol Biotechnol,* 1995, vol. 3, 1
- **ECKERT KA ; KUNKEL TA.** *Nucleic Acids Res,* 1990, vol. 18, 3739
- **SCHENK et al.** *Biospektrum,* 2006, vol. 3, 277-279
- An efficient random mutagenesis technique using an E.coli mutator strain. **GREENER A ; CALLAHAN M ; JERPSETH B.** In vitro mutagenesis protocols. Humana Press, 1996
- **STEMMER WPC.** *Nature,* 1994, vol. 370, 389
- **STEMMER WPC.** *Proc Natl Acad Sci USA,* 1994, vol. 91, 10747
- **REETZ MT ; JAEGER K-E.** *Topics Curr Chem,* 1999, vol. 200, 31
- Methods for optimizing industrial enzymes by directed evolution. **ZHAO H ; MOORE JC ; VOLKOV AA ; ARNOLD FH.** Manual of industrial microbiology and biotechnology. American Society for Microbiology, 1999

- **GOEDDEL.** Gene Expression Technology: Methods in Enzymology. Academic Press, 1990, vol. 185
- Buch Cloning Vectors. Elsevier, 1985
- **T. MANIATIS ; E.F. FRITSCH ; J. SAMBROOK.** Molecular Cloning: A Laboratory Manual. Cold Spring Harbor Laboratory, 1989
- **T.J. SILHAVY ; M.L. BERMAN ; L.W. ENQUIST.** Experiments with Gene Fusions. Cold Spring Harbor Laboratory, 1984
- **AUSUBEL, F.M. et al.** Current Protocols in Molecular Biology, Greene Publishing Assoc. Wiley Interscience, 1987
- Cloning Vectors. Elsevier, 1985
- Molecular Biology. Wiley Interscience, 1997
- **SAMBROOK et al.** Molecular Cloning: A Laboratory Manual. Cold Spring Harbor Laboratory, 1989
- **LEHRBUCH ; CHMIEL.** Bioprozeßtechnik 1. Einführung in die Bioverfahrenstechnik. Gustav Fischer Verlag, 1991
- **STORHAS.** Bioreaktoren und periphere Einrichtungen. Vieweg Verlag, 1994
- **HANDBUCH.** Manual of Methods für General Bacteriology. American Society für Bacteriology, 1981
- Applied Microbiol. Physiology, A Practical Approach. IRL Press, 1997, 53-73
- **HARLOW, E. ; LANE, D.** Antibodies: A Laboratory Manual. Cold Spring Harbor (N.Y.) Press, 1988
- **J. LALONDE ; A. MARGOLIN.** *Immobilization of Enzymes*
- **K. DRAUZ ; H. WALDMANN.** Enzyme Catalysis in Organic Synthesis. Wiley-VCH, 2002, vol. III, 991-1032
- Biotechology. VCH, vol. 3
- **ENZYMCODE.** *Eur. J. Biochem.,* 1999, vol. 264, 610-650
- **SAMBROOK, J. ; FRITSCH, E.F ; MANIATIS, T.** Molecular cloning: A Laboratory Manual. Spring Harbor Laboratory Press, 1989